# EUROPEAN PATENT APPLICATION

(11) **EP 1 710 299 A2**
(43) Date of publication of application: **11.10.2006**
(21) Application number: 05026823.4
(22) Date of filing: 23.12.1999
(51) Int. Cl.: C12N 5/10, C12N 15/12, C12N 15/19, C12N 15/63, C12N 15/64, C07K 14/47, C07K 14/52, C07K 14/705

(54) **Secreted proteins and nucleic acids encoding them**

(30) Priority: 30.12.1998 US 223546
(62) Divisional of application: 99968191.9
(71) Applicant: MILLENNIUM PHARMACEUTICALS, INC., Cambridge, Massachusetts 02139 (US)
(72) Inventor: Holtzman, Douglas A,, Seattle, WA 98119 (US)
(74) Representative: Williams, Gareth Owen

(57) **Abstract**

The invention provides isolated nucleic acid molecules, designated TANGO 224, TANGO 128, TANGO 140, TANGO 197, TANGO 212, TANGO 213, and TANGO 239. These nucleic acid molecules encode wholly secreted and transmembrane proteins. The invention also provides antisense nucleic acid molecules, expression vectors containing the nucleic acid molecules of the invention, host cells into which the expression vectors have been introduced, and nonhuman transgenic animals in which a nucleic acid molecule of the invention has been introduced or disrupted. The invention still further provides isolated polypeptides, fusion polypeptides, antigenic peptides and antibodies. Diagnostic, screening and therapeutic methods utilizing compositions of the invention are also provided.

## Description

### Cross Reference to Related Applications

This application is a continuation-in-part of co-pending Application No. 09/223,546 filed December 30, 1998, which is incorporated herein by reference in its entirety.

### Background of the Invention

Many secreted proteins, for example, cytokines and cytokine receptors, play a vital role in the regulation of cell growth, cell differentiation, and a variety of specific cellular responses. A number of medically useful proteins, including erythropoietin, granulocyte-macrophage colony stimulating factor, human growth hormone, and various interleukins, are secreted proteins. Thus, an important goal in the design and development of new therapies is the identification and characterization of secreted and transmembrane proteins and the genes which encode them.

Many secreted proteins are receptors which bind a ligand and transduce an intracellular signal, leading to a variety of cellular responses. The identification and characterization of such a receptor enables one to identify both the ligands which bind to the receptor and the intracellular molecules and signal transduction pathways associated with the receptor, permitting one to identify or design modulators of receptor activity, e.g., receptor agonists or antagonists and modulators of signal transduction.

### Summary of the Invention

The present invention is based, at least in part, on the discovery of cDNA molecules encoding TANGO 128, TANGO 140, TANGO 197, TANGO 212, TANGO 213, TANGO 224, and TANGO 239, all of which are either wholly secreted or transmembrane proteins. These proteins, fragments, derivatives, and variants thereof are collectively referred to as polypeptides of the invention or proteins of the invention. Nucleic acid molecules encoding polypeptides of the invention are collectively referred to as nucleic acids .of the invention.

The nucleic acids and polypeptides of the present invention are useful as modulating agents in regulating a variety of cellular processes. Accordingly, in one aspect, the present invention provides isolated nucleic acid molecules encoding a polypeptide of the invention or a biologically active portion thereof. The present invention also provides nucleic acid molecules which are suitable as primers or hybridization probes for the detection of nucleic acids encoding a polypeptide of the invention.

The invention features nucleic acid molecules which are at least 45% (or 55%, 65%, 75%, 85%, 95%, or 98%) identical to the nucleotide sequence of any of SEQ ID NOs:1, 3, 4, 6, 8, 10, 11, 13, 14, 16, 17, 19, 20, 22, 38, 39, 53, 55, 56, 58, 59, 61, 62, 64, 65, 67, 68, 70, 71, 73, or the nucleotide sequence of the cDNA of a clone deposited with ATCC as any of Accession Numbers 98999, 202171, 98965, and 98966 (the "cDNA of a clone deposited as any of ATCC 98999, 202171, 98965, and 98966"), or a complement thereof.

The invention features nucleic acid molecules which are at least 45% (or 55%, 65%, 75%, 85%, 95%, or 98%) identical to the nucleotide sequence of any of SEQ ID NOs:1, 3, 4, 6, 8, 10, 11, 13, 14, 16, 17, 19, 20, 22, 38, 39, 53, 55, 56, 58, 59, 61, 62, 64, 65, 67, 68, 70, 71, 73, or the nucleotide sequence of the cDNA of a clone deposited with ATCC as any of Accession Numbers 98999, 202171, 98965, and 98966 (the "cDNA of a clone deposited as any of ATCC 98999, 202171, 98965; and 98966"), or a complement thereof, wherein such nucleic acid molecules encode polypeptides or proteins that exhibit at least one structural and/or functional feature of a polypeptide of the invention.

The invention features nucleic acid molecules of at least 570, 600, 650, 700, 750, 800, 850, 900, 950, 1000, 1100, 1200, 1300, 1400,1500,1600,1700, 1800, 1900, 2000, 2100, 2200, 2300, 2400, 2500, 2600, 2700, 2800 or 2835 nucleotides of the nucleotide sequence of SEQ ID NO:1, the nucleotide sequence of the TANGO 128 cDNA clone of ATCC Accession No. 98999, or a complement thereof. The invention also features nucleic acid molecules comprising at least 25, 50, 100, 150, 200, 250, 300, 350, 400, 450, 500, 600, 650, 700, 750, 800, 850, 900, 950, 1000, 1050, 1100, 1150, 1200, 1250, 1300, 1350, 1400, 1450, 1500, 1550, 1600, 1650, 1700, 1750, 1800, 1850, 1900, 1950, 2000, 2050, 2100, 2150, 2200 or 2230 nucleotides of nucleic acids 1 to 2233 of SEQ ID NO:1, or a complement thereof.

The invention features nucleic acid molecules which include a fragment of at least 15, 25, 50, 75, 100, 150, 200, 250, 300, 350, 400, 450, 500, 550, 600, 650, 700, 750, 800, 850, 900, 950, 1000 or 1030 nucleotides of the nucleotide sequence of SEQ ID NO:3, or a complement thereof.

The invention features nucleic acid molecules of at least 250, 275, 300, 325, 350, 375, 400, 425, 450, 475, 500, 525, 550, 575, 600, 625, 650, 675, 700, 725, 750 or 760 nucleotides of the nucleotide sequence of SEQ ID NO:53, the nucleotide sequence of a mouse TANGO 128 cDNA, or a complement thereof. The invention features nucleic acid molecules comprising at least 25 30, 35, 40, 45, 50, 55, 60, 65, 70 or 77 nucleotides of nucleic acids 1 to 78 of SEQ ID NO:53, or a complement thereof. The invention features nucleic acid molecules comprising at least 25 30, 35, 40, 45, 50, 55 or 60 nucleotides of nucleic acids 257 to 318 of SEQ ID NO:53, or a complement thereof.

The invention features nucleic acid molecules comprising at least 225, 250, 275, 300, 325, 350, 375, 400, 425, 450, 475, 500, 525 or 550 nucleotides of the nucleotide sequence of SEQ ID NO:55, or a complement thereof. The invention also features nucleic acid molecules comprising at least 25, 30, 35, 40, 45, 50, 55 or 60 nucleotides of nucleic acids 46 to 107 of SEQ ID NO:55, or a complement thereof.

The invention features nucleic acid molecules of at least 425, 450, 475, 500, 525, 550, 600, 650, 700, 750, 800, 850, 900, 950, 1000, 1100, 1200, 1300 1400, 1500 or 1540 nucleotides of the nucleotide sequence of SBQ ED NO:4, the nucleotide sequence of a human TANGO 140-1 cDNA, the nucleotide sequence of the TANGO 140-1 cDNA clone of ATCC Accession No. 98999, or a complement thereof. The invention also features nucleic acid molecules comprising at least 25, 50, 100, 150, 200, 250, 300, 350 400, 450, 500 or 540 nucleotides of nucleic acids 1 to 545 of SEQ ID NO:4, or a complement thereof. The invention also features nucleic acid molecules comprising at least 25, 50, 100, 150, 200, 250, 300, 350, 400, 450, 500, 550 or 580 nucleotides of nucleic acids 980 to 1550 of SEQ ID NO:4, or a complement thereof.

The invention features nucleic acid molecules of at least 425, 450, 475, 500, 525, 550, 600, 650, 700, 750, 800, 850, 900, 950, 1000, 1100, 1200, 1300, 1400, 1500, 1650, 1700, 1750, 1800, 1850, 1900, 1950, 2000, 2050, 2100, 2150, 2200, 2300, 2350, 2400, 2450, 2500, 2550, 2600, 2650, 2700, 2750, 2800, 2850, 2900, 2950, 3000, 3050, 3100, 3150, 3200, 3250, 3300, 3350 or 3385 nucleotides of the nucleotide sequence of SEQ ID NO:6, the nucleotide sequence of a human TANGO 140-2 cDNA, the nucleotide sequence of the TANGO 140-2 cDNA clone of ATCC Accession No. 98999, or a complement thereof. The invention also features nucleic acid molecules comprising at least 25, 50, 100, 150, 200, 250, 300, 350 400, 450, 500 or 540 nucleotides of nucleic acids 1 to 545 of SEQ ID NO:6, or a complement thereof. The invention also features nucleic acid molecules comprising at least 25, 50, 100, 150, 200, 250, 300, 350, 400, 450, 500, 550, 600, 650, 700, 750, 800, 850, 900, 950, 1000, 1100, 1200, 1300, 1400, 1500, 1650, 1700, 1750, 1800, 1850, 1900, 1950, 2000, 2050, 2100, 2150, 2200, 2300, 2350 or 2400 nucleotides of nucleic acids 980 to 3385 of SEQ ID NO:6, or a complement thereof.

The invention features nucleic acid molecules comprising at least 75, 100, 150, 200, 250, 300, 350, 400, 450, 500, 550, 600 or 615 nucleotides of the nucleotide sequence of SEQ ID NO:38 or 39, or a complement thereof The invention features nucleic acid molecules comprising at least 25, 75, 100, 150, 200, 250, 300, 350, 400, 450, 500 or 545 nucleotides of nucleic acids 1 to 545 of SEQ ID NO:38 or 39, or a complement thereof.

The invention features nucleic acid molecales of at least 520, 550, 600, 650, 700, 750, 800, 850, 900, 950, 1000, 1100, 1200, 1300, 1400, 1500, 1600, 1700,1800, 1900. 2000, 2100, 2200, 2250 or 2270 nucleotides of the nurlootide sequence of SEQ ID NO:8, the nucleotide sequence of the TANGO 197 cDNA clone of ATCC Accession No. 98999, or a complement thereof The invention also features nucleic acid molecules composing at least 25, 50, 100, 150, 200, 250, 300, 350, 400, 450, 500, 550, 600, 650, 700, 750 or 785 nucleondes of nucleic acids 1 to 789 of SEQ ID NO:8, or a complement thereof. The invention also features nucleic acid molecules comprising at least 25, 50, 100, 150, 200, 250, 300, 350, 400, 450 or 500 nucleotides of nucleic acids 1164 to 1669 of SEQ ID NO:8, or a complement thereof. The invention also features nucleic acid molecules comprising at least 25, 50 or 80 nucleotides of nucleic acids 2190 to 2272 of SEQ ID NO:8, or a complement thereof.

The invention features nucleic acid molecules which include a fragment of at least 380, 400, 450, 500, 550, 600, 650, 700, 750, 800, 850, 900, 950, 1000, 1100, 1200, 1300, 1400, 1500, 1600, 1700, 1750 or 1770 nucleotides of the nucleotide sequence of SEQ ID NO:10, or a complement thereof. The invention also features nucleic acid molecules comprising at least 25, 50, 100, 150, 200, 250, 300, 350, 400, 450, 500, 550 or 575 nucleotides of nucleic acids 1 to 576 of SEQ ID NO:10, or a complement thereof.

The invention features nucleic acid molecules of at least 515, 550, 600, 650, 700, 750, 800, 850, 900, 950, 1000, 1100, 1200, 1300, 1400, 1500, 1600, 1700, 1800, 1900, 2000, 2100, 2200, 2250, 2250, 2300, 2350, 2400, 2450, 2500, 3550, 2600, 2650, 2700, 2750, 2800, 2850, 2900, 2950, 3000, 3050, 3100, 3150, 3200, 3250, 3300, 3500, 3550, 3600, 3650, 3700, 3750, 3800, 3850, 3900, 3950, 4000, 4050, 4100, 4150, 4200, 4250, 4300, 4350, 4400 or 4415 nucleotides of the nucleotide sequence of SEQ ID NO:56, the nucleotide sequence of a mouse TANGO 197 cDNA, or a complement thereof. The invention also features nucleic acid molecules comprising at least 25, 50, 100, 150, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550, 1600, 1650, 1700, 1750, 1800, 1850, 1900, 1950, 2000, 2050, 2100, 2200, 2250, 2300, 2350, 2400, 2450, 2500, 2550, 2600, 2650, 2700, 2750, 2800, 2850, 2900, 2950, 3000, 3050, 3100 or 3135 nucleotides of nucleic acids 1 to 3138 of SEQ ID NO:56, or a complement thereof. The invention also features nucleic acid molecules comprising at least 25, 50, 100, 150, 200, 250, 300 or 320 nucleotides of nucleic acids 4094 to 4417 of SEQ ID NO:56, or a complement thereof.

The invention features nucleic acid molecules which include a fragment of at least 390, 400, 450, 500, 550, 600, 650, 700, 750, 800, 850, 900, 950, 1000, 1100 or 1140 nucleotides of the nucleotide sequence of SEQ ID NO:58, or a complement thereof.

The invention features nucleic acid molecules of at least 545, 600, 650, 700, 750, 800, 850, 900, 950, 1000, 1100, 1200, 1300, 1400, 1500, 1600, 1700, 1800, 1900, 2000, 2100, 2200, 2250, 2250, 2300, 2350, 2400 or 2435 nucleotides of the nucleotide sequence of SEQ ID NO:11, the nucleotide sequence of the TANGO 212 cDNA clone of ATCC Accession No. 202171 or a complement thereof. The invention also features nucleic acid molecules comprising at least 25, 50, 100, 150, 200, 250, 300, 350, 400, 450, 500, 550, 600, 650, 700, 750, 800, 850, 900, 950, 1000, 1050, 1100, 1150, 1200, 1250, or 1270 nucleotides of nucleic acids 1 to 1273 of SEQ ID NO:11, or a complement thereof. The invention also features nucleic acid molecules comprising at least 25, 50, 100, 150, 200, 250, 300 or 320 nucleotides of nucleic acids 4094 to 4417 of SEQ ID NO:11, or a complement thereof.

The invention features nucleic acid molecules which include a fragment of at least 240, 275, 300, 350, 400, 450, 500, 550, 600, 650, 700, 750, 800, 850, 900, 950, 1000, 1100, 1150, 1200, 1250, 1300, 1350, 1400, 1450, 1500, 1550, 1600 or 1660 nucleotides of the nucleotide sequence of SEQ ID NO:13, or a complement thereof The invention also features nucleic acid molecules comprising at least 25, 50, 100, 150, 200, 250, 300, 350, 400, 450, 500, 550, 600, 650, 700, 750, 800, 850 or 900 nucleotides of nucleic acids 1 to 905 of SEQ ID NO:13, or a complement thereof.

The invention features nucleic acid molecules of at least 785, 800, 850, 900, 950, 1000, 1050, 1100, 1150 or 1180 nucleotides of the nucleotide sequence of SEQ ID NO:59, the nucleotide sequence of a mouse TANGO 212 cDNA, or a complement thereof. The invention also features nucleic acid molecules comprising at least 25, 50, 100, 150 or 190 nucleotides of nucleic acids 983 to 1180 of SEQ ID NO:59, or a complement thereof.

The invention features nucleic acid molecules which include a fragment of at least 570, 600, 650, 700, 750, 800, 850, 900, 950 or 998 nucleotides of the nucleotide sequence of SEQ ID NO:61, or a complement thereof. The invention also features nucleic acid molecules comprising at least 25, 50, 100, 150 or 180 nucleotides of nucleic acids 804 to 999 of SEQ ID NO:61, or a complement thereof.

The invention features nucleic acid molecules of at least 530, 600, 650, 700, 750, 800, 850, 900, 950, 1000, 1100, 1200, 1300, 1400 or 1495 nucleotides of the nucleotide sequence of SEQ ID NO: 14, the nucleotide sequence of the TANGO 213 cDNA clone of ATCC Accession No. 98965, or a complement thereof. The invention also features nucleic acid molecules comprising at least 25,50,100,150,200,250,300 or 360 nucleotides of nucleic acids 1 to 361 of SEQ ID NO: 14, or a complement thereof. The invention also features nucleic acid molecules comprising at least 25, 40, 50 or 60 nucleotides of nucleic acids 759 to 822 of SEQ ID NO:14, or a complement thereof.

The invention features nucleic acid molecules which include a fragment of at least 250, 275, 300, 350, 400, 450, 500, 550, 600, 650, 700, 750, 800 or 810 nucleotides of the nucleotide sequence of SEQ ID NO:16, or a complement thereof. The invention also features nucleic acid molecules comprising at least 25, 50, 100,150, 200, 250 or 300 nucleotides of nucleic acids 1 to 304 of SEQ ID NO:16, or a complement thereof. The invention also features nucleic acid molecules comprising at least 25, 40, 50 or 60 nucleotides ofnucleic acids 701 to 764 of SEQ ID NO:16, or a complement thereof.

The invention features nucleic acid molecules of at least 530, 600, 650, 700, 750, 800, 850, 900, 950, 1000, 1100, 1200, 1300, 1400, 1500, 1600, 1700, 1800, 1900, 2000, 2100 or 2150 nucleotides of the nucleotide sequence of SEQ ID NO:62, the nucleotide sequence of a mouse TANGO 213 cDNA, or a complement thereof. The invention also features nucleic acid molecules comprising at least 25, 50, 100, 150, 200, 250, 300, 350, 400, 450, 500, 550, 600, 650, 700, 750, 800, 850, 900, 950 or 1000 nucleotides of nucleic acids 1 to 101 of SEQ ID NO:62, or a complement thereof. The invention also features nucleic acid molecules comprising at least 25, 50, 100, 150, 200, 250, 300, 350, 400, 450, 500, 550, 600, 650, 700, 750, 800, 850, 900 or 920 nucleotides of nucleic acids 1227 to 2154 of SEQ ID NO:62, or a complement thereof.

The invention features nucleic acid molecules which include a fragment of at least 25, 50, 100, 150, 200, 250, 275, 300, 350, 400, 450, 500, 550 or 575 nucleotides of the nuclcotide sequence of SEQ ID NO:64, or a complement thereof.

The invention features nucleic acid molecules of at least 570, 600, 650, 700, 750, 800, 850, 900, 950, 1000, 1100, 1200, 1300, 1400, 1500, 1600, 1700, 1800, 1900, 2000, 2100, 2150, 2200, 2250, 2300, 2350, 2400, 2450, 2500, 2550, 2600, 2650 or 2680 nucleotides of the nucleotide sequence of SEQ ID NOs:17 or 65, the nucleotide sequence of a human TANGO 224 cDNA form 1 or form 2 respectively, the nucleotide sequence of the TANGO 213 cDNA clone of ATCC Accession Number 98966, or a complement thereof. The invention also features nucleic acid molecules comprising at least 25, 50, 100, 150, 200, 250 or 270 nucleotides of nucleic acids 1 to 272 of SEQ ID NO:17 or 65, or a complement thereof. The invention also features nucleic acid molecules comprising at least 25, 50, 100, 150, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500 or 1530 nucleotides of nucleic acids 573 to 2106 of SEQ ID NO:17 or 65, or a complement thereof.

The invention features nucleic acid molecules which include a fragment of at least 300, 350, 400, 450, 500, 550, 600, 650, 700, 750, 800, 850, 900, 950, 1000, 1050, 1100, 1150, 1200, 1250, 1300 or 1360 nucleotides of the nucleotide sequence of SEQ ID NO:19, or a complement thereof. The invention also features nucleic acid molecules comprising at least 25, 40, 50, 100, 150 or 200 nucleotides of nucleic acids 1 to 204 of SEQ ID NO:19, or a complement thereof. The invention also features nucleic acid molecules comprising at least 25, 40, 50, 100, 150, 200, 250, 300, 350, 400, 450, 500, 550, 600, 650, 700, 750, 800, 850, 900 or 930 nucleotides of nucleic acids 507 to 1440 of SEQ ID NO:19, or a complement thereof.

The invention features nucleic acid molecules which include a fragment of at least 570, 600, 650, 700, 750, 800, 850, 900, 950, 1000, 1100, 1200, 1300, 1400, 1500, 1600, 1700, 1800, 1900, 2000, 2100, 2150, 2200, 2250, 2300, 2350, 2400, 2450, 2500, 2550, 2600, 2650 or 2680 nucleotides of the nucleotide sequence of SEQ ID NO:67, or a complement thereof. The invention also features nucleic acid molecules comprising at least 25, 40, 50, 100, 150 or 200 nucleotides of nucleic acids 1 to 204 of SEQ ID NO:67, or a complement thereof. The invention also features nucleic acid molecules comprising at least 25, 40, 50, 100, 150, 200, 250, 300, 350, 400, 450, 500, 55.0, 600, 650, 700, 750, 800, 850, 900, 950, 1000, 1050, 1100, 1150, 1200, 1250, 1300, 1350, 1400, 1450, 1500 or 1530 nucleotides of nucleic acids 507 to 2038 of SBQ ID NO:67, or a complement thereof.

The invention features nucleic acid molecules of at least 550, 600, 650, 700, 750, 800, 850, 900, 950, 1000, 1100, 1200, 1300, 1400, 1500, 1600, 1700, 1800, 1900, 2000, 2100, 2150, 2200, 2250, 2300, 2350, 2400, 2450, 2500, 2550, 2600, 2650, 2700, 2750, 2800, 2850, 2900, 2950, 3000, 3050, 3100, 3150, 3200, 3250, 3300, 3350 or 3400 nucleotides of the nucleotide sequence of SBQ ID NOs:20 or 68, the nucleotide sequence of the TANGO 239 cDNA clone of ATCC Accession No. 98999, or a complement thereof. The invention also features nucleic acid molecules comprising at least 25, 50, 100, 150, 200, 250, 300, 350, 400, 450, 500, 550, 600, 650, 700, 750, 800, 850, 900, 950, 1000, 1100, 1200, 1300, 1400, 1500, 1600, 1700, 1800, 1900, 2000, 2100, 2150, 2200 or 2225 nucleotides of nucleic acids 1 to 2227 of SEQ ID NQs:20 or 6.8, or a complement thereof.

The invention features nucleic acid molecules which include a fragment of at least 25, 50, 100, 150, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550, 1600 or' 1650 nucleotides of the nucleotide sequence of SEQ ID NO:22, or a complement thereof.

The invention features nucleic acid molecules which include a fragment of at least 25, 50, 100, 150, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550, 1600, 1650, 1700, 1750, 1800, 1850, 1900, 1950, 2000 or 2050 nucleotides of the nucleotide sequence of SEQ ID NO:70, or a complement thereof.

The invention features nucleic acid molecules of at least 25, 50, 100, 150, 200, 250, 300, 350, 400, 450, 500, 550, 600, 650, 700, 750, 800, 850, 900, 950, 1000 or 1028 nucleotides of the nucleotide sequence of SEQ ID NOs:20 or 68, the nucleotide sequence of a mouse TANGO 239 cDNA, or a complement thereof.

The invention features nucleic acid molecules which include a fragment of at least 25, 50, 100, 150 or 160 nucleotides of the nucleotide sequence of SBQ ID NO:73, or a complement thereof.

The invention features nucleic acid molecules which include a fragment of at least 300 (325, 350, 375, 400, 425, 450, 500, 550, 600, 650, 700, 800, 900, 1000, or 1200) nucleotides of the nucleotide sequence of any of SEQ ID Nos: 1, 3, 4, 6, 8, 10, 11, 13, 14, 16, 17, 19, 20, 22, 38, 39, 53, 55, 56, 58, 59, 61, 62, 64, 65, 67, 68, 70, 71, 73, or the' nucleotide sequence of the cDNA of a clone deposited as any of ATCC 98999, 202171, 98965, and 98966, or a complement thereof.

The invention features nucleic acid molecules which include a fragment of at least 300 (325, 350, 375, 400, 425, 450, 500, 550, 600, 650, 700, 800, 900, 1000, or 1200) nucleotides of the nucleotide sequence of any of SEQ ID Nos: 1, 3, 4, 6, 8, 10, 11, 13, 14, 16, 17, 19, 20, 22, 38, 39, 53, 55, 56, 58, 59, 61, 62, 64, 65, 67, 68, 70, 71, 73, or the nucleotide sequence of the cDNA of a clone deposited as any of ATCC 98999, 202171, 98965, and 98966, or a complement thereof, wherein such nucleic acid molecules encode polypeptides or proteins that exhibit at least one structural and/or functional feature of a polypeptide of the invention.

The invention also features nucleic acid molecules which include a nucleotide sequence encoding a protein having an amino acid sequence that is at least 45% (or 55%, 65%, 75%, 85%, 95%, or 98%) identical to the amino acid sequence of any of SEQ ID NOs:2, 5, 7, 9, 12, 15, 18, 21, 54, 57, 60, 63, 66, 69, 72, or the amino acid sequence encoded by the cDNA of a clone deposited as any of ATCC 98999, 202171, 98965, and 98966, or a complement thereof.

The invention also features nucleic acid molecules which include a nucleotide sequence encoding a protein having an amino acid sequence that is at least 45% (or 55%, 65%, 75%, 85%, 95%, or 98%) identical to the amino acid sequence of any of SEQ ID NOs:2, 5, 7, 9, 12, 15, 18, 21, 54, 57, 60, 63, 66, 69, 72, or the amino acid sequence encoded by the cDNA of a clone deposited as any of ATCC 98999, 202171, 98965, and 98966, or a complement thereof, wherein the protein encoded by the nucleotide sequence also exhibits at least one structural and/or functional feature of a polypeptide of the invention.

In preferred embodiments, the nucleic acid molecules have the nucleotide sequence of any of SEQ ID NOs:1, 3, 4, 6, 8, 10, 11, 13, 14, 16, 17, 19, 20, 22, 38, 39, 53, 55, 56, 58, 59, 61, 62, 64, 65, 67, 68, 70, 71, 73, or the nucleotide sequence of the cDNA of a clone deposited as any of ATCC 98999, 202171, 98965, and 98966, or a complement thereof Also within the invention are nucleic acid molecules which encode a fragment of a polypeptide having the amino acid sequence of any of SEQ ID NOs:2, 5, 7, 9, 12, 15, 18, 21, 54, 57, 60, 63, 66, 69, 72, the fragment including at least 15 (20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390 or 400) contiguous amino acids of any of SEQ ID NOs:2, 5, 7, 9, 12, 15, 18, 21, 54, 57, 60, 63, 66, 69, 72, or the polypeptide encoded by the cDNA of a clone deposited as any of ATCC 98999, 202171, 98965, and 98966.

Also within the invention are nucleic acid molecules which encode a fragment of a polypeptide having the amino acid sequence of any of SEQ ID NOs:2, 5, 7, 9, 12, 15, 18, 21, 54, 57, 60, 63, 66, 69, 72, the fragment including at least 15 (20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390 or 400) contiguous amino acids of any of SEQ ID NOs:2, 5, 7, 9, 12, 15, 18, 21, 54, 57, 60, 63, 66, 69, 72, or the polypeptide encoded by the cDNA of a clone deposited as any of ATCC 98999, 202171, 98965, and 98966, wherein the fragment exhibits at least one structural and/or functional feature of a polypeptide of the invention.

The invention includes nucleic acid molecules which encode a naturally occurring allelic variant of a polypeptide comprising the amino acid sequence of any of SEQ ID NOs:2, 5, 7, 9, 12, 15, 18, 21, 54, 57, 60, 63, 66, 69, 72, or an amino acid sequence encoded by the cDNA of a clone deposited as any of ATCC 98999, 202171, 98965, and 98966, or a complement thereof, wherein the nucleic acid molecule hybridizes under stringent conditions to a nucleic acid molecule having a nucleic acid sequence encoding any of SEQ ID NOs:2, 5, 7, 9, 12, 15, 18, 21, 54, 57, 60, 63, 66, 69, 72, or a complement thereof.

The invention includes nucleic acid molecules which encode a naturally occurring allelic variant of a polypeptide comprising the amino acid sequence of any of SEQ ID NOs:2, 5, 7, 9,12,15, 18, 21, 54, 57, 60, 63, 66, 69, 72, or an amino acid sequence encoded by the cDNA of a clone deposited as any of ATCC 98999, 202171, 98965, and 98966, or a complement thereof, wherein the nucleic acid molecule hybridizes under stringent conditions to a nucleic acid molecule having a nucleic acid sequence encoding any of SEQ ID NOs:2, 5, 7, 9, 12, 15, 18, 21, 54, 57, 60, 63, 66, 69, 72, or a complement thereof, wherein such nucleic acid molecules encode polypeptides or proteins that exhibit at least one structural and/or functional feature of a polypeptide of the invention.

Also within the invention are isolated polypeptides or proteins having an amino acid sequence that is at least about 65%, preferably 75%, 85%, 95%, or 98% identical to the amino acid sequence of any of SEQ ID NOs:2, 5, 7, 9, 12, 15, 18, 21, 54, 57, 60, 63, 66, 69, or 72.

Also within the invention are isolated polypeptides or proteins having an amino acid sequence that is at least about 65%, preferably 75%, 85%, 95%, or 98% identical to the amino acid sequence of any of SEQ ID NOs:2, 5, 7, 9, 12, 15, 18, 21, 54, 57, 60, 63, 66, 69, 72, wherein the polypeptides or proteins also exhibit at least one structural and/or functional feature of a polypeptide of the invention.

Also within the invention are isolated polypeptides or proteins which preferably are encoded by a nucleic acid molecule having a nucleotide sequence that is at least about 65%, 75%, 85%, or 95% identical the nucleic acid sequence encoding any of SEQ ID NOs:2, 5, 7, 9, 12, 15, 18, 21, 54, 57, 60, 63, 66, 69, 72, wherein the polypeptides or proteins preferably also exhibit at least one structural and/or functional feature of a polypeptide of the invention, and isolated polypeptides or proteins which are encoded by a nucleic acid molecule having a nucleotide sequence which hybridizes under stringent hybridization conditions to a nucleic acid molecule having the sequence of any of SEQ ID NOs:1, 3, 4, 6, 8, 10, 11, 13, 14, 16, 17, 19, 20, 22, 38, 39, 53, 55, 56, 58, 59, 61, 62, 64, 65, 67, 68, 70, 71, 73, or a complement thereof, or the non-coding strand of the cDNA of a clone deposited as any of ATCC 98999, 202171, 98965, and 98966,

Also within the invention are polypeptides which are naturally occurring allelic variants of a polypeptide that includes the amino acid sequence of any of SEQ ID NOs:2, 5, 7, 9, 12, 15, 18, 21, 54, 57, 60, 63, 66, 69, 72, or an amino acid sequence encoded by the cDNA of a clone deposited as any of ATCC 98999, 202171, 98965, and 98966, wherein the polypeptide is encoded by a nucleic acid molecule which hybridizes under stringent conditions to a nucleic acid molecule having the sequence of any of SEQ ID Nos:1, 3, 4, 6, 8, 10, 11, 13, 14, 16, 17, 19, 20, 22, 38, 39, 53, 55, 56, 58, 59, 61, 62, 64, 65, 67, 68, 70, 71, 73, or a complement thereof.

Also within the invention are polypeptides which are naturally occurring allelic variants of a polypeptide that includes the amino acid sequence of any of SEQ ID NOs:2, 5, 7, 9, 12, 15, 18, 21, 54, 57, 60, 63, 66, 69, 72, or an amino acid sequence encoded by the cDNA of a clone deposited as any of ATCC 98999, 202171, 98965, and 98966, wherein the polypeptide is encoded by a nucleic acid molecule which hybridizes under stringent conditions to a nucleic acid molecule having the sequence of any of SEQ ID Nos:1, 3, 4, 6, 8, 10, 11, 13, 14, 16, 17, 19, 20, 22, 38, 39, 53, 55, 56, 58, 59, 61, 62, 64, 65, 67, 68, 70, 71, 73, or a complement thereof, wherein such nucleic acid molecules encode polypeptides or proteins that exhibit at least one structural and/or functional feature of a polypeptide of the invention.

The invention also features nucleic acid molecules that hybridize under stringent conditions to a nucleic acid molecule comprising the nucleotide sequence of any of SEQ ID NOs:1, 3, 4, 6, 8, 10, 11, 13, 14, 16, 17, 19, 20, 22, 38, 39, 53, 55, 56, 58, 59, 61, 62, 64, 65, 67, 68, 70, 71, 73, of the cDNA of a clone deposited as any of ATCC 98999, 202171, 98965, and 98966, or a complement thereof, wherein preferably such nucleic acid molecules encode polypeptides or proteins that exhibit at least one structural and/or functional feature of a polypeptide of the invention. In other embodiments, the nucleic acid molecules are at least 300 (325, 350; 375, 400, 425, 450, 500, 550, 600, 650, 700, 800, 900,1000, or 1290) nuclootidos in length and hybridize under stringent conditions to a nucleic acid molecule comprising the nucleotide sequence of any of SBQ ID NOs:1, 3, 4, 6, 8, 10, 11, 13, 14, 16, 17, 19, 20, 22, 38, 39, 53, 55, 56, 55, 59, 61, 62, 64, 65, 67, 68, 70, 71, 73, of the cDNA of a clone deposited as any of ATCC 98999, 202171, 98965, and 98966, or a complement thereof.

In certain preferred embodiments, the isolated nucleic acid molecules encode a cytoplasmic, transmembrane, or extracellular domain of a polypeptide of me invention.

In another embodiment, the invention provides an isolated nucleic acid molecule which is antisense to the coding strand of a nucleic acid of the invention.

Another aspect of the invention provides vectors, e.g., recombinant expression vectors, comprising a nucleic acid molecule of the invention. In another embodiment the invention provides host cells containing such a vector, or engineered to contain a nuclcioc acid of the invention and/or to express a nucleic acid of the invention. The invention also provides methods for producing a polypeptide of the invention by culturing, in a suitable medium, a host cell of the invention such that the polypeptide of the invention is produced.

Another aspect of this invention features isolated or recombinant proteins and polypeptides of the invention. Preferred proteins and polypeptides possess at least one biological activity possessed by the corresponding naturally-occurring human polypeptide. An activity, a biological activity, and a functional activity of a polypeptide of the invention refers to an activity exerted by a protein or polypeptide of the invention on a responsive cell as determined *in vivo*, or *in vitro,* according to standard techniques. Such activities can be a direct activity, such as an association with or an enzymatic activity on a second protein or an indirect activity, such as a cellular signaling activity mediated by interaction of the protein with a second protein. Thus, such activities include, *e*.*g*., (1) the ability to form protein-protein interactions with proteins in the signaling pathway of the naturally-occurring polypeptide; (2) the ability to bind a ligand of the naturally-occurring polypeptide; (3) the ability to bind to an intracellular target of the naturally-occurring polypeptide. Other activities include, *e*.*g*., (1) the ability to modulate cellular proliferation; (2) the ability to modulate cellular differentiation; (3) the ability to modulate chemotaxis and/or migration; and (4) the ability to modulate cell death.

In one embodiment, a polypeptide of the invention has an amino acid sequence sufficiently identical to an identified domain of a polypeptide of the invention. As used herein, the term "sufficiently identical" refers to a first amino acid or nucleotide sequence which contains a sufficient or minimum number of identical or equivalent (e.g., with a similar side chain) amino acid residues or nucleotides to a second amino acid or nucleotide sequence such that the first and second amino acid or nucleotide sequences have a common structural domain and/or common functional activity. For example, amino acid or nucleotide sequences which contain a common structural domain having about 65% identity, preferably 75% identity, more preferably 85%, 95%, or 98% identity are defined herein as sufficiently identical.

In one embodiment, the isolated polypeptide of the invention lacks both a transmembrane and a cytoplasmic domain. In another embodiment, the polypeptide lacks both a transmembrane domain and a cytoplasmic domain and is soluble under physiological conditions.

The polypeptides of the present invention, or biologically active portions thereof, can be operably linked to a heterologous amino acid sequence to form fusion proteins. The invention further features antibodies that specifically bind a polypeptide of the invention such as monoclonal or polyclonal antibodies.

In addition, the polypeptides of the invention or biologically active portions thereof, or antibodies of the invention, can be incorporated into pharmaceutical compositions, which optionally include pharmaceutically acceptable carriers.

In another aspect, the present invention provides methods for detecting the presence of the activity or expression of a polypeptide of the invention in a biological sample by contacting the biological sample with an agent capable of detecting an indicator of activity such that the presence of activity is detected in the biological sample.

In another aspect, the invention provides methods for modulating activity of a polypeptide of the invention comprising contacting a cell with an agent that modulates (inhibits or stimulates) the activity or expression of a polypeptide of the invention such that activity or expression in the cell is modulated. In one embodiment, the agent is an antibody that specifically binds to a polypeptide of the invention.

In another embodiment, the agent modulates expression of a polypeptide of the invention by modulating transcription, splicing, or translation of an mRNA encoding a polypeptide of the invention, In yet another embodiment, the agent is a nucleic acid molecule having a nucleotide sequence that is antisense to the coding strand of an mRNA encoding a polypeptide of the invention.

The present invention also provides methods to treat a subject having a disorder characterized by aberrant activity of a polypeptide of the invention or aberrant expression of a nucleic acid of the invention by administering an agent which is a modulator of the activity of a polypeptide of the invention or a modulator of the expression of a nucleic acid of the invention to the subject. In one embodiment, the modulator is a protein of the invention. In another embodiment, the modulator is a nucleic acid of the invention. In other embodiments, the modulator is a peptide, peptidomimetic, or other small organic molecule. The present invention also provides diagnostic assays for identifying the presence or absence of a genetic lesion or mutation characterized by at least one of: (i) aberrant modification or mutation of a gene encoding a polypeptide of the invention, (ii) mis-regulation of a gene encoding a polypeptide of the invention, and (iii) aberrant post-translational modification of a polypeptide of the invention wherein a wild-type form of the gene encodes a polypeptide having the activity of the polypeptide of the invention.

In another aspect, the invention provides a method for identifying a compound that binds to or modulates the activity of a polypeptide of the invention. In general, such methods entail measuring a biological activity of the polypeptide in the presence and absence of a test compound and identifying those compounds which alter the activity of the polypeptide.

The invention also features methods for identifying a compound which modulates the expression of a polypeptide or nucleic acid of the invention by measuring the expression of the polypeptide or nucleic acid in the presence and absence of the compound.

Other features and advantages of the invention will be apparent from the following detailed description and claims.

### Brief Description of the Drawings

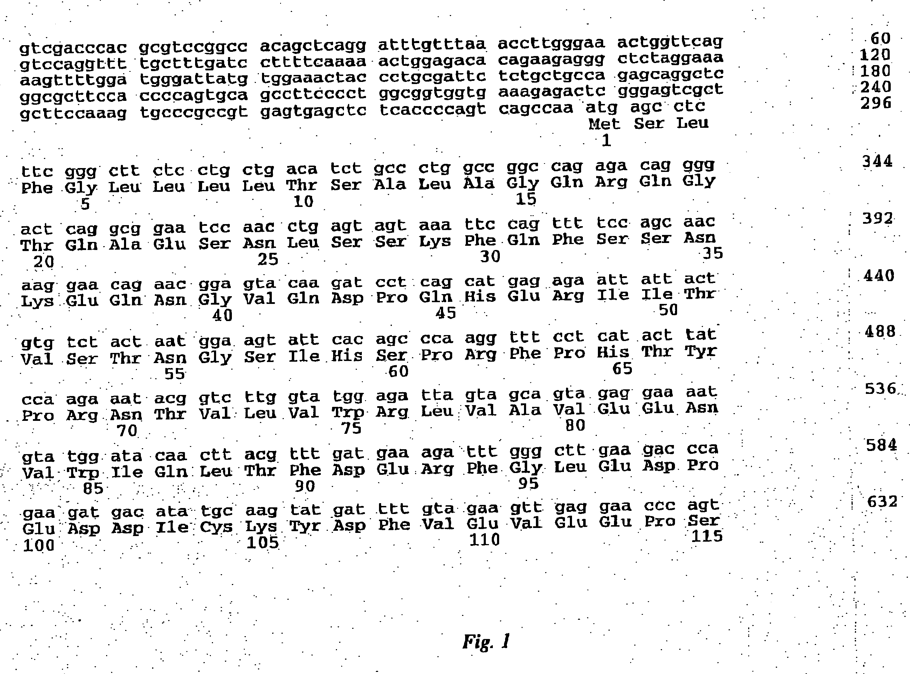

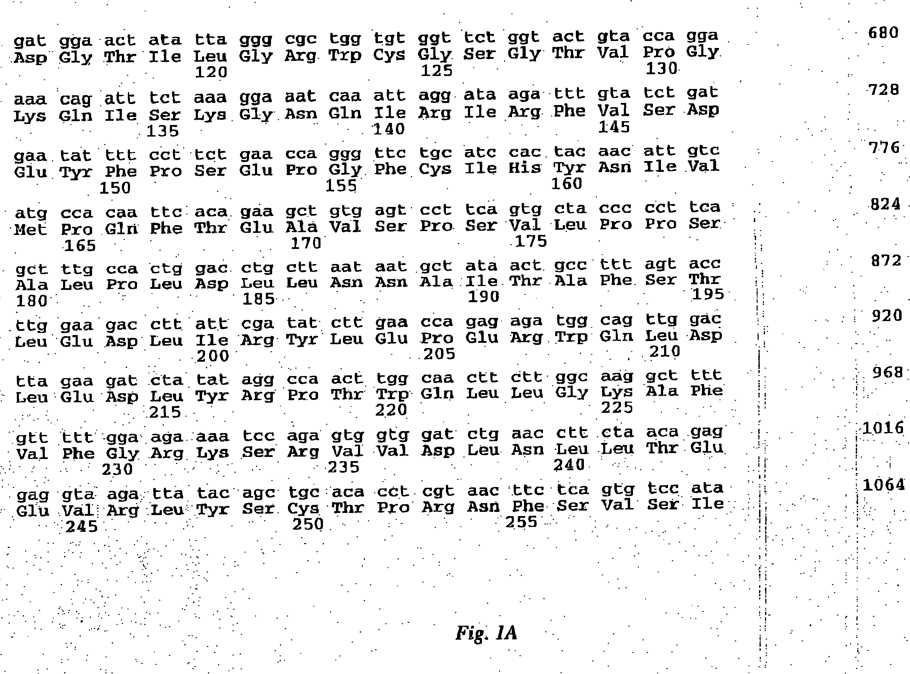

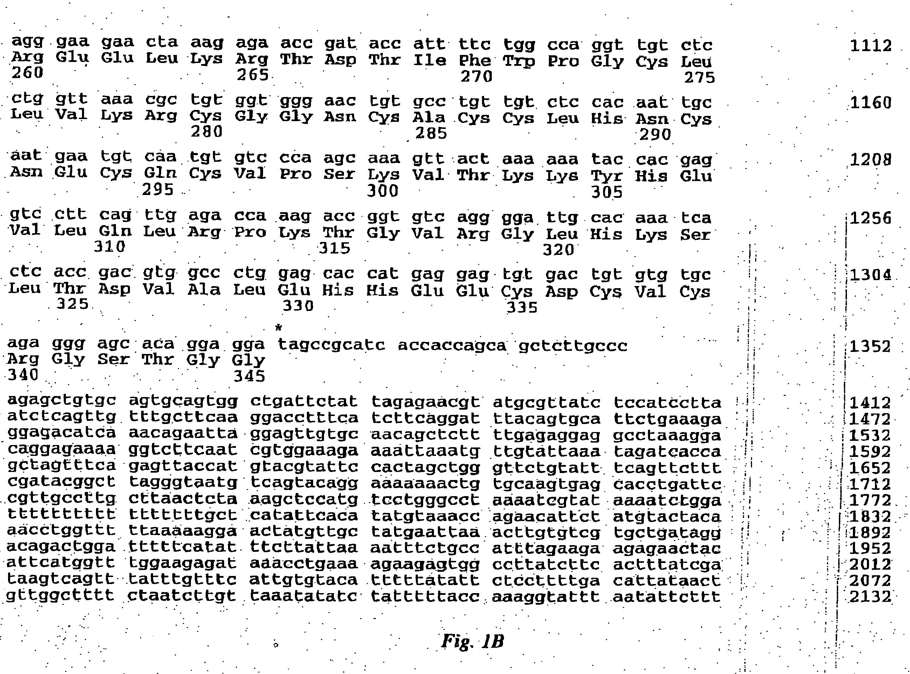

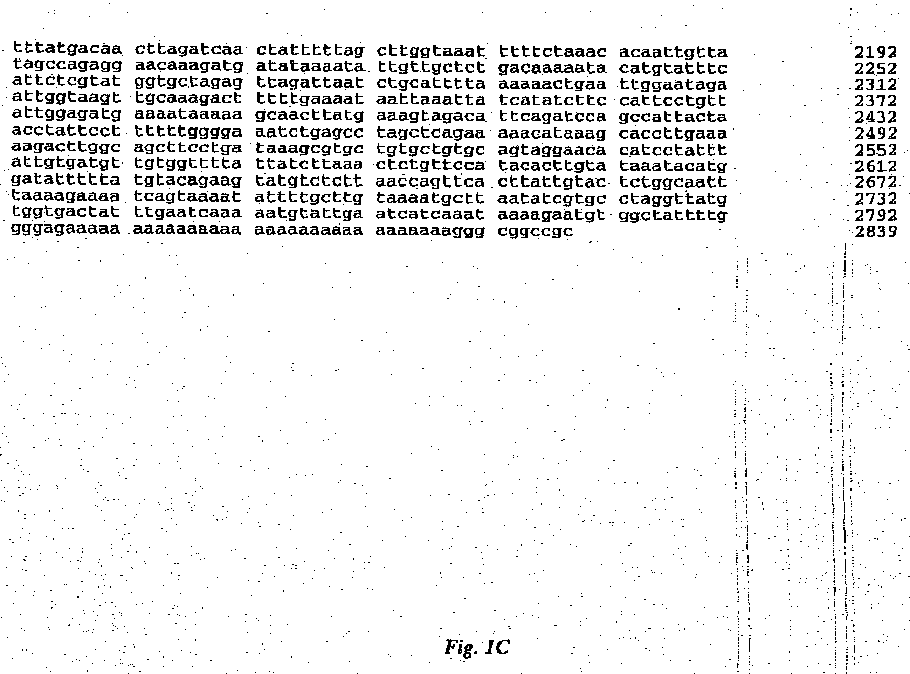

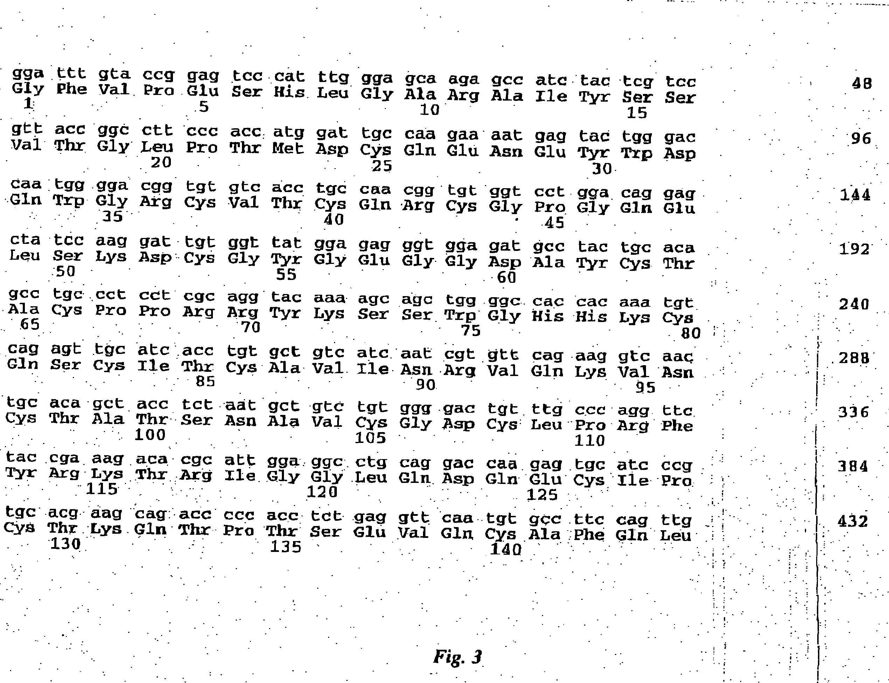

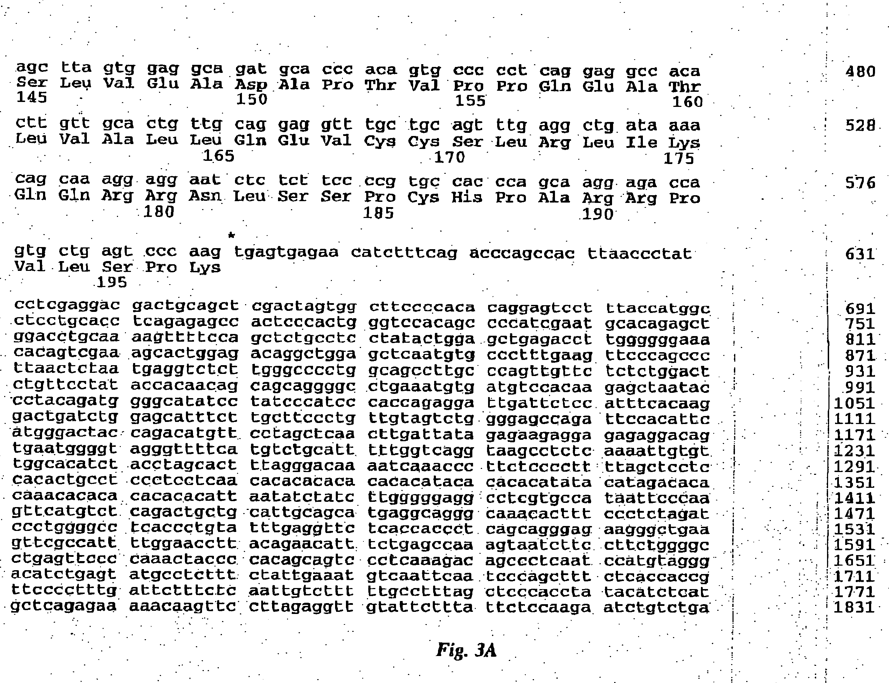

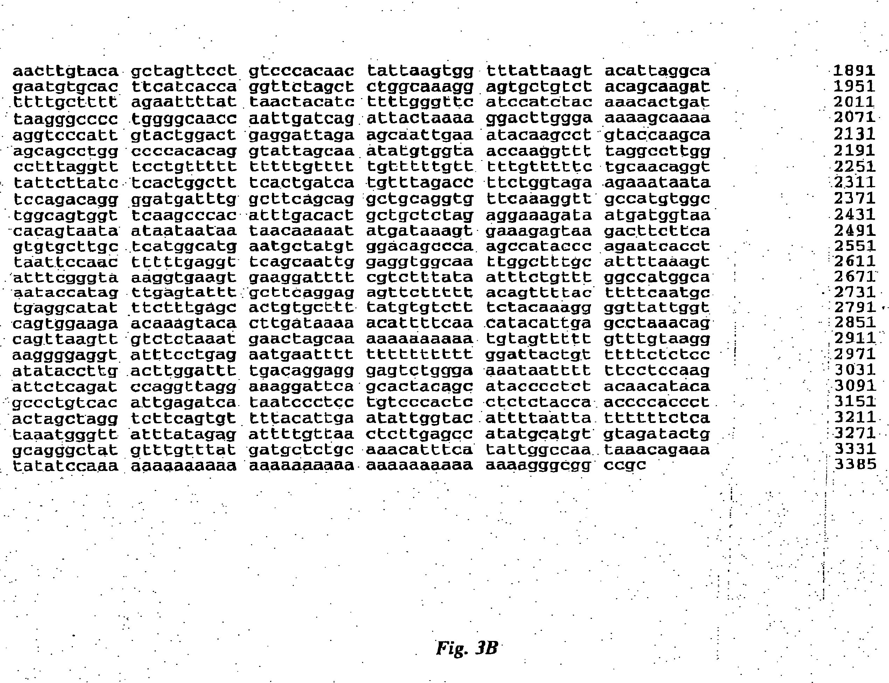

*Figure 1* depicts the cDNA sequence of human TANGO 128 (SBQ ID NO:1) and predicted amino acid sequence of TANGO 128 (SEQ ID NO:2). The open reading frame of SEQ ID NO:1 extends from nucleotide 288 to 1322 of SEQ ID NO:1 (SEQ ID NO:3).

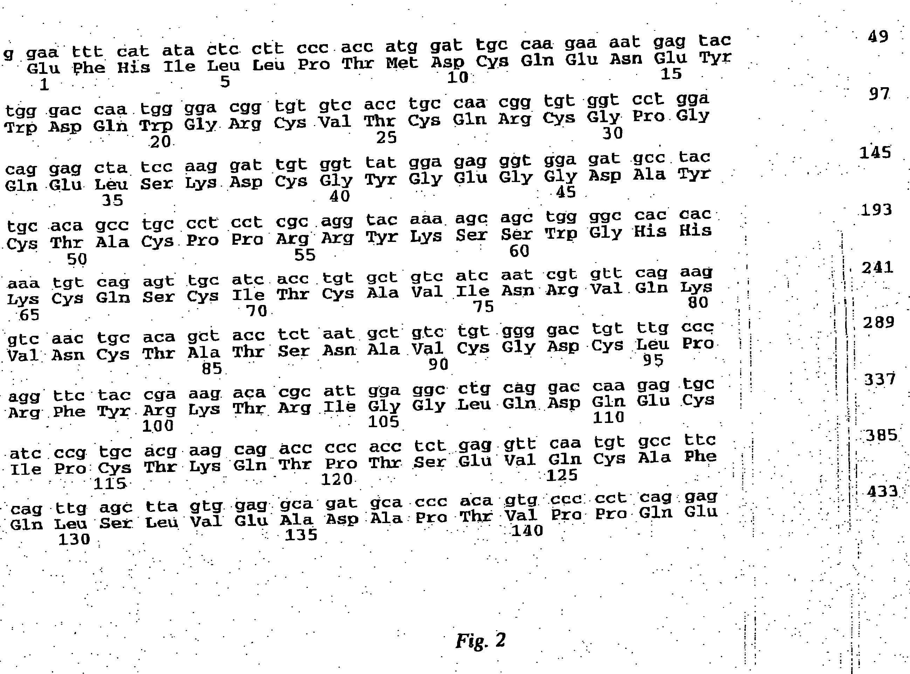

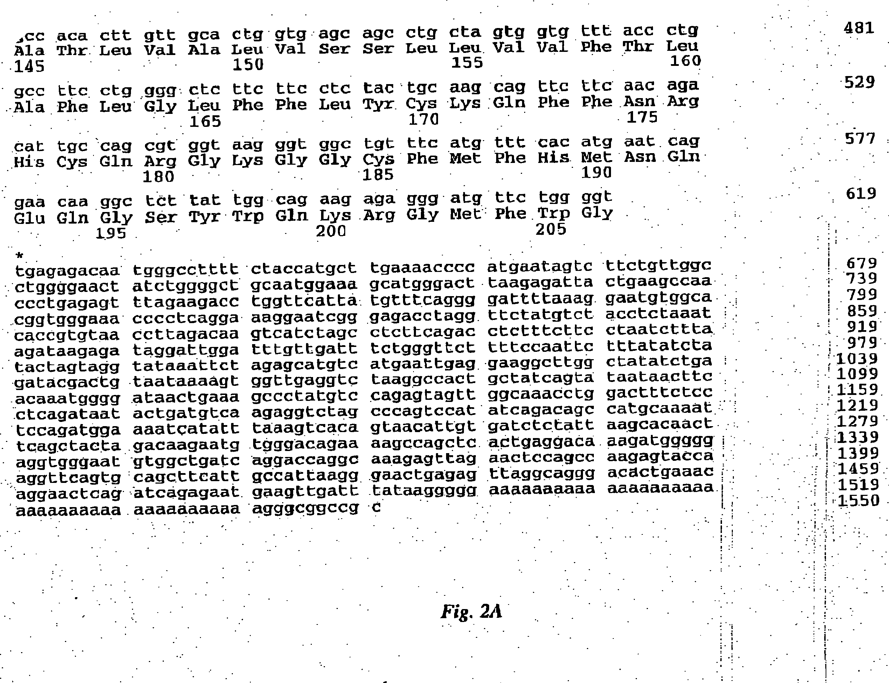

*Figure 2* depicts the cDNA sequence of human TANGO 140-1 (SEQ ID NO:4) and predicted amino acid sequence of TANGO 140-1 (SEQ ID NO:5). The open reading frame of SEQ ID NO:4 extends from nucleotide 2 to 6.19 of SEQ ID NO:4 (SEQ ID NO:38).
. *Figure* 3 depicts the cDNA sequence of human TANGO 140-2 (SEQ ID NO:6) and predicted amino acid sequence of TANGO 140-2 (SEQ ID NO:7). The open reading frame of SEQ ID NO:6 extends from nucleotide 1 to 591 of SEQ ID NO:6 (SEQ ID NO:39).

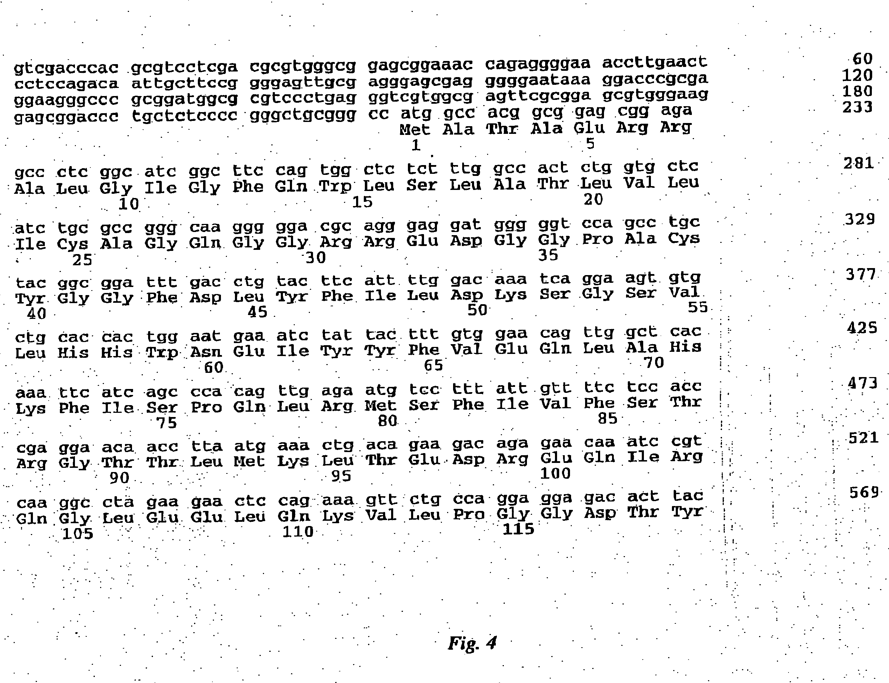

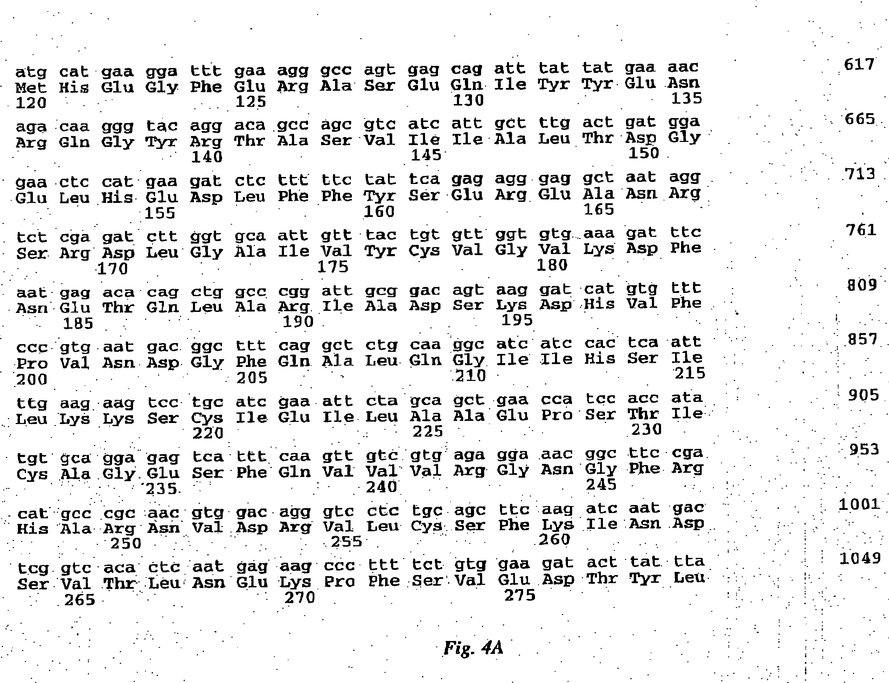

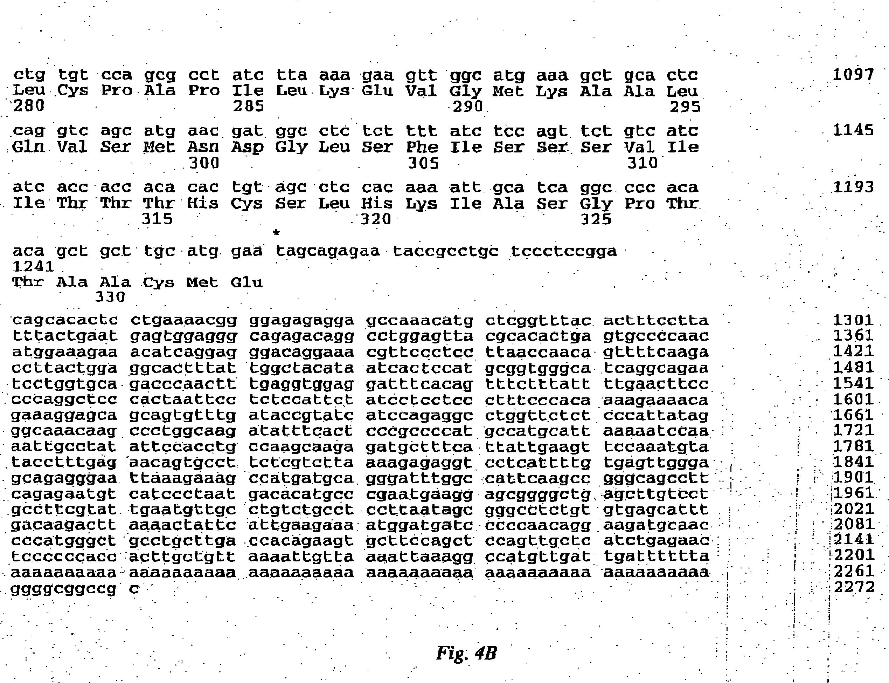

*Figure 4* depicts the cDNA sequence of human TANGO 197 (SEQ ID NO:8) and predicted amino acid sequence of TANGO 197 (SEQ ID NO:9). The open reading frame of SEQ ID NO:8 extends from nucleotide 213 to 1211 of SEQ ID NO:8 (SEQ ID NO:10).

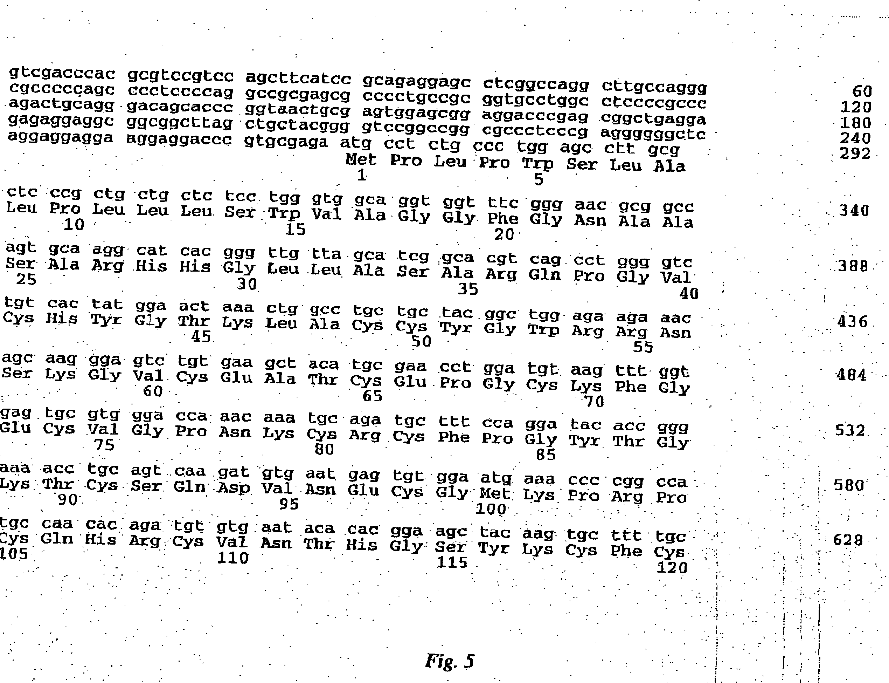

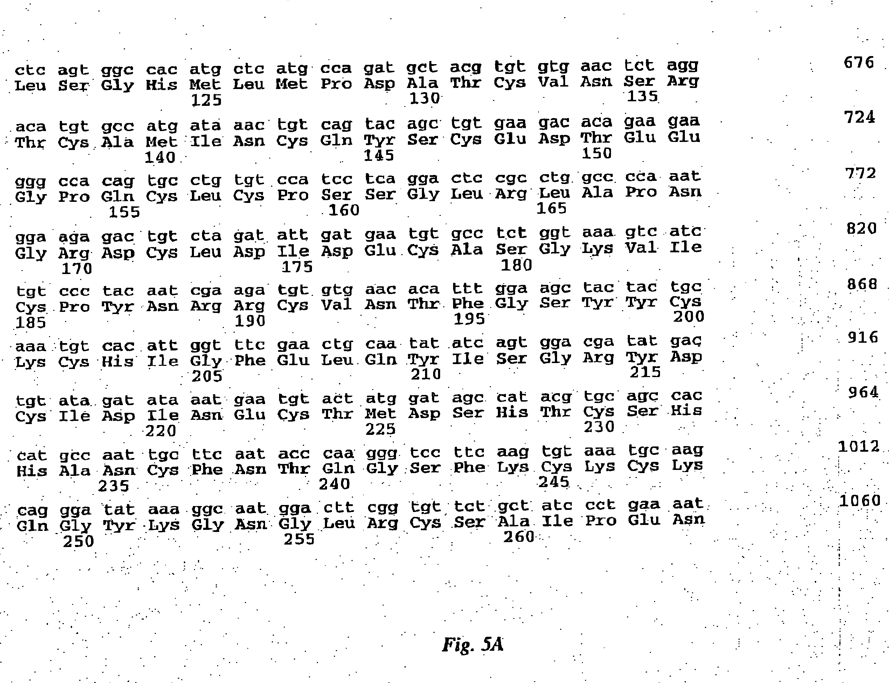

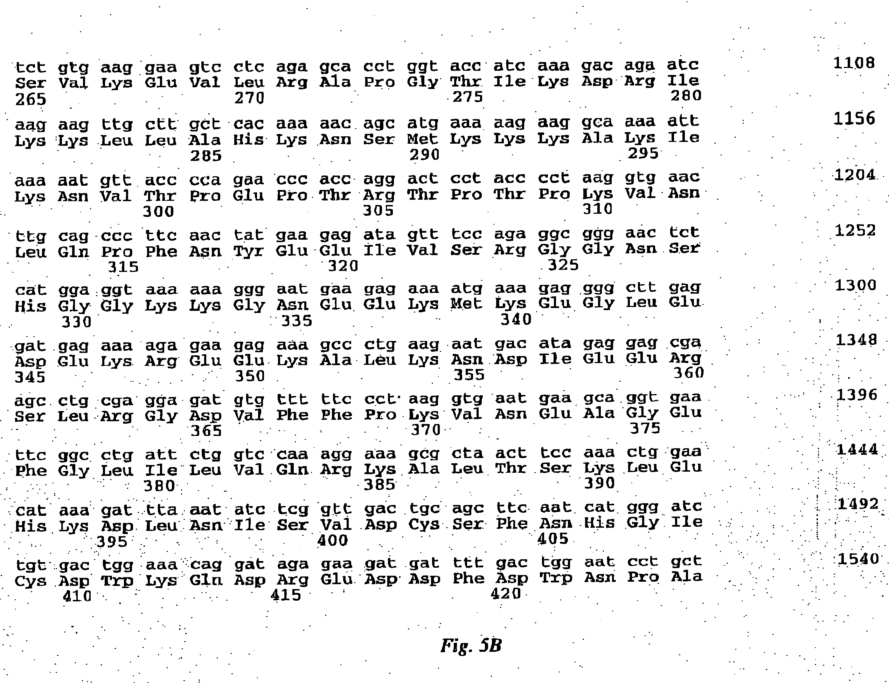

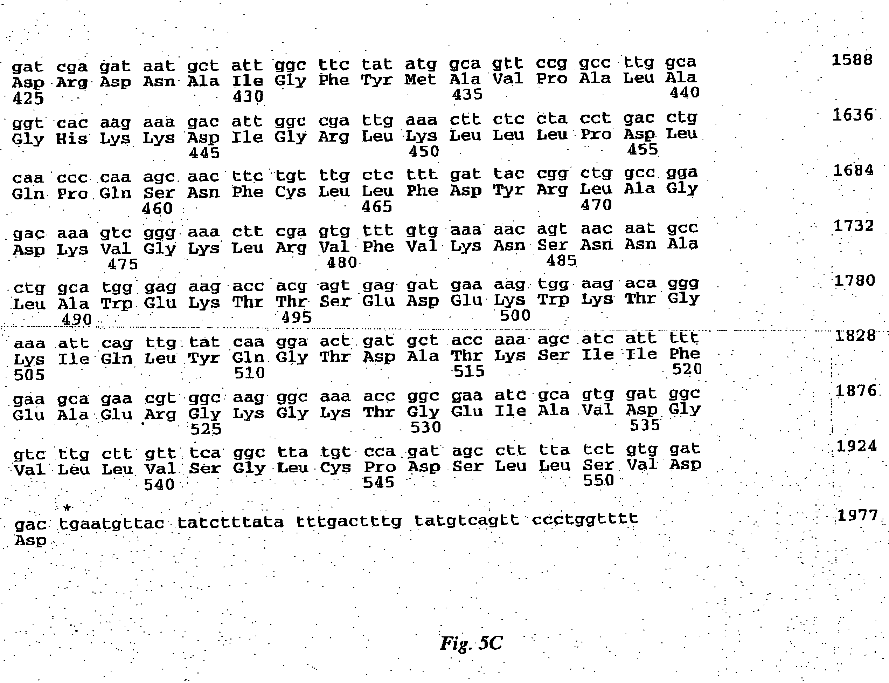

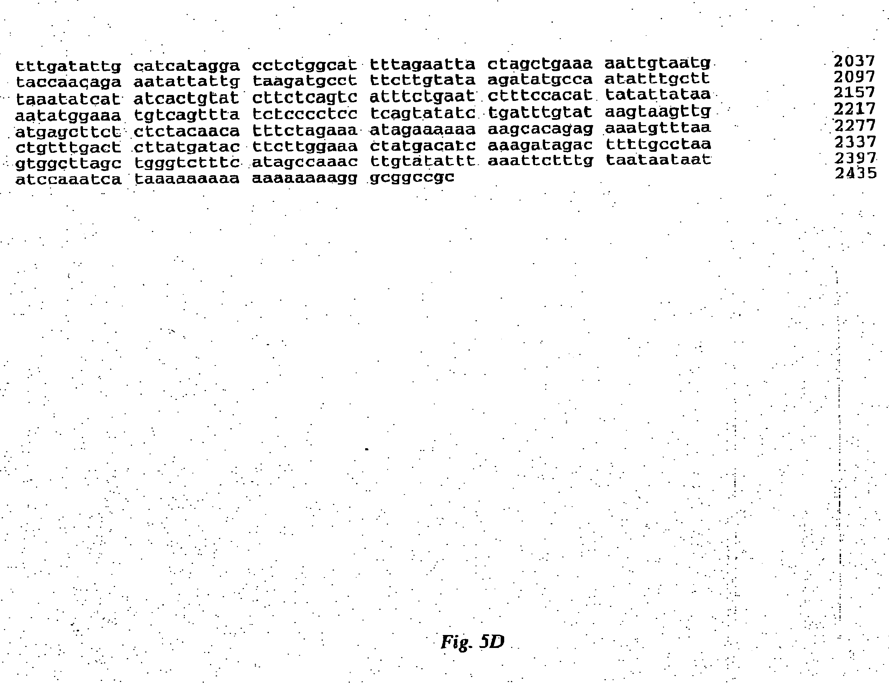

*Figure 5* depicts the cDNA sequence of human TANGO 212 (SEQ ID NO:11) and predicted amino acid sequence of TANGO 212 (SEQ ID NO:12). The open reading frame of SEQ ID NO:11 extends from nucleotide 269 to 1927 of SEQ ID NO:11 (SEQ ID NO:13).

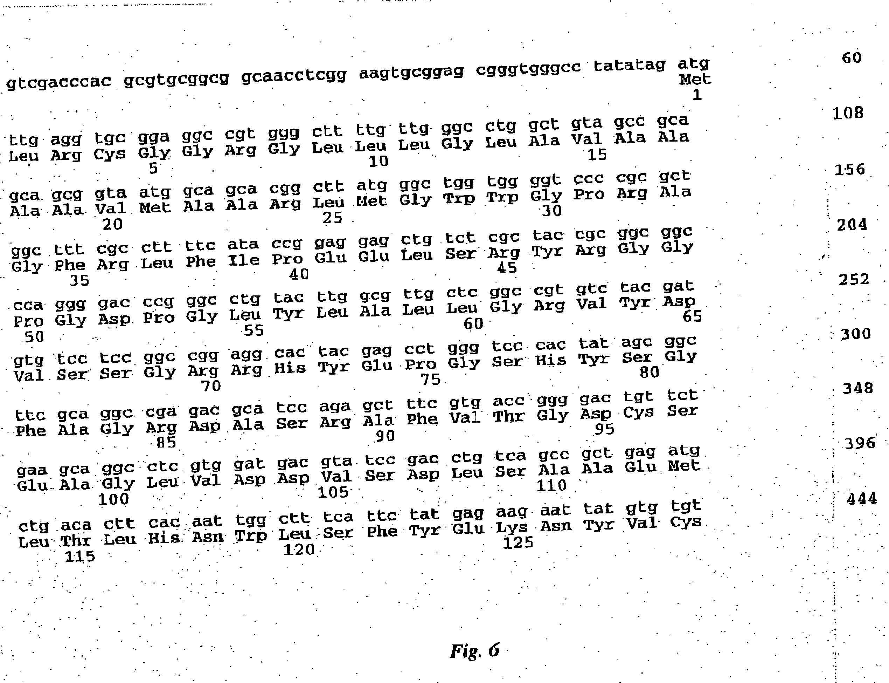

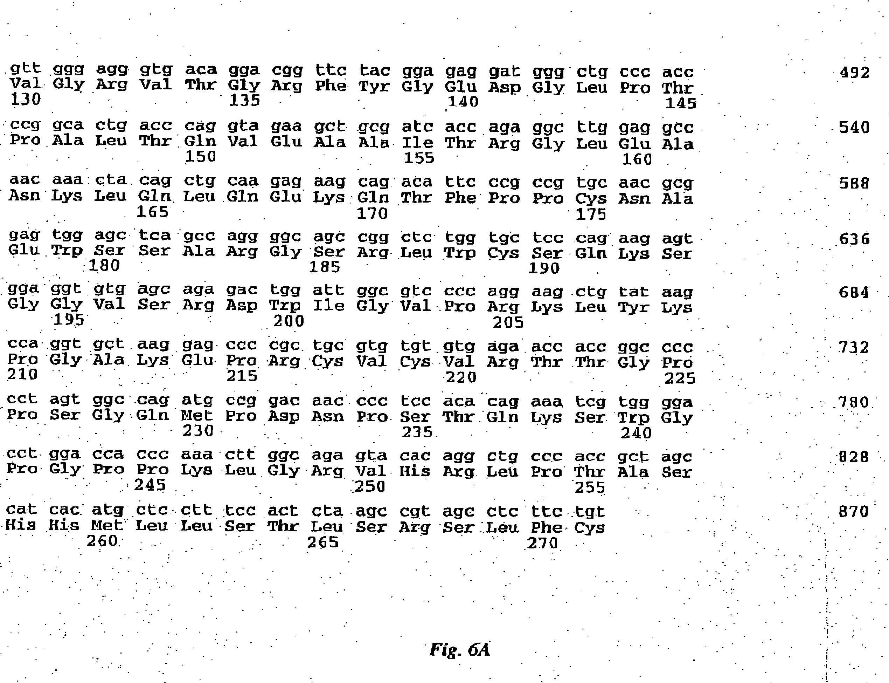

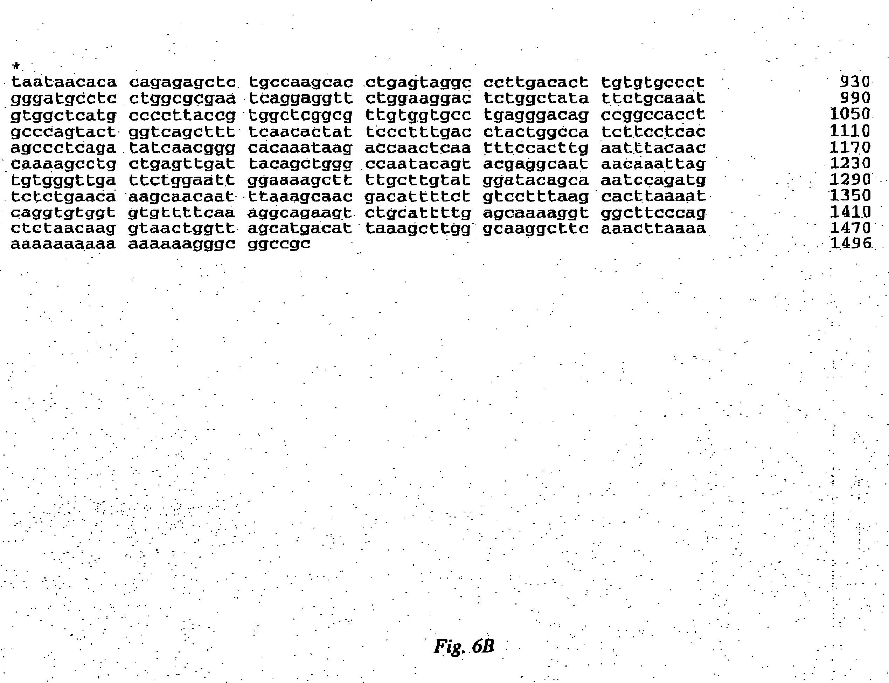

*Figure 6* depicts the cDNA sequence of human TANGO 213 (SEQ ID NO:14) and predicted amino acid sequence of TANGO 213 (SEQ ID NO:15). The open reading frame of SEQ ID NO:14 extends from nucleotide 58 to 870 of SEQ ID NO:14 (SEQ ID NO:16).

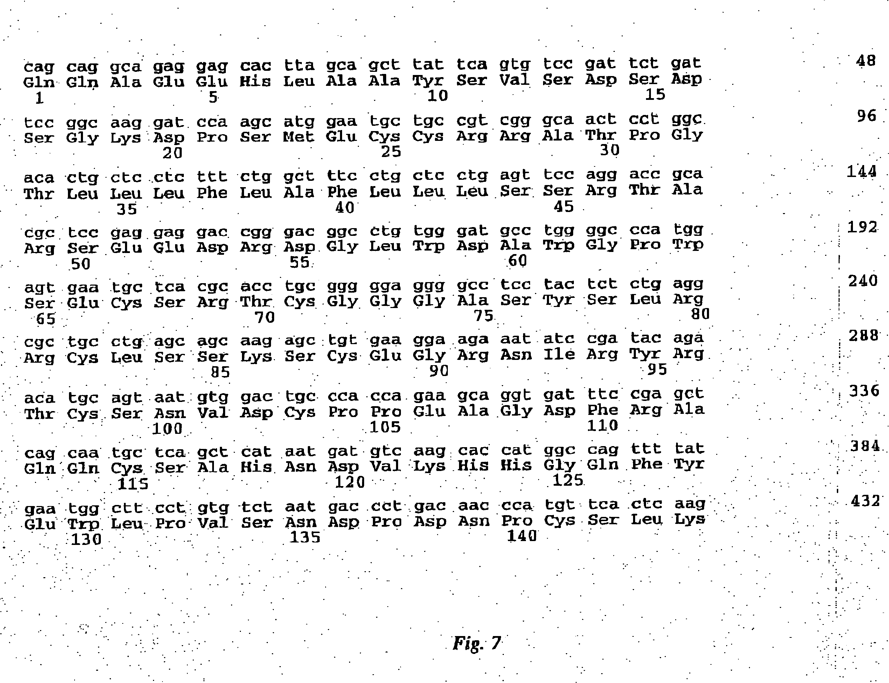

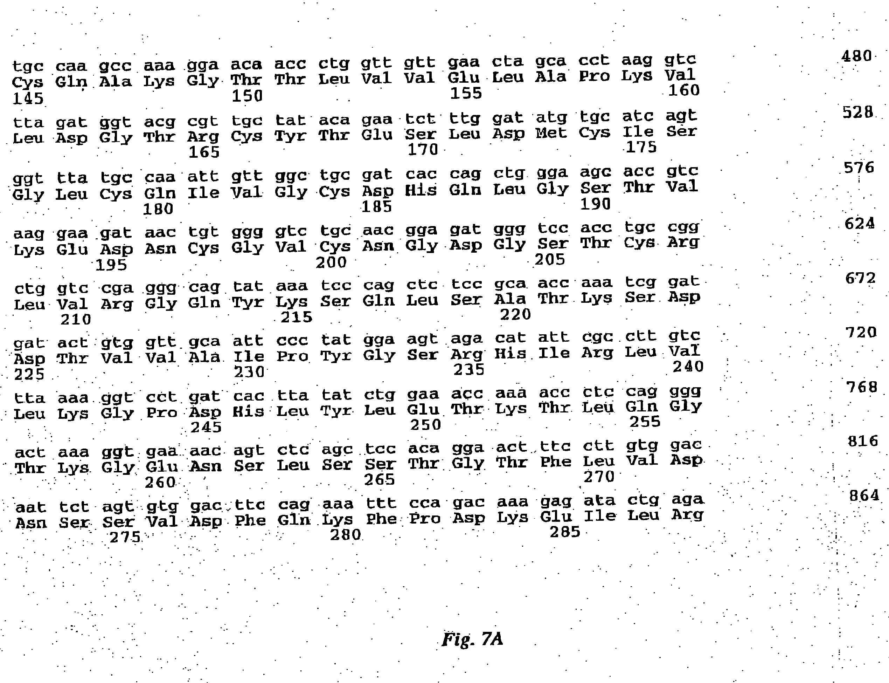

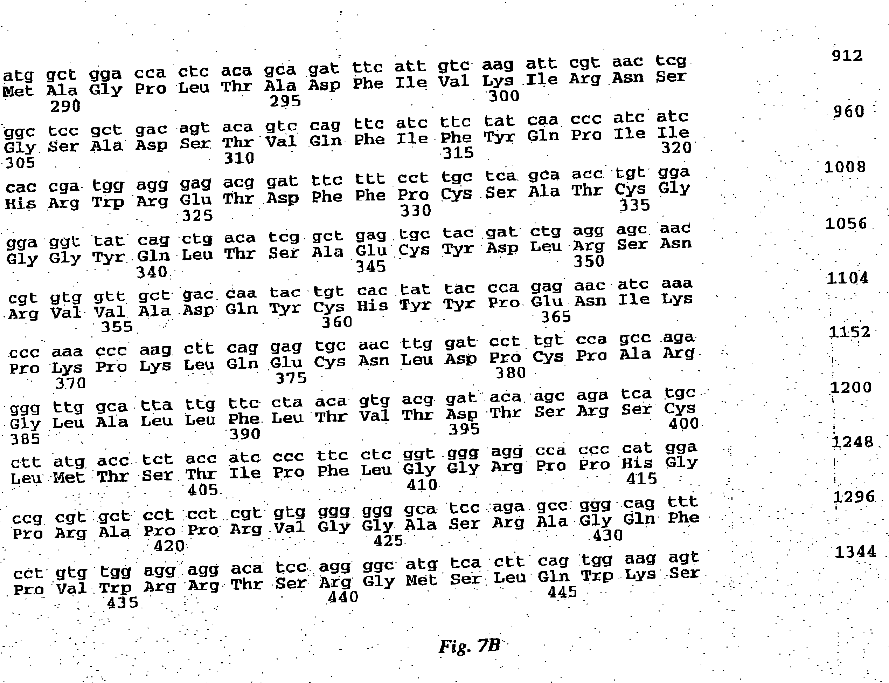

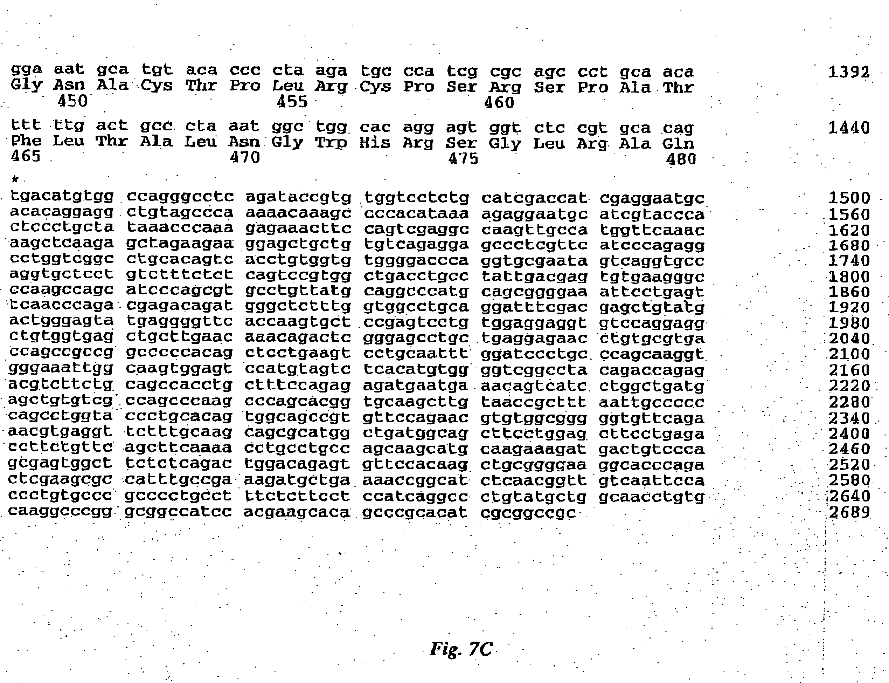

*Figure 7* depicts the cDNA sequence of human TANGO 224, form 1 (SEQ ID NO:17) and predicted amino acid sequence of TANGO 224, form 1 (SEQ ID NO:18). The open reading frame of SEQ ID NO:17 extends from nucleotide 1 to 1440 of SEQ ID NO:17 (SEQ ID NO:19).

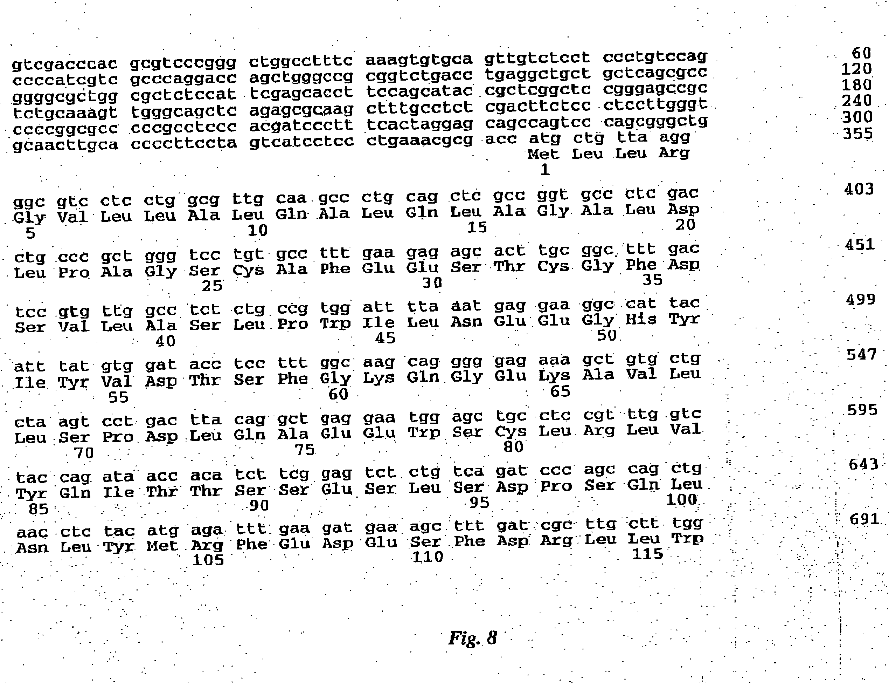

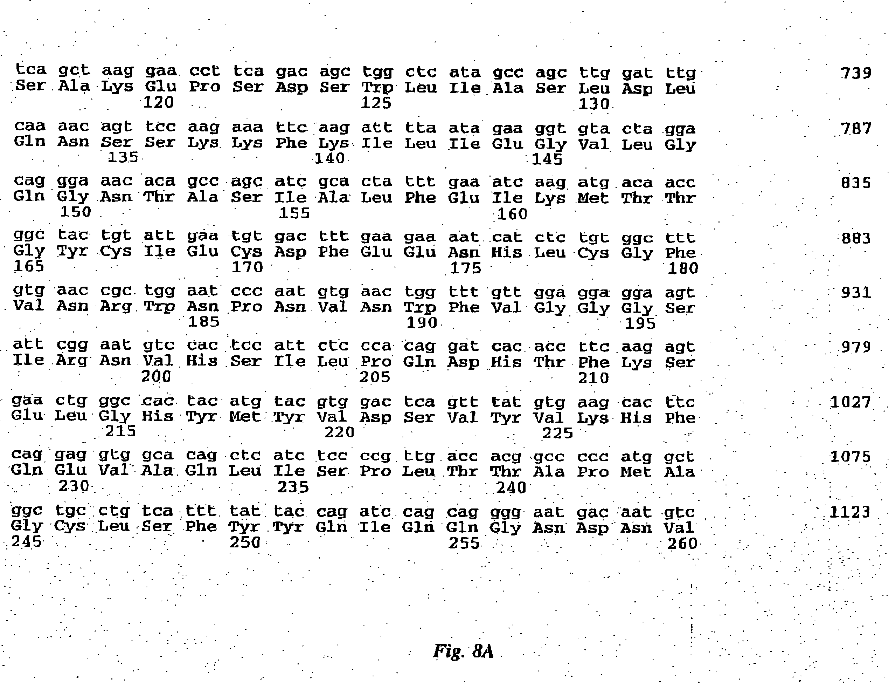

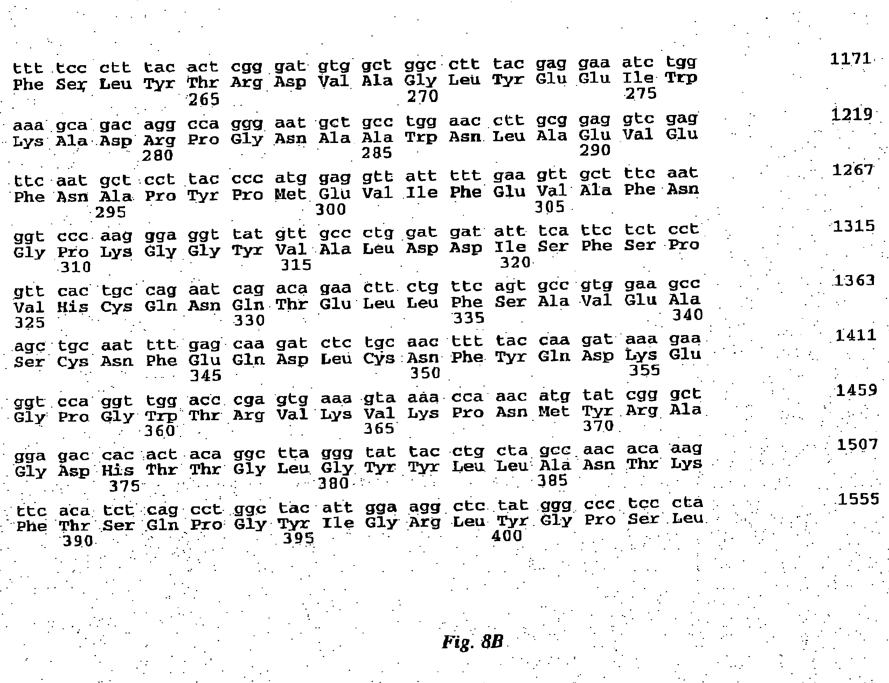

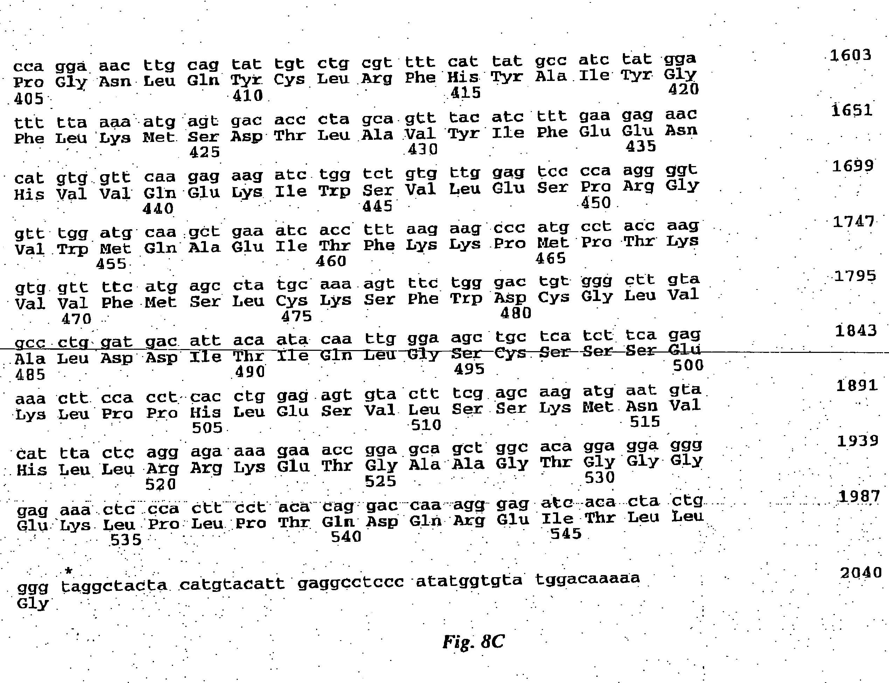

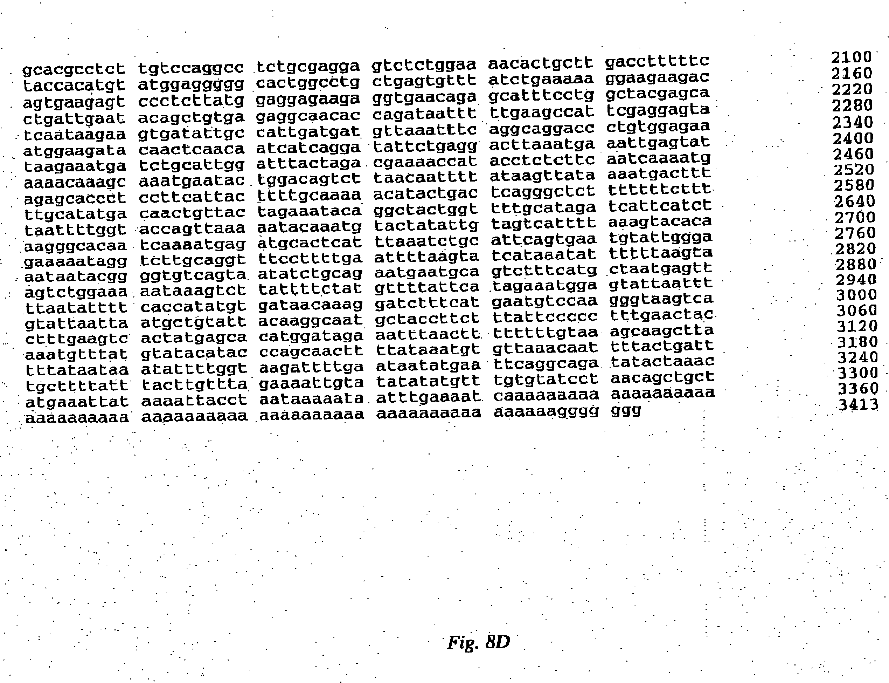

*Figure* 8 depicts the cDNA sequence of human TANGO 239, form 1 (SEQ ID NO:20) and predicted amino acid sequence of TANGO 239, form 1(SEQ ID NO:21). The open reading frame of SEQ ID NO:20 extends from nucleotide 344 to 1990 of SEQ ID NO:20 (SEQ ID NO:22).

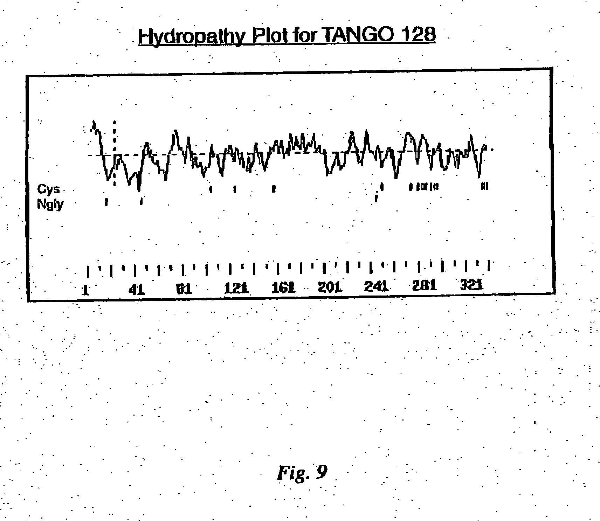

*Figure 9* depicts a hydropathy plot of a human TANGO-128. Relatively hydrophobic residues are above the dashed horizontal line, and relatively hydrophilic residues are below the dashed horizontal line. The cysteine residues (cys) and potential N-glycosylation sites (Ngly) are indicated by short vertical lines just below the hydropathy trace.

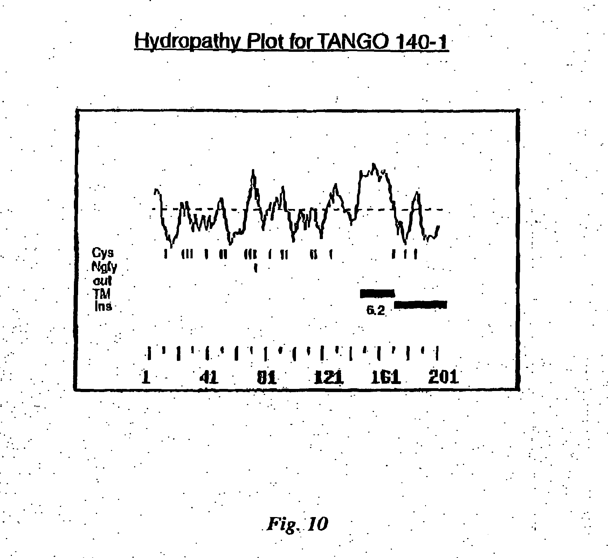

*Figure 10* depicts a hydropathy plot of a human TANGO 140-1. Relatively hydrophobic residues are above the dashed horizontal line, and relatively hydrophilic residues are below the dashed horizontal line. The cysteine residues (cys) and potential N-glycosylation sites (Ngly) are indicated by short vertical lines just below the hydropathy trace.

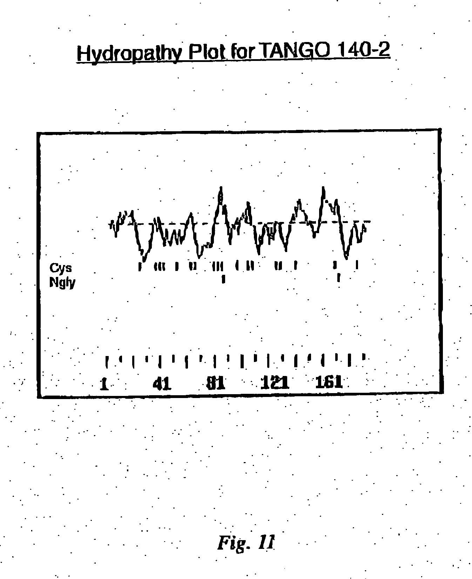

*Figure 11* depicts a hydropathy plot of a human TANGO 140-2. Relatively hydrophobic residues are above the dashed horizontal line, and relatively hydrophilic residues are below the dashed horizontal line. The cysteine residues (cys) and potential N-glycosylation sites (Ngly) are indicated by short vertical lines just below the hydropathy trace.

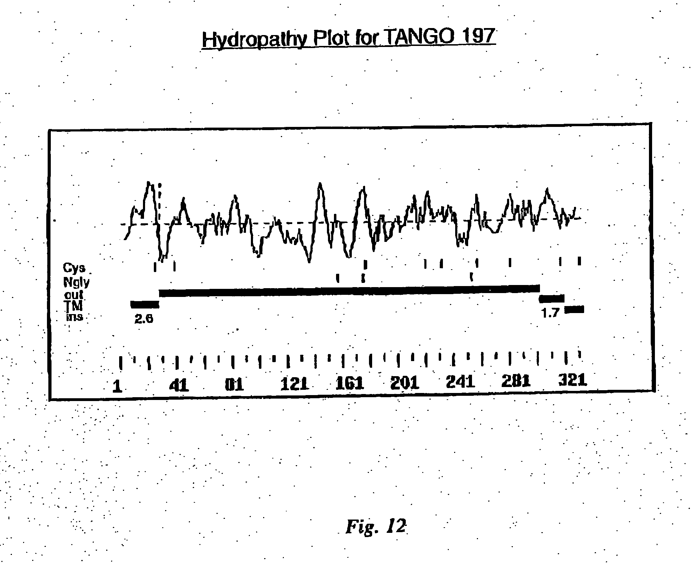

*Figure 12* depicts a hydropathy plot of a human TANGO 197. Relatively hydrophobic residues are above the dashed horizontal line, and relatively hydrophilic residues are below the dashed horizontal line. The cysteine residues (cys) and potential N-glycosylation sites (Ngly) are indicated by short vertical lines just below the hydropathy trace.

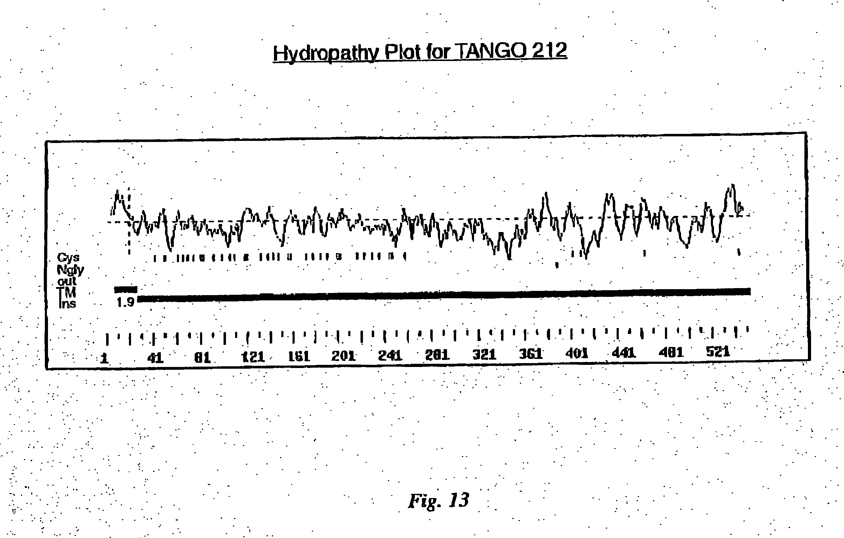

*Figure 13* depicts a hydropathy plot of a human TANGO 212. Relatively hydrophobic residues are above the dashed horizontal line, and relatively hydrophilic residues are below the dashed horizontal line. The cysteine residues (cys) and potential N-glycosylation sites (Ngly) are indicated by short vertical lines just below the hydropathy trace.

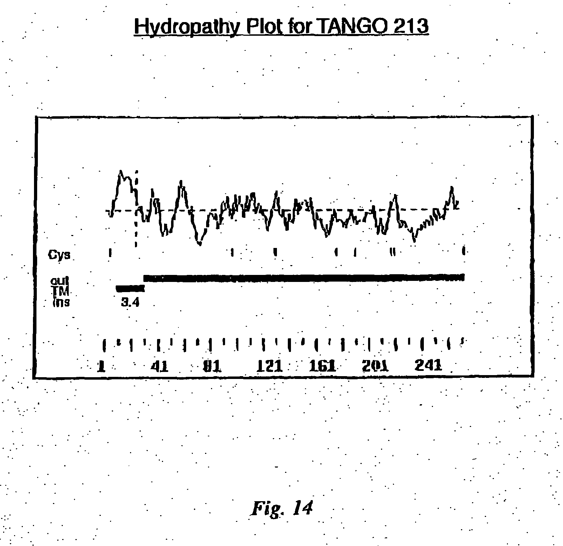

*Figure 14* depicts a hydropathy plot of a human TANGO 213. Relatively hydrophobic residues are above the dashed horizontal line, and relatively hydrophilic residues are below the dashed horizontal line. The cysteine residues (cys) and potential N-glycosylation sites (Ngly) are indicated by short vertical lines just below the hydropathy trace.

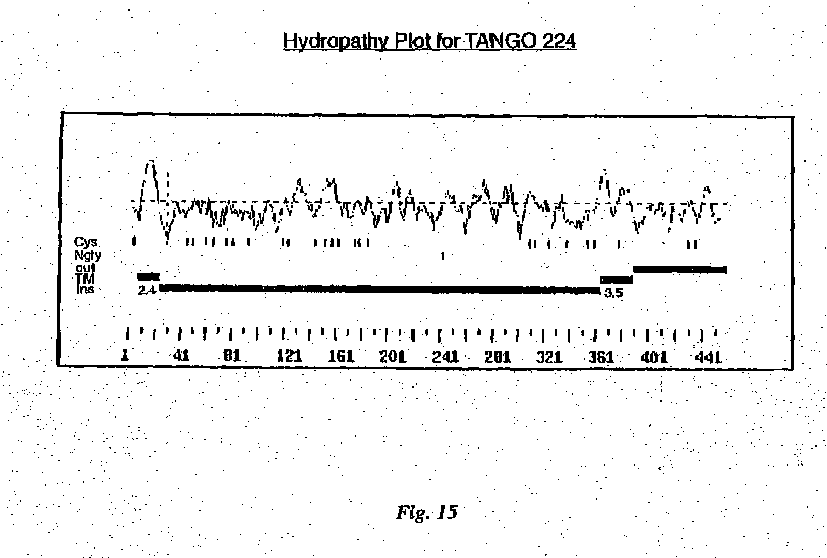

*Figure 15* depicts a hydropathy plot of a human TANGO 224. Relatively hydrophobic residues are above the dashed horizontal line, and relatively hydrophilic residues are below the dashed horizontal line. The cysteine residues (cys) and potential N-glycosylation sites (Ngly) are indicated by short vertical lines just below the hydropathy trace.

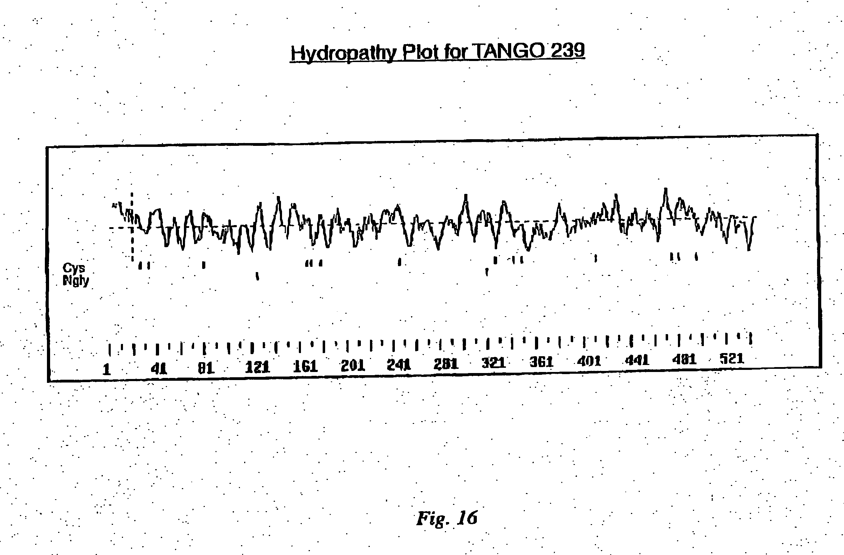

*Figure 16* depicts a hydropathy plot of a human TANGO 239. Relatively hydrophobic residues are above the dashed horizontal line, and relatively hydrophilic residues are below the dashed horizontal line. The cysteine residues (eys) and potential N-glycosylation sites (Ngly) arc indicated by short vertical lines just below the hydropathy trace.

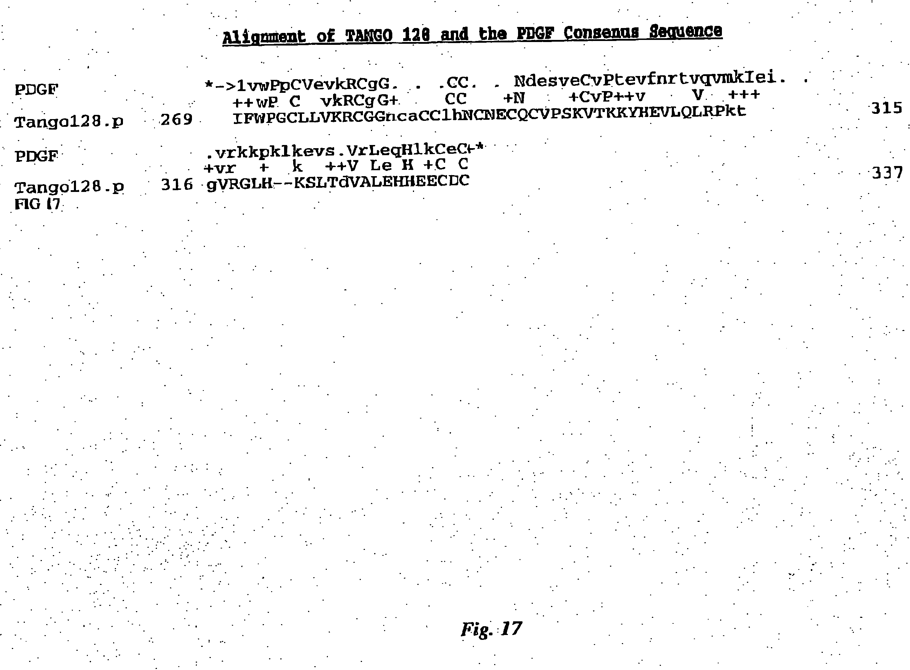

*Figure 17* depicts the alignment of amino acids 269 to 337 of TANGO 128 (amino acids 269 to 337 of SEQ ID NO:2)(SEQ ID NO: X) and the platelet derived growth factor (PDGF) consensus sequence (SEQ ID NO:40). In these alignments, an uppercase letter between the two sequences indicates an exact match, and a (+) indicates a conservative amino acid substitution.

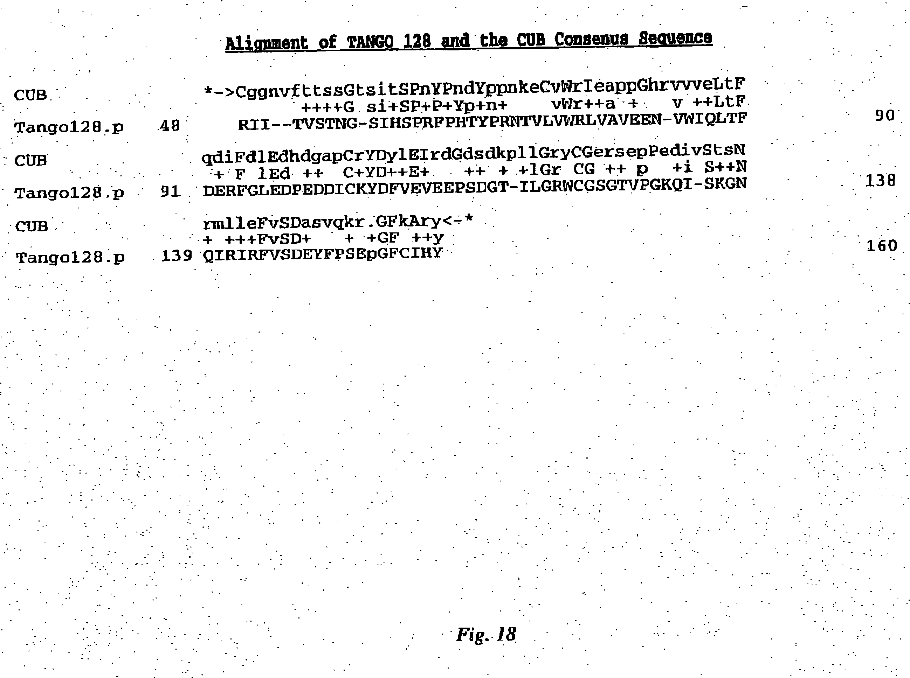

*Figure 18* depicts the alignment of amino acids 48 to 160 of TANGO 128 (amino acids 48 to 160 of SEQ ID NO:2)(SEQ ID NO: X) and the CUB consensus sequence (SEQ ID NO:41). In these alignments, an uppercase letter between the two sequences indicates an exact match, and a (+) indicates a conservative amino acid substitution.

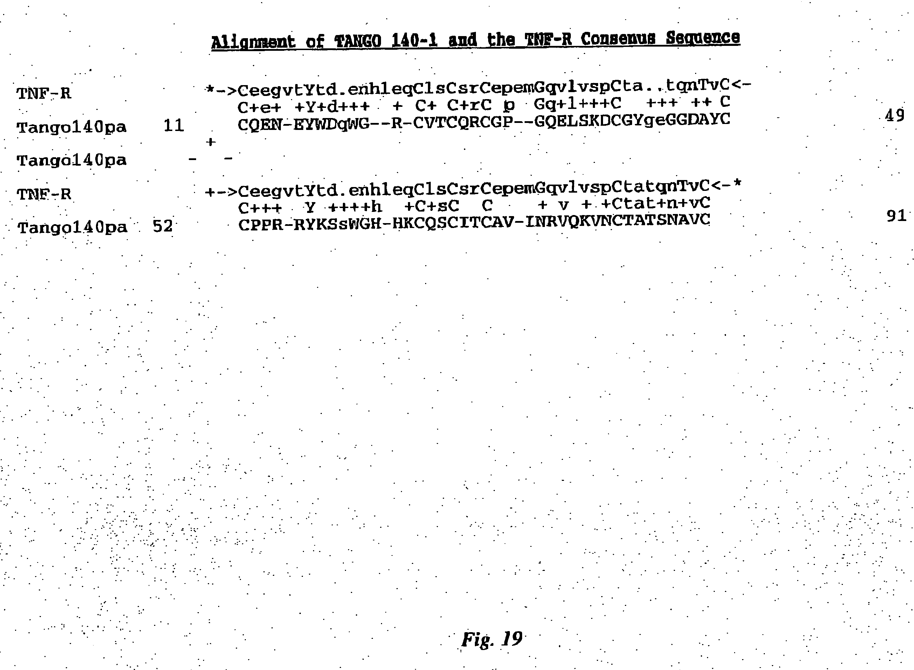

*Figure 19* depicts the alignment of amino acids 11 to 49 (SEQ ID NO: X) and amino acids 52 to 91 (SEQ ID NO: X) of TANGO 140-1 (SEQ ID NO:5) with the tumor necrosis factor receptor (TNF-R) consensus sequence (SEQ ID NO:42). In these alignments, an uppercase letter between the two sequences indicates an exact match, and a (+) indicates a conservative amino acid substitution.

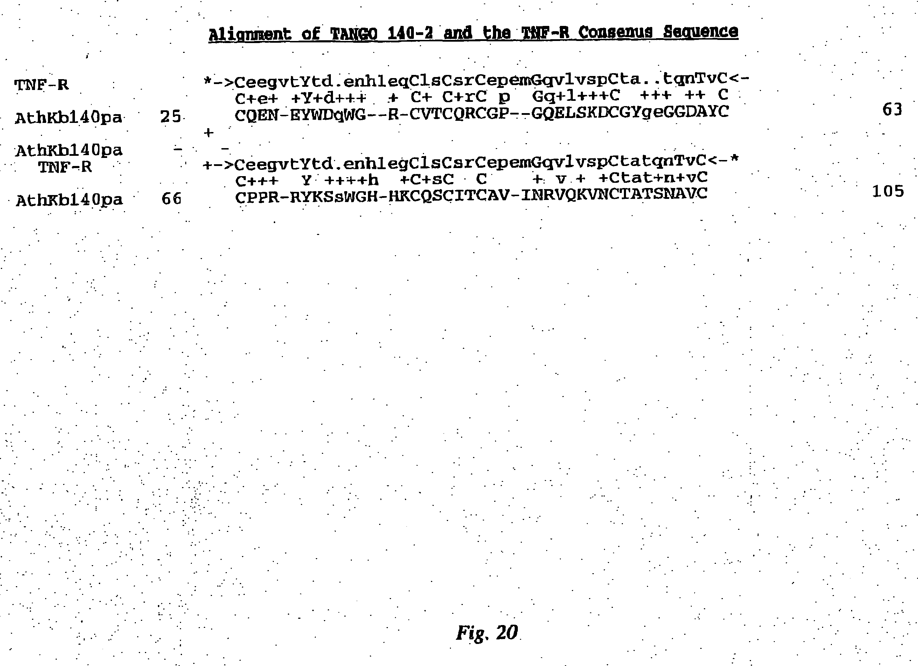

Figure *20* depicts the alignment of amino acids 25 to 63 (SEQ ID NO: X) and amino acids 66 to 105 (SEQ ID NO: X) of TANGO 140-2 (SEQ ID NO:7) with the turnor necrosis factor receptor (TNF-R) consensus sequence (SEQ ID NO:42). In these alignments, an uppercase letter between the two sequences indicates an exact match, and a (+) indicates a conservative amino acid substitution.

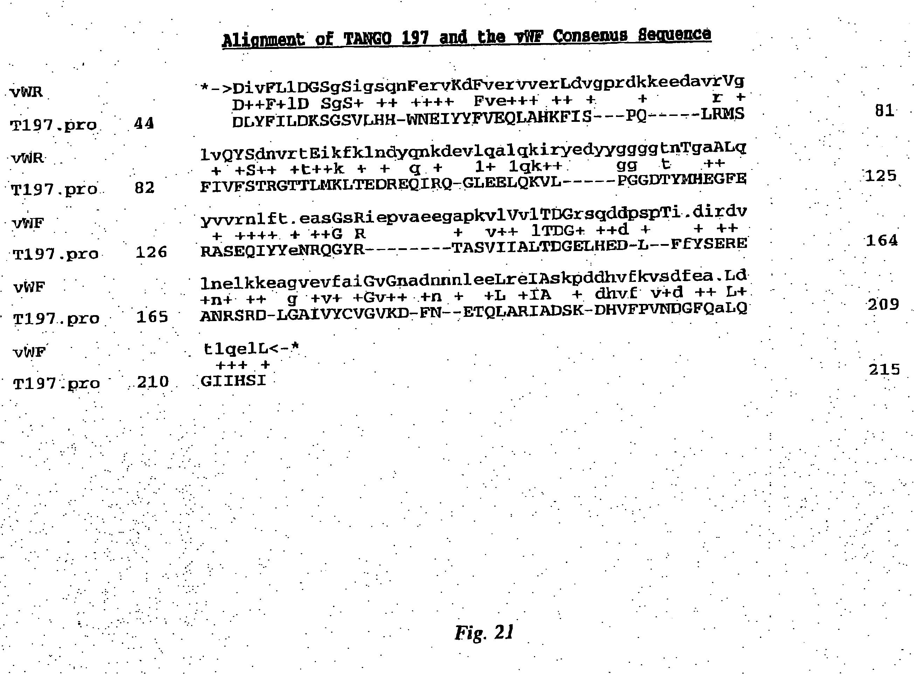

*Figure 21* depicts the alignment of amino acids 44 to 215 of TANGO 197 (amino acids 44 to 215 of SEQ ID NO:9)(SEQ ID NO: X) and the von Willebrand Factor (vWF) consensus sequence (SEQ ID NO:43). In these alignments, an uppercase letter between the two sequences indicates an exact match, and (+) indicates a conservative amino acid substitution.

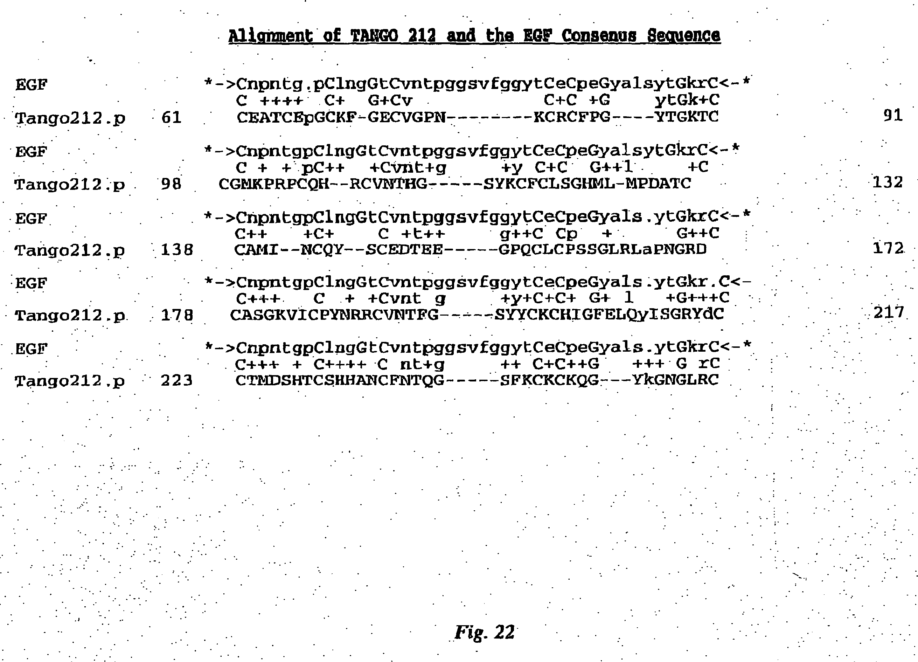

*Figure 22* depicts the alignment of amino acids 61 to 91 (SEQ ID NO: X), amino acids 98 to 132 (SEQ ID NO: X), amino acids 138 to 172 (SEQ ID NO: X), amino acids 178 to 217 (SEQ ID NO: X), and amino acids 223 to 258 (SEQ ID NO: X) of TANGO 212 (SEQ ID NO:12) and the epidermal growth factor (EGF) consensus sequence (SEQ ID NO:44). In these alignments, an uppercase letter between the two sequences indicates an exact match, and a (+) indicates a conservative amino acid substitution.

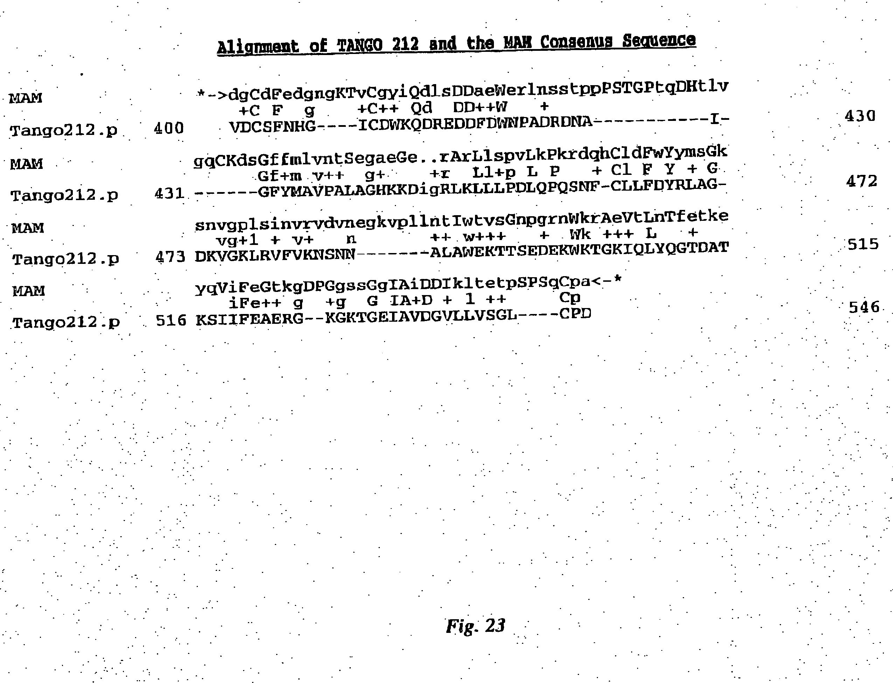

*Figure 23* depicts the alignment of amino acids 400 to 546 of TANGO 212 (amino acids 400 to 546 of SEQ ID NO:12)(SEQ ID NO: X) and the MAM consensus sequence (SEQ ID NO:45). In these alignments, an uppercase letter between the two sequences indicates an exact match, and a (+) indicates a conservative amino acid substitution.

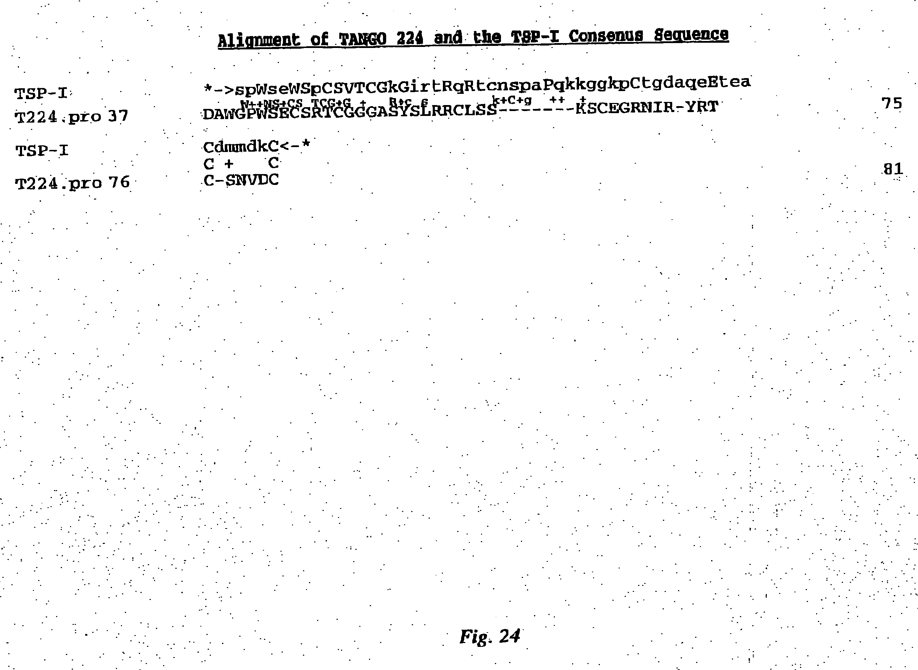

*Figure 24* depicts the alignment of amino acids 37 to 81 of TANGO 224, form 1 (amino acids 37 to 81 of SEQ ID NO:18)(SEQ ID NO: X) and the thrombospondin type-I (TSP-I) consensus sequence (SEQ ID NO:46). In these alignments, an uppercase letter between the two sequences indicates an exact match, and a (+) indicates a conservative amino acid substitution.

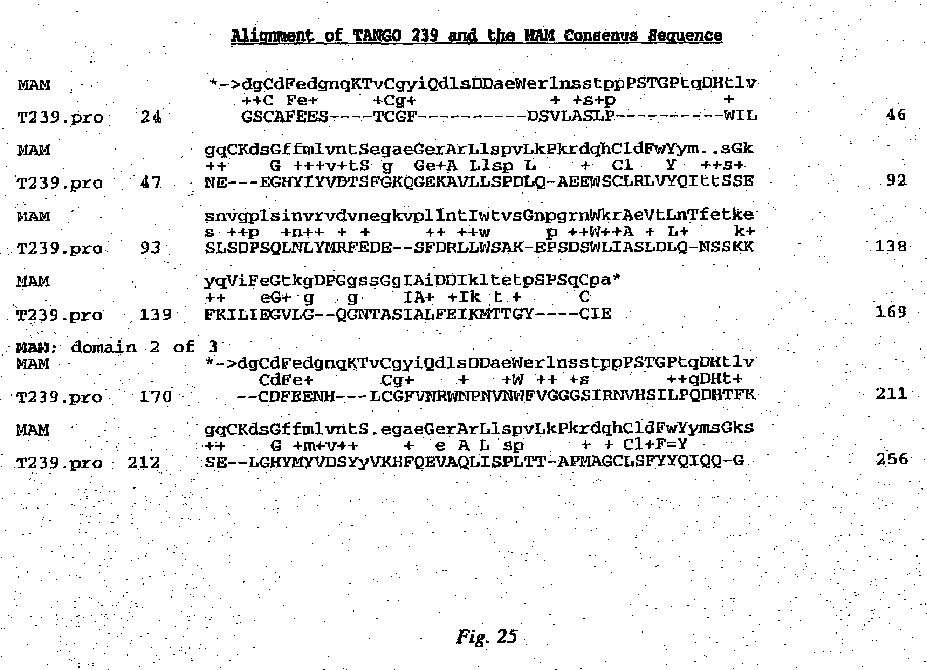

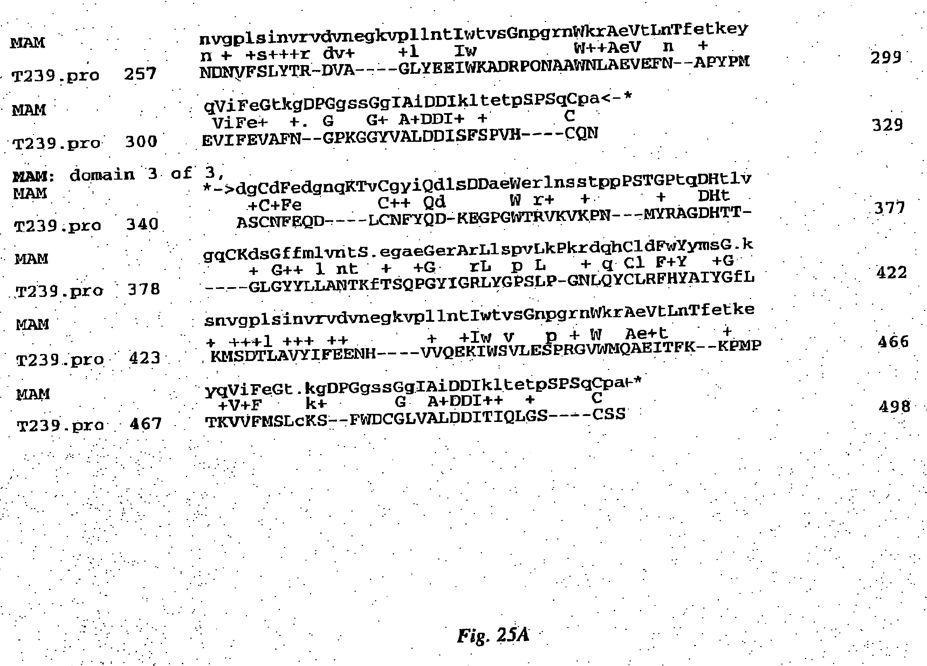

*Figure 25* depicts the alignment of amino acids 24 to 169 (SEQ ID NO: X), amino acids 170 to 329 (SEQ ID NO: X) and amino acids 340 to 498 (SEQ ID NO: X) of TANGO 239 (SEQ ID NO:21) and the MAM consensus sequence (SEQ ID NO:45). In these alignments, an uppercase letter between the two sequences indicates an exact match, and a (+) indicates a conservative amino acid substitution.

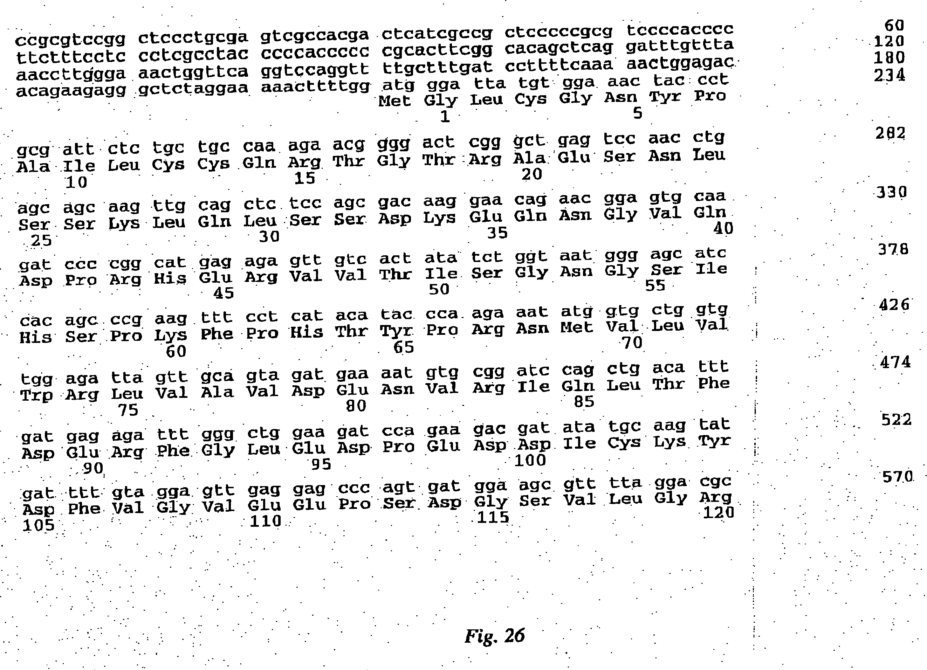

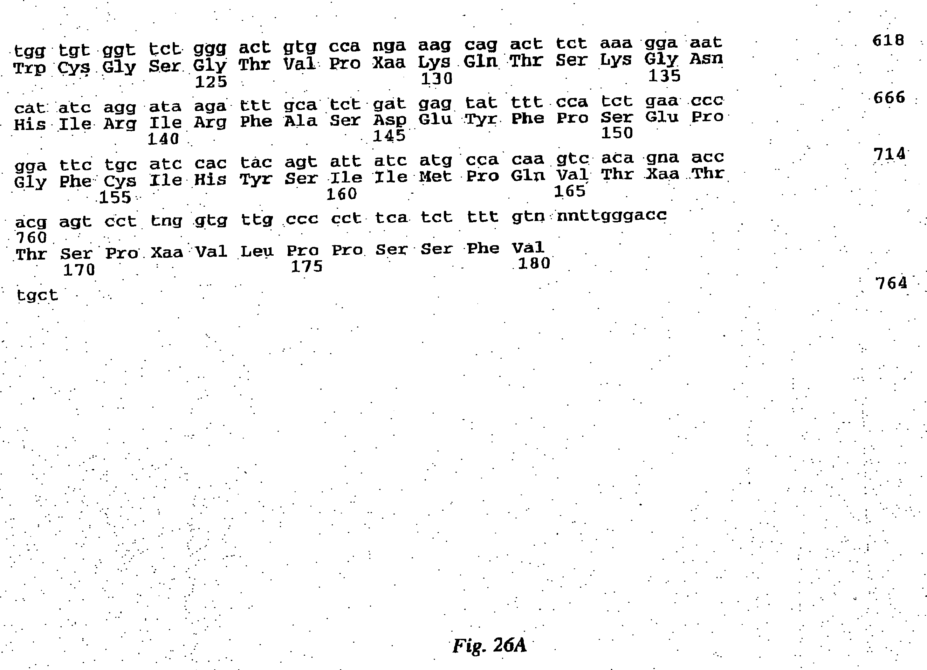

*Figure 26* depicts the cDNA sequence of mouse TANGO 128 (SEQ ID NO:53) and predicted amino acid sequence of mouse TANGO 128 (SEQ ID NO:54). The open reading frame of SEQ ID NO:53 comprises from nucleotides 211 to 750 of SEQ ID NO:53 (SEQ ID NO:55).

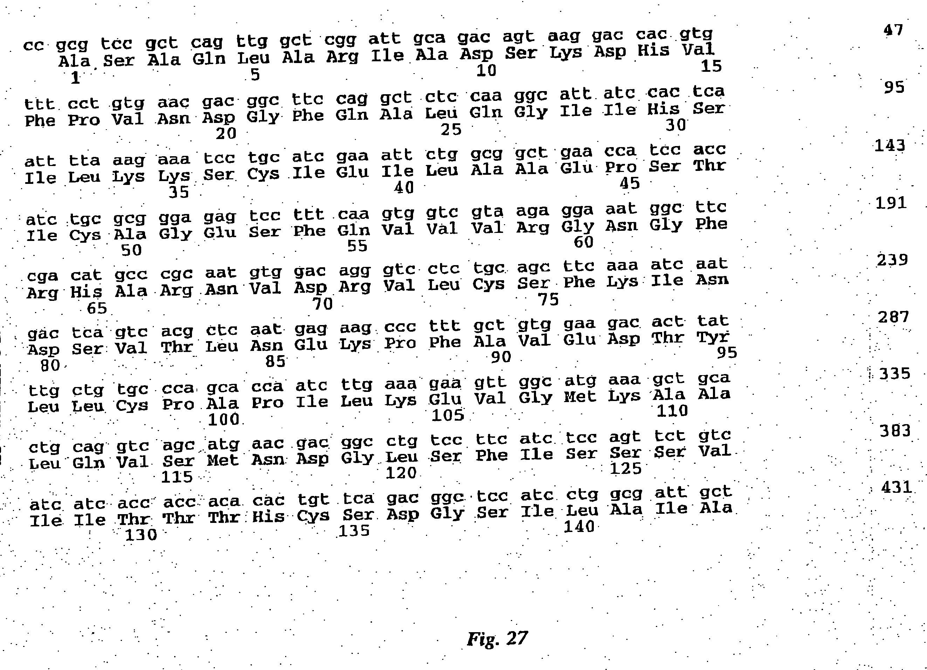

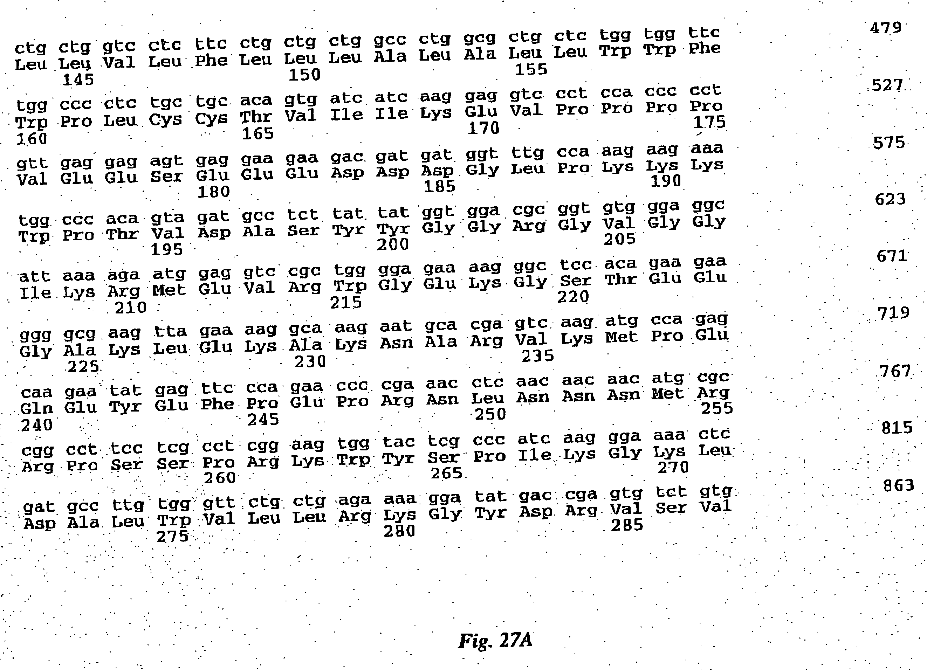

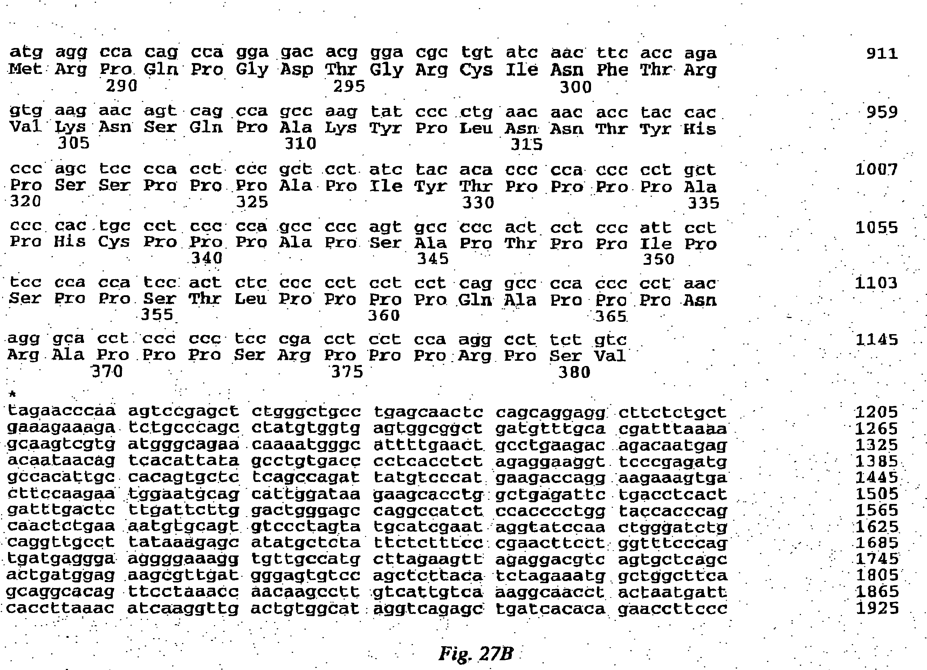

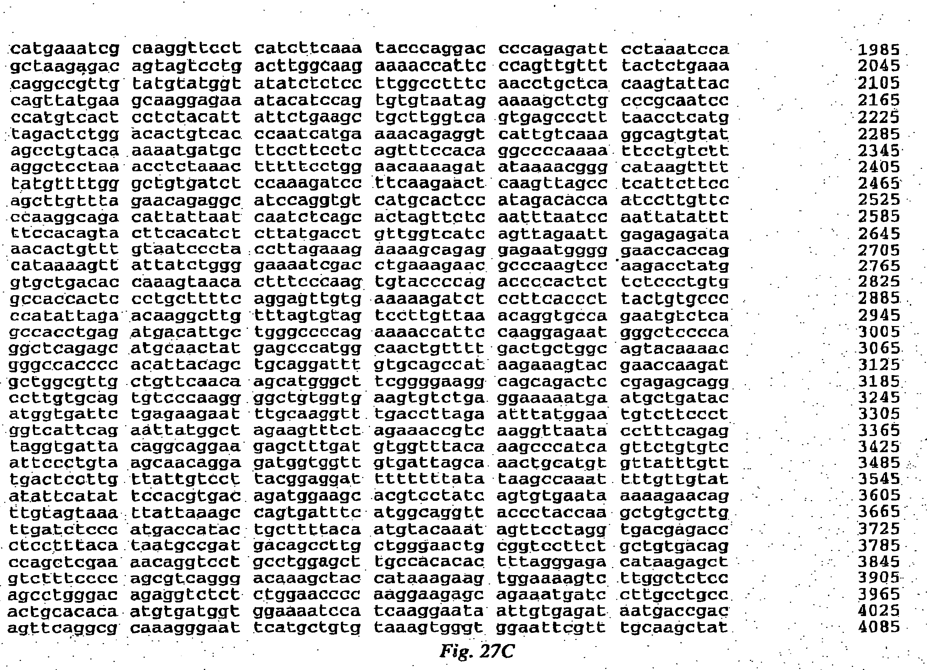

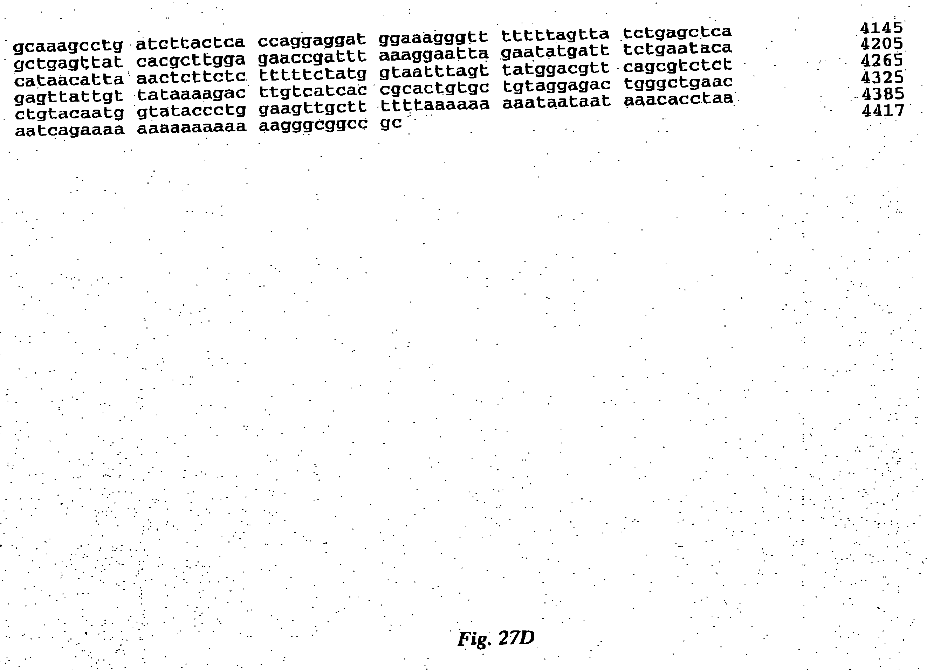

*Figure 27* depicts the cDNA sequence of mouse TANGO 197 (SEQ ID NO:56) and predicted amino acid sequence of mouse TANGO 197 (SEQ ID NO:57). The open reading frame of SEQ ID NO:56 extends from nucleotide 3 to 1145 of SEQ ID NO:56 (SEQ ID NO:58).

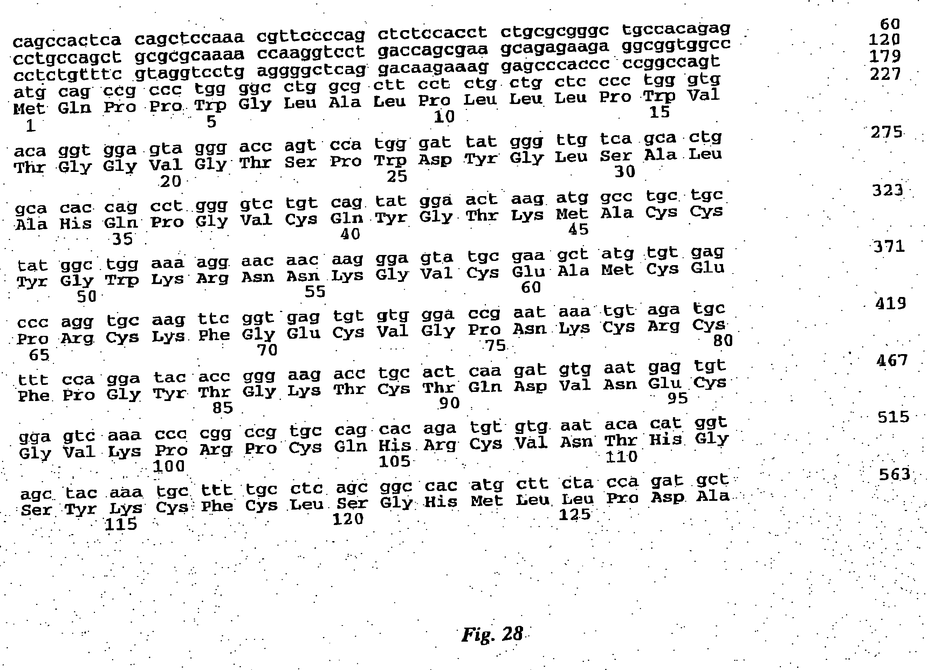

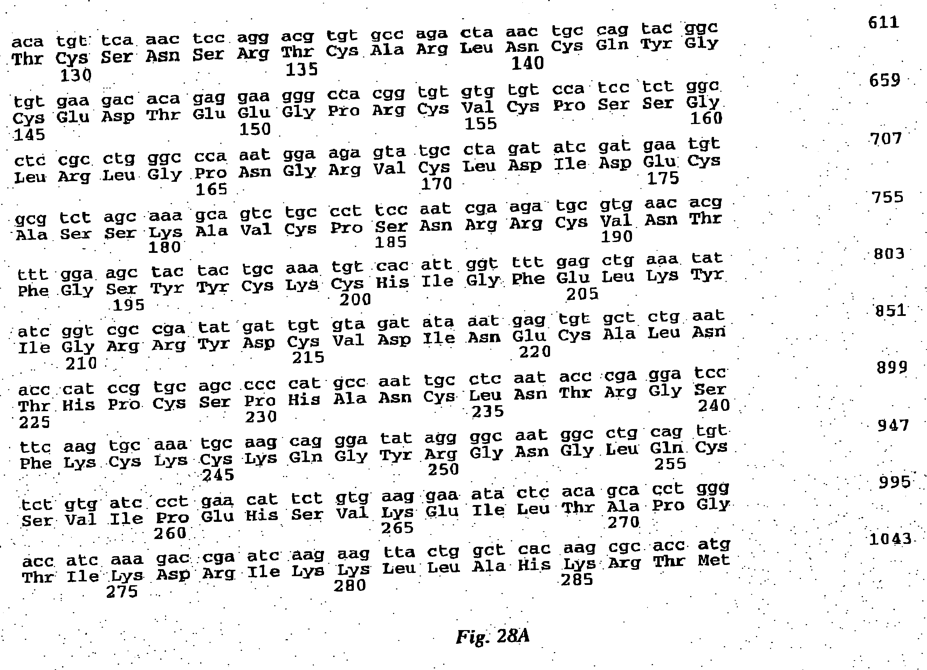

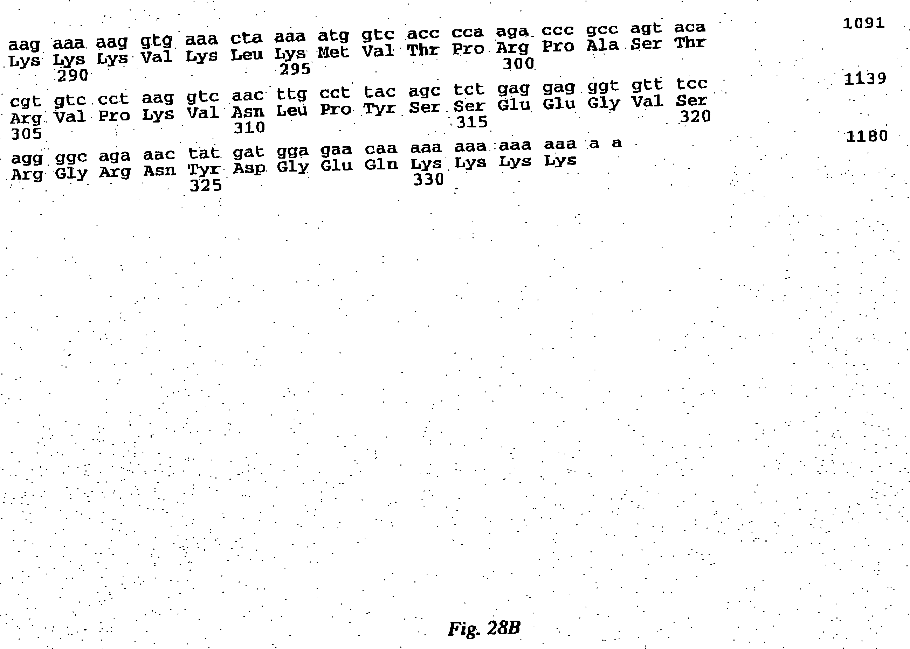

*Figure 28* depicts the cDNA sequence of mouse TANGO 212 (SEQ ID NO:59) and predicted amino acid sequence of mouse TANGO 212 (SEQ ID NO:60). The open reading frame of SEQ ID NO:60 extends from nucleotide 180 to 1179 of SEQ ID NO:60 (SEQ ID NO:61).

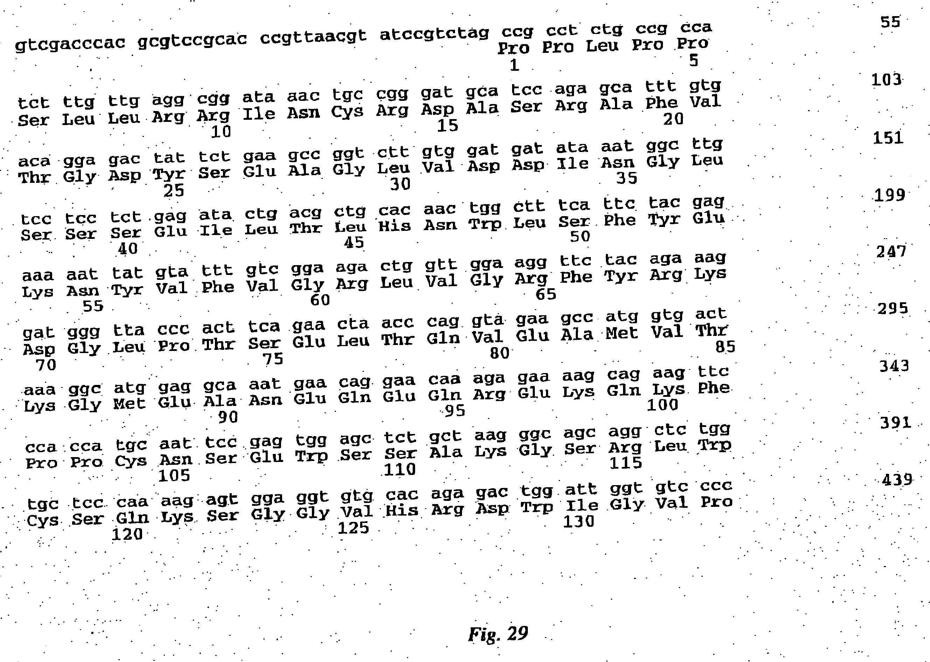

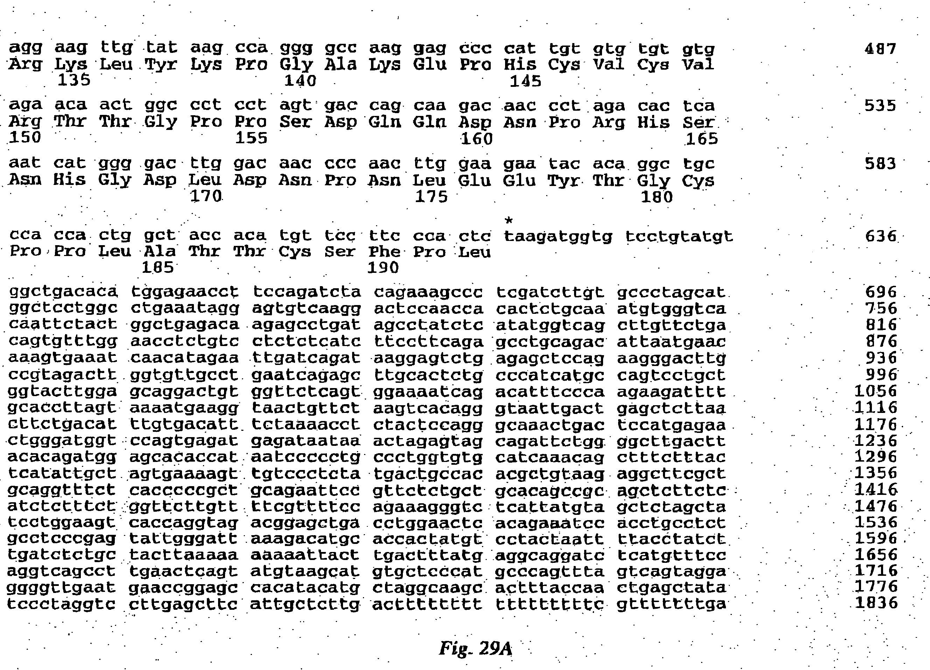

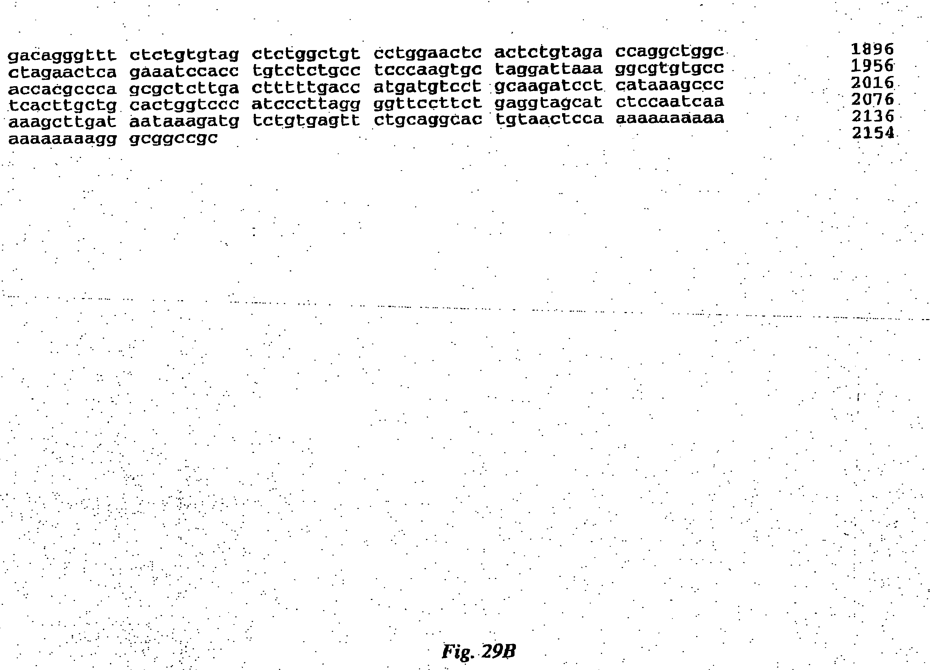

*Figure 29* depicts the cDNA sequence of mouse TANGO 213 (SEQ ID NO:62) and predicted amino acid sequence of mouse TANGO 213 (SEQ ID NO:63). The open reading frame of SEQ ID NO:62 extends from nucleotide 41 to 616, of SEQ ID NO:62 (SEQ ID NO:64).

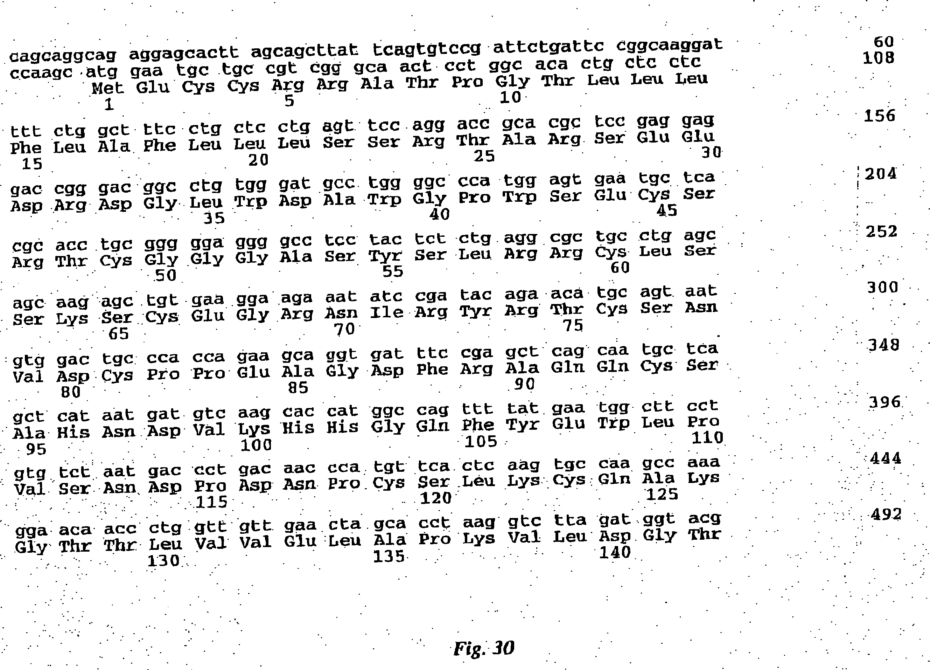

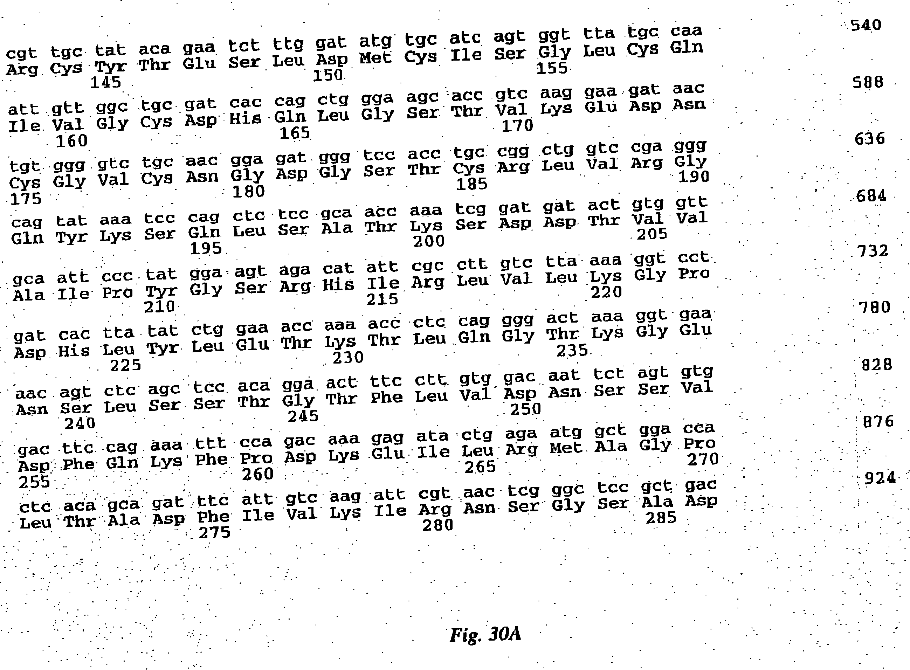

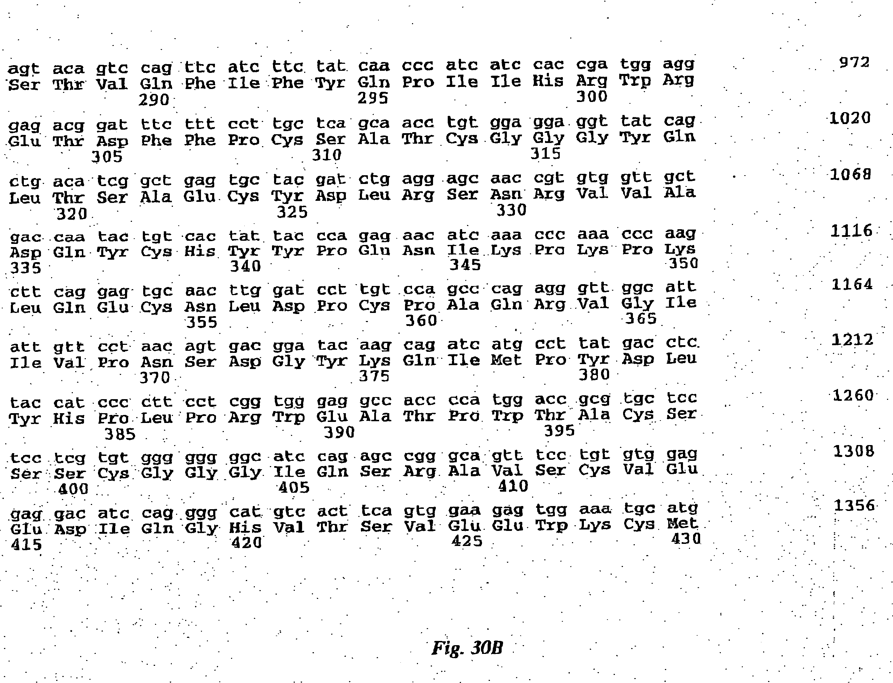

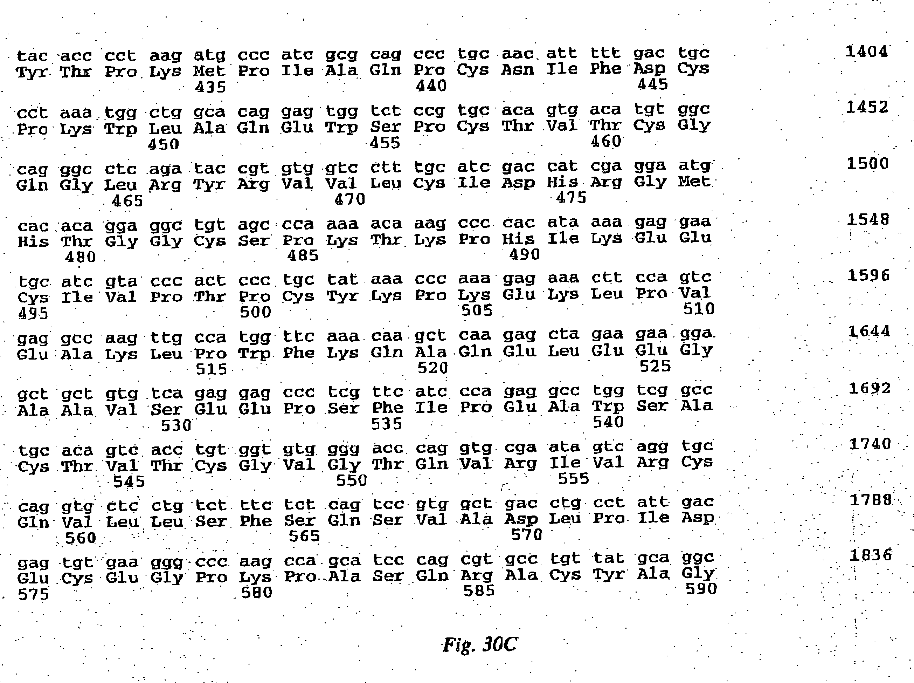

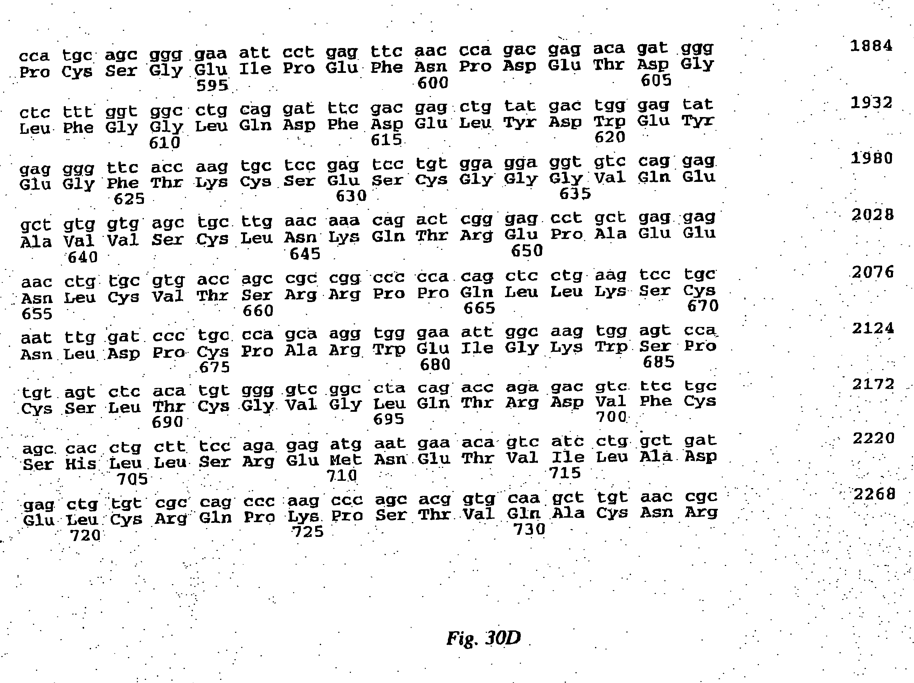

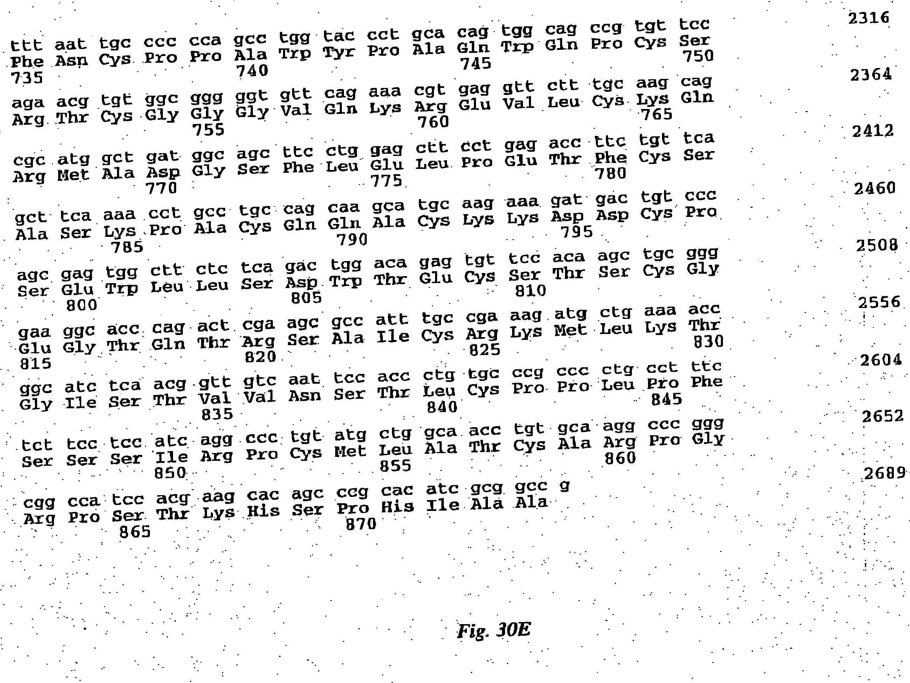

*Figure 30* depicts the cDNA sequence of human TANGO 224, form 2 (clone Athsa25a8) (SEQ ID NO:65) and predicted amino acid sequence of human TANGO 224, form 2 (clone Atbsa25a8)(SEQ ID NO;66). The open reading frame of SEQ ID NO:65 extends from nucleotide 67 to 2690 of SEQ ID NO:65 (SEQ ID NO:67).

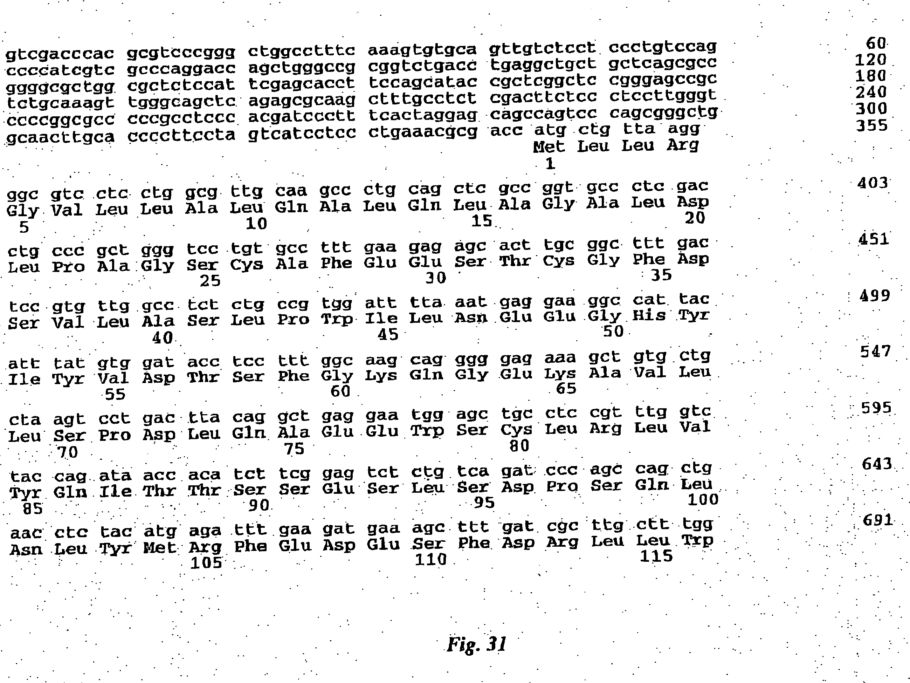

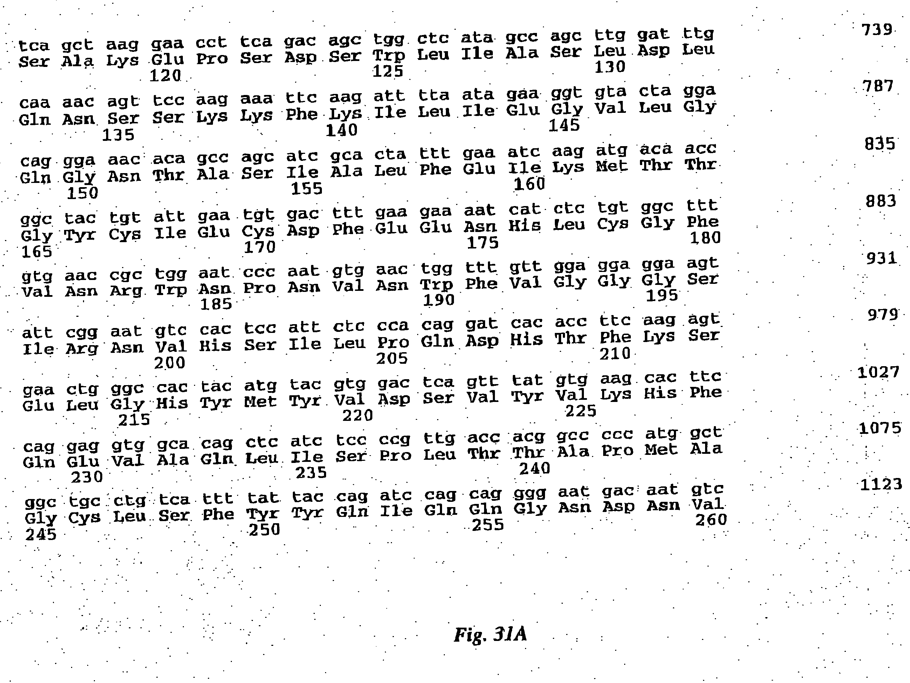

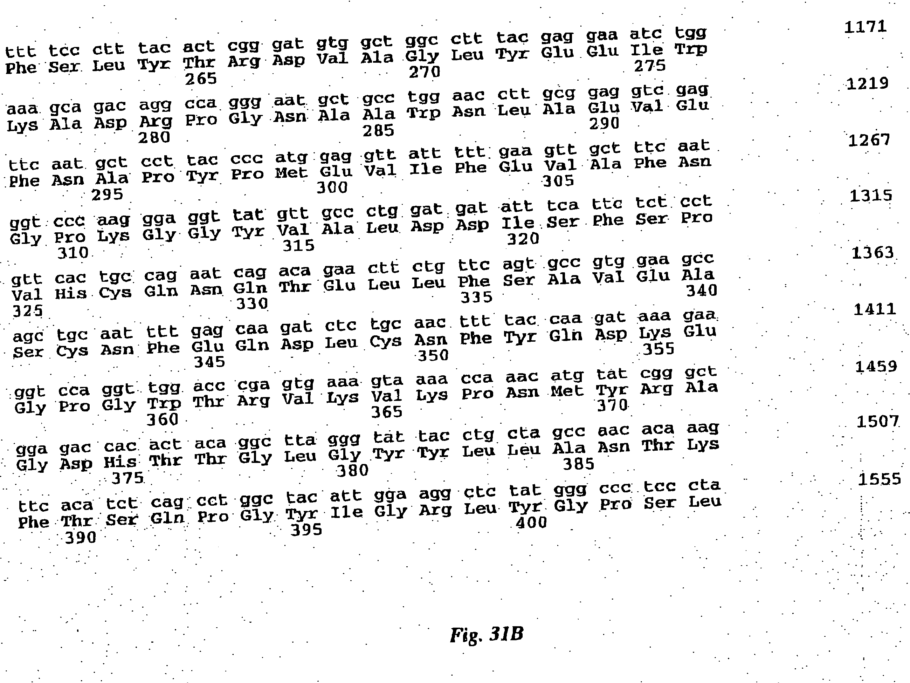

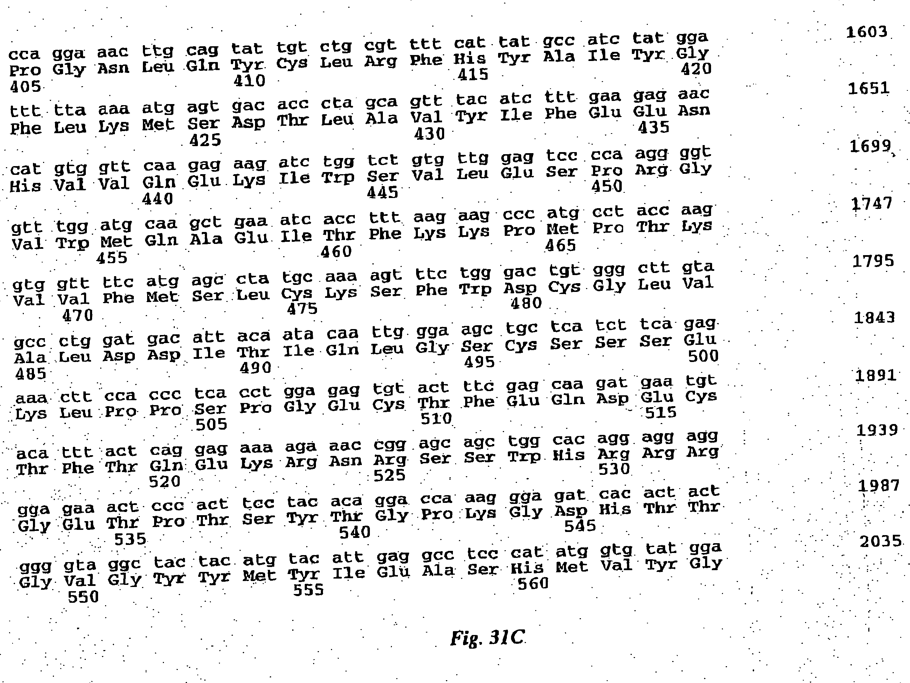

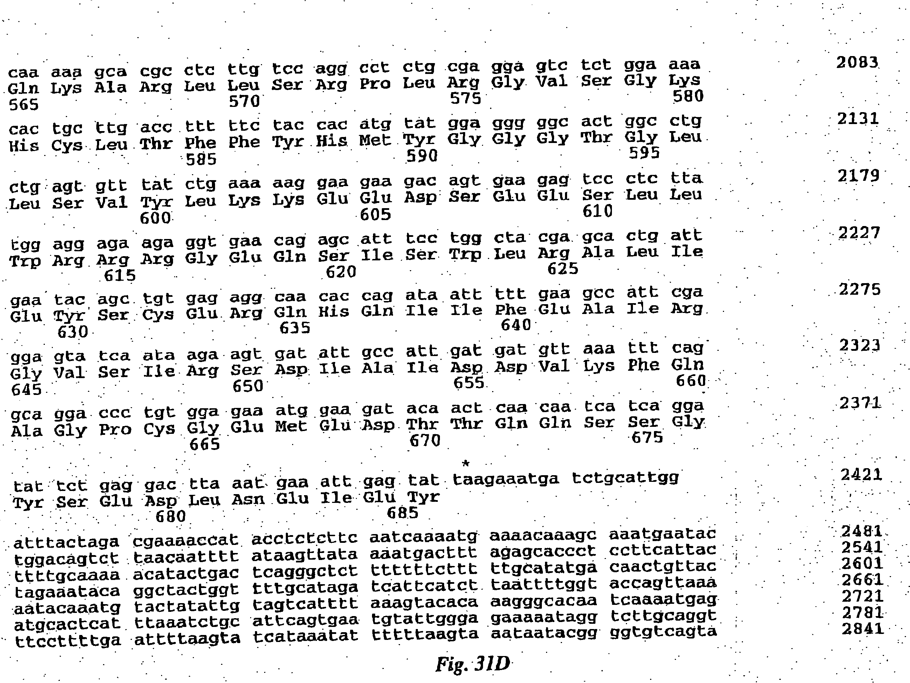

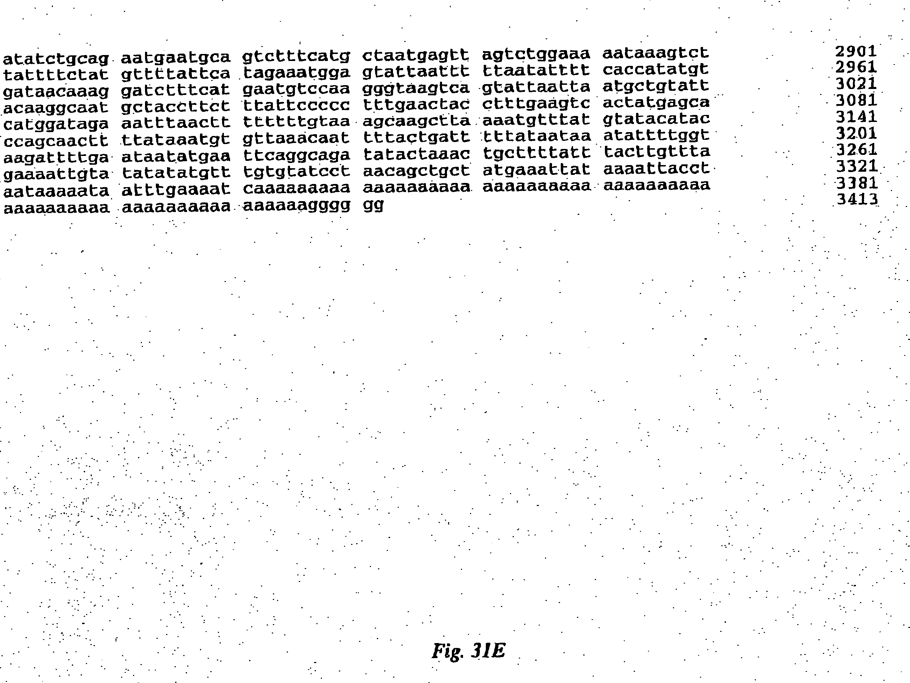

*Figure 31* depicts the cDNA sequence of human TANGO 239, form 2 (clone Athxe3b8)(SEQ ID NO:68) and predicted amino acid sequence of human TANGO 239, form 2 (clone Athxe3bS)(SEQ ID NO:69). The open reading frame of SEQ ID NO:68 extends from nucleotide 344 to 2401 of SEQ ID NO:68 (SEQ ID NO:70).

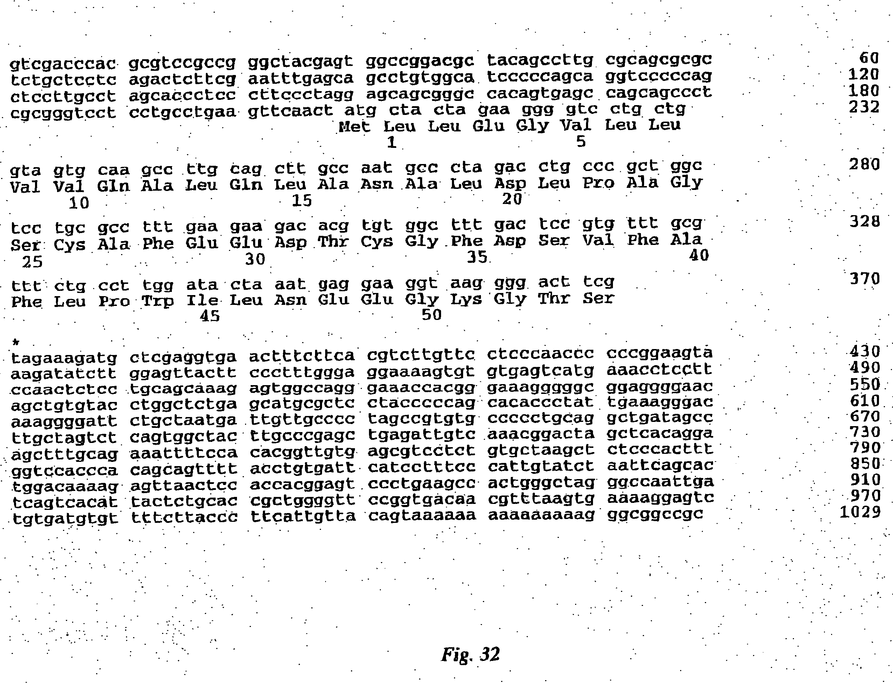

*Figure 32* depicts the cDNA sequence of mouse TANGO 239 (SEQ ID NO:71) and predicted amino acid sequence of mouse TANGO 239 (SEQ ID NO:72). The open reading frame of SEQ ID NO:71 extends from nucleotide 209 to 370 of SEQ ID NO:71 (SEQ ID NO:73).

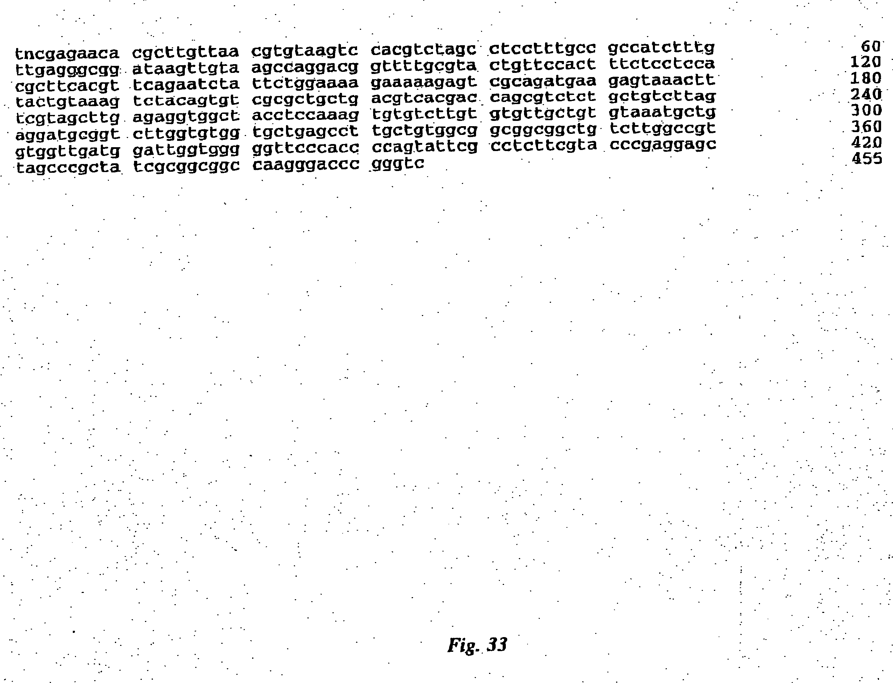

*Figure 33* depicts the cDNA sequence of rat TANGO 213 (SEQ ID NO:).

### Description of the Preferred Embodiments

The present invention is based on the discovery of cDNA molecules encoding TANGO 128, TANGO 140, TANGO 197, TANGO 212, TANGO 213, TANGO 224, and TANGO 239, all of which are either wholly secreted or transmembrane proteins.

### TANGO 128

In one aspect, the present invention is based on the discovery of cDNA molecules which encode a novel family of proteins having sequence identity to vascular endothelial growth factor (VFGF), referred to herein as TANGO 128 proteins.

The TANGO 128 proteins and nucleic acid molecules comprise a family of molecules having certain conserved structural and functional features. As used harem, the term "family" is intended to mean two or more proteins or nucleic acid molecules having a common structural domain and having sufficient amino acid or nucleotide sequence identity as defined herein. Family members can be from either the same or different species. For example, a family can comprises two or more proteins of human origin, or can comprise one or more proteins of human origin and one or more of non-human origin. Members of the same family may also have common structural domains.

For example, the VEGF family to which the TANGO 128 proteins of the invention bear sequence identity, are a family of mitogens which contain a platelet-derived growth factor (PDGF) domain having conserved cysteine residues. These cysteine residues form intra- and inter-chain disulfide bonds which can affect the structural integrity of the protein. Thus, included within the scope of the invention are TANGO 128 proteins having a platelet-derived growth factor (PDGF) domain. As used herein, a PDGF-domain refers to an amino acid sequence of about 55 to 80, preferably about 60 to 75, 65 to 70, and more preferably about 69 amino acids in length. A PDGF domain of TANGO 128 extends, for example, from about amino acids 269 to 337 of SEQ ID NO:2. (SEQ ID NO:75).

Conserved amino acid motifs, referred to herein as "consensus patterns" or "signature patterns", can be used to identify TANGO 128 family members (and/or PDGF family members) having a PDGF domain. For example, the following signature pattern can be used to identify TANGO 128 family members: P - x - C -[LY] - x (3) - R -C- [GSTA] - G - x (0, 3) - C- C (SEQ ID NO:46). The signature patterns or consensus patterns described herein arc described according to the following designation: all amino acids are indicated according to their universal single letter designation; "x" designates any amino acid; x(n) designates n number of amino acids, e.g., x (2) designates any two amino acids, e.g., x (1, 3) designates any of one to three amino acids; and, amino acids in brackets indicates any one of the amino acids within the brackets, e.g., [LV] indicates any of one of either L (leucine) or V (valine). TANGO 128 has such a signature pattern at about amino acids 272 to 287 of SEQ ID NO:2 (SEQ ID NO:74).

A PDGF domain further contains at least about 2 to 10, preferably, 3 to 9, 4 to 8, or 6 to 7 conserved cysteine residues. By alignment of a TANGO 128 family member with a PDGF consensus sequence (SEQ ID NO:40), conserved cysteine residues can be found. For example, as shown in Figure 17, there is a first cysteine residue in the PDGF consensus sequence (SEQ ID NO:40) that corresponds to a cysteine residue at amino acid 274 of TANGO 128 (SBQ ID NO:2); there is a second cysteine residue in the PDGF consensus sequence (SEQ ID NO:40) that corresponds to a cysteine residue at amino acid 280 of TANGO 128 (SEQ ID NO:2); there is a third cysteine residue in the PDGF consensus sequence (SEQ ID NO:40) that corresponds to a cysteine residue at amino acid 286 of TANGO 128 (SEQ ID NO:2); there is a fourth cysteine residue in the PDGF consensus sequence (SEQ ID NO:40) that corresponds to a cysteine residue at amino acid 287 of TANGO 128 (SEQ ID NO:2); there is a fifth cysteine residue in the PDGF consensus sequence (SEQ ID NO:40) that corresponds to a cysteine residue at amino acid 296 of TANGO 128 (SEQ ID NO:2); there is a sixth cysteine residue in the PDGF consensus sequence (SEQ ID NO:40) that corresponds to a cysteine residue at amino acid 335 of TANGO 128 (SEQ ID NO:2); and/or there is a seventh cysteine residue in the PDGF consensus sequence (SEQ ID NO:40) that corresponds to a cysteine residue at amino acid 337 of TANGO 128 (SEQ ID NO:2). The PDGF consensus sequence is also available from the HMMer version 2.0 software as Accession Number PF00341. Software for HMM-based profiles is available from http://www.esc.ucsc.edu/research/compbio/sam.html and from http://genome.wustl.edu/eddy/hmmer.html.

The present invention also features TANGO 128 proteins having a CUB domain. The CUB domain is associated with various developmentally regulated proteins and as such is likely to be involved in developmental processes. As used herein, a CUB domain refers to an amino acid sequence of about 90 to about 140, preferably about 100 to 125, 110 to 115, and more preferably about 113 amino acids in length. A CUB domain of TANGO 128 extends, for example, from about amino acids 48 to 160 of SEQ ID NO:2. (SEQ ID NO:77) An alignment of TANGO 128 and the CUB consensus sequence is shown in Figure 18.

Conserved amino acid motifs, referred to herein as "consensus patterns" or "signature patterns", can be used to identify TANGO 128 family members having a CUB domain. For example, the following signature pattern can be used to identify TANGO 128 family members: GS - x (3, 11) -[ST] - [PLYA] - x (2)-P - x (2,3) - Y - x (6, 8) - [WY] - x (9, 11) - [LVIF] - x - [LIF] - x (7,10) - C (SEQ ID NO:47). The signature patterns or consensus patterns described herein are described according to the following designation: all amino acids are indicated according to their universal single letter designation; "x" designates any amino acid; x(n) designates "n" number of amino acids, e.g., x (2) designates any two amino acids, e.g., x (2, 3) designates any of two to three amino acids; and, amino acids in brackets indicates any one of the amino acids within the brackets, e.g., [ST] indicates any of one of either S (serine) or T (threonine). TANGO 128 has such a signature pattern at about amino acids 56 to 104 of SEQ ID NO:2 (SEQ ID NO:76).

A CUB domain further contains at 2 or more conserved cysteine residues which are likely to form disulfide bonds which affect the structural integrity of the protein.

Also included within the scope of the present invention are TANGO 128 proteins having a signal sequence. As used herein, a "signal sequence" includes peptide of at least about 15 or 20 amino acid residues in length which occurs at the N-terminus of secretory and membrane-bound proteins and which contains at least about 70% hydrophobic amino acid residues such as alanine, leucine, isoleucine, phenylalanine, proline, tyrosine, tryptophan, or valine. In a preferred embodiment, a signal sequence contains at least about 15 to 40 amino acid residues, preferably about 15-30 amino acid residues, and has at least about 60-80%, more preferably 65-75%, and more preferably at least about 70% hydrophobic residues. A signal sequence serves to direct a protein containing such a sequence to a lipid bilayer.

In certain embodiments, a TANGO 128 family member has the amino acid sequence of SEQ ID NO:2, and the signal sequence is located at amino acids 1 to 20, 1 to 21, 1 to 22, 1 to 23 or 1 to 24. In such embodiments of the invention, the domains and the mature protein resulting from cleavage of such signal peptides are also included herein. For example, the cleavage of a signal sequence consisting of amino acids 1 to 22 of SEQ ID NO:2 (SEQ ID NO:23) results in a mature TANGO 128 protein corresponding to amino acids 23 to 345 of SEQ ID NO:2 (SEQ ID NO:29). The signal sequence is normally cleaved during processing of the mature protein.

In one embodiment, a TANGO 128 protein of the invention includes a PDGF domain and/or a CUB domain. In another embodiment, a TANGO 128 protein of the invention includes a PDGF domain, a CUE domain, a signal sequence, and is secreted.

Various features of human and mouse TANGO 128 are summarized below.

### HUMAN TANGO 128

The cDNA encoding human TANGO 128 was isolated by homology screening. Briefly, a clone encoding a portion of TANGO 128 was identified through high throughput screening of a mesangial cell library and showed homology to the VEGF family. An additional screen of the mcsangial cell library was performed to obtain a clone comprising full length human TANGO 128. Human TANGO 128 includes a 2839 nucleotide cDNA (Figure 1; SEQ ID NO:1). It is noted that me nucleotide sequence depicted in SEQ ID NO: 1 contains *Sal I* and *Not I* adapter sequences on the 5' and 3' ends, respectively ((5' GTCGACCCACGCGTCCG 3' (SEQ ID NO:), and 5' GGGCGCCCGC 3' (SBQ ID NO:), respectively). Thus, it is to be understood that the nucleic acid molecules of the invention include not only those sequences with such adaptor sequences but also the nucleic acid sequences described herein lacking the adaptor sequences. The open reading frame of this cDNA, nucleotides 288 to 1322 (SEQ ID NO:3), encodes a 345 ammo acid secreted protein (Figure 1; SEQ ID NO:2).

The signal peptide prediction program SIGNALP (Nielsen et al. (1997) *Protein Engineering* 10:1-6) predicted that human TANGO 128 includes a 22 amino acid signal peptide (amino acids 1 to amino acid 22 of SEQ ID NO:2)(SEQ ID NO:23) preceding the mature TANGO 128 protein (corresponding to amino acid 23 to amino acid 345 of SEQ ID NO:2)(SEQ ID NO:29).

Human TANGO 128 includes a PDGF domain from about amino acids 269 to 337 of SEQ ID NO:2 (SEQ ID NO:75). Human TANGO 128 further includes a CUB domain (about amino acids 48 to 160 of SEQ ID NO:2)(SEQ ID NO:77).

A clone, EpDH237, which encodes human TANGO 128 was deposited as part of EpDHMix1 with the American Type Culture Collection (ATCC, 10801 University Boulevard, Manassas, VA 20110-2209) on November 20, 1998 which was assigned Accession Number 98999. This deposit will be maimainod under the terms of the Budapest . Treaty on the International Recognition of the Deposit of Microorganisms for the Purposes of Patent Procedure. This deposit was made merely as a convenience to those of skill in the art and is not an admission that a deposit is required under 35 U.S.C. §112.

Figure 9 depicts a hydropathy plot of human TANGO 128. Relatively hydrophobic residues are above the horizontal line, and relatively hydrophilic residues are below the horizontal line. As shown in the hydropathy plot, the hydrophobic region at the beginning of the plot which corresponds to about amino acids 1 to 22 of SEQ ID NO:2 is the signal sequence of TANGO 128 (SEQ ID NO:23). The cysteine residues (cys) and potential N-glycosylation sites (Ngly) are indicated by short vertical lines just below the hydropathy trace.

Northern analysis of human TANGO 128 mRNA expression revealed the presence of approximately a 3.8 kb transcript that is expressed in a wide range of tissues including heart, brain, placenta, lung, liver, skelctal muscle, kidney, pancreas, spleen, prostate, testis, ovary, small intestine, colon, and peripheral blood leukocytes. The highest levels of expression were seen in the pancreas, kidney and ovary. An additional TANGO 128 transcript of approximately 3 kb is seen in the ovary, prostate, pancreas, and kidney.

The human gene for TANGO 128 was mapped on radiation hybrid panels to the long arm of chromosome 4, in the region q28-31, Flanking markers for this region are WI-3936 and AFMCO27ZB9. The FGC (fibrinogen gene cluster), GYP (glycophorin cluster), IL15 (interlukin 15), TDO2 (tryptophab oxygenase), and MLR (mineralcorticoid receptor) genes also map to this region of the human chromosome. This region is syntenio to mouse chromosome 8. The Q (quinky), pdw (proportional dwarf), and lyll (lymphoblastomic leukemia) loci also map to this region of the mouse chromosome. Il15 (interlukin 15), mlr (mineral cvrticoid receptor), ucp (uncoupling protein), and clgn (calmegin) genes also map to this region of the mouse chromosome.

TANGO 128 protein binds to endothelial cells with high affinity: In vitro studies of AP-T128 binding to bACE cells (novine adrenal cortical capillary endothelial cells) were performed with Phospha-Light chemiluminescent assay system (Tropix, Inc. Bedford, MA). bACE cells were plated into gelatinized 96-well plates (3000 cells/well) and allowed to grow to confluency. The cells were then fixed with acetone. AP-hT128 was incubated with the cells for 1 hour. Specific binding was detected with a microplate luminometer according to the manufacturer's instruction.

The binding studies indicated high affinity to bovine adrenal capillary endothelial cells in culture. Half-maximal binding occurred with approximately 0.5 nM AP-T128. AP-T128 was capable of exhibiting binding to adrenal cortex, ovary (medulla), mucosal layer of colon, and bronchial epithelium of lung in the mouse.

Recombinant TANGO 128 protein stimulates endothelial cell proliferation in vitro: The ability of A1 protein to stimulate the growth of endothelial cells was tested by bovine adrenal capillary endothelial (bACE) cell proliferation assay. Briefly, cultured bovine capillary endothelial cells dispersed with 0.05% trypsin/0.53 mM EDTA were plated onto gelatinized (Difco) 24-well culture plates (12,500 cell/well) in DMEM containing 10% bovine calf serum (BCS) and incubated for 24 hours. The media was replaced with 0.5 ml DMEM containing 5% bovine calf serum and either buffer only or buffer containing AP-hT128 were added. After 72 hours, the cells were counted with Coulter Counter. By cell count, there is a modest increase in bACE cells after 3 days. TANGO 128 was shown to exhibit proliferative activity on endothelial cells in vitro. Preliminary studies show that AP-T128 has mitogenic activity on primary bovine adrenal cortical capillary endothelial cells (bACE cells).

### Mouse TANGO 128

A mouse homolog of human TANGO 128 was identified. A cDNA encoding mouse TANGO 128 was identified by analyzing the sequences of clones present in a mouse osteoblast lipolysaccharide (LPS) stimulated cDNA library. This analysis led to the identification of a clone, jtmoal14h01, encoding full-length mouse TANGO 128. The murine TANGO 128 cDNA of this clone is 764 nucleotides long (Figure 26; SEQ ID NO:53). It is noted that the nuclcotide sequence depicted in SEQ ID NO:53 contains *Sal I* and *Not I* adapter sequences on the 5' and 3' ends, respectively ((GTCGACCCACGCGT CCG (SEQ ID NO:), and GGGCGGCCGC (SEQ ID NO:), respectively). Thus, it is to be understood that the nucleic acid molecules of the invention include not only those sequences with such adaptor sequences but also the nucleic acid sequences described herein lacking the adaptor sequences. The open reading frame of this cDNA, comprises nucleotides 211 to 750 of SEQ ID NO:53 (SEQ ID NO:55), and encodes a 179 amino acid secreted protein (Figure 26; SEQ ID NO:54).

In one embodiment of a nucleotide sequence of mouse Tango 128, the nucleotide at position 595 is a guanine (G) (SEQ ID NO: 78). In this embodiment, the amino acid at position 129 is glycine (G)(SEQ ID NO:79) In another embodiment of a nucleotide sequence of mouse Tango 128, the nucleotide a position 595 is a cytosine (C) (SEQ ID NO: 80). In this embodiment, the amino acid at position 129 is arginine (R)(SEQ ID NO:81) In another embodiment of a nucleotide sequence of mouse Tango 128, the nucleotide at position 595 is a thymidine (T) (SEQ ID NO:82). In this embodiment, the amino acid at position 129 is a stop codon (Opal) and results in a polypeptide of 128 aa in length (SEQ ID NO:83).

In one embodiment of a nucleotide sequence of mouse Tango 128, the nucleotide at position 710 is a thymidine (T) (SQ ID NO:84). In this embodiment, the amino acid at position 167 is valine (V)(SEQ ID NO:85) In another embodiment of a nucleotide sequence of mouse Tango 128, the nucleotide at position 710 is a cytosine (C) (SEQ ID NO:86). In this embodiment, the amino acid at position 167 is alanine (A)(SEQ ID NO:87). In another embodiment of a nucleotide sequence of mouse Tango 128, the nucleotide at position 710 is adenine (A)(SEQ ID NO:88). In this embodiment, the amino acid at position 167 is glutamine (E)(SEQ ID NO:89). In another embodiment of a nucleotide sequence of mouse Tango 128, the nucleotide at position 710 is guanine (G)(SEQ ID NO:90). In this embodiment, the amino acid at position 167 is glycine (G)(SEQ ID NO:91).

In one embodiment of a nucleotide sequence of mouse Tango 128, the nucleotide at position 725 is a thymidine (T) (SQ ID NO:92). In this embodiment, the amino acid at position 172 is leucine (L)(SEQ ID NO:93) In another embodiment of a nucleotide sequence of mouse Tango 128, the nucleotide at position 725 is a cytosine (C) (SEQ ID NO:94). In this embodiment, the amino acid at position 172 is serine (S)(SBQ ID NO:95) In another embodiment of a nucleotide sequence of mouse Tango 128, the nucleotide at position 725 is a adenine (A) (SEQ ID NO;96). In this embodiment, the amino acid at position 172 is a stop codon (Amber) and results in a polypeptide of 171 aa in length (SEQ ID NO:97). In another embodiment of a nucleotide sequence of mouse Tango 128, the nucleotide at position 725 is a guanine (G) (SEQ ID NO:98). In this embodiment, the amino acid at position 172 is tryptophan (SEQ ID NO:99).

In situ tissue screening was performed on mouse adult and embryonic tissue to analyze the expression of mouse TANGO 128 mRNA. Of the tissues tested, expression in the adult mouse was highest in the reproductive tract, testes and ovary.

In the case of adult expression, the following results were obtained: For the testis, a signal outlining some seminiferous tubules was detected which possibly included the lamina propria which contains fibromyocytes (myoid cells). In the placenta, a signal was detected in the labyrinthine tissue, In the ovaries, a strong, multifocal signal was detected. A weak signal was detected from the capsule of the adrenal gland. In the spleen, a ubiquitous signal was detected which was slighter higher in the non-follicular spaces. A weak, ubiquitous signal was detected in the submandibular gland. Weak expression was also seen in a number of other tissues. For example, a very weak signal was detected in the olfactory bulb of the brain. A very weak ubiquitous signal only slightly above background was detected in the colon, small intestine, and liver. A multifocal signal was detected in brown and white fat. No signal was detected in the following tissues: eye and harderian gland, spinal cord, stomach, thymus, skeletal muscle, bladder, heart, lymph node, lung, pancreas, and kidney.

Embryonic expression was seen in a number of tissues. The highest expressing tissue was the capsule of the kidney which was seen at E14.5 and continues to P1.5. Adult kidney did not show this expression pattern. Other tissues with strong expression include the frontal cortex and developing cerebellum of the brain, various cartilage structures of the head including Meckel's cartilage and the spinal column. Numerous tissues with a smooth muscle component also showed expression including the small intestine and stomach as well as the diaphragm at early embryonic stages, E13.4 and E14.5. At E13.5, signal in the brain was seen in areas adjacent to the ventricles, which includes the roof of the midbrain and the roof of the neopallial cortex. A stronger signal was observed from the skin of the snout and follicles of vibrissae extending to the epithelium of the mouth and tongue. A diffuse signal around developing clavicle, hip, and vertebrae was suggestive of muscle expression. A signal did not appear to be expressed from developing bone or cartilage except in the case of the spinal column where there may have been some cartilage expression. Large airways of the lung were positive as is the diaphragm, stomach and intestines. A signal from the digestive tract appeared to be associated with smooth muscle. At E14.5, the expression pattern was nearly identical to that seen at E13.5 except kidney expression was now apparent. Signal was restricted to the capsule and was the strongest expressing tissue. The capsule of the adrenal gland had expression but to a lesser extent than that seen in the kidney. The developing musculature of the feet had strong expression as well. At E16.5, signal in the muscle and skin was decreased. Diaphragm expression was no longer apparent but the smooth muscle of the intestine was still seen. Strongest signal was seen in the skin and muscle of the snout and feet, capsule of the kidney, the frontal cortex, and the cerebellar promordium. Signal from lung had decreased and become ubiquitous. At E17.5, signal was most apparent in the frontal cortex and cerebellar primordium of the brain, the snout, Meckel's cartilage, submandibular gland, spinal column, and capsule of the kidney which had the strongest signal. Signal was also seen from the smooth muscle of the gut. At E18.5, the pattern was nearly identical to that seen at E17.5. At P1.5, the pattern was very similar to that seen at E17.5 and 18.5 with strongest signal seen from Meckel's cartilage, basiocippital and basisphenoid bone, spinal column, developing cerebellum, and capsule of the kidney. By this stage of development, expression in most other tissues and organs had dropped to nearly background levels.

Human and murine TANGO 128 sequences exhibit considerable similarity at the protein, nucleic acid, and open reading frame levels. An alignment (made using the ALIGN software (Myers and Miller (1989) CABIOS, ver. 2.0); BLOSUM 62 scoring matrix; gap penalties -12/-4), reveals a protein identity of 77.8%. The human and murine TANGO 128 full length cDNAs are 83.3% identical, as assessed using the same software and parameters as indicated (without the BLOSUM 62 scoring matrix). In the respective ORFs, calculated in the same fashion as the full length cDNAs, human and murine TANGO 128 are 81.3% identical.

### Uses of TANGO 128 Nucleic Acids, Polypeptides, and Modulators Thereof

The TANGO 128 proteins of the invention bear some similarity to the VEGF family of growth factors. Accordingly, TANGO 128 proteins likely function in a similar manner as members of the VEGF family. Thus, TANGO 128 modulators can be used to treat any VEGF-associated disorders and modulate normal VEGF functions.

VBGF family members play a role in angiogenesis and endothelial cell growth. For example, VEGF is an endothelial cell specific mitogen and has been shown to be a potent angiogenic factor. Ferrara et al. (1992) *Endocr. Rev.* 13:18-32. Thus, several studies have reported that VEGF family members can serve as regulators of normal and pathological angiogenesis. Olofsson et al. (1996) *Proc. Natl. Acad. Sci, USA* 93:2576-2581; Berse et al. (1992) *Mol. Biol. Cell.* 3:211-220; Shweiki et al. (1992) *Nature* 359:843-845. Similarly, the TANGO 128 proteins of the invention likely play a role in angiogenesis. Accordingly, the TANGO 128 proteins, nucleic acids, and/or modulators of the invention are useful angiogenic modulators. For example, the TANGO 128 proteins, nucleic acids and/or modulators can be used in the treatment of wounds, e.g., modulate wound healing, and/or the regrowth of vasculature, e.g., the regrowth of vasculature into ischemic organs, e.g., such as in coronary bypass. In addition, TANGO 128 proteins, nucleic acids and/or modulators can be used to promote growth of cells in culture for cell based therapies.

Angiogenesis is also involved in pathological conditions including the growth and metastasis of tumors. In fact, tumor growth and metastasis have been shown to be dependent on the formation of new blood vessels. Accordingly, TANGO 128 polypeptides, nucleic acids and/or modulators thereof can be used to modulate angiogenesis in proliferative disorders such as cancer, (*e*.*g*., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leimyosarcoma, rhabdotheliosarcoma, colon sarcoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hematoma, bile duct carcinoma, melanoma, choriocarcinoma, semicoma, embryonal carcinoma, Wilms' tumor, cervical cancer, testicular tumor, lung carcinoma, small cell carcinoma, bladder carcinoma, epithelial carcinoma, glioma, astrocytoma, medulloblastoma, cramopharyngioma, ependynoma, pinealoma, hemangioblastoma, and retinoblastoma.

Because TANGO 128 is expressed in the reproductive tract, particularly in the ovaries and testis, the TANGO 128 polypeptides, nucleic acids and/or modulators thereof can be used to modulate the function, morphology, proliferation and/or differentiation of cells in the tissues in which it is expressed. For example, such molecules can be used to treat or modulate disorders associated with the testis including, without limitation, the Klinefelter syndrome (both the classic and mosaic forms), XX male syndrome, variococele, germinal cell aplasia (the Sertoli cell-only syndrome), idiopathic azoospermia or severe oligospermia, crpytochidism, and immotile cilia syndrome, or testicular cancer (pnmary germ cell tumors of the testis). In another example, TANGO 128 polypeptides, nucleic acids, or modulators thereof, can be used to treat testicular disorders, such as unilateral testicular enlargment (e.g., nontuberculous, granulomatous orchitis), inflammatory diseases resulting in testicular dysfunction (e.g., gonorrhea and mumps), and tumors (e.g., germ cell tumors, interstitial cell tumors, androblastoma, testicular lymphoma and adenomatoid tumors).

For example, the TANGO 128 polypeptides, nucleic acids and/or modulators thereof can be used modulate the function, morphology, proliferation and/or differentiation of the ovaries. For example, such molecules can be used to treat or modulate disorders associated with the ovaries, including, without limitation, ovarian tumors, McCune-Albright syndrome (polyostotic fibrous dysplasia). For example, the TANGO 128 polypeptides, nucleic acids and/or modulators can be used in the treatment of infertility.

The TANGO 128 polypeptides, nucleic acids and/or modulators thereof can be used to modulate the function, morphology, proliferation and/or differentiation of cells in the tissues of the reproductive tract other than the ovaries and testis. For example, such molecules can be used to treat or modulate disorders associated with the female reproductive tract including, without limitation, uterine disorders, e.g., hyperplasia of the endometrium, uterine cancers (e.g., uterine leiomyomoma, uterine cellular leiomyoma, leiomyosarcoma of the uterus, malignant mixed mullerian Tumor of uterus, uterine Sarcoma), and dysfunctional uterine bleeding (DUB).

### TANGO 140

In another aspect, the present invention is based on the discovery of cDNA molecules which encode a novel family of proteins referred to herein as TANGO 140 proteins. Described herein are TANGO 140-1 (SEQ ID NO:4), and TANGO 140-2 (SEQ ID NO:6) nucleic acid molecules and the corresponding polypeptides which the nucleic acid molecules encode (SEQ ID NO:5 and SEQ ID NO:7, respectively).

The TANGO 140 proteins and nucleic acid molecules comprise a family of molecules having certain conserved structural and functional features. As used herein, the term "family" is intended to mean two or more proteins or nucleic acid molecules having a common structural domain and having sufficient amino acid or nucledtide sequence identity as defined herein. Family members can be from either the same or different species. For example, a family can comprises two or more proteins of human origin, or can, comprise one or more proteins of human origin and one or more of non-human origin. Members of the same family may also have common structural domains.

For example, the tumor necrosis factor receptor (TNF-R) family to which the TANGO 140 proteins of the invention bear sequence similarity, are a family of cell surface proteins which function as receptors for cytokines and which contain conserved patterns of cysteine residues. Conserved cysteine residues, as used herein, refer to cysteine residues which are maintained within TANGO 140 family members (and/or TNF-R family members). This cysteine pattern is referred to herein as a tumor necrosis factor receptor (TNF-R) domain. These cysteine residues can form disulfide bonds which can affect the structural integrity of the protein. Thus, included within the scope of the invention are TANGO 140 proteins having at least one to four TNF-R domains, preferably two TNF-R domains. As used herein, a TNF-R domain refers to an amino acid sequence of about 25 to 50, preferably about 30 to 45, 30 to 40, and more preferably about 35 to 39 or 40 amino acids in length. A TNF-R domain of TANGO 140-1 extends, for example, from about amino acid 11 to amino acid 49 (SEQ ID NO:100) and/or from about amino acid 52 to amino acid 91 of SEQ ID NO:5 (SEQ ID NO:101); a TNF-R domain of TANGO 140-2 extends, for example, from about amino acid 25 to amino acid 63 (SEQ ID NO:102) and/or from about amino acid 66 to amino acid 105 of SEQ ID NO:7 (SEQ ID NO:103).

Conserved amino acid motifs, referred to herein as "consensus patterns" or "signature patterns", can be used to identify TANGO 140 family members (and/or TNF-R family members) having a TNF-R domain. For example, the following signature pattern can be used to identify TANGO 140 family members: C - x (4, 6) - [FYH] - x (5, 10) - C -x (0, 2) - C - x (2, 3) - C - x (7, 11) - C - x (4, 6) - [DNEQSKP] - x (2) - C (SEQ ID NO:48). The signature patterns or consensus patterns described herein are described according to Prosite Signature designation. Thus, all amino acids are indicated according to their universal single letter designation; "x" designates any amino acid; x(n) designates "n" number of amino acids, e.g., x (2) designates any two amino acids, e.g., x (4, 6) designates any four to six amino acids; and, amino acids in brackets indicates any one of the amino acids within the brackets, e.g., [FYH] indicates any of one of either F (phenylalanine), Y (tyrosine) or H (histidine). This consensus sequence can also be obtained as Prosite Accession Number FDOC00561. TANGO 140-1 has such a signature pattern at about amino acids 11 to 49 (SEQ ID NO:100) and at about amino acids 52 to 91 of SEQ ID NO:5 (SEQ ID NO:101). TANGO 140-2 has such a signature pattern at about amino acids 25 to 63 (SEQ ID NO:102) and at amino acids 66 to 105 of SEQ ID NO:7 (SEQ ID NO:103).

A TNF-R domain further contains at least about 2 to 10, preferably, 3 to 8, or 4 to 6 conserved cysteine residues. By alignment of a TANGO 140 family member with a TNF-R consensus sequence, conserved cysteine residues can be found. For example, as shown in Figure 19, there is a first cysteine residue in the TNF-R consensus sequence that corresponds to a cysteine residue at amino acid 11 of the first TNF-R domain of TANGO 140-1 (SEQ ID NO:5); there is a second cysteine residue in the TNF-R consensus sequence that corresponds to a cysteine residue at amino acid 23 of the first TNF-R domain of TANGO 140-1 (SEQ ID NO:5); there is a third cysteine residue in the TNF-R consensus sequence that corresponds to a cysteine residue at amino acid 26 of the first TNF-R domain of TANGO 140-1 (SEQ ID NO:5); there is a fourth cysteine residue in the TNF-R consensus sequence that corresponds to a cysteine residue at amino acid 29 of the first TNF-R domain of TANGO 140-1 (SBQ ID NO-5); there is a fifth cysteine residue in the TNF-R consensus sequence that corresponds to a cysteine residue at amino acid 39 of the first INF-R domain of TANGO 140-1 (SEQ ID NO:5); and/or there is a sixth cysteine residue in the TNF-R consensus sequence that corresponds to a cysteine residue at amino acid 49 of the first TNF-R domain of TANGO 140-1 (SEQ ID NO:5). In addition, conserved cysteine residues can be found at amino acids 52, 66, 69, 72, 83 and/or 91 of the second TNF-R domain of TANGO 140-1 (SEQ ID NO:5). Moreover, as shown in Figure 20, conserved cysteine residues can be found at amino acids 25, 37, 40, 43, 53 and/or 63 of the first TNF-R domain of TANGO 140-2 (SEQ ID NO:7); and at amino acids 66, 80, 83, 86, 97 and/or 105 of TANGO-140-2 (SEQ ID NO:7). The TNF-R consensus sequence is available from the HMMer version 2.0 software as Accession Number PF00020. Software for HMM-based profiles is available from http://www.csc.ucsc.edu/research/compbio/sam.html and from http://genome.wustl.edu/eddy/hmmer.html.

The present invention also includes TANGO 140 proteins having a transmembrane domain. As used herein, a transmembrane domain refers to an amino acid sequence having at least about 25 to about 40 amino acid residues in length and which contains at least about 65-70% hydrophobic amino acid residues such as alanine, leucine, isoleueine, phenylalanine, proline, tyrosine, tryptophan, or valine. In a preferred embodiment, a transmembrane domain contains at least about 30-35 amino acid residues, preferably about 30-35 amino acid residues, and has at least about 60-80%, more preferably 65-75%, and more preferably at least about 68% hydrophobic residues. An example of a transmembrane domain includes from about amino acids 147 to 170 of TANGO 140-1 (SEQ ID NO:5)(SEQ ID NO:36).

Thus, in one embodiment, a TANGO 140 protein includes at least one TNF-R domain, preferably two, three or four TNF-R domains and is secreted. In another embodiment, a TANGO 140 protein of the invention includes at least one TNF-R domain, preferably two, three or four TNF-R domains, a transmembrane domain and is a membrane bound protein.

Various features of human TANGO 140-1 and 140-2 are summarized below.

### Human TANGO 140-1

A cDNA encoding a portion of human TANGO 140-1 was identified by screening a stimulated human mesangial library. Human TANGO 140-1 includes a 1550 nucleotide cDNA (Figure 2; SEQ ID NO:4). It is noted that the nucleotide sequence depicted in SEQ ID NO:4 contains a *Not I* adapter sequence on the 3' end (5' GGGCGGCCGC 3')(SEQ ID NO:). Thus, it is to be understood that the nucleic acid molecules of the invention include not only those sequences with such adaptor sequences but also the nucleic acid sequences described herein lacking the adaptor sequences. The open reading frame of TANGO 140-1 comprises nucleotides 2 to 619 of SEQ ID NO:4 (SEQ ID NO:38), and encodes a 206 amino acid putative membrane protein (Figure 2; SEQ ID NO:5).

In one embodiment, human TANGO 140-1 includes an extracellular domain (about amino acids 1 to 146 of SEQ ID NO:5)(SEQ ID NO:35), a transmembrane (TM) domain (amino acids 147 to 170 of SEQ ID NO:5)(SEQ ID NO:36); and a cytoplasmic domain (amino acids 171 to 206 of SEQ ID NO:5)(SEQ ID NO:37) Alternatively, in another embodiment, a human TANGO 140-1 protein contains an extracellular domain at amino acid residues 1 to 146 of SEQ ID NO:5 (SEQ ID NO:), a transmembrane domain at amino acid residues 147 to 170 of SEQ ID NO:5 (SEQ ID NO:), and a cytoplasmic domain at amino acid residues 171 to 206 of SEQ ID NO:5 (SEQ ID NO:).

The extracellular region of human TANGO 140-1 includes TNF-R domains from about amino acids 11 to 49 of SEQ ID NO:5 (SEQ ID NO:100) and from about amino acids 52-91 of SEQ ID NO:5 (SEQ ID NO:101).

A clone, EpDH137, which encodes human TANGO 140-1 was deposited as part of EpDHMix1 with the American Type Culture Collection, 10801 University Boulevard, Manassas, VA 20110-2209) on November 20, 1998 which was assigned Accession Number 98999. This deposit will be maintained under the terms of the Budapest Treaty on the International Recognition of the Deposit of Microorganisms for the Purposes of Patent Procedure. This deposit was made merely as a couvenience for those of skill in the art and is not an admission that a deposit is required under 35 U.S.C. §112.

Figure 10 depicts a hydropathy plot of human TANGO 140- 1. Relatively hydrophobic residues are above the horizontal line, and relatively hydrophilie residues are below the horizontal line. As shown in the hydropathy plot, amino acids 147 to 170 of SEQ ID NO:5 (SEQ ID NO:36) correspond to a transmembrane domain of TANGO 140-1. The cysteine residues (cys) and potential N-glycosyiation sites (Ngly) are indicated by short vertical lines just below the hydropathy trace.

### HUMAN TANGO 140-2

An additional clone having significant homology to human TANGO 140-1 was identified. The clone was sequenced and is likely to be a splice variant of TANGO 140-1. This variant is referred to herein as TANGO 140-2. The human TANGO 140-2 includes a 3385 nucleotide cDNA (Figure 3; SEQ ID NO:6). It is noted that the nucleotide sequence depicted in SEQ ID NO:6 contains a *Not I* adapter sequence on the 3' end (5' GGGCGG CCGC 3' (SEQ ID NO:)). Thus, it is to be understood that the nucleic acid molecules of the invention include not only those sequences with such adaptor sequences but also the nucleic acid sequences described herein lacking the adaptor sequences. The open reading frame of TANGO 140-2 comprises nucleotides 2 to 619 of SEQ ID NO:6 (SEQ ID NO:38), and encodes a 198 amino acid putative secreted protein (Figure 3; SEQ ID NO:7).

Human TANGO 140-2 also includes TNF-R domains from about amino acids 25 to 63 of SEQ ID NO:7 (SEQ ID NO:102), and from about amino acids 66 to 105 of SEQ ID NO:7 (SEQ ID NO:103).

A clone, EpDH185, which encodes human TANGO 140-2 was deposited as part of EpDHMix1 with the American Type Culture Collection (10801 University Boulevard, Manassas, VA 20110-2209) on November 20, 1998 which was assigned Accession Number 98999. This deposit will be maintained under the terms of the Budapest Treaty an the International Recognition of the Deposit of Microorganisms for the Purposes of Patent Procedure. This deposit was made merely as a convenience for those of skill in the art and is not an admission that a deposit is required under 35 U.S.C. §112.

Figure 11 depicts a hydropathy plot of TANGO 140-2. Relatively hydrophobic residues are above the horizontal line, and relatively hydrophilic residues are below the horizontal line. As shown in the hydropathy plot, TANGO 140-2 does not have a transmembrane domain. The cysteine residues (cys) and potential N-glycosylatiott sites (Ngly) are indicated by short vertical lines just below the hydropathy trace.

### Uses of TANGO 140 Nucleic Acids. Polypeptides, and Modulators Thereof

The TANGO 140 proteins of the invention comprise a family of proteins having sequence similarity to members of the TNF-R superfamily. Thus, the TANGO 140 proteins of the invention are members of the TNF-R superfamily. Accordingly, TANGO 140 proteins likely function in a similar manner as members of the TNF-R family and TANGO 140 modulators can be used to treat any TNF-R/NGF-R-associated disorders.

For example, members of the tumor necrosis factor receptor (TNF-R) superfamily regulate a diverse range of cellular processes including cell proliferation, programmed cell death and immune responses. TNF-R family members are cell surface proteins which function as receptors for cytokines. Mallet et al. (1991) *Immunology Today* 12:220-223. For example, the binding of NGF to NGF-R causes neuronal differentiation and survival. Barde (1989) *Neuron* 2:1525-1534. Similarly, the TANGO 140 molecules of the invention can modulate neuronal differentiation and survival.

NGF (nerve growth factor) induces, *inter alia*, neurite outgrowth and promotes survival of embryonic sensory and sympathetic neurons. Nerve growth factor (NGF) is also involved in the development and maintenance of the nervous system. Thus, TANGO 140 polypeptides, nucleic acids and/or modulators thereof can be used to modulate the function, morphology, proliferation and/or differentiation of cells in the nervous system. Such molecules may be used in the treatment of neural disorders, including, without limitation, epilepsy, muscular dystrophy, and neurodegenerative diseases such, as Alzheimer's disease, Parkinson's disease, and Huntington's disease).

In addition, both TGF-α and TGF-β bind to TGF-RI and TGF-RII, leading to a diverse range of effects including inflammation and tumor cell death. Beutler et al. (1989) *Ann. Rev. Immunol*. 7:625-665; Sprang (1990) *Trends Biochem. Sci*. 15:366-368. Thus, the TANGO 140 proteins of the invention are likely to bind directly or indirectly to a soluble protein, e.g., a cytokine, or membrane-bound protein, and play a role in modulating inflammation, cell proliferation, and/or apoptosis.

In light of the similarity of TANGO 140, TANGO 140 polypeptides, nucleic acids and/or modulators thereof can be used to treat TANGO 140 associated disorders which can include TNF-related disorders (*e*.*g*.; acute myocarditis, myocardial infarction, congestive heart failure, T cell disorders (*e*.*g*., dermatitis, fibrosis)), immunological differentiative and apoptotic disorders (*e*.*g*., hyper-proliferative syndromes such as systemic lupus erythematosus (lupus)), and disorders related to angiogenesis (*e*.*g*., tumor formation and/or metastasis, cancer). Examples of types of cancers include benign tumors, neoplasms or tumors (such as carcinomas, sarcomas, adenomas or myeloid lymphoma tumors, e.g., fibrosarcoma, myxosarcoma, liposarcoma, chondrosarcoma, osteogenic sarcoma, chordoma, angiosafeoma, endothetiosarcoma, lymphangiosareoma, lymphangioendotheliosarcoma, synovioma, mesothelioma, Ewing's tumor, leimyosarcoma, rhabdotheliosarcoma, colon sarcoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adeaocarcinomas, cystadenocarcinoma, medullary carcinoma, bronchogenic carcinonia, renal cell carcinoma, hematoma, bile duct carcinoma, melanoma, chonocarcinoma, semicoma, embryonal carcinoma, Wilms' tumor, cervical cancer, testicular tumor, lung carcinoma, small cell carcinoma, bladder carcinoma, epithelial carcinoma, glioma, astrocytoma, medulloblastoma, craniopharyngioma, ependynoma, pinealoma, hemangioblastoma, retinoblastoma), leukemias, (*e*.*g*. acute lymphocytic leukemia), acute myelocytic leukemia (myelolastic, promyelocytic, myelomonocytic, monocytic and erythroleukemia), chronic leukemias (chronic myelocytic (granulocytic) leukemia and chronic lymphocytic leukemia), or polycythemia vera, or lymphomas (Hodgkin's disease and non-Hodgkin's diseases), multiple myelomas and Waldenström's macroglobulinemia.

Moreover, as TANGO 140 is expressed in a stimulated mesangial library, the TANGO 140 polypeptides, nucleic acids and/or modulators thereof can be used to modulate the function, morphology, proliferation and/or differentiation of cells in the tissues in which it is expressed. Mesangial cells are known to play an important role in maintaining structure and function of the glomerulus and in the pathogeuesis of glomerular diseases. Moreover, the local production of chemokines by mesangial cells has been linked to inflammatory processes within the glomerulus. Also, it is known that high glucose directly increases oxidative stress in glomerular mesangial cells, a target cell of diabetic nephropathy. Thus, TANGO 140 polypeptides, nucleic acids and/or modulators thereof can be used to modulate the function, morphology, proliferation and/or differentiation of cells in the kidney. Such molecules can also be used to treat disorders associated with abnormal or aberrant metabolism or function of cells in the kidney. Therefore, such molecules can be used to treat or modulate renal (kidney) disorders, such as glomerular diseases (e.g., acute and chronic glomerulonephritis, rapidly progressive glomerulonephritis, nephrotic syndrome, focal proliferative glomerulonephritis, glomerular lesions associated with systemic disease, such as systemic lupus erythematosus, Goodpasture's syndrome, multiple myeloma, diabetes, neoplasia, sickle cell disease, and chronic inflammatory diseases), tubular diseases (e.g., acute tubular necrosis and acute renal failure, polycystic renal diseasemedullary sponge kidney, medullary cystic disease, nephrogenic diabetes, and renal tubular acidosis), tubulointerstitial disesses (e.g., pyelonephritis, drug and toxin induced tubulointerstitial nephritis, hypercalcemic nepbropathy, and hypokalemic nephropathy) acute and rapidly progressive renal failure, chronic renal failure, nephrolithiasis, vascular diseases (e.g., hypertension and nephrosclerosis, microangiopathic hemolytic anemia, atheroembolic renal disease, diffuse cortical necrosis, and renal infarcts), or tumors (e.g., renal cell carcinoma and nephroblastoma).

### TANGO 197

In one aspect, the present invention is based on the discovery of cDNA molecules which encode a novel family of proteins referred to herein as TANGO 197 proteins.

The TANGO 197 proteins and nucleic acid molecules comprise a family of molecules having certain conserved structural and functional features. As used herein, the term "family" is intended to mean two or more proteins or nucleic acid molecules having a common structural domain and having sufficient amino acid or nucleotide sequence identity as defined herein. Family members can be from either the same or different species. For example, a family can comprise two or more proteins of human origin, or can comprise one or more proteins of human origin and one or more of non-human origin. Members of the same family may also have common structural domains.

For example, the type A module superfamily, which includes proteins of the extracellular matrix and various proteins with adhesive function, have a von Willebmd factor type A (vWF) domain. This domain allows for the interaction between various cells and/or extracellular matrix (ECM) components. Thus, included within the scope of the invention are TANGO 197 proteins having a von Willebrand factor type A (vWF) domain. As used herein, a vWF domain refers to an amino acid sequence of about 150 to 200, preferably about 160 to 190, 170 to 180, and more preferably about 172 to 175 amino acids in length. A vWF domain of TANGO 197 extends, for example, from about amino acids 44 to 215 of SEQ ID N0:9 (SEQ ID NO:105).

Conserved amino acid motifs, referred to herein as "consensus patterns" or "signature patterns", can be used to identify TANGO 197 family members having a vWF domain. For example, the following signature pattern can be used to identify TANGO 197 family members: D - x (2) - F -[ILV] - x - D - x - S - x (2,3) - [ILV]- x (10, 12) - F (SEQ ID NO:49). The signature patterns or consensus patterns described herein are described according to the following designation: all amino acids are indicated according to their universal single letter designation; "x" designates any amino acid, x(n) designates "n" number of amino acids, e.g., x (2) designates any two amino acids, e.g., x (2, 3) designates any of two to three amino acids; and, amino acids in brackets indicates any one of the amino acids within the brackets, e.g., [ILV] indicates any of one of either I (isoleucine), L (leucine) or V (valine), TANGO 197 has such a signature pattern at about amino acids 44 to 65 of SEQ ID NO:9 (SEQ ID NO:104).

An alignment of TANGO 197 and the vWF consensus sequence is shown in Figure 21. The vWF consensus sequence is available from the HMMer 2.0 software as Accession Number PF00092. Software for HMM-based profiles is available from http://www.csc.ucsc.edu/research/compbio/sarn.html and from http://genome.wustl. edu/eddy/hmmer.html.

Also included within the scope of the present invention ate TANGO 197 proteins having a signal sequence. As used herein, a "signal sequence" includes a peptide of at least about 15 or 20 amino acid residues in length which occurs at the N-terminus of secretory and membrane-bound proteins and which contains at least about 70% hydrophobic amino acid residues such as alanine, leucine, isoleucine, phenylalanine, proline, tyrosine, tryptophan, or valine. In a preferred embodiment, a signal sequence contains at least about 15 to 40 amino acid residues, preferably about 15-30 amino acid residues, and has at least about 60-60%, more preferably 65-75%, and more preferably at least about 70% hydrophobic residues. A signal sequence serves to direct a protein containing such a sequence to a lipid bilayer.

In certain embodiments, a TANGO 197 family member has the amino acid sequence of SEQ ID NO:9, and the signal sequence is located at amino acids 1 to 25, 1 to 26, 1 to 27, 1 to 28, or 1 to 29. In such embodiments of the invention, the domains and the mature protein resulting from cleavage of such signal peptides are also included herein. Thus, in another embodiment, a TANGO 197 protein contains a signal sequence of about amino acids 1 to 27 of SEQ ID NO:2 ((SEQ ID NO:24) which results in an extracellular domain consisting of amino acids 28 to 301 of SEQ ID NO:2 (SEQ ID NO:), and a mature TANGO 197 protein corresponding to amino acids 28 to 333 of SEQ ID NO:2 (SBQ ID NO:). The signal sequence is normally cleaved during processing of the mature protein.

Various features of human and mouse TANGO 197 are summarized below.

### HUMAN TANGO 197

A cDNA encoding a portion of human TANGO 197 was ideritified by screening a human fetal lung library. An additional screen of an osteoclast library was performed to obtain a clone comprising a full length human TANGO 197. Human TANGO 197 includes a 2272 nucleotide cDNA (Figure 4; SEQ ID NO:8). It is noted that the nucleotide sequence depicted in SEQ ID NO:8 contains *Sal I* and *Not I* adapter sequences on the 5' and 3' ends, respectively ((GTCGACCCACGCGTCCT (SEQ ID NO:), and GGGCGGCCGC (SEQ ID NO:), respectively). Thus, it is to be understood that the nucleic acid molecules of the invention include not only those sequences with such adaptor sequences but also the nucleic acid sequences described herein lacking the adaptor sequences. The open reading frame of this cDNA, nucleotides 213 to 1211 (SEQ ID NO:10), encodes a 333, amino acid transmembrane protein (Figure 4; SEQ ID NO:9).

The signal peptide prediction program SIGNALP (Nielsen et al. (1997) *Protein Engineering* 10:1-6) predicted that human TANGO 197 includes a 27 amino acid signal peptide (amino acids 1 to about amino acid 27 of SEQ ID NO:9)(SEQ ID NO:24) preceding the mature TANGO 197 protein (corresponding to about amino acid 28 to amino acid, 333 of SEQ ID NO:9)(SEQ ID NO:30).

Human TANGO 197 includes a vWF domain from about amino acids 44 to 215 of SEQ ID NO:9.

A clone, EpDH213, which encodes human TANGO 197 was deposited as part of EpDHMix1 with the American Type Culture Collection (ATCC, 10801 University Boulevard, Manassas, VA 20110-2209) on November 20,1998 which was assigned Accession Number 989,99. This deposit will be maintained under the terms of the Budapest Treaty on the International Recognition of the Deposit of Microorganisms for the Purposes of Patent Procedure. This deposit was made merely as a convenience to those of skill in the art and is not an admission that a deposit is required under 35 U.S.C. §112.

Figure 12 depicts a hydropathy plot of human TANGO 197. Relatively hydrophobic residues are above the horizontal line, and relatively hydrophilic residues are below the horizontal line. As shown in the hydropathy plot, the hydrophobic region at the beginning of the plot which corresponds to about amino acids 1 to 27 of SEQ ID NO:9 (SEQ ID NO:24) is the signal sequence of TANGO 197. The cysteine residues (cys) and potential N-glycosylation sites (Ngly) are indicated by short vertical lines just below the hydropathy trace.

In one embodiment, human TANGO 197 protein is a transmembrane protein that contains an extracellular domain at amino acid residues 28-301 of SEQ ID NO:9 (SEQ ID NO:), a transmembrane domain at amino acid residues 302 to 319 of SEQ ID NO:9 (SEQ ID NO:), and a cytoplasmic domain at amino acid residues 320 -333 of SBQ ID NO:9 (SEQ ID NO:). Alternatively, in another embodiment, a human TANGO 197 protein contains an extracellular domain at amino acid residues 320 to 333 of SEQ ID NO:9 (SEQ ID NO:), a transmembrane domain at amino acid residues 302 to 319 of SEQ ID NO:9 (SEQ ID NO:), and a cytoplasmic domain at amino acid residues 1 to 301 of SEQ ID NO:9 (SEQ ID NO:).

Northern analysis of human TANGO 197 mRNA expression revealed expression in a wide variety of tissues such as brain, skeletal muscle, colon, thymus, spleen, kidney, liver, and the small intestine. The highest levels of expression were seen in tissues such as the heart, placenta and lung. There was no expression of the transcript in peripheral blood leukocytes.

### Mouse TANGO 197

A mouse homolog of human TANGO 197 was identified. A cDNA encoding mouse TANGO 197 was identified by analyzing the sequences of clones present in a mouse testis (Sertoli TM4 cells) cDNA library. This analysis led to the identification of a clone, jtmzb062c08, encoding full-length mouse TANGO 197. The murine TANGO 197 cDNA of this clone is 4417 nucleotides long (Figure 27; SEQ ID NO:56). It is noted that the nucleotide sequence depicted in SEQ ID NO:56 contains a *Not I* adapter sequence on the 3' end (5' GGGCGGCCGC 3' (SEQ ID NO:)). Thus, it is to be understood that the nucleic acid molecules of the invention include not only those sequences with such adaptor sequences but also the nucleic acid sequences described herein lacking the adaptor sequences. The open reading frame of this cDNA, comprises nucleotides 3-1145 of SEQ ID NO:56 (SEQ ID NO:58), encodes a 381 amino acid transmembrane protein (Figure 27; SEQ ID NO:57).

In one embodiment, mouse TANGO 197 protein is a transmembrane protein that contains an extracellular domain at amino acid residues 161 to 381 of SEQ ID NO:57 (SEQ ID NO:), a transmembrane domain at amino acid residues 139 to 160 of SEQ ID NO:57 (SEQ ID NO:), and a cytoplasmic domain at amino acid residues 1 to 138 of SEQ ID NO:57 (SEQ ID NO:). Alternatively, in another embodiment, a mouse TANGO 197 protein contains an extracellular domain at amino acid residues 1 to 139 of SEQ ID NO:57 ( SEQ ID NO:), a transmembrane domain at amino acid residues 139 to 160 of SEQ ID NO:57 (SEQ ID NO:), and a cytoplasmic domain at amino acid residues 161 to 381 of SEQ ID NO:57 (SEQ ID NO:).

Expression of mouse TANGO 197 mRNA was detected by a library array procedure. Briefly, the library array procedure entailed preparing a PCR mixture by adding to the standards reagents (Taq Polymerase, dNTPs, and PCR buffer) a vector primer, a primer internal to the gene of interest, and an aliquot of a library in which expression was to be tested. This procedure was performed with many libraries at a time in a 96 well PCR tray, with 80 or more wells containing libraries and a control well in which the above primers were combined with the clone of interest itself. The control well served as an indicator of the fragment size to be expected in the library wells, in the event the clone of interest was expressed within. Amplification was performed in a PCR machine, employing standard PCR conditions for denaturing, annealing, and elongation, and the resultant mixture was mixed with an appropriate loading dye and run on an ethidium bromide-stained agarose gel. The gel was later viewed with UV light after the DNA loaded within its lanes had time to migrate into the gels. Lanes in which a band corresponding with the control band was visible indicated the libraries in which the clone of interest was expressed.

Results of the library array procedure revealed strong expression in the choroid plexus, 12.5 day whole mouse embryo, LPS-stimulated osteoblast tissue, hyphae stimulated long term bone marrow cells. Weak expression was detected in TM4 (Sertoli cells), from testis, esophagus, LPS-stimulated osteoblast tissue. No expression was detected in diffetentiated 3T3, 10.5 day mouse fetus, mouse kidney fibrosis model, nephrotoxic serum (NTS), LPS-stimulated heart, LPS-stimulated osteoblasts, lung, mouse insulidoma (Nit-1), normal/hyperplastic islets (pancreas), normal spleen, 11.5 day mouse, LPS-stimulated lung, hypertropic heart, LPS-stimulated kidney, LPS-stimulated lymph node, mc/9 mast cells, 13.5 day mouse, LPS-sfimulated anchored heart, normal thymus, Th2-ovarian-Tg, Balb C liver (bile duct ligation d2), normal heart, brain polysome (MPB), LPS-stimulated anchored liver, brain (EAE d10 model), th1-ovarian-Tg, heart, hypothalamus, lone term bone, marrow cells, megakaryocyte, LPS-stimulated splecn, hyphae-stimulated long term bone marrow, lung, angiogenic pancreatic islets, Th2, brain, LPS-stimulated thymus, LPS-stimulated microghal cells, testes (random-primed), tumor pancreatic islets, LPS-stimulated brain, LPS-stimulated alveolar macrophage cell line, mouse lung bleomycin model, pregnant uterus, and hypothalamus nuclei.

Human and murine TANGO 197 sequences exhibit considerable similarity at the protein, nucleic acid, and open reading frame levels. An alignment (made using the ALIGN software {Myers and Miller (1989) CABIOS, ver. 2.0}; BLOSUM 62 scoring matrix; gap penalties -12/-4), reveals a protein identity of 88.0 %. The human and murine TANGO 197 full length cDNAs are 52.8% identical, as assessed using the same software and parameters as indicated (without the BLOSUM 62 scoring matrix). In the respective QRFs, calculated in the same fashion as the full length cDNAs, human and murine TANGO 197 are 51.6% identical.

### Uses of TANGO 197 Nucleic Acids, Polypeptides, and Modulators Thereof

As TANGO 197 exhibits expression in the lung, TANGO 197 polypeptides, nucleic acids, or modulators thereof, can be used to treat pulmonary (lung) disorders, such as atelectasis, pulmonary congestion or edema, chronic obstructive airway disease (e.g., emphysema, chronic bronchitis, bronchial asthma, and bronchiectasis), diffuse interstitial diseases (e.g., saicoidosis, pneumoconiosis, hypersensitivity pneumonitis, Goodpasture's syndrome, idiopathic pulmonary hemosiderosis, pulmonary alveolar proteinosis, desquamative interstitial pneumonitis, chronic interstitial pneumonia, fibrosing alveolitis, hamman-rich syndrome, pulmonary eosinophilia, diffuse interstitial fibrosis, Wegener's granulomatosis, lymphomatoid granulomatosis, and lipid pneumonia), or tumors (e.g., bronchogenic carcinoma, bronchiolovlveolar carcinoma, bronchial carcinoid, hamartoma, and mesenchymal tumors).

Morever, as a species isoform of TANGO 197 was also isolated from a testis library, therefore TANGO 197 polypeptides, nucleic acids, or modulators thereof, can be used to treat testicular disorders, such as unilateral testicular enlargment (e.g., nontuberculous, granulomatous orchitis), inflammatory diseases resulting in testicular dysfunction (e.g., gonorrhea and mumps), and tumors (e.g., germ cell tumors, interstitial cell tumors, androblastoma, testicular lymphoma and adenomatoid tumors).

Furthermore, as TANGO 197 is expressed in the testis, the TANGO 197 polypeptides, nucleic acids and/or modulators thereof can be used to modulate, for example and without limitation, Klinefelter syndrome (both the classic and mosaic forms), XX male syndrome, vsriococele, germinal cell aplasia (the Sertoli cell-only syndrome), idiopathic azoospermia or severe oligospermia, crpytochidism, and immotile cilia syndrome, or testicular cancer (primary germ cell tumors of the testis), In another example, TANGO 197 polypeptides, nucleic acids, or modulators thereof, can be used to treat testicular disorders, such as unilateral testicular enlargment (e.g., nontuberculous, granulomatous orchitis), inflammatory diseases resulting in testicular dysfunction (e.g., gonorrhea and mumps), and tumors (e.g., germ cell tumors, interstitial cell tumors, androblastoma, testicular lymphoma and adenomatoid tumors).

As discussed above, the vWF domain of TANGO 197 is involved in cellular adhesion and interaction with extracellular matrix (ECM) components. Proteins of the type A modulc superfamily which incorporate a vWF domain participate in multiple BCM and cell/ECM interactions. For example, proteins having a vWF domain have been found to play a role in cellular adhesion, migration, homing, pattern formation and/or signal transduction after interaction with several different ligands (Colombatti et al. (1993) *Matrix* 13:297-306).

Similarly, the TANGO 197 proteins of the invention likely play a role in various extracellular matrix interactions, e.g., matrix binding, and/or cellular adhesion. Thus, a TANGO 197 activity is at least one or more of the following activities: 1) regulation of extracellular matrix structuring; 2) modulation of cellular adhesion, either *in vitro* or *in vivo;* 3) regulation of cell trafficking and/or migration. Accordingly, the TANGO 197 proteins, nucleic acid molecules and/or modulators can be used to modulate cellular interactions such as cell-cell and/or cell-matrix interactions and thus, to treat disorders associated with abnormal collutar interactions.

TANGO 197 polypeptides, nucleic acids and/or modulators thereof can also be used 5 to modulate cell adhesion in proliferative disorders, such as cancer. Examples of types of cancers include benign tumors, neoplasms or tumors (such as carcinomas, sarcomas, adenomas or myeloid lymphoma tumors, e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leimyosarcoma, rhabdotheliosarcoma, colon sarcoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas; cystadenocarcinoma, medullary carcinoma, bronchogenic carcinoma, renal cell carcinoma, hematoma, bile duct carcinoma, melanoma, chonocatcinoma, semicoma, embryonal carcinoma, Wilms' tumor, cervical cancer, testicular tumor, lung carcinoma, small cell carcinoma, bladder carcinoma, epithelial carcinoma, glioma, astrocytoma, medulloblastoma, craniopharyngioma, ependynoma, pinealoma, hemangioblastoma, retinoblastoma), leukemias, (*e*.*g*. acute lymphocytic leukemia), acute inyelocytic leukemia (myelolastic, promyelocytic, myelomonocytic, monocytic and erythroleukemia), chronic leukemias (chronic myelocytic (granulocytic) leukernia and chronic lymphocytic leukemia), or polycythemia vera, or lymphomas (Hodgkin's disease and non-Hodgkin's diseases), multiple myclomas and Waldenström's macroglobulinemia.

### TANGO 212

In another aspect, the present invention is based on the discovery of cDNA molecules which encode a novel family of proteins referred to herein as TANGO 212 proteins.

The TANGO 212 proteins and nucleic acid molecules camprise a family of molecules having certain conserved structural and functional features. As used herein, the term "family" is intended to mean two or more proteins or nucleic acid molecules having a common structural domain and having sufficient amino acid or nucleotide sequence identity as defined herein. Family members can be from either the same or different species. For example, a family can comprises two or more proteins of human origin, or can comprise one or more proteins of human origin and one or more of non-human origin. Members of the same family may also have common structural domains.

For example, the EGF family to which the TANGO 212 proteins of the invention bear sequence similarity, are a family of mitogens which contain a conserved pattern of cysteine residues. Conserved cysteine residues, as used herein, refer to cysteine residues which are maintained within TANGO 212 family members (and/or EGF family members). This cysteine pattern is referred to herein as an epidermal growth factor (EGF) domain. These cysteine residues form disulfide bonds which can affect the structural integrity of the protein. Thus, included within the scope of the invention are TANGO 212 proteins having at least one, preferably two, three, four, or five EGF domain(s). As used herein, an EGF-domain refers to an amino acid sequence of about 25 to 50, preferably about 30 to 45, 30 to 40, and more preferably about 31, 35, 36 to 40 amino acids in length.

Conserved amino acid motifs, referred to herein as "consensus patterns" or "signature patterns", can be used to identify TANGO 212 family members (and/or EGF family members) having an EGF domain. For example, the following signature pattern referred to herein as a EGF-like conscensus sequence, can be used to identify TANGO 212 family members; C - x - C - x (5, 11) - G - x (2, 3) - C (SEQ ID NO:50). The signature patterns or consensus patterns described herein are described according to the following designations: all amino acids are indicated according to their universal single letter designation; "x" designates any amino acid; and, x(n) designates "n" number of amino acids, e.g., x (2) designates any two amino acids, e.g., x(2,3) designates any two to three amino acids. TANGO 212 has such a signature pattern at about amino acids 80 to 91 (SEQ ID NO:106), amino acids 156 to 172 (SEQ ID NO:107), amino acids 200 to 217 (SEQ ID NO: 108) and/or amino acids 245 to 258 of SEQ ID NO:12 (SEQ ID NO:109). An EGF domain of TANGO 212 extends, for example, from about amino acids 61 to 91 of SEQ ID NO:12 (SEQ ID NO:110), from about amino acids 98 to 132 of SEQ ID NO:12 (SEQ ID NO:111), from about amino acids 138 to 172 of SEQ ID NO:12 (SEQ ID NO:112), from about amino acids 178 to 217 of SEQ ID NO:12 (SEQ ID NO:113), and/or from about amino acids 223 to 258 of SEQ ID NO:12 (SEQ ID NO:114).

An EGF domain further contains at least about 2 to 10, preferably, 3 to 9, 4 to 8, or 6 to 7 conserved cysteine residues. By alignment of a TANGO 212 family member with an EGF-like consensus sequence, conserved cysteine residues can be found. For example, as shown in Figure 22, there is a first cysteine residue in the EGF-like consensus sequence that corresponds to a cysteine residue at amino acid 61 of the first EGF domain of TANGO 212 (SEQ ID NO: 12); there is a second cysteine residue in the EGF-like consensus sequence that corresponds to a cysteine residue at amino acid 69 of the first BGF domain of TANGO 212 (SEQ ID NO:12); there is a third cysteine residue in the EGF-like consensus sequence that corresponds to a cysteine residue at amino acid 74 of the first EGF domain of TANGO 212 (SEQ ID NO:12); there is a fourth cysteine residue in the EGF-like consensus sequence that corresponds to a cysteine residue at amino acid 80 of the first EGF domain of TANGO 212 (SEQ ID NO:12); there is a fifth cysteine residue in the EGF-like consensus sequence that corresponds to a cysteine residue at amino acid 82 of the first EGF domain of TANGO 212 (SEQ ID NO:12); and/or there is a sixth cysteine residue in the EGF-like consensus sequence that corresponds to a cysteine residue at amino acid 91 of the first EGF-domain of TANGO 212 (SEQ ID NO:12). In addition, conserved cysteine residues can be found at amino acids 98, 105, 109, 118, 120 and/or 132 of the second EGF domain of TANGO 212 (SEQ ID NO:12); at amino acids 138, 143, 147, 156, 158 and/or 172 of the third EGF domain of TANGO 212 (SEQ ID NO:12); at amino acids 178, 185, 191, 200, 202 and/or 217 of the fourth EGF domain of TANGO 212 (SBQ ID N0:12); and at amino acids 223, 230, 236, 245, 247 and/or 258 of the fifth EGF domain of TANGO 212 (SEQ ID NO:12). The EGF-like consensus sequence is available from the HMMer version 2.0 software as Accession Number PF00008. Software for HMM-based profiles is available from http://www.csc.ucsc.edu/research/compbio/sam.html and from http://genome:wustl.edu/cddy/hmmer.html.

The present invention also features TANGO 212 proteins having a MAM domain. The MAM domain is associated with various adhesive proteins and as such is likely to have adhesive function. Within MAM domains are conserved cysteine residues which play a role in the adhesion of a MAM domain to other proteins. As used herein, a MAM domain refers to an amino acid sequence of about 120 to about 170, preferably about 130 to 160, 140 to 150, and more preferably about 145 to 147 amino acids in length.

Conserved amino acid motifs, referred to herein as "consensus patterns" or "signature patterns", can be used to identify TANGO 212 family members having a MAM domain. For example, the following signature pattern can be used to identify TANGO 212 family members: G - x - [LIVMFY] (2) - x (3) - [STA] - x (10, 11) - [LV] - x (4,6)-[LIVMF] - x (6, 7) - C - [LIVM] - x (3) - [LIVMFY] - x (3,4) - [GSC] (SEQ ID NO:51). The signature patterns or consensus patterns described herein are described according to the following designations: all amino acids are indicated according to their universal single letter designation; "x" designates any amino acid; x(n) designates "n" number of amino acids, e.g., x (2) designates any two amino acids, e.g., x (6, 7) designates any six to seven amino acids; and, amino acids in brackets indicates any one of the amino acids within the brackets, e.g., [STA] indicates any of one of either S (serine), T (threonine) or A (alanine). TANGO 212 has such a signature pattern at about amino acids 431 to 472 of SEQ ID NO:12 (SBQ ID NO:115).

A MAM domain further contains at least about 2 to 6, preferably, 3 to 5, more preferably 4 conserved cysteine residues. By alignment of a TANGO 212 family member with a MAM consensus sequence, conserved cysteine residues can be found. For example, as shown in Figure 23, there is a first cysteine residue in the MAM consensus sequence that corresponds to a cysteine residue at amino acid 402 of TANGO 212 (SBQ ID NO:12); there is a second cysteine residue in the MAM consensus sequence that corresponds to a cysteine residue at amino acid 409 of TANGO 212 (SEQ ID N0:12); there is a third cysteine residue in the MAM consensus sequence that corresponds to a cysteine residue at amino acid 463 of TANGO 212 (SBQ ID NO:I2); and/or there is a fourth cysteine residue in the MAM consensus sequence that corresponds to a cysteine residue at amino acid 544 of TANGO 212 (SEQ ID NO:12). The MAM consensus sequence is available from the HMMer version 2.0 software as Accession Number PF00629. Software for HMM-based profiles is available from http://www.csc.ucsc.edu/research/compbio/sam.html and from http://genome.wustl.edu/eddy/hmmer.html.

Also included within the scope of the present invention are TANGO 212 proteins having a signal sequence. As used herein, a signal sequence includes a peptide of at least about 15 or 20 amino acid residues in length which occurs at the N-terminus of secretory and membrane-bound proteins and which contains at least about 75% hydrophobic amino acid residues such as alanine, leucine, isoleucine, phenylalanine, proline, tyrosine, tryptophan, or valine. In a preferred embodiment, a signal sequence contains at least about 15 to 40 amino acid residues, preferably about 15-30 amino acid residues, and has at least about 65-85%, more preferably 70-80%, and more preferably at least about 75% hydrophobic residues. A signal sequence serves to direct a protein containing such a sequence to a lipid bilayer.

In certain embodiments, a TANGO 212 family member has the amino acid sequence of SEQ ID NO:12, and the signal sequence is located at amino acids 1 to 16, 1 to 17, 1 to 18, 1 to 19, or 1 to 20. In such embodiments of the invention, the domains and the mature protein resulting from cleavage of such signal peptides are also included herein. For example, the cleavage of a signal sequence consisting of amino acids 1 to 18 of SEQ ID NO:12 (SEQ ID NO:25) results in a mature TANGO 212 protein corresponding to amino acids 19 to 553 of SBQ ID NO:12 (SEQ ID NO:31). The signal sequence is normally cleaved during processing of the mature protein.

In one embodiment, a TANGO 212 protein of the invention includes at least one EGF domain, preferably two, three, four, or five EGF domains and a MAM domain. In another embodiment, a TANGO 212 protein of the invention includes at least one EGF domain, preferably two, three, four, or five EGF domains, a MAM domain, a signal sequence, and is secreted.

Various features of human and mouse TANGO 212 are summarized below.

### Human TANGO 212

A cDNA encoding human TANGO 212 was identified by screening a human fetal lung library. A clone, comprising TANGO 212, was selected for complete sequencing based on its ability to direct the secretion of a protein of approximately 30 kDa in ³⁵-S labeled supernatants of 293T cells.

TANGO 212 includes a 2435 nucleotide cDNA (Figure 5; SEQ ID NO:11). It is noted that the nucleotide sequence depicted in SEQ ID NO:11 contains *Sal I* and *Not I* adapter sequences on the 5' and 3' ends, respectively ((GTCGACCCACGCGTCCG (SEQ ID NO:), and GGGCGGCCGC (SEQ ID NO:), respectively). Thus, it is to be understood that the nucleic acid molecules of the invention include not only those sequences with such adaptor sequences but also the nucleic acid sequences described herein lacking the adaptor sequences. The open reading frame of this cDNA, nucleotides 269 to 1927 (SEQ ID NO:13), encodes a 553 amino acid secreted protein (Figure 5; SEQ ID NO:12).

The signal peptide prediction grogram SIGNALP (Nielsen et al. (1997) *Protein Engineering* 10:1-6) predicted that human TANGO 212 includes an 18 amino acid signal peptide (amino acids 1 to about amino acid 18 of SEQ ID NO:12) (SEQ ID NO:25) preceding the mature TANGO 212 protein (corresponding to about amino acid 19 to amino acid 553 of SEQ ID NO:12)(SEQ ID NO:31). Human TANGO 212 is predicted to have a molecular weight of approximately 61 kDa prior to cleavage of its signal peptide and a molecular weight of approximately 59 kDa subsequent to cleavage of its signal peptide. In addition, gel analysis of³⁵-S labeled supernatants of 293T cells transfected with TANGO 212 expression plasmid identified a band at approximately 30 kDa. Thus, further processing of human TANGO 212 is likely to occur.

Secretion of TANGO 212 was detected by transfection using SPOT analysis (SignalP Optimized Tool, or "SPOT"). Briefly, SPOT based analysis was performed using software (termed developed to identify signal peptide encoding RNAs, all forward orientation open reading frames in the DNA sequences and phrap (see http://bozeman.mbt.washington.edu/ phrap.docs/phrap.html) pre-assembled DNA sequences from the library, starting with ATG and continuing for at least 19 non-stop codons, were translated. Signal peptides in the translated sequences were then predicted using the computer algorithm SignalP (Nielsen, H. et al.(1997) Protein Engineering 10:1-6), and those sequences scoring YES were saved. Open reading frames containing signal peptides with fewer than 20 amino acids after the predicted cleavage site were discarded. The translated sequences scoring YES in the SignalP analysis were then compared against a non-redundant protein database using BLAST 1.4, PAM10 matrix with score cut-offs (parameters S and S2) set to 150. Translated sequences with a match under these conditions were discarded.

Human TANGO 212 includes five EGF domains from about amino acids 61 to 91 (SBQ ID NO:110), amino acids 98 to 132 (SEQ ID NO:111), amino acids 138 to 172 (SEQ ID NO:112), amino acids 178 to 217 (SEQ ID NO:113), and amino acids 223 to 258 of SEQ ID NO:12 (SEQ ID NO:114). Human TANGO 212 further includes a MAM domain (about amino acids 400 to 546 of SEQ ID NO:12)(SEQ ID NO:116).

A clone, EpDH202, which encodes human TANGO 212 was deposited with the American Type Culture Collection (ATCC, 10801 University Boulevard, Manassas, VA 20110-2209) on September 10, 1998 and assigned Accession Number 202171. This deposit will be maintained under the terms of the Budapest Treaty on the International Recognition of the Deposit of Microorganisms for the Purposes of Patent Procedure. This deposit was made merely as a convenience to those of skill in the art and is not an admission that a deposit is required under 35 U.S.C. §112.

Figure 13 depicts a hydropathy plot of human TANGO 212. Relatively hydrophobic residues are above the horizontal line, and relatively hydrophilic residues are below the horizontal line. As shown in the hydropathy plot, the hydrophobic region at the beginning of the plot which corresponds to about amino acids 1 to 18 of SEQ ID NO:12 is the signal sequence of TANGO 212 (SEQ ID NO:25), cleavage of which yields the mature protein of lengtht 19-553 (SBQ ID NO:31). The cysteine residues (cys) and potential N-glycosylation sites (Ngly) are indicated by short vertical lines just below the hydropathy trace.

Northern analysis of human TANGO 212 mRNA expression revealed that is expressed at a very high level in placenta, strong levels in fetal lung and kidney, and at a low level in adult lung. No expression was seen in adult heart, liver, brain, skeletal muscle, kidney, pancreas, spleen, thymus, prostate, testis, ovary, small intestine, colon, peripheral blood leukocytes, or fetal brain and liver.

### Mouse TANGO 212

A mouse homolog of human TANGO 212 was identified. A cDNA encoding mouse TANGO 212 was identified by analyzing the sequences of clones present in a mouse osteoblast LPS stimulated cDNA library. This analysis led to the identification of a clone, jtmoa103g01, encoding mouse TANGO 212. The murine TANGO 212 cDNA of this clone is 1180 nucleotides long (Figure 28; SEQ ID NO:59). The open reading frame of this cDNA, comprises nucleotides 180 to 1179 of SEQ ID NO:59 (SEQ TD NO:61), and encodes a polypeptide comprising the 334 amino acid secreted sequence depicted in Figure 28 (SEQ ID NO:60).

In situ tissue screening was performed on mouse adult and embryonic tissue to analyze for the expression of mouse TANGO 212 mRNA. Of the adult tissues tested, only the renal medulla (kidney and medullary collecting tubules) was positive. Expression was observed primarily in the embryo. Signal was observed at E13.5 in the lung, skin (especially the upper lip), diaphragm, and muscle of the abdominal cavity and skin. This pattern remained through E18.5 with increasing lung expression. Muscle expression was still apparent at E18.5 but decreased to near background levels by postnatal day 1.5 with residual expression in the upper lip. No signal was detected in the following tissues: lung, diaphragm (smooth muscle), heart, liver, pancreas, thymus, eye, brain, bladder, small intestine, skeletal muscle, colon, placenta. In the case of embryonic mouse expression during the period of E13.5 through E16.5, expression was observed in the skin - especially upper lip/snout area, in the lung-multifocal at 13.5 but became more ubiquitous and more intense, muscle and diaphragm, skin, limbs (especially 13.5 and 14.5), and the abdominal wall. At E18.5, the expression observed was the same as for 13.5 through 16.5 but decreasing in muscle and skin (except upper lip). At P1.5, the expression signal decreased to almost background levels except in the upper lip.

Human and murine TANGO 212 sequences exhibit considerable similarity at the protein, nucleic acid, and open reading frame levels. An alignment (made using the ALIGN software {Myers and Miller (1989) CABIOS, ver. 2.0}; BLOSUM 62 scoring matrix; gap penalties -12/-4), reveals a protein identity of 77.2%. The human and murine TANGO 212 full length cDNAs are 80.5% identical, as assessed using the same software and parameters as indicated (without the BLOSUM 62 scoring matrix). In the respective ORFs, calculated in the same fashion as the full length cDNAs, human and murine TANGO 212 are 83.3% identical.

### Use of TANGO 212 Nucleic Acids, Polypeptides, and Modulators Thereof

The TANGO 212 proteins of the invention comprise a family of proteins having the hallmarks of a secreted protein of the EGF family. Accordingly, TANGO 212 proteins likely function in a similar manner as members of the EGF family. Thus, TANGO 212 modulators can be used to treat EGF-associated disorders.

For example, the TANGO 212 proteins likely play a role in tissue regeneration and/or wound healing. *In vitro* studies with several members of the EGF family such as EGF and TGF-α have shown that these proteins influence a number of cellular processes involved in soft tissue repair leading to their categorization as wound hormones in wound healing. The affects of these proteins include cellular proliferation and chemotaxis. Thus, the TANGO 212 proteins of the invention likely affect various cells associated with wound healing. Effects that the TANGO 212 proteins have on various cells include proliferation and chemotaxis. Accordingly, the TANGO 212 proteins, nucleic acids and/or modulators of the invention are useful in the treatment of wounds and/or the modulation of proliferative disorders, e.g., cancer.

Because TANGO 212 is expressed in the kidney, the TANGO, 212 polypeptides, nucleic acids and/or modulators thereof can be used to modulate the function, morphology, proliferation and/or differentiation of cells in the tissues in which it is expressed. Such molecules can also be used to treat disorders associated with abnormal or aberrant metabolism or function of cells in the tissues in which it is expressed. Such molecules can be used to treat or modulate renal (kidney) disorders, such as glomerular diseases (e.g., acute and chronic glomerulonephritis, rapidly progressive glomerulonephritis, nephrotic syndrome, focal proliferative glomerulonephritis, glomerular lesions associated with systemic disease, such as systemic lupus erythematosus, Goodpasture's syndrome, multiple myeloma, diabetes, neoplasia, sickle cell disease, and chronic inflammatory diseases), tubular diseases (e.g., acute tubular necrosis and acute renal failure, polycystic renal diseasemedullary sponge kidney, medullary cystic disease, nephrogenic diabetes, and renal tubular acidosis), tubulointerstitial diseases (e.g., pyelonephritis, drug and toxin induced tubulointerstitial nephritis, hypercalcemic nephropathy, and hypokalemic nephropathy) acute and rapidly progressive renal failure, chronic renal failure, nephrolithiasis, vascular diseases (e.g., hypertension and nephrosclerosis, microangiopathic hemolytic anemia, atheroembolic renal disease, diffuse cortical necrosis, and renal infarets), or tumors (e.g., renal cell carcinoma and nephroblastoma).

### TANGO 213

In another aspect, the present invention is based on the discovery of cDNA molecules which encode a novel family of proteins having sequence similarity to progesterone binding protein, referred to herein as TANGO 213 proteins.

The TANGO 213 proteins and nucleic acid molecules comprise a family of molecules having certain conserved structural and functional features. As used herein, the term "family" is intended to mean two of more proteins or nucleic acid molecules having a common structural domain and having sufficient amino acid or nucleotide sequence identity as defined herein. Family members can be from either the same or different species. For example, a family can comprises two or more proteins of human origin or can comprise one or more proteins of human origin and one or more of non-human origin. Members of the same family may also have common structural domains.

Also included within the scope of the present invention are TANGO 213 proteins having a signal sequence. As used herein, a signal sequence includes a peptide of at least about 15 or 20 amino acid residues in length which occurs at the N-terminus of secretory and membrane-bound proteins and which contains at least about 70% hydrophobic amino acid residues such as alanine, leucine, isoleucine, phenylalanine, proline, tyrosine, tryptophan, or valine. In a preferred embodiment, a signal sequence contains at least about 15 to 40 amino acid residues, preferably about 15-30 amino acid residues, and has at least about 60-80%, more preferably 65-75%, and more preferably at least about 70% hydrophobic residues. A signal sequence serves to direct a protein containing such a sequence to a lipid bilayer.

In certain embodiments, a TANGO 213 family member has the amino acid sequence of SEQ ID NO:15, and the signal sequence is located at amino acids 1 to 20, 1 to 22, 1 to 22, or 1 to 23. In such embodiments of the invention, the domains and the mature protein resulting from cleavage of such signal peptides are also included herein. For example, the cleavage of a signal sequence consisting of amino acids 1 to 22 of SEQ ID NO:15 (SEQ ID NO:26) results in a mature TANGO 213 protein corresponding to amino acids 23 to 371 of SEQ ID NO:12 (SEQ ID NO:32). The signal sequence is normally cleaved during processing of the mature protein.

In particular, BLASTP analysis using the amino acid sequence of TANGO 213 (SEQ ID NO:15) revealed sequence similarity between TANGO 213 and several steroid binding-proteins including 51% sequence identity between TANGO 213 and human progesterone binding protein (GenBack Accession No. Y12711). Thus, the TANGO 213 proteins of the invention are likely to function similarly to steroid binding-proteins. Steroid binding protein activities include the ability to form protein-protein interactions with steroid hormones in signaling pathways, and/or the ability to modulate intracellular ion levels, e.g., sodium and/or calcium levels. Accordingly, TANGO 213 proteins, nucleic acids and/or modulators can be used to treat steroid binding protein-associated disorders.

Various features of human and mouse TANGO 213 are summarized below.

### HUMAN TANGO 213

A cDNA encoding human TANGO 213 was isolated by screening a human mesangial cell library, Human TANGO 213 includes a 1496 nucleotide cDNA (Figure 6; SEQ ID NO:14). It is noted that the nucleotide sequence depicted in SEQ ID NO:14 contains *Sal I* and *Not I* adapter sequences on the 5' and 3' ends, respectively ((GTCGACCCACGCGTGCG (SEQ ID NO:), and GGGCGGCCGC (SEQ ID NO:), respectively), Thus, it is to be understood that the nucleic acid molecules of the invention include not only those sequences with such adaptor sequences but also the nucleic acid sequences described herein lacking the adaptor sequences. The open reading frame of this cDNA, nucleotides 58 to 870 (SEQ ID NO:16), encodes a 271 amino acid secreted protein (Figure 6; SEQ ID NO:15).

The signal peptide prediction program SIGNALP (Nielsen et al. (1997) *Protein Engineering* 10:1-6) predicted that human TANGO 213 includes a 22 amino acid signal peptide (amino acids 1 to about amino acid 22 of SEQ ID NO:15)(SEQ ID NO:26) preceding the mature TANGO 213 protein (corresponding to about amino acid 23 to amino acid 271 of SEQ ID NO:15)(SEQ ID NO:32). Human TANGO 213 is predicted to have a molecular weight of approximately 29.5 kDa prior to cleavage of its signal peptide and a molecular weight of approximately 27.5 kDa subsequent to cleavage of its signal peptide.

A clone, EpDH156, which encodes human TANGO 213 was deposited with the American Type Culture Collection (ATCC, 10801 University Boulevard, Manassas, VA 20110-2209) on October 30,1998 and assigned Accession Number 98965. This deposit will be maintained under the terms of the Budapest Treaty on the International Recognition of the Deposit of Microorganisms for the Purposes of Patent Procedure. This deposit was made merely as a convenience to those of skill in the art and is not an admission that a deposit is required under 35 U.S.C. §112.

Figure 14 depicts a hydropathy plot of human TANGO 213. Relatively hydrophobic residues are above the horizontal line, and relatively hydrophilic residues are below the horizontal line. As shown in the hydropathy plot, the hydrophobic region at the beginning of the plot which corresponds to about amino acids 1 to 22 of SEQ ID NO:15 is the signal sequence of TANGO 213 (SEQ ID NO:26). The cysteine residues (cys) and potential N-glycosylation sites (Ngly) are indicated by short vertical lines just below the hydropathy trace.

Northern analysis of human TANGO 213 mRNA expression revealed expression at a very high level in testis and kidney. Expression at lower levels was also seen in all other tissues including adult heart, liver, brain, skeletal muscle, kidney, pancreas, spleen, thymus, prostate, ovary, small intestine, colon, and peripheral blood leukocytes. Low levels of expression were observed in lung.

The human gene for TANGO 213 was mapped on radiation hybrid panels to the long arm of chromosome 17, in the region p13.3. Flanking markers for this region are WI-5436 and WI-6584. The MDCR (Miller-Dicker syndrome), PEDF (pigment epithelium derived factor), and PFN1(profillin 1) genes also map to this region of the human chromosome. This region is syntenic to mouse chromosome 11, locus 46(g), The ti (tipsy) loci also maps to this region of the mouse chromosome. The pfn1 (profilin 1), htt (5-hydroxytryptamine (serotonin) transporter), acrb (acetylcholine receptor beta) genes also map to this region of the mouse chromosome.

### Mouse and Rat TANGO 213

A mouse homolog of human TANGO 213 was identified. A cDNA encoding mouse TANGO 213 was identified by analyzing the sequences of clones present in a mouse testis cDNA library. This analysis led to the identification of a clone, jtmz213a01, encoding mouse TANGO 213, The murine TANGO 213 cDNA of this clone is 2154 nucleotides long (Figure 29; SEQ ID NO:62). It is noted that the nucleotide sequence depicted in SEQ ID NO:62 contains a *Not I* adapter sequence on the 3' end (5' GGGCGGCCGC 3')(SEQ ID NO:), respectively). Thus, it is to be understood that the nucleic acid molecules of the invention include not only those sequences with such adaptor sequences but also the nucleic acid sequences described herein lacking the adaptor sequences. The open reading frame of this cDNA comprises nucleotides 41 to 616 of SEQ ID NO:62 (SEQ ID NO:64) and encodes a protein comprising the 192 amino acid sequence protein depicted in Figure 29 (SEQ ID NO:63).

A rat homolog of human TANGO 213 was identified. A cDNA encoding rat TANGO 213 was identified by analyzing the sequences of clones present in a rat testis cDNA library. This analysis led to the identification of a clone encoding rat TANGO 213. The rat TANGO 213 cDNA of this clone is 455 nucleotides long (Figure 33; SEQ ID NO:).

In situ tissue screening was performed on mouse adult and embryonic ussue to analyze for the expression of mouse TANGO 213 mRNA. The strongest expression was observed in the seminiferous tubules of the testes. Moderate or weak expression is observed in several other adult tissues including the liver, kidney, and placenta. A weak, ubiquitous signal was observed in brain, heart, liver, kidney, adrenal gland, and the spleen. A signal was observed in the ovaries. A ubiquitous signal was seen in the labyrinth zone and slightly higher signal in the zone of giant cells. No signal was detected in the following tissues: spinal cord, eye and harderian gland, submandibular gland, white fat, brown fat, stomach, lung, colon, small intestine, thymus, lymph node, pancreas, skeletal muscle, and bladder. Embryonic expression is negligible. A weak signal was observed in the developing liver and CNS. The signal in the CNS was near background levels. Specifically, at B13.5, a weak, ubiquitous signal observed in the liver. At E14.5 and E15.5, a weak, ubiquitous signal was observed in the liver, brain, and spinal cord. At E16.5, B18.S and P1.5, the signal in liver and CNS was even less pronounced and was almost at background levels, Library array expression studies were earned out as descnbcd above for mouse TANGO 197. Strong expression was detected in the choroid plexus 12.5 day whole mouse embryo, TM4 (Sertoli cells), from testis, esophagus, and kidney fibrosis library. Weak expression was detected in LPS-stimulated osteoblast tissue, 10.5 day whole mouse embryo, and in 11.5 day whole mouse embryo. No expression was detected in differential 3T3, 10.5 day mouse fetus, mouse kidney fibrosis model nephrotoxic serum (NTS), LPS-stimulated heart, LPS-stimulated osteoblasts, lung, mouse insulinoma (Nit-1), mouse normal/hyperplastic islets (pancreas), normal spleen, 11.5 day mouse, LPS-stimulated lung, Lung, LPS-stimulated osteoblasts, BL6 Lung, day 15, 3 hour inflammation model, BDL Day 10 (balb C liver), hypertropic heart, LPS-stimulated lung, LPS-stimulated kidney, LPS-stimulated lymph node, Balb C liver (bile duct ligation d2), mc/9 mast cells, 13.5 day mouse, LPS-stimulated anchored heart, normal thymus, Th2-ovarian-Tg, Balb C liver (bile duct ligation d2), mc/9 mast cells, normal heart, brain polysome (MPB), LPS-stimulated anchored liver, brain (EAE d10 model), th1-ovarian-Tg, heart, hypothalamus, lone term bone, marrow cells, LPS-stimulated lung, megakaryocyte, LPS-stimulated spleen, hyphae-stimulated long term bone marrow, lung, angiogenic pancreatic islets, Th2, brain, LPS-stimulated thymus, LPS-stimulated microglial cells, testes, tumor pancreatic islets, LPS-stimulated brain, LPS-stimulated alveolar macrophage cell line, mouse lung bleomycin model d7, pregnant uterus, and hypothalamus nuclei.

Human and murine TANGO 213 sequences exhibit considerable similarity at the protein, nucleic acid, and open reading frame levels. An alignment (made using the ALIGN software {Myers and Miller (1989) CABIOS, ver. 2.0}; BLOSUM 62 scoring matrix; gap penalties -12/-4), reveals a protein identity of 64.6%. The human and murine TANGO 213 full length cDNAs are 68.8% identical, as assessed using the same software and parameters as indicated (without the BLOSUM 62 scoring matrix). In the respective ORFs, calculated in the same fashion as the full length cDNAs, human and murine TANGO 213 are 77.1% identical.

### Uses of TANGO 213 Nucleic Acids, Polypeptides, and Modulators Thereof

The TANGO 213 proteins and nucleic acid molecules of the invention have at least one "TANGO 213 activity" (also referred to herein as "TANGO 2 13 biological activity").

TANGO 213 activity refers to an activity exerted by a TANGO 213 protein or nucleic acid molecule on a TANGO 213 responsive cell *in vivo* or *in vitro*. Such TANGO 213 activities include at least one or more of the following activities: 1) interaction of a TANGO 213 protein with a TANGO 213-target molecule; 2) activation of a TANGO 213 target molecule; 3) modulation of cellular proliferation; 4) modulation of cellular differentiation; or 5) modulation of a signaling pathway. Thus, the TANGO 213 proteins, nucleic acids and/or modulators can be used for the treatment of a disorder characterized by aberrant TANGO 213 expression and/or an aberrant TANGO 213 activity, such as proliferative and/or differentiative disorders.

As TANGO 213 is expressed in the kidney, the TANGO 213 polypeptides, nucleic acids and/or modulators thereof can be used to modulate the function, morphology, proliferation and/or differentiation of cells in the tissues in which it is expressed. Such molecules can also be used to treat disorders associated with abnormal or aberrant metabolism or function of cells in the tissues in which it is expressed. Such can be used to treat or modulate renal (kidney) disorders, such as glomerular diseases (e.g., acute and chronic glomerulonephritis, rapidly progressive glomerulonephritis, nephrotic syndrome, focal proliferative glomerulonephritis, glomerular lesions associated with systemic disease, such as systemic lupus crythematosus, Goodpasture's syndrome, multiple myeloma, diabetes, neoplasia, sickle cell disease, and chronic inflammatory diseases), tubular diseases (e.g., acute tubular necrosis and acute renal failure, polycystic renal disease, medullary sponge kidney, medullary cystic disease, nephrogenic diabetes, and renal tubular acidosis), tubulointerstitial diseases (e.g., pyelonephritis, drug and toxin induced tubulointerstitial nephritis, hypercalcemic nephropathy, and hypokslemie nephropathy) acute and rapidly progressive renal failure, chronic renal failure, nephrolithiasis, vascular diseases (e.g., hypertension and nephrosclerosis, microangiopathic hemolytic anemia, atheroembolic renal disease, diffuse cortical necrosis, and renal infarcts), or tumors (e.g., renal cell carcinoma and nephroblastoma).

Furthermore, as TANGO 213 is expressed in the testis, the TANGO 213 polypeptides, nucleic acids and/or modulators thereof can be used to modulate the function, morphology, proliferation and/or differentiation of cells in the tissues in which it is expressed. For example, such molecules can be used to treat or modulate disorders associated with the testis including, without limitation, the Klinefelter syndrome (both the classic and mosaic forms), XX male syndrome, variococele, germinal cell aplasia (the Sertoli cell-only syndrome), idiopathic azoospermia or severe oligospermia, crpytochidism, and immotile cilia syndrome, or testicular cancer (primary germ cell tumors of the testis). In another example, TANGO 213 polypeptides, nucleic acids, or modulators thereof, can be used to treat testicular disorders, such as unilateral testicular enlargmant (e.g., nontuberculous, granulomatous orchitis), inflammatory diseases resulting in testicular dysfunction (e.g., gonorrhea and mumps), and tumors (e.g., germ cell tumors, interstitial cell turnors, androblastoma, testicular lymphoma and adenomatoid tunors).

### TANGO 224

In another aspect, the present invention is based on the discovery of cDNA molecules which encode a novel family of proteins referred to herein as TANGO 224 proteins.

The TANGO 224 proteins and nucleic acid molecules comprise a family of molecules having certain conserved structural and functional features. As used herein, the term "family" is intended to mean two or more proteins or nucleic acid molecules having a common structural domain and having sufficient amino acid or nucleotide sequence identity as defined herein. Family members can be from either the same or different species. For example, a fmnily can comprises two or more proteins of human origin, or can comprise one or more proteins of human origin and one or more of non-human origin. Members of the same family may also have common structural domains.

For example, the TANGO 224 proteins of the invention include a thrombospondin type I (TSP-I) domain. The TSP-I domain is involved in the binding to both soluble and matrix macromolecules (e.g,, sulfated glycoconjugates). As used herein, a thrombospondin type I (TSP-I) domain refers to an amino acid sequence of about 30 to about 60, preferably about 35 to 55, 40 to 50, and more preferably about 45 amino acids in length. TANGO 224 has such a signature pattern at about amino acids 42 to 81 of SEQ ID NO:18 (SEQ ID NO:117).

Conserved amino acid motifs, referred to herein as "consensus patterns" or "signature patterns", can be used to identify TANGO 224 family members having a TSP-1 domain. For example, the following signature pattern can be used to identify TANGO 224 family members: W - S - x - C [SD] - x (2) - C - x (2) - G - x (3, 5) - R - x (7, 15) - C - x (9, 11) - C - x (4, 5) - C (SEQ ID NO:52). The signature patterns or consensus patterns described herein are described according to the following designations: all amino acids are indicated according to their universal single letter designation; "x" designates any amino acid; x(n) designates "n" number of amino acids, e.g., x (2) designates any two amino acids, e.g., x (3, 5) designates any three to five amino acids; and, amino acids in brackets indicates any one of the amino acids within the brackets, e.g., [SD] indicates any of one of either S (serine) or D (aspartic acid). A TSP-I domain of TANGO 224 extends, for example, from about amino acids 37 to 81 of SEQ ID NO:18 (SEQ ID NO:118).

A TSP-I domain further contains at least about 4 to 9, preferably, 5 to 8, more preferably 6 conserved cysteine residues. By alignment of a TANGO 224 family member with a TSP-I consensus sequence, conserved cysteine residues can be found. For example, as shown in Figure 24, there is a first cysteine residue in the TSP-I consensus sequence that corresponds to a cysteine residue at amino acid 45 of TANGO 224 (SEQ ID NO:18); there is a second cysteine residue in the TSP-I consensus sequence that corresponds to a cysteine residue at amino acid 49 of TANGO 224 (SBQ ID NO:18); there is a third cysteine residue in the TSP-I consensus sequence that corresponds to a cysteine residue at amino acid 60 of TANGO 224 (SEQ ID NO:18); there is a fourth cysteine residue in the TSP-I consensus sequence that corresponds to a cystein residue at amino acid 66 of TANGO 224 (SEQ ID NO:18); there is a fifth cysteine reside in the TSP-I consensus sequence that corresponds to a cysteine residue at amino acid 76 of TANGO 224 (SEQ ID NO:18); and/or there is a sixth cysteine residue in the TSP-I consensus sequence that corresponds to a cysteine residue at amino acid 81 of TANGO 2m24 (SEQ ID NO:18). The TSP-I consensus sequence is available from the HMMer version 2.0 software as Accession Number PF00090, Software for HMM-based profiles is available from http://www.csc.ucsc.edu/research/ compbio/sam.html and from http://genome.wustl.edu/eddy/hmmer.html.

For example, the TANGO 224 proteins of the invention include a Furin-like cysteine rich domain (Accession number:PF00757). The consensus sequence for the Furin-like cysteine rich domain is: C-Xaa(3)-C-Xaa-G-G-Xaa(n)-C-Xaa(5)-D-G, wherein C is cysteine, Xaa is any amino acid, G is glycine, n is about 5 to 15, preferably 6 to 14, more preferably about 7 to 12, and D is aspartic acid. As used herein, a Furin-like cysteine rich domain refers to an amino acid sequence of about 80 to 160, preferably of about 100 to 150, and more preferably about 110 to 130, amino acids in length. Human TANGO 224, form 2 has such a signature pattern at about amino acids 707-829 of SEQ ID NO:66 (SEQ ID NO:).

Also included within the scope of the present invention are TANGO 224 proteins having a signal sequence. As used herein, a signal sequence includes a peptide of at least about 15 or 20 amino acid residues in length which occurs at the N-terminus of secretory and membrane-bound proteins and which contains at least about 70% hydrophobic amino acid residues such as alanine, leucine, isoleucine, phenylalsnine, proline, tyrosine. tryptophan, or valine. In a preferred emboditnent, a signal sequence contains at least about 15 to 40 amino acid residues, preferably about 15-30 amino acid residues, and has at least about 60-80%, more preferably 65-75%, and more preferably at least about 70% hydrophobic residues. A signal sequence setves to direct a protein containing such a sequence to a lipid bilayer.

In certain embodiments, a TANGO 224 family member has the amino acid sequence of SEQ ID NO:18, and the signal Sequence is located at amino acids to 26, 1 to 27,1 to 28,1 to 29 or 1 to 30. In such embodiments of the invention, the domains and the mature protein resulting from cleavage of such signal peptides are also included herein. For example, the cleavage of a signal sequence consisting of amino acids 1 to 28 of SEQ ID NO:18 (SEQ ID NO:27) results in a mature TANGO 224 protein corresponding to amino acids 29 to 458 of SEQ ID NO:18 (SEQ ID NO:33). The signal sequence is normally cleaved during processing of the mature protein.

A cDNA encoding human TANGO 224 was identified by screening a human fetal spleen library. A clone comprising human TANGO 224 was selected for complete sequencing. In one embodiment, TANGO 224 is referred to as TANGO 224, form 1. Human TANGO 224, form 1 comprises a 2689 nucleotide cDNA (Figure 7; SEQ ID NO:17). The open reading frame of this TANGO 224, form 1 cDNA clone comprises nucleotides 1 to 1440 (SEQ ID NO:19), and encodes a secreted protein comprising the 480 amino acid sequence depicted in Figure 7 (SEQ ID NO:18).

Another cDNA clone comprising human TANGO 224, was also obtained. TANGO 224 clone includes a 2691 nucleotide cDNA (Figure 30; SEQ ID NO:65), and encodes a human TANGO 224 and is referred to as human TANGO 224, form 2. The open reading frame of human TANGO 224, form 2 cDNA clone comprises nucleotides 67 to 2690 (SEQ ID NO:67), and encodes a secreted protein comprising the 874 amino acid sequence depicted in Figure 30 (SEQ ID NO:66).

The signal peptide prediction program SIGNALP (Nielsen et al. (1997) *Protein Engineering* 10:1-6) predicted that human TANGO 224 form 1 includes an 28 amino acid signal peptide (amino acids 1 to about amino acid 28 of SEQ ID NO:18) (SEQ ID NO:27) preceding the mature TANGO 224 protein (corresponding to about amino acid 29 to amino acid 458 of SEQ ID NO:18)(SEQ ID NO:33). Human TANGO 224 is predicted to have a molecular weight of approximately 50 kDa prior to cleavage of its signal peptide and a molecular weight of approximately 47 kDa subsequent to cleavage of its signal peptide.

The signal peptide prediction pro graift SIGNALP (Nielsen ct al, (1997) *Protein Engineering* 10:1-6) predicted that human TANGO 224 form 2 includes an 28 amino acid signal peptide (amino acids 1 to about amino acid 28 of SEQ ID NO:18)(SEQ ID NO:27) preceding the mature TANGO 224, form 2 protein (corresponding to about amino acid 29 to amino acid 874 of SEQ ID NO:18)(SEQ ID NO:). Human TANGO 224 is predicted to have a molecular weight of approximately 131 kDa prior to cleavage of its signal peptide and a molecular weight of approximately 127 kDa subsequent to cleavage of its signal peptide.

Human TANGO 224, form 1 includes a TSP-I domain from about amino acids 37 to 81 of SEQ ID NO:18 (SEQ ID NO:118).

Human TANGO 224, form 2 includes a TSP-I domain from about amino acids 37 to 81 of SEQ ID NO:18 (SEQ ID NO:118). Human TANGO 224, form 2 has a Furin-like cysteine rich domain from amino acids 707 to 829 of SEQ ID NO:66 (SEQ ID NO:).

A clone, EpDH210, which encodes human TANGO 224, form 1 was deposited with the American Type Culture Collection (ATCC, 10801 University Boulevard, Manassas, VA 20110-2209) on October 30,1998 and was assigned Accession Number 98966. This deposit will be maintained under the terms of the Budapest Treaty on the International Recognition of the Deposit of Microorganisms for the Purposes of Patent Procedure. This deposit was made merely as a convenience to those of skill in the at and is not an admission that a deposit is required under 35 U.S.C. §112.

Figure 15 depicts a hydropathy plot of human TANGO 224. Relatively hydrophobic residues are above the horizontal line, and relatively hydrophilic residues are below the horizontal line. As shown in the hydropathy plot, the hydrophobic region at the beginning of the plot which corresponds to about amino acids 1 to 28 of SEQ ID NO:18 is the signal sequence of TANGO 224 (SEQ ID NO:27). The cysteine residues (cys) and potential N-glycosylanon sites (Ngly) are indicated by short vertical lines just below the hydropathy trace.

Northern analysis of human TANGO 224 mRNA expression using TANGO 224 form 2 nucleotide sequence as a probe revealed expression of TANGO 224 mRNA in the spleen, prostate, ovary and colon. Only weak expression was detected in testis, small intestine, and peripheral blood leukocytes. No expression was detected in the thymus.

Library Array Expression studies were performed as described above for the mouse TANGO 128 gene, except that human tissues were tested. Strong expression was obtained in the pituitary and fetal spleen. Only weak expression was detected in the primary osteoblasts, umbilical smooth muscle treated and the bronchial smooth muscle. No expression was detected in kidney, testes, Prostate, HMC-1 control (mast cell line), fetal dorsal spinal cord, human colon to liver metastasis, erythroblasts from CD34+ Blood, human spinal cord (ION 3), HUVEC TGF-B (h. umbilical endothelia), hUVEC (h. umbilical endothalia), human spinal cord (ION 3), brain K563 (red blood cell line), uterus, Hep-G2 (human insulinvma), human normal colon, human colon to liver metastasis, skin, D HUVEC controls (umbilical endothelial cells), human colon (inflammatory bowel disease), melanoma (G361 cell line), adult bone arrow CD34+ cells, HPK; human lung, mammary gland, normal breast epithelium, colon to liver metastasis (CHT128), normal breast, bone marrow (CD34+), W138 (H. embryonic Lung), Th1 cells, HUVEC untreated (umbilical endothelium), liver, spleen, normal human ovarian epithelia, colon to liver metastasis 5 (CHT133), PTH-treated osteoblasts, ovarian ascites, lung squamous cell, carcinoma (MDA 261), Th2 cells, colon (WUM 23), thymus, heart, small intestine, normal megakaryoctyes, colon carcinoma (NDR109), lung adenocarcinoma (PIT245), IBD Colon (WUM6), brain-subcortical white matter (ION2), prostate tumor xenograft A12, trigeminal ganglia 9 week fetus, thymus, retinal pigmentosa epithelia, bone marrow, colon carcinoma (NDR103), lung squamous cell carcinoma (PIT299), cervical cancer, normal rostate, Prostate tumor xenograft K10, Lumbrosacaral spinal cord, A549 control, stomach, retina, Th-1 induced T cell, colon carcinoma (NDR82), d8 dendritic ells, spinal cord, ovarian epithelial tumor, prostate cancer to liver metastasis IHH3, lumbrosacaral dorsal root ganglia, salivary gland, skeletal muscle, HMC-1 (human mast cell line), Th-2 induced T-eell, colon carcinoma (NDR097), H6, megakaryocytes, H7, dorsal root ganglia (ION 6,7,8), H8. HUVEC L-NAME (umbilical endothelia), H9. prostate cancer to liver metastasis JHH4, H10. Dorsal root ganglia (ION 6, 7, 8),

### Use of TANGO 224 Nucleic Acids, Polypeptides, and Modulators Thereof

As discussed above, the TSP-I domain of TANGO 224 is involved in matrix interactions. Thus, the TANGO 224 proteins of the invention likely play a role in various matrix interactions, e.g., matrix binding. Thus, a TANGO 224 activity ia at least one or more of the following activities: 1) regulation of extracellular matrix structuring; 2) modulation of cellular adhesion, either *in vitro* or *in vivo*; 3) regulation of cell trafficking and/or migration. Accordingly, the TANGO 224 proteins, nucleic acid molecules and/or modulators can be used to modulate cellular interactions such as cell-cell and/or cell-matrix interactions and thus, to treat disorders associated with abnormal cellular interactions.

As TANGO 224 was originally found in a fetal spleen library,- TANGO 228 nucleic acids, proteins, and modulators thereof can be used to modulate the proliferation, differentiation, and/or function of cells that form the spleen, e.g., cells of the splenic connective tissue, e.g., splenic smooth muscle cells and/or endothelial cells of the splenic blood vessels. TANGO 224 nucleic acids, proteins, and modulators thereof can also be used to modulate the proliferation, differentiation, and/or function of cells that are processed, e.g., regenerated or phagocytized within the spleen, e.g., erythrocytes and/or B and T lymphocytes and macrophages. Thus, TANGO 224 nucleic acids, proteins, and modulators thereof can be used to treat spleen, e.g., the fetal spleen, associated diseases and disorders. Examples of splenic diseases and disorders include e.g., splenic lymphoma and/or splenomegaly, and/or phagocytotic disorders, e.g., those inhibiting macrophage engulfment of bacteria and viruses in the bloodstream.

### TANGO 239

In another aspect, the present invention is based on the discovery of cDNA molecules which encode a novel family of proteins referred to herein as TANGO 239 proteins.

The TANGO 239 proteins and nucleic acid molecules comprise a family of molecules having certain conserved structural and functional features. As used herein, the term "family" is intended to mean two or more proteins or nucleic acid molecules having a common structural domain and having sufficient amino acid or nuclootide sequence identity as defined herein. Family members can be from either the same or different species. For example, a family can comprises two or more proteins of human origin, or can comprise one or more proteins of human origin and one or more of non-human origin. Members of the same family may also have common structural domains.

For example, the present invention features TANGO 239 proteins having at least one, preferably two or three, MAM domain(s). The MAM domain is associated with various adhesive proteins and as such is likely to have adhesive function. Within MAM domains are conserved cysteine residues which play a role in the adhesion of a MAM domain to other proteins. As used herein, a MAM domain refers to an amino acid sequence of about 130 to about 170, preferably about 140 to 165, and more preferably about 145, 146 to 159 or 160 amino acids in length.

Conserved amino acid motifs, referred to herein as "consensus patterns" or "signature patterns", can be used to identify TANGO 239 family members having a MAM domain. For example, the following signature pattern can be used to identify TANGO 239 family members: G - x - [LIVMFY] (2) - x (3) - [STA] - x (10,11) - [LV] - x (4,6)-[LIVMF] - x (6, 7) - C - [LIVM] - x (3) - [LIVMFY] - x (3, 4) - [GSC] (SEQ ID NO:51). The signature patterns or consensus patterns described herein are described according to the following designations: all amino acids are indicated according to their universal single letter designation; "x" designates any amino acid; x(n) designates "n" number of amino acids, e.g., x (2) designates any two amino acids, e.g., x (6, 7) designates any six to seven amino acids; and, amino acids in brackets indicates any one of the amino acids within the brackets, e.g., [STA] indicates any of one of either S (serine), T (threonine) or A (alanine). TANGO 239 has such a signature pattern at about amino acids 50 to 90 (SEQ ID NO:119), amino acids 215 to 256 (SEQ ID NO:120) and/or amino acids 380 to 420 of SEQ ID NO:21 (SEQ ID NO:121).

A MAM domain further contains at least about 2 to 6, preferably, 3 to 5, more preferably 4 conserved cysteine residues. By alignment of a TANGO 239 family member with a MAM consensus sequence, conserved cysteine residues can be found. For example, as shown in Figure 25, there is a first cysteine residue in the MAM consensus sequence that corresponds to a cysteine residue at amino acid 26 of the first MAM domain of TANGO 239 (SEQ ID NO:21); there is a second cysteine residue in the MAM consensus sequence that corresponds to a cysteine residue at amino acid 33 of TANGO 239 (SEQ ID NO:21); there is a third cysteine residue in the MAM consensus sequence that corresponds to a cysteine residue at amino acid 80 of TANGO 239 (SEQ ID NO:21); and/or there is a fourth cysteine residue in the MAM consensus sequence that corresponds to a cysteine residue at amino acid 167 of TANGO 239 (SEQ ID NO:21). In addition, conserved cysteine residues can be found at amino acids 170, 178, 246 and/or 327 of the second MAM domain of TANGO 239 (SEQ ID NO:21); and at amino acids 342, 349, 411 and/or 496 of the third MAM domain of TANGO 239 (SEQ ID NO:21). The MAM consensus sequence is available from the HMMer version 2.0 software as Accession Number PF00629. Software for HMM-based profiles is available from http://www.csc.ucsc.edu/research/compbio /sam.html and from http://genomc.wustl.edu/eddy/hmmer.html. A MAM domain of TANGO 239 extends, for example, from about amino acids 26 to 169 of SEQ ID NO:21 (SEQ ID NO:122), from about amino acids 170 to 329 of SEQ ID NO:21 (SEQ ID NO:123), from about amino acids 342 to 498 of SEQ ID NO:21 (SEQ ID NO:124), and/or from about amino acids 509 to 666 of SEQ ID NO:21 (SEQ ID NO:).

Also included within the scope of the present invention are TANGO 239 proteins having a signal sequence. As used herein, a signal sequence includes a peptide of at least about 15 or 20 acid residues in length which occurs at the N-terminus of secretory and membrane-bound proteins and which contains at least about 70% hydrophobic amino acid residues such as alanine, leucine, isoleucine, phenylalanine, proline, tyrosine, tryptophan, or valine. In a preferred embodiment, a signal sequence contains at least about 15 to 40 amino acid residues, preferably about 15-30 amino acid residues, and has at least about 60-80%, more preferably 65-75%, aud more preferably at least about 70% hydrophobic residues. A signal sequence serves to direct a protein containing such a sequence to a lipid bilayer.

In certain embodiments, a TANGO 239 family member has the amino acid sequence of SEQ ID NO:21, and the signal sequence is located at amino acids 1 to 16,1 to 17, 1 to 18, 1 to 19, and 1 to 20. In such embodiments of the invention, the domains and the mature protein resulting from cleavage of such signal peptides are also included herein. For example, the cleavage of a signal sequence consisting of amino acids 1 to 18 of SEQ ID NO:21 (SEQ ID NO:) results in a mature TANGO 239 protein corresponding to amino acids 19 to 686 of SEQ ID NO:2 (SEQ ID NO:). The signal sequence is normally cleaved during processing of the mature protein.

Various features of human, TANGO 239, form 1 and form 2, and mouse TANGO are summarized below.

### HUMAN TANGO 239 Form 1

A cDNA encoding human TANGO 239 was identified by screening an IL-1β stimulated astrocyte library. A clone, comprising human TANGO 239, was selected for complete sequencing based on its ability to direct the secretion of a protein of approximately 60 kDa in ³⁵-S labeled supernatants of 293T cells.

TANGO 239 includes a 3413 nucleotide cDNA (Figure 8; SEQ ID NO:20). In one embodiment, TANGO 239 is referred to as TANGO 239, form 1. The open reading frame of this TANGO 239, form 1 cDNA comprises nucleotides 344 to 1990 (SEQ ID NO:22), and encodes a secreted protein comprising the 550 amino acid depicted in Figure 8 (SEQ ID NO:21). It is noted that the nucleotide sequence depicted in SEQ ID NO:20 contains *Sal I* and *Not I* adapter sequences on the 5' and 3' ends, respectively ((GTCGACCCACGCGTC CC (SEQ ID NO:), and GGGCGGCCGC (SEQ ID NO:), respectively) Thus, it is to be understood that the nucleic acid molecules of the invention include not only those sequences with such adaptor sequences but also the nucleic acid sequences described herein lacking the adaptor sequences.

The signal peptide prediction program SIGNALP (Nielsen et al. (1997) *Protein Engineering* 10:1-6) predicted that human TANGO 239, form 1 includes an 18 amino acid signal peptide (amino acids 1 to about amino acid 18 of SEQ ID NO:21)(SEQ ID NO:28) preceding the mature TANGO 239, form 1 protein (corresponding to about amino acid 19 to amino acid 550 of SEQ ID NO:21)(SEQ ID NO:34). Human TANGO 239, form 1 is predicted to have a molecular weight of approximately 61.5 kDa prior to cleavage of its signal peptide and a molecular weight of approximately 59.5 kDa subsequent to cleavage of its signal peptide.

Human TANGO 239, form 1 includes three MAM domains from about amino acids 24 to 169 (SEQ ID NO:122), amino acids 170 to 329 (SEQ ID NO:123), and amino acids 340 to 496 of SBQ ID NO:21 (SBQ ID NO:124).

Figure 16 depicts a hydropathy plot of human TANGO 239, form 1. Relatively hydrophobic residues are above the horizontal line, and relatively hydrophilic residues are below the horizontal line. As shown in the hydropathy plot, the hydrophobic region at the beginning of the plot which corresponds to about amino acids 1 to 18 of SEQ ID NO:21 is the signal sequence of TANGO 239, form 1 (SEQ ID NO:28). The cysteine residues (cys) and potential N-glyrosylation sites (Ngly) are indicated by short vertical lines just below the hydropathy trace.

A clone, EpDH233, which encodes human TANGO 239 form 1 was deposited as part of EpDHMix1 with the American Type Culture Collection (ATCC, 10801 University Boulevard, Manassas, VA 20110-2209) on November 20,1998 which was assigned Accession Number 98999. This deposit will be maintained under the terms of the Budapest Treaty on the International Recognition of the Deposit of Microorganisms for the Purposes of Patent Procedure. This deposit was made merely as a convenience to those of skill in the art and is not an admission that a deposit is required under 35 U.S.C. §112.

### HUMAN TANGO 239 Form 2

A cDNA encoding full length human TANGO 239 was identified by screening an IL-1β stimulated astrocyte library. A clone comprising human TANGO 239 was selected for complete sequencing based on its ability to direct the secretion of a protein of approximately 102.9 kDa in ³⁵-S labeled supernatants of 293T cells.

Human TANGO 239 includes a 3413 nucleotide cDNA (Figure 31; SEQ ID NO:68). In one embodiment, human TANGO 239 is referred to as TANGO 239, form 2. The open reading frame of this TANGO 239, form 2 cDNA comprises nucleotides 344 to 2395 (SEQ ID NO:70), and encodes a secreted protein comprising the 686 amino acid depicted in Figure 31 (SEQ ID NO:69). It is noted that the nucleotide sequence depicted in SEQ ID NO:70 contains *Sal I* adaptor sequences and adapter sequences on the 5' and 3' ends, respectively ((GTCGACCCACGCGTCCC (SEQ ID NO:), and GGGGGG (SEQ ID NO:), respectively). Thus, it is to be understood that the nucleic acid molecules of the invention include not only those sequences with such adaptor sequences but also the nucleic acid sequences described herein lacking the adaptor sequences.

The signal peptide prediction program SIGNALP (Nielsen et al. (1997) *Protein Engineering* 10:1-6) predicted that human TANGO 239, form 2 includes an 18 amino acid signal peptide (amino acids 1 to about amino acid 18 of SEQ ID NO:)(SEQ ID NO:125) preceding the mature TANGO 239, form 2 protein (corresponding to about amino acid 19 to amino acid 686 of SEQ ID NO:126)(SEQ ID NO:126). Human TANGO 239, form 2 is predicted to have a molecular weight of approximately 102.9 kDa prior to cleavage of its signal peptide and a molecular weight of approximately 100 kDa subsequent to cleavage of its signal peptide.

Human TANGO 239, form 2 includes four MAM domains from about amino acids 26 to 169 of SEQ ID NO:126, (SEQ ID NO:122), amino acids 170 to 329 of SEQ ID NO:126 (SEQ ID NO:123), amino acids 340 to 496 of SEQ ID NO:126 (SEQ ID NO:124), and amino acids 509 to 666 of SEQ ID NO:126. (SEQ ID NO:).

Northern analysis of human TANGO 239 mRNA expression using TANGO 239, form 2 nucleotide sequence as a probe revealed that TANGO 239 mRNA was highly expressed in skeletal muscle, placenta, and peripheral blood leukocytes. Expression was moderate in colon, thymus, kidney. Weak expression was observed in the liver, small intestine, and lung. No expression was detected in the brain, heart and spleen.

### Mouse TANGO 239

A mouse homologue of human TANGO 239 was identified, Mouse TANGO 239 was identified by analyzing the sequences of clones present in a mouse inflammation model cDNA library. This analysis led to the identification of a clone, jymua038a02, encoding full-length mouse TANGO 239. The murine TANGO 239 cDNA of this clone is 1029 nucleotides long (Figure 32; SEQ ID NO:71). It is noted that the nucleotide sequence depicted in SEQ ID NO:71 contains *Sal I* and *Not* I adapter sequences on the 5' and 3' ends, respectively ((GTCGACCCACGCGTCCC (SEQ ID NO:), and GGGCGGCCGC (SEQ ID NO:), respectively). Thus, it is to be understood that the nucleic acid molecules of the invention include not only those sequences with such adaptor sequences but also the nucleic acid sequences described herein lacking the adaptor sequences. The open reading frame of this cDNA, nucleotides 209 to 370 of SEQ ID NO:71 (SEQ ID NO:73), encodes a 54 amino acid secreted protein (Figure 32; SEQ ID NO:72).

In situ tissue screening was performed on mouse adult and embryonic tissue to analyze for the expression of mouse TANGO 239 mRNA. In summary, expression in the adult mouse appeared to be restricted to bone structures. The in-situ screen only detected expression in developing bones of embryos starting at E14.5. Expression was weak but was clearly detectable in the skull, scapula, sternum, vertebrae, incisor teeth, and femur. Adult tissues did not include bone or cartilage. Photoemulsion technique will be necessary to determine whether expression is from osteoblasts, osteoclasts, or chondrocytes. No signal was detected in the following tissues: brain (included a sense control), spinal cord, eye and harderian gland, submandibular gland, white fat, brown fat, stomach, heart (included a sense control), lung (included a sense control), liver (included a sense control), kidney (included a sense control), adrenal gland, .colon, small intestine, thymus, lymph node, spleen, pancreas (included a sense control), skeletal muscle, bladder, testes, ovaries, placenta (included a sense control). In the case of embryonic expression, the following results were obtained: At E13.5, no signal was observed. At E14.5, a weak signal was observed outlining the vertebrae, incisors, and femur (included a sense control). At E15:5, most developing bone structures appeared to be outlined including the skull, Meckel's cartilage, scapula, vertebrae, primordium of basisphenoid bone, and femur (included a sense control). At E16.5 and E18.5, most developing bone structures had a weak signal in a pattern which oufline the bone structures (included a sense control). At P1.5, a weak signal was associated with many developing bone structures. The most noticeable structures included the skull, basisphenoid bone, vertebrae, Meckel's cartilage and/or incisor teeth of the upper and lower jaw, sternum, scapula, and femur (included a sense control).

Human and murine TANGO 239 sequences exhibit considerable similarity at the protein, nucleic acid, and open reading frame levels. An alignment (made using the ALIGN software {Myers and Miller (1989) CABIOS, ver. 2.0}; BLOSUM 62 scoring matrix; gap penalties -12/-4), reveals a protein identity of 79.6%. The human and murine TANGO 239 full length cDNAs are 58.8% identical, as assessed using the same software and parameters as indicated (without the BLOSUM 62 scoring matrix). In the respective ORFs, calculated in the same fashion as the full length cDNAs, human and murine TANGO 239 are 77.2% identical.

### Uses of TANGO 239 Nucleic Acids. Polypeptides, and Modulators Thereof

As discussed above, the MAM domains of human TANGO 239 have adhesion function. Thus, the human TANGO 239 proteins of the invention likely play a role in cellular adhesion and therefore, human TANGO 239 proteins, nucleic acid molecules and/or modulators can he used to modulate cellular adhesion.

As human TANGO 239 was originally identified in an astrocyte library, human TANGO 239 nucleic acids, proteins, and modulators thereof can be used to modulate the proliferation, activation, development, differentiation, and/or function of glial cells *e*.*g*., astrocytes. Human TANGO 239 nucleic acids, proteins and modulators thereof can be used to treat glial cell-related disorders, *e.g*., astrocytoma and glioblastoma

As TANGO 239 exhibits expression in the lung, TANGO 239 polypeptides, nucleic acids, or modulators thereof, can be used to treat pulmonary (lung) disorders, such as atelectasis, pulmonary congestion or edema, chronic obstructive airway disease (*e.g*., emphysema, chronic bronchitis, bronchial asthma, and bronchier-tasis), diffuse interstitial diseases (e.g., sarcoidosis, pneumoconiosis, hypersensitivity pneumomtis, Goodpasture's syndrome, idiopathic pulmonary hemosiderosis, pulmonary alveolar proteinosis, desquamative interstitial pneumonitis, chronic interstitial pneumonia, fibrosing alveolitis, hamman-rich syndrome, pulmonary eosinophilia, diffuse interstitial fibrosis, Wegener's granulomatosis, lymphomatoid granulomatosis, and lipid pneumonia), or tumors (e.g., bronchogenic carcinoma, bronchiolovlveolar carcinoma, bronchial carcinoid, hamartoma, and mesenchymal tumors).

As TANGO 239 exhibits expression in the small intestine, TANGO 239 polypeptides, nucleic acids, or modulators thereof, can be used to treat intestinal disorders, such as ischemic bowel disease, infective enterocolitis, Crohn's disease, benign tumors, malignant tumors (e.g., argentaffinomas, lymphomas, adenocarcinomas, and sarcomas), malabsorption syndromes (e.g., celiac disease, tropical sprue, Whipple's disease, and abetalipoproteinemia), obstructive lesions, hemias, intestinal adhesions, intussusception, or volvulus.

As TANGO 239 exhibits expression in the spleen, TANGO 239 nucleic acids, proteins, and modulators thereof can be used to modulate the proliferation, differentiation, and/or function of cells that form the spleen, e.g., cells of the splenic connective tissue, e.g., splenic smooth muscle cells and/or endothelial cells of the splenic blood vessels. TANGO 239 nucleic acids, proteins, and modulators thereof can also be used to modulate the proliferation, differentiation, and/or function of cells that are processed, *e*.*g*., regenerated or phagocytized within the spleen, e.g., erythrocytes and/or B and T lymphocytes and macrophages. Thus TANGO 239 nucleic acids, proteins, and modulators thereof can be used to treat spleen, *e*.*g*., the fetal spleen, associated diseases and disorders. Examples of splenic diseases and disorders include *e*.*g*., splenic lymphoma and/or splenomegaly, and/or phagocytotic disorders, *e*.*g*., those inhibiting macrophage engulfment of bacteria and viruses in the bloodstream.

As TANGO 239 exhibits expression in the heart, TANGO 239 nucleic acids, proteins, and modulators thereof can be used to treat heart disorders, e.g., ischemic heart disease, atherosclerosis, hypertension, angina pectoris, Hypertrophic Cardiomyopathy, and congenital heart disease.

As TANGO 239 exhibits expression in bone structures, TANGO 239 nucleic acids, proteins, and modulators thereof can be used to modulate the proliferation, differentiation, and/or function of bone and cartilage cells, *e*.*g*., chondrocytes and osteoblasts, and to treat bone and/or cartilage associated diseases or disorders. Examples of bone and/or cartilage diseases and disorders include bone and/or cartilage injury due to for example, trauma (*e*.*g*., bone breakage, cartilage tearing), degeneration (*e*.*g*., osteoporosis), degeneration of joints, *e*.*g*., arthritis, *e.g*., osteoarthritis, and bone wearing.

Other TANGO 239 activities include at least one or more of the following activities: 1) modulation of cellular adhesion, either *in vitro* or *in vivo*; 2) regulation of cell trafficking and/or migration; 3) modulation of cellular proliferation; 4) modulation of inflammation; and/or 5) modulation of a signaling pathway. Thus, TANGO 239 proteins, nucleic acids and/or modulators can be used to treat a disorder characterized by aberrant TANGO 239 expression and/or an aberrant TANGO 239 activity.

Tables 1 and 2 below provide summaries of sequence information for the human TANGO molecules described herein.

Tables 3 and 4 below provide summaries of sequence information for the mouse TANGO molecules described herein.

**TABLE 1: Summary of Human TANGO 128, TANGO 140, TANGO 197, TANGO 212, TANGO 213, TANGO 224, and TANGO 239 Nucleotide Sequence Information.**

| Gene | cDNA | ORF | Protein | Figure | Accession No. |
|---|---|---|---|---|---|
| TANGO 128 | SEQ ID NO:1 | SEQ ID NO:3 | SEQ ID NO:2 | Fig. 1 | ATCC 98999 |
| TANGO 140-1 | SEQ ID NO:4 | SEQ ID NO:38 | SEQ ID NO:5 | Fig. 2 | ATCC 98999 |
| TANGO 140-2 | SEQ ID NO:6 | SEQ ID NO:39 | SEQ ID NO:7 | Fig. 3 | ATCC 999 |
| TANGO 197 | SEQ ID NO:8 | SEQ ID NO:10 | SEQ ID NO:9 | Fig. 4 | ATCC 98999 |
| TANGO 212 | SEQ ID NO:11 | SEQ ID NO:13 | SEQ ID NO:12 | Fig. 5 | ATCC 202171 |
| TANGO 213 | SBQ ID NO;14' | SBQ ID NO:16 | SBQ ID NO:15 | Fig. 6 | ATCC 98965 |
| TANGO 224 Form 1 | SEQ ID NO:17 | SEQ ID NO:19 | SEQ ID NO:18 | Fig. 7 | ATCC 98966 |
| TANGO 224 Form 2 | SEQ ID NO:65 | SEQ ID NO:67 | SEQ ID NO:66 | Fig.30 | |
| TANGO 239 Form 1 | SBQ ID NO:20 | SEQ ID NO:22 | SEQ ID NO:21 | Fig. 8 | ATCC 989999 |
| TANGO 239 Form 2 | SEQ ID NO:68 | SEQ ID NO:127 | SEQ ID NO:126 | Fig. 31 | |

**TABLE 2: Summary of Domains of Human TANGO 128, TANGO 140, TANGO 197, TANGO 212, TANGO 213, TANGO 224, and TANGO 239 Proteins**

| Protein | Signal Sequence | Mature Protein | Extracellular Domain | Transmembrane Domain | Cytoplasmic Domain |
|---|---|---|---|---|---|
| TANGO 128 | as 1-22 SEQ ID NO:23 | aa 23-345 SEQ ID NO:29 | | | |
| TANGO 140-1 | | | aa 1-146 SEQ ID NO:35 | aa 147-170 SEQ ID NO:36 | aa 171-206 SEQ ID NO:37 |
| TANGO 197 | aa 1-27 SEQ ID NO:24 | aa 28-333 SEQ ID NO:30 | aa 28-301 SEQ ID NO: | aa 302-319 SEQ ID NO: | aa 320-333 SEQ ID NO: |
| TANGO 212 | aa 1-18 SEQ ID NO:25 | aa 19-553 SEQ ID NO:31 | | | |
| TANGO 213 | aa 1-22 SEQ ID NO:26 | aa 23-271 SEQ ID NO:32 | | | |
| TANGO 224 Form I | aa 1-28 SEQ ID NO:27 | aa 29-458 SEQ ID NO:33 | | | |
| TANGO 224 Form 2 | aa 1-28 SEQ ID | sa 29-874 SEQ ID NO: | | | |
| | NO:27 | | | | |
| TANGO 239 Form 1 | aa 1-18 SEQ ID NO:28 | aa 19-550 SEQ ID NO:34 | | | |
| TANGO 239 Form 2 | aa-1-18 SEQ ID NO:125 | aa 19-686 SEQ ID NO:126 | | | |

**TABLE 3: Summary of Mouse TANGO 128, TANGO 197, TANGO 212, TANGO 213, and TANGO 239 Sequence Information.**

| Gene | cDNA | ORF | Protein | Figure |
|---|---|---|---|---|
| Mouse TANGO 128 | SEQ ID NO: 53 | SEQ ID NO:55 | SEQ ID NO:54 | Fig. 26 |
| Mouse TANGO 197 | SEQ ID NO:56 | SEQ ID NO:58 | SEQ ID NO:57 | Fig. 27 |
| Mouse TANGO 212 | SEQ ID NO:59 | SEQ ID NO:61 | SEQ ID NO:60 | Fig. 28 |
| Mouse TANGO 213 | SEQ ID NO:62 | SEQ ID NO:64 | SEQ ID NO:63 | Fig. 29 |
| Mouse TANGO 239 | SEQ ID NO:71 | SEQ ID NO:73 | SEQ ID NO:72 | Fig. 32 |

**TABLE 4: Summary of Domains of TANGO 197, TANGO 212, and TANGO 239 Proteins**

| Protein | Signal Sequence | Mature Protein | Extracellular Domain | Transmembrane Domain | Cytoplasmic Domain |
|---|---|---|---|---|---|
| Mouse TANGO 197 | | aa 1-381 SEQ ID NO: | aa 161-381 SEQ ID NO: | aa 139-160 SEQ ID NO: | aa 1-138 SEQ ID NO: |
| Mouse TANGO 212 | aa 1-18 SEQ ID NO: | as 19-553 SEQ ID NO: | | | |
| Mouse TANGO 239 | aa 1-18 SEQ ID NO: | aa 19-54 SEQ ID NO: | | | |

Various aspects of the invention are described in further detail in the following subsections.

### I. Isolated Nucleic Acid Molecules

One aspect of the invention pertains to isolated nucleic acid molecules that encode a polypeptide of the invention or a biologically active portion thereof, as well as nucleic acid molecules sufficient for use as hybridization probes to identify nucleic acid molecules encoding a polypeptide of the invention and fragments of such nucleic acid molecules suitable for use as PCR primers for the amplification or mutation of nucleic acid molecules. As used herein, the term "nucleic acid molecule" is intended to include DNA molecules (e.g., cDNA or genomic DNA) and RNA molecules (e.g., mRNA) and analogs of the DNA or RNA generated using nucleotide analogs. The nucleic acid molecule can be single-stranded or double-stranded, but preferably is double-stranded DNA.

An "isolated" nucleic acid molecule is one which is separated from other nucleic acid molecules which are present in the natural source of the nucleic acid molecule. Preferably, an "isolated" nucleic acid molecule is free of sequences (preferably protein encoding sequences) which naturally flank the nucleic acid (i.e., sequences located at the 5' and 3' ends of the nucleic acid) in the genomic DNA of the organism from which the nucleic acid is derived. For example, in various embodiments, the isolated nucleic acid molecule can contain less than about 5 kB, 4 kB, 3 kB, 2 kB, 1 kB, 0.5 kB or 0.1 kB of nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material, or culture medium when produced by recombinant techniques, or substantially free of chemical precursors or other chemicals when chemically synthesized.

A nucleic acid molecule of the present invention, e.g., a nucleic acid molecule having the nucleotide sequence of SEQ ID NO:1, 3, 4, 6, 8, 10, 11, 13, 14, 16, 17, 19, 20, 22, 38, 39, 53, 55, 56, 58, 59, 61, 62, 64, 65, 67, 68, 70, 71, 73, or a complement thereof, can be isolated using standard molecular biology techniques and the sequence information provided herein. Using all or a portion of the nucleic acid sequences of SEQ ID NO:1, 3, 4, 6, 8, 10, 11, 13, 14, 16, 17, 19, 20, 22, 38, 39, 53, 55, 56, 58, 59, 61, 62, 64, 65, 67, 68, 70, 71, 73, as a hybridization probe, nucleic acid molecules of the invention can be isolated using standard hybridization and cloning techniques (e.g., as described in Sambrook ot al., eds., *Molecular Cloning: A Laboratory Manual, 2nd ed., Cold Spring Harbor Laboratory*, Cold Spring Harbor Laboratory Press, Cold Spring Harbor, NY, 1989).

A nucleic acid molecule of the invention can be amplified using cDNA, mRNA or genomic DNA as a template and appropriate oligonucleotide primers according to standard PCR amplification techniques. The nucleic acid so amplified can be cloned into an appropriate vector and characterized by DNA sequence analysis. Furthermore, oligonucleotides corresponding to all or a portion of a nucleic acid molecule of the invention can be prepared by standard synthetic techniques, *e*.*g*., using an automated DNA synthesizer.

In another preferred embodiment, an isolated nucleic acid molecule of the invention comprises a nucleic acid molecule which is a complement of the nucleotide sequence of SEQ ID NO:1,3,4, 6, 8, 10, 11, 13, 14, 16, 17, 19, 20, 22, 38, 39, 53, 55, 56, 58, 59, 61, 62, 64, 65, 67, 68, 70, 71, 73, or a portion thereof. A nucleic acid molecule which is complementary to a given nucleotide sequence is one which is sufficiently complementary to the given nucleotide sequence that it can hybridize to the given nucleotide sequence thereby forming a stable duplex.

Moreover, a nucleic acid molecule of the invention can comprise only a portion of a nucleic acid sequence encoding a full length polypeptide of the invention for example, a fragment which can be used as a probe or primer or a fragment encoding a biologically active portion of a polypeptide of the invention. The nucleotide sequence determined from the cloning one gene allows for the generation of probes and primers, designed for use in identifying and/or cloning homologues in other cell types, e.g., from other tissues, as well as homologues from other maramats. The probe/primer typically comprises substantially purified oligonucleotide. The oligonucleotide typically comprises a region of nucleotide sequence that hybridizes under stringent conditions to at least about 12, preferably about 25, more preferably about 50, 75, 100, 125, 150, 175, 200, 250, 300, 350 or 400 consecutive nucleotides of the sense or anti-sense sequence of SEQ ID NO:1, 3, 4, 6, 8, 10, 11, 13, 14, 16, 17, 19, 20, 22, 38, 39, 53, 55, 56, 58, 59, 61, 62, 64, 65, 67, 68, 70, 71, 73, or of a naturally occurring mutant of SEQ ID NO:1, 3, 4, 6, 8, 10, 11, 13, 14, 16, 17, 19, 20 or 22.

Probes based on the sequence of a nucleic acid molecule of the invention can be used to detect transcripts or genomic sequences encoding the same protein molecule encoded by a selected nucleic acid molecule. The probe comprises a label group attached thereto, e.g., a radioisotope, a fluorescent compound, an enzyme, or an enzyme co-factor. Such probes can be used as part of a diagnostic test kit for identifying cells or tissues which mis-express the protein, such as by measuring levels of a nucleic acid molecule encoding the protein in a sample of cells from a subject, e.g., detecting mRNA levels or determining whether a gene encoding the protein has been mutated or deleted.

A nucleic acid fragment encoding a biologically active portion of a polypeptide of the invention can be prepared by isolating a portion of any of SEQ ID NOs:1, 3, 4, 6, 8, 10, 11, 13, 14, 16, 17, 19, 20, 22, 38, 39, 53, 55, 56, 58, 59, 61, 62, 64, 65, 67, 68, 70, 71, 73, expressing the encoded portion of the polypeptide protein (e.g., by recombinant expression *in vitro*) and assessing the activity of the encoded portion of the polypeptide.

The invention further encompasses nucleic acid molecules that differ from the nucleotide sequence of SEQ ID NO:1, 3, 4, 6, 8, 10, 11, 13, 14, 16, 17, 19, 20, 22, 38, 39, 53, 55, 56, 58, 59, 61, 62, 64, 65, 67, 68, 70, 71, 73, due to degeneracy of the genetic code and thus encode the same protein as that encoded by the nucleotide sequence of SEQ ID NO:1, 3, 4, 6, 8, 10, 11, 13, 14, 16, 17, 19, 20, 22, 38, 39, 53, 55, 56, 58, 59, 61, 62, 64, 65, 67, 68, 70, 71, 73.

In addition to to the nucleotide sequences of SEQ ID NOs:1, 3, 4, 6, 8, 10, 11, 13, 14, 16, 17, 19, 20, 22, 38, 39, 53, 55, 56,.58, 59, 61, 62, 64, 65, 67, 68, 70, 71, 73, it will be appreciated by those skilled in the art that DNA sequence polymorphisms that lead to changes in the amino acid sequence may exist within a population (e.g., the human population). Such genetic polymorphisms may exist among individuals within a population due to natural allelic variation. An allde is one of a group of genes which occur alternatively at a given genetic locus. For example, TANGO 128 has been mapped to chromosome 4, between flanking markers WI-3936 and AFMCO27ZB9, and therefore, TANGO 128 family members can include nucleotide sequence polymorphisms (*e*.*g*., nucleotide sequences that vary from SEQ ID NO:X) that map to this chromosome 4 region (*i*.*e*., between markers WI-3936 and AFMCO27ZB9).. For example, TANGO 213 has been mapped to chromosome 17, in the region p13.3, between flanking markers WI-5436 and WI-6584, and therefore, TANGO 213 family members can include nucleotide sequence polymorphisms (*e.g*., nuoleotide sequences that vary from SEQ ID NO:X) that map to this chromosome 17 region (*i.e*., between markers WI-5436 and WI-6584). As used herein, the phrase allelic variant refers to a nucleotide sequence which occurs at a given locus or to a polypeptide encoded by the nucleotide sequence. As used herein, the terms "gene" and "recombinant gene" refer to nucleic acid molecules comprising an open reading frame encoding a polypeptide of the invention. Such natural allelic variations can typically result in 1-5% variance in the nucleotide sequence of a given gene. Alternative alleles can be identified by sequencing the gene of interest in a number of different individuals. This can be readily carried out by using hybridization probes to identify the same genetic locus in a variety of individuals. Any and all such nucleotide, variations and resulting amino acid polymorphisms or variations that are the result of natural allelic variation and that do not alter the functional activity are intended to be within the scope of the invention.

Moreover, nucleic acid molecules encoding proteins of the invention from other species (homologues), which have a nucleotide sequence which differs from that of the human protein described herein are intended to be within the scope of the invention. Nucleic acid molecules corresponding to natural allelic variants and homologues of a cDNA of the invention can be isolated based on their identity to the human nucleic acid molecule disclosed herein using the human cDNAs, or a portion thereof, as a hybridization probe according to standard hybridization techniques under stringent hybridization conditions. For example, a cDNA encoding a soluble form of a membrane-bound protein of the invention isolated based on its hybridization to a nucleic acid molecule encoding all or part of the membrane-bound form. Likewise, a cDNA encoding a membrane-bound form can be isolated based on its hybridization to a nucleic acid molecule encoding all or part of the soluble form.

Accordingly, in another embodiment, an isolated nucleic acid molecule of the invention is at least 100 (125, 150, 175, 200, 225, 250, 275, 300 325, 350, 375, 400, 425, 450, 500, 550, 600, 650, 700, 800, 900, 1000, or 1290) nucleotides in length and hybridizes under stringent conditions to the nucleic acid molecule comprising the nucleotide sequence, preferably the coding sequence, of SEQ ID NO:1, 3, 4, 6, 8, 10, 11, 13, 14, 16, 17, 19, 20, 22, 38, 39, 53, 55, 56, 58, 59, 61, 62, 64, 65, 67, 68, 70, 71, 73, or complement thereof.

As used herein, the term "hybridizes under stringent conditions" is intended to describe conditions for hybridization and washing under which nucleotide sequences at least 60% (65%, 70%, preferably 75%) identical to each other typically remain hybridized to each other. Such stringent conditions are known to those skilled in the art and can be found in *Current Protocols in Molecular Biology*, John Wiley & Sons, N.Y. (1989), 6.3.1-6.3.6. A preferred, non-limiting example of stringent hybridization conditions are hybridization in 6X sodium chloride/sodium citrate (SSC) at about 45 C, followed by one or more washes in 0.2 X SSC, 0.1% SDS at 50-65 C. Preferably, an isolated nucleic acid molecule of the invention that hybridizes under stringent conditions to the sequence of SEQ ID NO:1, 3, 4, 6, 8, 10, 11, 13, 14, 16, 17, 19, 20, 22, 38, 39, 53, 55, 56, 58, 59, 61, 62, 64, 65, 67, 68, 70, 71, 73, or complement thereof, corresponds to a naturally-occurring nucleic acid molecule. As used herein, a "naturally-occurring" nucleic acid molecule refers to an RNA or DNA molecule having a nucleotide sequence that occurs in nature (e.g., encodes a natural protein), Representative species that hybridize under such conditions to one or more of the sequences above include, but are not limited to, SEQ ID Nos:78, 80, 82, 84, 86, 88, 90, 92, 94, 96, and 98, which in particular hybridize to the TANGO 128 sequences listed above (SEQ ID NO:1).

In addition to naturally-occurring allelic variants of a nucleic acid molecule of the invention sequence that may exist in the population, the skilled artisan will further appreciate that changes can be introduced by mutation thereby leading to changes in the amino acid sequence of the encoded protein, without altering the biological activity of the protein. For example, one can make nucleotide substitutions leading to amino acid substitutions at "non-essential" amino acid residues. A "non-essential" amino acid residue is a residue that can be altered from the wild-type sequence without altering the biological -activity, whereas an "essential" amino acid residue is required for biological activity. For example, amino acid residues that are not conserved or only semi-conserved among homologues of various species may be non-essential for activity and thus would be likely targets for alteration. Alternatively, amino acid residues that are conserved, among the homologues of various species (e.g., murine and human) may be essential for activity and thus would not be likely targets for alteration. For example, representative species of the mouse TANGO 128 presented for illustrative purposes only and not by way of limitation, include but are not limited to, SEQ ID Nos 78, 80, 82, 84, 86, 88, 90, 92, 94, 96, and 98.

Accordingly, another aspect of the invention pertains to nucleic acid molecules encoding a polypeptide of the invention that contain changes in amino acid residues that are not essential for activity. Such polypeptides differ in amino acid sequence from SEQ ID NO:2, 5, 7, 9, 12, 15, 18, 21, 54, 57, 60, 63, 66, 69, 72, yet retain biological activity. In one embodiment, the isolated nucleic acid molecule includes a nucleotide sequence encoding a protein that includes an amino acid sequence that is at least about 45% identical, 65%, 75%, 85%, 95%, or 98% identical to the amino acid sequence of SEQ ID NO:2, 5, 7, 9, 12, 15, 18, 21, 54, 57, 60, 63, 66, 69, 72.

An isolated nucleic acid molecule encoding a variant protein can be created by introducing one or more nucleotide substitutions, additions or deletions into the nucleotide sequence of SEQ ID NO:1, 3, 4, 6, 8, 10, 11, 13, 14, 16, 17, 19, 20, 22, 38, 39, 53, 55, 56, 58, 59, 61, 62, 64, 65, 67, 68, 70, 71, 73, such that one or more amino acid substitutions, additions or deletions are introduced into the encoded protein. Preferably such variant proteins retain or exhibit at least one structural or biological activity of the polyeptides of the invention. Mutations can be introduced by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Alternatively, mutations can be introduced randomly along all or part of the coding sequence, such is by saturation mutagenesis, and the resultant mutants can be screened for biological activity to identify mutants that retain activity. Following mutagenesis, the encoded protein can be expressed recombinantly and the activity of the protein can be determined. In a preferred embodiment, a mutant polypeptide thai is a variant of a polypeptide of the invention can be assayed for: (1) the ability to form protein:protein interactions with proteins in a signaling pathway of the polypeptide of the invention; (2) the ability to bind a ligand of the polypeptide of the invention; or (3) the ability to bind to an intracellular target protein of the polypeptide of the invention. In yet another preferred embodiment, the mutant polypeptide can be assayed for the ability to modulate cellular proliferation, cellular migration or chemotaxis, or cellular differentiation.

The present invention encompasses antisense nucleic acid molecules, i.e., molecules which are complementary to a sense nucleic acid encoding a polypeptide of the invention, e.g., complementary to the coding strand of a double-stranded cDNA molecule or complementary to an mRNA sequence. Accordingly, an antisense nucleic acid can hydrogen bond to a sense nucleic, acid. The antisense nucleic acid can be complementary to an entire coding strand, or to only a portion thereof, .e.g., all or part of the protein coding region (or open reading frame). An antisense nucleic acid molecule can be antisense to all or part of a non-coding region of the coding strand of a nucleotide sequence encoding a polypeptide of the invention. The non-coding regions ("5' and 3' untranslated regions") are the 5' and 3' sequences which flank the coding region and are not translated into amino acids.

An antisense oligonucleotide can be, for example, about 5, 10, 15, 20, 25, 30, 35, 40, 45 or 50 nucleotides in length. An antisense nucleic acid of the invention can be constructed using chemical synthesis and enzymatic ligation reactions using procedures known in the art. For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate detivatives and acridine substituted nucleotides can be used. Examples of modified nucleotides which can be used to generate the amisense nucleic acid include 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thioundinc, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-miouracil, 5-methyluracil, uracil-5-oxyacetic acid methylester, uracil-5-oxyacetic acid (v), 5-methyl-2-thiomacil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

The antisense nucleic acid molecules of the invention are typically administered to a subject or generated *in situ* such that they hybridize with or bind to cellular mRNA and/or genomic DNA encoding a selected polypeptide of the invention to thereby inhibit expression, e.g., by inhibiting transcription and/or translation. The hybridization can be by conventional nucleotide complementarity to form a stable duplex, or, for example, in the case of an antisense nucleic acid molecule which binds to DNA duplexes, through specific interactions in the major groove of the double helix. An example of a route of administration of antisense nucleic acid molecules of the invention includes direct injection at a tissue site. Alternatively, antisense nucleic acid molecules can be modined to target selected cells and then administered systemically. For example, for systemic administration, antisense molecules can be modified such that they specifically bind to receptors or antigens expressed on a selected cell surface, e.g., by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the antisense molecules, vector constructs in which the antisense nucleic acid molecule is placed under the control of a strong pol II or pol III promoter are preferred.

An antisense nucleic acid molecule of the invention can be an α-anomeric nucleic acid molecule. An α-anometic nucleic acid molecule forms specific double-stranded hybrids with complementary RNA in which, contrary to the usual β-units, the strands run parallel to each other (Gaultier et al. (1987) *Nucleic Acids Res*. 15:6625-6641). The antisense nucleic acid molecule can also comprise a 2'-o-methylribonucleotide (Inoue et al. (1987) *Nucleic Acids Res*. 15:6131-6148) or a chimeric RNA-DNA analogue (Inoue et al. (1987) *FEBS Lett,* 215:327-330).

The invention also encompasses ribozymes. Ribozymes are catalytic RNA molecules with ribonuclease activity which are capable of cleaving a single-stranded nucleic acid, such as an mRNA, to which they have a complementary region. Thus, ribozymes (e.g., hammerhead ribozymes (described in Haselhoff and Gerlach (1988) *Nature* 334:585-591)) can be used to catalytically cleave mRNA transcripts to thereby inhibit translation of the protein encoded by the mRNA. A ribozyme having specificity for a nucleic acid molecule encoding a polypeptide of the invention can be designed based upon the nucleotide sequence of a cDNA disclosed herein. For example, a derivative of a *Tetrahymena* L-19 IVS RNA can be constructed in which the nucleotide sequence of the active site is complementary to the nucleotide sequence to be cleaved in a Cech et al. U.S. Patent No. 4,987,071; and Cech et al. U.S. Patent No. 5,116,742. Alternatively, an mRNA encoding a polypeptide of the invention can be used to select a catalytic RNA having a specific ribonuclease activity from a pool of RNA molecules. *See, e*.*g*., Bartel and Szostak (1993) *Science* 261:1411-1418.

The invention also encompasses nucleic acid molecules which form triple helical structures. For example, expression of a polypeptide of the invention can be inhibited by targeting nucleotide sequences complementary to the regulatory region of the gene encoding the polypeptide (e.g., the promoter and/or enhancer) to form triple helical structures that prevent transcription of the gene in target cells. *See generally* Helene (1991) *Anticancer Drug Des.* 6(6):569-84; Holene (1992) *Ann*. *N.Y. Acad. Sci.* 660:27-36; and Maher (1992) *Bioassays* 14(12):807-15.

In various embodiments, the nucleic acid molecules of the invention can be modified at the base moiety, sugar moiety or phosphate backbone to improve, e.g., the stability, hybridization, or solubility of the molecule. For example, the deoxyribose phosphate backbone of the nucleic acids can be modified to generate peptide nucleic acids (*see* Hyrup et al. (1996) *Bioorganic & Medicinal Chemistry* 4(1): 5-23). As used herein, the terms "peptide nucleic acids" or "PNAs" refer to nucleic acid mimics, e.g., DNA mimics, in which the deoxyribose phosphate backbone is replaced by a pseudopeptide backbone and only the four natural nuclcobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup et al. (1996), *supra*; Perry-O'Keefe ct al. (1996) *Proc. Natl. Acad. Sci. USA* 93: 14670-575.

PNAs can be used in therapeutic and diagnostic applications. For example, PNAs can be used as antisense or antigene agents for sequence-specific modulation of gene expression by, e.g., inducing transcription or translation arrest or inhibiting replication. PNAs can also be used. e.g., in the analysis of single base pair mutations in a gene by, e.g., PNA directed PCR clamping; as artificial restriction enzymes when used in combination with other enzymes, e.g., S 1 nucleases (Hyrup (1996), *supra*; or as probes or primers for DNA sequence and hybridization (Hyrup (1996), *supra*; Perry-O'Keefe et al. (1996) *Proc. Natl. Acad. Sci. USA* 93: 14670-675).

In another embodiment, PNAs can be modified, e.g., to enhance their stability or cellular uptake, by attaching lipophilic or other helper groups to PNA, by the formation of PNA-DNA chimeras, or by the use of liposomes or other techniques of drug delivery known in the art. For example, PNA-DNA chimeras can be generated which may combine the advantageous properties of PNA and DNA. Such chimeras allow DNA recognition enzymes, e.g., RNAse H and DNA polymerases, to interact with the DNA portion while the PNA portion would provide high binding affinity and specificity. PNA-DNA chimeras can be linked using linkers of appropriate lengths selected in terms of base stacking, number of bonds between the nucleobases, and orientation (Hyrup (1996), *supra*). The synthesis of PNA-DNA chimeras can be performed as described in Hyrup (1996), *supra*, and Finn et al. (1996) *Nucleic Acids Res*. 24(17):3357-63. For example, a DNA chain can be synthesized on a solid support using standard phosphoramidite coupling chemistry and modified nucleoside analogs. Compounds such as 5'-(4-methoxytrityl)amino-5'-deoxy-thymidine phosphoramidite can be used as a link between the PNA and the 5' end of DNA (Mag et al. (1989) *Nucleic Acids Res*. 17:5973-88). PNA monomers are then coupled in a stepwise manner to produce a chimeric molecule with a 5' PNA segment and a 3' DNA segment (Finn et al. (1996) *Nucleic Acids Res*. 24(17):3357-63). Alternatively, chimeric molecules can be synthesized with a 5' DNA segment and a 3' PNA segment (Peterser et al. (1975) *Biocirganic Med. Chem. Lett*. 5:1119-11124).

In other embodiments, the oligonucleotide may include other appended groups such as peptides (e.g., for targeting host cell receptors *in vivo*), or agents facilitating transport across the cell membrane (*see*, *e.g.,* Letsinger et al. (1989) *Proc. Natl. Acad. Sci. USA* 86:6553-6556; Lemaitre et al. (1987) *Proc. Natl. Acad. Sci. USA* 84:648-652; PCT Publication No. W0 88/09810) or the blood-brain barrier *(see, e.g*., PCT Publication No. W0 89/10134). In addition, oligonucleotides can be modified with hybridization-triggered cleavage agents *(see, e.g.,* Krol et al. (1988) *Bio*/*Techniques* 6:958-976) or intercalating agents (*see, e.g*., Zon (1988) *Pharm. Res.* 5:539-549). To this end, the oligonucleotide may be conjugated to another molecule, e.g., a peptide, hybridization triggered cross-linking agent, transport agent, hybridization-triggered cleavage agent, etc.

### II. Isolated Proteins and Antibodies

One aspect of the invention pertains to isolated proteins, and biologically active portions thereof, as well as polypeptide fragments suitable for use as immunogens to raise antibodies directed against a polypeptide of the invention. In one embodiment, the native polypeptide can be isolated from cells or tissue sources by an appropriate purification scheme using standard protein purification techniques. In another embodiment, polypeptides of the invention are produced by recombinant DNA techniques. Alternative to recombinant expression, a polypeptide of the invention can be synthesized chemically using standard peptide synthesis techniques.

An "isolated" or "purified" protein or biologically active portion thereof is substantially free of cellular material or other contaminating proteins from the cell or tissue source from which the protein is derived, or substantially free of chemical precursors or other chemicals when chemically synthesized. The language "substantially free of cellular material" includes preparations of protein in which the protein is separated from cellular components of the cells from which it is isolated or recombinantly produced. Thus, protein that is substantially free of cellular material includes preparations of protein having less than about 30%, 20%, 10%, or 5% (by dry weight) of heterologous protein (also referred to herein as a "contaminating protein"). When the protein or biologically active portion thereof is recombinantly produced, it is also preferably substantially free of culture medium, i.e., culture medium represents less than about 20%, 10%, or 5% of the volume of the protein preparation. When the protein is produced by chemical synthesis, it is preferably substantially free of chemical precursors or other chemicals, i.e., it is separated from chemical precursors or other chemicals which are involved in the synthesis of the protein. Accordingly such preparations of the protein have less than about 30%, 20%, 10%, 5% (by dry weight) of chemical precursors or compounds other than the polypeptide of interest.

Biologically active portions of a polypeptide of the invention include polypeptides comprising amino acid sequences sufficiently identical to or derived from the amino acid sequence of the protein (e.g., the amino acid sequence shown in any of SEQ ID NOs:2, 5, 7, 9, 12, 15, 18, 21, 54, 57, 60, 63, 66, 69, 72), which include fewer amino acids than the full length protein, and exhibit at least one activity of the corresponding full-length protein. Typically, biologically active portions comprise a domain or motif with at least one activity of the corresponding protein. A biologically active portion of a protein of the invention can be a polypeptide which is, for example, 10, 25, 50, 100, or more amino acids in length. Moreover, other biologically active portions, in which other regions of the protein are deleted, can be prepared by recombinant techniques and evaluated for one or more of the functional activities of the native form of a polypeptide of the invention.

Preferred polypeptides have the amino acid sequence of SBQ ID NO:2, 5, 7, 9, 12, 15,18, 21, 54, 57, 60, 63, 66, 69, 72 Other useful proteins are substantially identical (e.g., at least about 45%, preferably 55%, 65%, 75%, 85%, 95%, or 99%) to any of SEQ ID NO:2, 5, 7, 9, 12, 15, 18, 21, 54, 57, 60, 63, 66, 69, 72 and retain the functional activity of the protein of the corresponding naturally-occurring protein yet differ in amino acid sequence due to natural allelic variation or mutagenesis.

To determine the percent identity of two amino acid sequences or of two nucleic acids, the sequences are aligned for optimal comparison purposes (e.g., gaps can be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (i.e., % identity = # of identical positions/total # of positions (*e*.*g*., overlapping positions) x 100). In one embodiment the two sequences are the same length.

The determination of percent identity between two sequences can be accomplished using a mathematical algorithm. A preferred, non-limiting example of a mathematical algorithm utilized for the comparison of two sequences is the algorithm of Karlin and Altschul (1990) *Proc. Natl. Acad. Sci. USA* 87:2264-2268, modified as in Karlin and Altschul (1993) *Proc. Natl. Acad. Sci. USA* 90:5873-5877. Such an algorithm is incorporated into the NBLAST and XBLAST programs of Altschul, et al. (1990) *J. Mol. Biol*. 215:403-410. BLAST nucleotide searches can be performed with the NBLAST program, score = 100, wordlength = 12 to obtain nucleotide sequences homologous to a nucleic acid molecules of the invention. BLAST protein searches can be performed with the XBLAST program, score = 50, wordlength = 3 to obtain amino and sequences homologous to a protein molecules of the invention. To obtain gapped alignments for comparison purposes, Gapped BLAST can be utilized as described in Altschul et al. (1997) *Nucleic Acids Res.* 25:3389-3402. Alternatively, PSI-Blast can be used to perform an iterated search which detects distant relationships between molecules. *Id.* When utilizing BLAST, Gapped BLAST, and PSI-Blast programs, the default parameters of the respective programs (e.g., XBLAST and NBLAST) can be used. *See* http://www.ncbi.nlm.nih.gov. Another preferred, non-limiting example of a mathematical algorithm utilized for the comparison of sequences is the algorithm of Myers and Miller, (1988) *CABIOS* 4:11-17. Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used.

The percent identity between two sequences can be determined using techniques similar to those described above, with or without allowing gaps. In calculating percent identity, only exact matches are counted.

The invention also provides chimeric or fusion proteins. As used herein, a "chimeric protein" or "fusion protein" comprises all or part (preferably biologically active) of a polypeptide of the invention operably linked to a heterologous polypeptide (i.e., a polypeptide other than the same polypeptide of the invention). Within the fusion protein, the term "operably linked" is intended to indicate that the polypeptide of the invention and the heterologous polypeptide are fused in-frame to each other. The heterologous polypeptide can be fused to the N-terminus or C-termiaus of the polypeptide of the invention. One useful fusion protein is a GST fusion protein in which the polypeptide of the invention is fused to the C-terminus of GST sequences. Such fusion proteins can facilitate the purification of a recombinant polypeptide of the invention.

In another embodiment, the fusion protein contains a heterologous signal sequence at its N-terminus. For example, the native signal sequence of a polypeptide of the invention can be removed and replaced with a signal sequence from another protein. For example, the gp67 secretory sequence of the baculovirus envelope protein can be used as a heterologous signal sequence *(Current Protocols in Molecular Biology*, Ausubel et al., eds., John Wiley & Sons, 1992). Other examples of eukaryotic heterologous signal sequences include the secretory sequences of melittin and human placental alkaline phosphatase (Stratagene; La Jolla, California). In yet another example, useful prokaryotic heterologous signal sequences include the phoA secretory signal (Sambrook et al., *supra*) and the protein A secretory signal (Pharmacia Biotech; Pisrataway, New Jersey).

In yet another embodiment, the fusion protein is an immunoglobulin fusion protein in which all or part of a polypeptide of the invention is fused to sequences derived from a member of the immunoglobulin protein family. The immunoglobulin fusion proteins of the invention can be incorporated into pharmaceutical compositions and administered to a subject to inhibit an interaction between a ligand (soluble or membrane-bound) and a protein on the surface of a cell (receptor), to thereby suppress signal transduction *in vivo*. The immunoglobulin fusion protein can be used to affect the bioavailability of a cognate ligand of a polypeptide of the invention. Inhibition of ligand/receptor interaction may be useful therapeutically, both for treating proliferative and differentiative disorders and for modulating (e.g. promoting or inhibiting) cell survival. Moreover, the immunoglobulin fusion proteins of the invention can be used as immunogens to produce antibodies directed against a polypeptide of the invention in a subject, to purify ligands and in screening assays to identify molecules which inhibit the interaction of receptors with ligands.

Chimeric and fusion proteins of the invention can be produced by standard recombinant DNA techniques. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence (*see, e*.*g*., Ausubel et al., *supra*). Moreover, many expression vectors are commercially available that already encode a fusion moiety (e.g., a GST polypeptide). A nucleic acid encoding a polypeptide of the invention can be cloned into such an expression vector such that the fusion moiety is linked in-frame to the polypeptide of the invention.

A signal sequence of a polypeptide of the invendon (SBQ ID NO:2, 5, 7, 9, 12, 15, 18, 21, 23, 24, 24, 26, 27, 28, or 125) can be used to facilitate secretion and isolation of the secreted protein or other proteins of interest. Signal sequences are typically characterized by a core of hydrophobic amino acids which are generally cleaved from the mature protein during secretion in one or more cleavage events. Such signal peptides contain processing sites that allow cleavage of the signal sequence from the mature proteins as they pass through the secretory pathway. Thus, the invention pertains to the described polypeptides having a signal sequence, as well as to the signal sequence itself and to the polypeptide in the absence of the signal sequence (i.e., the cleavage products). In one embodiment, a nucleic acid sequence encoding a signal sequence of the invention can be operably linked in an expression vector to a protein of interest, such as a protein which is ordinarily not secreted or is otherwise difficult to isolate. The signal sequence directs secretion of the protein, such as from a eukaryotic host into which the expression vector is transformed, and the signal sequence is subsequently or concurrently cleaved. The protein can then be readily purified from the extracellular medium by art recognized methods. Alternatively, the signal sequence can be linked to the protein of interest using a sequence which facilitates purification, such as with a GST domain.

In another embodiment, the signal sequences of the present invention can be used to identify regulatory sequences, e,g., promoters, enhancers, repressors. Since signal sequences are the most amino-terminal sequences of a peptide, it is expected that the nucleic acids which flank the signal sequence on its amino-terminal side will be regulatory sequences which affect transcription. Thus, a nucleotide sequence which encodes all or a portion of a signal sequence can be used as a probe to identify and isolate signal sequences and their flanking regions, and these flanking regions can be studied to identify regulatory elements therein.

The present invention also pertains to variants of the polypeptides of the invention. Such variants have an altered amino acid sequence which can function as either agoiusts (mimetics) or as antagonists. Variants can be generated by mutagenesis, e.g., discrete point mutation or truncation. An agonist can retain substantially the same, or a subset, of the biological activities of the naturally occurring form of the protein. An antagonist of a protein can inhibit one or more of the activities of the naturally occurring form of the protein by, for example, competitively binding to a downstream or upstream member of a cellular signaling cascade which includes the protein of interest. Thus, specific biological effects can be elicited by treatment with a variant of limited function Treatment of a subject with a variant having a subset of the biological activities of the naturally occurring form of the protein can have fewer side effects in a subject relative to treatment with the naturally occurring form of the protein.

Variants of a protein of the invention which function as either agonists (mimetics) or as antagonists can be identified by screening combinatorial libraries of mutants, e.g., truncation mutants, of the protein of the invention for agonist or antagonist activity. In one embodiment, a variegated library of variants is generated by combinatorial mutagenesis at the nucleic acid level and is encoded by a variegated gene library. A variegated library of variants can be produced by, for example, enzymatically ligating a mixture of synthetic oligonucleotides into gene sequences such that a degenerate set of potential protein sequences is expressible as individual polypeptides, or alternatively, as a set of larger fusion proteins (e.g., for phage display). There are a variety of methods which can be used to produce libraries of potential variants of the polypeptides of the invention from a degenerate oligonucleotide sequence. Methods for synthesizing degenerate oligonucleotides are known in the art (*see*, *e*.*g*., Narang (1983) *Tetrahedron* 39:3; Itakura et al. (1984) *Annu. Rev. Biochem*. 53:323; Itakuta et al. (1984) *Science* 198:1056; Ike et al. (1983) *Nucleic Acid Res*. 11:477).

In addition, libraries of fragments of the coding sequence of a polypeptide of the invention can be used to generate a variegated population of polypeptides for screening and subsequent selection of variants. For example, a library of coding sequence fragments can be generated by treating a double stranded PCR fragment of the coding sequence of interest with a nuclease under conditions wherein nicking occurs only about once per molecule, denaturing the double stranded DNA, renaturing the DNA to form double stranded DNA which can include sense/antisense pairs from different nicked products, removing single stranded portions from reformed duplexes by treatment with S1 nuclease, and ligating the resulting fragment library into an expression vector. By this method, an expression library can be derived which encodes N-terminal and internal fragments of various, sizes of the protein of interest.

Several techniques are known in the art for screening gene products of combinatorial libraries made by point mutations or truncation, and for screening cDNA libraries for gene products having a selected property. The most widely used techniques, which are amenable to high through-put analysis, for screening large gene libraries typically include cloning the gene library into replicable expression vectors, transforming appropriate cells, with the resulting library of vectors, and expressing the combinatorial genes under conditions in which detection of a desired activity facilitates isolation of the vector encoding the gene whose product was detected. Reeursive ensemble mutagenesis (REM), a technique which enhances the frequency of functional mutants in the libraries, can be used in combination with the screening assays to identify variants of a protein of the invention (Arkin and Yourvan (1992) *Proc. Natl. Acad Sci. USA 89*:7811-7815; Delgrave et al. (1993) *Protein Engineering* 6(3):327-331).

An isolated polypeptide of the invention, or a fragment thereof, can be used as an immunogen to generate antibodies using standard techniques for polyclonal and monoclonal antibody preparation. The full-length polypeptide or protein can be used or, alternatively, the invention provides antigenic peptide fragments for use as immunogens. The antigenic peptide of a protein of the invention comprises at least 8 (preferably 10,15,20, or 30) amino acid residues of the amino acid sequence of SEQ ID NO:2,5, 7,9,12,15,18,21,54, 57, 60, 63, 66, 69, 72, and encompasses an epitope of the protein such that an antibody raised against the peptide forms a specific immune complex with the protein.

Preferred epitopes encompassed by the antigenic peptide are regions that are located on the surface of the protein, e.g., hydrophilic regions. Figures 8-14 are hydrophobicity plots of the proteins of the invention. These plots or similar analyses can be used to identify hydrophilic regions.

An immunogen typically is used to prepare antibodies by immunizing a suitable subject, (e.g., rabbit, goat, mouse or other mammal). An appropriate immunogenic preparation can contain, for example, recombinantly expressed or chemically synthesized polypeptide. The preparation can further include an adjuvant, such as Freund's complete or incomplete adjuvant, or similar immunostimulatory agent.

Accordingly, another aspect of the invention pertains to antibodies directed against a polypeptide of the invention. The term "antibody" as used herein refers to immunoglobulin molecules and immunologically active portions of immunoglobulin molecules, *i.e*., molecules that contain an antigen binding site which specifically binds an antigen, such as a polypeptide of the invention, *e.g*., an epitope of a polypeptide of the invention. A molecule which specifically binds to a given polypeptide of the invention is a molecule which binds the polypeptide, but does not substantially bind other molecules in a sample, *e*.*g*., a biological sample, which naturally contains the polypeptide. Examples of immunologically active portions of immunoglobulin molecules include F(ab) and F(ab')₂ fragments which can be generated by treating the antibody with an enzyme such as pepsin. The invention provides polyclonal and monoclonal antibodies. The term "monoclonal antibody" or "monoclonal antibody composition", as used herein, refers to a population of antibody molecules that contain only one species of an antigen binding site capable of immunoreacting with a particular epitope.

Polyclonal antibodies can be prepared as described above by immunizing a suitable subject with a polypeptide of the invention as an immunogen. Preferred polyclonal antibody compositions are ones that have been selected for antibodies directed against a polypeptide or polypeptides of the invention. Particularly preferred polyclonal antibody preparations are ones that contain only antibodies directed against a polypeptide or polypeptides of the invention. Particularly preferred immunogen compositions are those that contain no other human proteins such as, for example, immunogen compositions made using a non-human host cell for recombinant expression of a polypeptide of the invention. In such a manner, the only human epitope or epitopes recognized by the resulting antibody compositions raised against this immunogen will be present as part of a polypeptide or polypeptides of the invention.

The antibody titer in the immunized subject can be monitored over time by standard techniques, such as with an enzyme linked immunosorbent assay (ELISA) using immobilized polypeptide. If desired, the antibody molecules can be isolated from the mammal (*e*.*g*., from the blood) and further purified by well-known techniques, such as protein A chromatography to obtain the IgG fraction. Alternatively, antibodies specific for a protein or polypeptide of the invention can be selected for (*e*.*g*., partially purified) or purified by, *e.g*., affinity chromatography. For example, a recombinantly expressed and purified (or partially purified) protein of the invention is produced as described herein, and covalently or non-covalently coupled to a solid support such as, for example, a chromatography column. The column can then be used to affinity purify antibodies specific for the proteins of the invention from a sample containing antibodies directed against a large number of different epitopes, thereby generating a substantially purified antibody' composition, *i.e*., one that is substantially free of contaminating antibodies. By a substantially purified antibody composition is meant, in this context, that the antibody sample contains at most only 30% (by dry weight) of contaminating antibodies directed against epitopes other than those on the desired protein or polypeptide of the invention, and preferably at most 20%, yet more preferably at most 10%, and most preferably at most 5% (by dry weight) of the sample is contaminating antibodies. A purified antibody composition means that at least 99% of the antibodies in the composition are directed against the desired protein or polypeptide of the invention.

At an appropriate time after immunization, *e*.*g*., when the specific antibody titers are highest, antibody-producing cells can be obtained from the subject and used to prepare monoclonal antibodies by standard techniques, such as the hybridoma technique originally described by Kohler and Milstein (1975) *Nature 256*:495-497, the human B cell hybridoma technique (Kozbor et al. (1983) *Immunol. Today* 4:72), the EBV-hybhdoma technique (Cole et al. (1985), *Monoclonal Antibodies and Cancer Therapy,* Alan R. Liss, Inc., pp. 77-96) or trioma techniques. The technology for producing hybridomas is well known (*see generally Current Protocols in Immunology* (1994) Coligan et al. (eds.) John Wiley *&* Sons, Inc., New York, NY). Hybridoma cells producing a monoclonal antibody of the invention are detected by screening the hybridoma culture supernatants for antibodies that bind the polypeptide of interest, *e.g.,* using a standard ELISA assay.

Alternative to preparing monoclonal antibody-secreting hybridomas, a monoclonal antibody directed against a polypeptide of the invention can be identified and isolated by screening a recombinant combinatorial immunoglobulin library (*e.g.,* an antibody phage display library) with the polypeptide of interest. Kits for generating and screening phage display libraries are commercially available (*e*.*g*., the Pharmacia *Recombinant Phage Antibody System,* Catalog No. 27-9400-01; and the Stratagene *SurfZAP™ Phage Display Kit,* Catalog No. 240612). Additionally, examples of methods and reagents particularly amenable for use in generating and screening antibody display library can be found in, for example, U.S. Patent No. 5,223,409; PCT Publication No. WO 92/18619; PCT Publication No. WO 91/17271; PCT Publication No. WO 92/20791; PCT Publication No. WO 92/15679; PCT Publication No. WO 93/01288; PCT Publication No. WO 92/01047; PCT Publication No. WO 92/09690; PCT Publication No. WO 90/02809; Fuchs et al. (1991) *Bio*/*Technology* 9:1370-1372; Hay et al. (1992) *Hum. Antibod. Hybridomas* 3:81-85; Huse et al. (1989) *Science* 246:1275-1281; Griffiths et al. (1993) *EMBO J.* 12:725-734.

Additionally, recombinant antibodies, such as chimeric and humanized monoclonal antibodies, comprising both human and non-human portions, which can be made using standard recombinant DNA techniques, are within the scope of the invention. A chimeric antibody is a molecule in which different portions are derived from different animal species, such as those having a variable region derived from a murine mAb and a human immunoglobulin constant region. (See, *e*.*g*., Cabilly et al., U.S. Patent No. 4,816,567; and Boss et al., U.S. Patent No. 4,816397, which are incorporated herein by reference in their entirety.) Humanized antibodies are antibody molecules from non-human species having one or more complementarily determining regions (CDRs) from the non-human, species and a framework region from a human immunoglobulin molecule. (See, *e*.*g*., Queen, U.S. Patent No. 5,585,089, which is incorporated herein by reference in its entirety.) Such chimeric and humanized monoclonal antibodies can be produced by recombinant DNA techniques known in the art, for example using methods described in PCT Publication No. WO 87/02671; European Patent Application 184,187; European Patent Application 171,496; European Patent Application 173,494; PCT Publication No. WO 86/01533; U.S. Patent No. 4,816,567; European Patent Application 125,023; Better et al. (1988) *Science* 240:1041-1043; Liu et al. (1987) *Proc. Natl. Acad. Sci. USA* 84:3439-3443; Liu et al. (1987) *J. Immunol*. 139:3521-3526; Sun et al. (1987) *Proc. Natl. Acad. Sci. USA* 84:214-218; Nishimura et al. (1987) *Canc. Res.* 47:999-1005; Wood et al. (1985) *Nature* 314:446-449; and Shaw et al. (1988) *J. Natl. Cancer Inst*. 80:1553-1559); Morrison (1985) *Science* 229:1202-1207; Oi et al. (1986) *Bio*/*Techniques* 4:214; U.S. Patent 5,225,539; Jones et al. (1986) *Nature* 321:552-525; Verhoeyan et al. (1988) *Science* 239:1534; and Beidler et al. (1988) *J. Immunol.* 141:4053-4060.

Completely human antibodies are particularly desirable for therapeutic treatment of human patients. Such antibodies can be produced, for example, using transgenic mice which are incapable of expressing endogenous immunoglobulin heavy and light chains genes, but which can express human heavy and light chain genes. The transgenic mice are immunized in the normal fashion with a selected antigen, *e.g*., all or a portion of a polypeptide of the invention. Monoclonal antibodies directed against the antigen can be obtained using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA and IgE antibodies. For an overview of this technology for producing human antibodies, see Lonberg and Huszar (1995, *Int. Rev. Immunol*. 13:65-93). For a detailed discussion of this technology for producing human antibodies and human monoclonal antibodies and protocols for producing such antibodies, *see, e*.*g*., U.S. Patent 5,625,126; U.S. Patent 5,633,425; U.S. Patent 5,569,825; U.S. Patent 5,661,016; and U.S. Patent 5,545,806, In addition, companies such as Abgenix, Inc. (Fremont, CA), can be engaged to provide human antibodies directed against a selected antigen using technology similar to that described above.

Completely human antibodies which recognize a selected epitope can be generated using a technique referred to as "guided selection." In this approach a selected non-human monoclonal antibody, *e*.*g*., a mouse antibody, is used to guide the selection of a completely human antibody recognizing the same epitope. (Jespers et al. (1994) *Bio*/*technology* 12:899-903).

An antibody directed against a polypeptide of the invention (*e*.*g*., monoclonal antibody) can be used to isolate the polypeptide by standard techniques, such as affinity chromatography or immunoprecipitation. Moreover, such an antibody can be used to detect the protein (*e*.*g*., in a cellular lysate or cell supernatant) in order to evaluate the abundance and pattern of expression of the polypeptide. The antibodies can also be used diagnostically to monitor protein levels in tissue as part of a olinical testing procedure, *e*.*g*., to, for example, determine the efficacy of a given treatment regimen. Detection can be facilitated by coupling the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, allsaline phosphatase, beta-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

Further, an antibody (or fragment thereof) can be conjugated to a therapeutic moicty such as a cytotoxin, a therapeutic agent or a radioactive metal ion. A cytotoxin or cytotoxic agent includes any agent that is detrimental to cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents include, but arc not limited to, antimetabolites (*e*.*g*., methotrexate, 6-mercaptopurine, 6-thioguanine, cytarabine, 5-fluorouracil decarbazine), alkylating agents (*e*.*g*., mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (*e*.*g*., daunorubicin (formerly daunomycin) and doxorubicin), antibiotics (*e*.*g*., dactinomycin (formerly actinomycin), blcomycin, mithramycin, and anthramycin (AMC)), and anti-mitotic agents (*e*.*g*., vincristine and vinblastine).

The conjugates of the invention can be used for modifying a given biological response, the drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, alpha.-interferon, beta.-interferon, nerve growth factor, platelet derived growth factor, tissue plasminogen activator, or, biological response modifiers such as, for example, lymphokines, interleukin-1 ("IL-1"), interleukin-2 ("IL-2"), interleukin-6 ("IL-6"), granulocyte macrophase colony stimulating factor ("GM-CSF"), granulocyte colony stimulating factor ("G-CSF"), or other growth factors.

Techniques for conjugating such therapeutic moiety to antibodies are well known, see, e.g., Amon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies 84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev., 62:119-58 (1982).

Alternatively, an antibody can be conjugated to a second antibody to form an antibody heteroconjugate as described by Segal in U.S. Patent No. 4,676,980.

Accordingly, in one aspect, the invention provides substantially purified antibodies or fragment thereof, and non-human antibodies or fragments thereof, which antibodies or fragments specifically bind to a polypeptide comprising an amino acid sequence selected from the group consisting of: the amino acid sequence of any one of SEQ ID NOs:2, 5, 7, 9, 12, 15, 18, 21, 54, 57, 60, 63, 66, 69, 72, or an amino acid sequence encoded by the cDNA of a clone deposited as any ofATCC 98999, 202171, 98965, and 98966, respectively; a fragment of at least 15 amino acid residues of the amino acid sequence of any one of SEQ ID NOs: 2, 5, 7, 9, 12, 15, 18, 21, 54, 57, 60, 63, 66, 69, 72, an amino acid sequence which is at least 95% identical to the amino acid sequence of any one of SEQ ID NOs: 2, 5, 7, 9, 12, 15, 18, 21, 54, 57, 60, 63, 66, 69, 72, wherein the percent identity is determined using the ALIGN program of the GCG software package with a PAM120 weight residue table, a gap length penalty of 12, and a gap penalty of 4; and an amino acid sequence which is encoded by a nucleic acid molecule which hybridizes to the nucleic acid molecule consisting of any one of SEQ ID NOs:1, 3, 4, 6, 8, 10, 11, 13, 14, 16, 17, 19, 20, 22, 38, 39, 53, 55, 56, 58, 59, 61, 62, 64, 65, 67, 68, 70, 71, 73, or the cDNA of a clone deposited as any of ATCC 98999, 202171, 98965, and 98966, or a complement thereof, under conditions of hybridization of 6X SSC at 45°C and washing in 0.2 X SSC, 0.1% SDS at 65°C. In various embodiments, the substantially purified antibodies of the invention, or fragments thereof, can be human, non-human, chimeric and/or humanized antibodies.

In another aspect, the invention provides non-human antibodies or fragments thereof, which antibodies or fragments specifically bind to a polypeptide comprising an amino acid sequence selected from the group consisting of: the amino acid sequence of any one of SEQ ID NOs:2, 5, 7, 9, 12, 15, 18, 21, 54, 57, 60, 63, 66, 69, 72, or an amino acid sequence encoded by the cDNA of a clone deposited as any of ATCC 98999, 202171, 98965, and 98966, respectively; a fragment of at least 15 amino acid residues of the amino acid sequence of any one of SEQ ID NOs: 2, 5, 7, 9, 12, 15, 18, 21, 54, 57, 60, 63, 66, 69, 72, an amino acid sequence which is at least 95% identical to the amino acid sequence of any one of SEQ ID NOs: 2, 5, 7, 9, 12, 15, 18, 21, 54, 57, 60, 63, 66, 69, 72, wherein the percent identity is determined using the ALIGN program of the GCG software package with a PAM120 weight residue table, a gap length penalty of 12, and a gap penalty of 4; and an amino acid sequence which is encoded by a nucleic acid molecule which hybridizes to the nucleic acid molecule consisting of any one of SEQ ID NOs:1, 3, 4, 6, 8, 10, 11, 13, 14, 16, 17, 19, 20, 22, 38, 39, 53, 55, 56, 58, 59, 61, 62, 64, 65, 67, 68, 70, 71, 73, or the cDNA of a clone deposited as any of ATCC 98999, 202171, 98965, and 98966, or a complement thereof, under conditions of hybridization of 6X SSC at 45°C and washing in 0.2 X SSC, 0.1% SDS at 65°C. Such non-human antibodies can be goat, mouse, sheep, horse, chicken, rabbit, or rat antibodies. Alternatively, the non-human antibodies of the invention can be chimeric and/or humanized antibodies. In addition, the non-human antibodies of the invention can be polyclonal antibodies or monoclonal antibodies.

In still a further aspect, the invention provides monoclonal antibodies or fragments thereof, which antibodies or fragments specifically bind to a polypeptide comprising an amino acid sequence selected from the group consisting of: the amino acid sequence of any one of SEQ ID NOs:2, 5, 7, 9, 12, 15, 18, 21, 54, 57, 60, 63, 66, 69, 72, or an amino acid sequence encoded by the cDNA of a clone deposited as any of ATCC 98999, 202171, 98965, and 98966, respectively; a fragment of at least 15 amino acid residues of the amino acid sequence of any one of SEQ ID NOs: 2, 5, 7, 9, 12, 15, 18, 21, 54, 57, 60, 63, 66, 69, 72, an amino acid sequence which is at least 95% identical to the amino acid sequence of any one of SEQ ID NOs: 2, 5, 7, 9, 12, 15, 18, 21, 54, 57, 60, 63, 66, 69, 72, wherein the percent identity is determined using the ALIGN program of the GCG software package with a PAM120 weight residue table, a gap length penalty of 12, and a gap penalty of 4; and an amino acid sequence which is encoded by a nucleic acid molecule which hybridizes to the nucleic acid molecule consisting of any one of SEQ ID NOs:1, 3, 4, 6, 8, 10, 11, 13, 14, 16, 17, 19, 20, 22, 38, 39, 53, 55, 56, 58, 59, 61, 62, 64, 65, 67, 68, 70, 71, 73, or the cDNA of a clone deposited as any of ATCC 98999, 202171, 98965, and 98966, or a complement thereof, under conditions of hybridization of 6X SSC at 45°C and washing in 0.2 X SSC, 0.1% SDS at 65°C. The monoclonal antibodies can be human, humanized, chimeric and/or non-human antibodies.

The substantially purified antibodies or fragments thereof specifically bind to a signal peptide, a secreted sequence, an extracellular domain, a transmembrane or a cytoplasmic domain cytoplasmic membrane of a polypeptide of the invention. In a particularly preferred embodiment, the substantially purified antibodies or fragments thereof, the non-human antibodies or fragments thereof, and/or the monoclonal antibodies or fragments thereof, of the invention specifically bind to a secreted sequence or an extracellular domain of the amino acid sequence of SEQ ID NOs:2, 5, 9, 12, 15, 18, 66, 21, 126. Preferably, the secreted sequence or extracellular domain to which the antibody, or fragment thereof, binds comprises from about amino acids 23-345 of SEQ ID NO:2 (SEQ ID NO:), from amino acids 1-146 of SEQ ID NO:5 (SEQ ID NO:35), from about amino acids 28-301 of SEQ ID NO:9 (SEQ ID NO:), from about amino acids 19-553 of SEQ ID NO:12 (SEQ ID NO:), from about amino acids 23-271 of SEQ ID NO:15 (SEQ ID NO:), from about amino acids 29-458 of SEQ ID NO:18 (SEQ ID NO:), from about amino acids 29-874 of SEQ ID NO:9 (SEQ ID NO:) and amino acid residues 1 to 146 of SEQ ID NO:35.

Any of the antibodies of the invention can be conjugated to a therapeutic moiety or to a detectable substance. Non-limiting examples of detectable substances that can be conjugated to the antibodies of the invention are an enzyme, a prosthetic group, a fluorescent material, a luminescent material, a bioluminescent material, and a radioactive material.

The invention also provides a kit containing an antibody of the invention conjugated to a detectable substance, and instructions for use. Still another aspect of the invention is a pharmaceutical composition comprising an antibody of the invention and a pharmaceutically acceptable carrier. In preferred embodiments, the pharmaceutical composition contains an antibody of the invention, a therapeutic moiety, and a pharmaceutically acceptable carrier.

Still another aspect of the invention is a method of making an antibody that specifically recognizes TANGO 128, TANGO 140, TANGO 197, TANGO 212, TANGO 213, TANGO 224, and TANGO 239, the method comprising immunizing a mammal with a polypeptide. The polypeptide used as an immungen comprises an amino acid sequence selected from the group consisting of the amino acid sequence of any one of SEQ ID NOs:2, 5, 7, 9, 12, 15, 18, 21, 54, 57, 60, 63, 66, 69, 72, or an amino acid sequence encoded by the cDNA of a clone deposited as any of ATCC 98999, 202171, 98965, and 98966, respectively; a fragment of at least 15 amino acid residues of the amino acid sequence of any one of SEQ ID NOs: 2, 5, 7, 9, 12, 15, 18, 21, 54, 57, 60, 63, 66, 69, 72, an amino acid sequence which is at least 95% identical to the amino acid sequence of any one of SEQ ID NOs: 2, 5, 7, 9, 12, 15, 18, 21, 54, 57, 60, 63, 66, 69, 72, wherein the percent identity is determined using the ALIGN program of the GCG software package with a PAM120 weight residue table, a gap length penalty of 12, and a gap penalty of 4; and an amino acid sequence which is encoded by a nucleic acid molecule which hybridizes to the nucleic acid molecule consisting of any one of SEQ ID NOs:1, 3, 4, 6, 8, 10, 11, 13, 14, 16, 17, 19, 20, 22,38,39,53,55,56.58,59,61,62,64,65,67,68, 70, 71, 73, or the cDNA of a clone deposited as any of ATCC 98999,202171, 98965, and 98966, or a complement thereof, under conditions of hybridization of 6X SSC at 45°C and washing in 02 X SSC, 0.1% SDS at 65°C. After immunization, a sample is collected from the mammal that contains an antibody that specifically recognizes GPVI. Preferably, the polypeptide is recombinantly produced using a non-human host cell, Optionally, the antibodies can be further purified from the sample using techniques well known to those of skill in the art. The method can further comprise producing a monoclonal antibody-producing cell from the cclls of the mammal. Optionally, antibodies are collected from the antibody-producing cell.

### III. Recombinant Expression Vectors and Host Cells

Another aspect of the invention pertains to vectors, preferably expression vectors, containing a nucleic acid encoding a polypeptide of the invention (or a portion thereof). As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors, expression vectors, are capable of directing the expression of genes to which they are operably linked. In general, expression vectors of utility in recombinant DNA techniques are often in the form of plasmids (vectors). However, the invention is intended to include such other forms of expression vectors, such as viral vectors (e.g., replication defective retroviruses, adenoviruses and adeno-associated viruses), which serve equivalent functions.

The recombinant expression vectors of the invention comprise a nucleic acid of the invention in a form suitable for expression of the nucleic acid in a host cell. This means that the recombinant expression vectors include one or more regulatory sequences, selected on the basis of the host cells to be used for expression, which is operably linked to the nucleic acid sequence to be expressed. Within a recombinant expression vector, "operably linked" is intended to mean that the nucleotide sequence of interest is linked to the regulatory sequence(s) in a manner which allows for expression of the nuclobtide sequence (e.g., in an *in vitro* transcription/translation system or in a host cell when the vector is introduced into the host cell). The term "regulatory sequence" is intended to include proinoters, enhancers and other expression control elements (e.g., polyadenylation signals). Such regulatory sequences are described, for example, in Goeddel, Gene *Expression Technology: Methods in Enzymology* 185, Academic Press, San Diego, CA (1990). Regulatory sequences include those which direct constitutive expression of a nucleotide sequence in many types of host cell and those which direct expression of the nucleotide sequence only in certain host cells (e.g., tissue-specific regulatory sequences). It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein.

The recombinant expression vectors of the invention can be designed for expression of a polypeptide of the invention in prokaryotic (e.g., *E. coli*) or eukaryotic cells (e.g., insect cells (using baculovirus expression vectors), yeast cells or mammalian cells). Suitable host cells are discussed further in Goeddel, *supra*. Alternatively, the recombinant expression vector can be transcribed and translated *in vitro,* for example using T7 promoter regulatory sequences and T7 polymerase.

Expression of proteins in prokaryotes is most often carried out in *E. coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith and Johnson (1988) *Gene* 67:31-40), pMAL (New England Biolabs, Beverly, MA) and pRIT5 (Pharmacia, Piscataway, NJ) which fuse glutathione S-transferase (GST), inaltose E binding protein, or protein A, respectively, to the target recombinant protein.

Examples of suitable inducible non-fusion *E. coli* expression vectors include pTrc (Amaan et aL, (1988) *Gene* 69:301-315) and pET 11d (Studier et al., *Gene Expression Technology: Methods in Enzymology* 185, Academic Press, San Diego, California (1990) 60-89). Target gene expression from the pTrc vector relies on host RNA polymerase transcription from a hybrid trp-lac fusion promoter. Target gene expression from the pET 11d vector relies on transcription from a T7 gn10-lac fusion promoter mediated by a coexpressed viral RNA polymeme (T7 gnl). This viral polymerase is supplied by host strains BL21 (DE3) or HMS 174(DE3) from a resident ë prophage harboring a T7 gn1 gene under the transcriptional control of the lacUV 5 promoter.

One strategy to maximize recombinant protein expression in *E. coli* is to express the protein in a host bacteria with an impaired capacity to protsolytically cleave the recombinant protein (Gottesman, *Gene Expression Technology: Methods in Enzymology* 185, Academic Press, San Diego, California (1990) 119-128). Another strategy is to alter the nucleic acid sequence of the nucleic acid to be inserted into an expression vector so that the individual codons for each amino acid are those preferentially utilized in *E. coli* (Wada et al. (1992) *Nucleic Acids Res.* 20:2111-2118). Such alteration of nucleic acid sequences of the invention can be carried out by standard DNA synthesis techniques.

In another embodiment, the expression vector is a yeast expression vector. Examples of vectors for expression in yeast *S. cerivisae* include pYepSec1 (Baldari et al. (1987) *EMBO J.* 6:229-234), pMFa (Kurjan and Herskowitz, (1982) *Cell* 30:933-943), pJRY88 (Schultz et al. (1987) *Gene* 54:113-123), pYES2 (Invitrogen Corporation, San Diego, CA), and pPicZ (Invitrogen Corp, San Diego, CA).

Alternatively, the expression vector is a baculovirus expression vector. Baculovirus vectors available for expression of proteins in cultured insect cells (e.g., Sf9 cells) include the pAc series (Smith et al. (1983) *Mol. Cell Biot* 3:2156-2165) and the pVL series (Lucklow and Summers (1989) *Virology* 170:31-39).

In yet another embodiment, a nucleic acid of the invention is expressed in mammalian cells using a mammalian expression vector. Examples of mammalian expression vectors include pCDM8 (Seed (1987) *Nature* 329:840) and pMT2PC (Kaufman et al. (1987) *EMBO J.* 6:187-195). When used in mammalian cells, the expression vector's control functions are often provided by viral regulatory elements. For example, commonly used promoters are derived from polyoma, Adenovirus 2, cytomegalovirus and Simian Virus 40. For other suitable expression systems for both prokaryotic and eukaryotic cells see chapters 16 and 17 of Sambrook et al., *supra*.

In another embodiment, the recombinant mammalian expression vector is capable of directing expression of the nucleic acid preferentially in a particular cell type (e.g., tissue-specific regulatory elements are used to express the nucleic acid). Tissue-specific regulatory elements are known in the art. Non-limiting examples of suitable tissue-specific promoters include the albumin promoter (liver-specific; Pinkert et al. (1987) *Genes Dev*. 1:268-277), lymphoid-specific promoters (Calauie and Eaton (1988) *Adv. Immunol*. 43:235-275), in particular promoters of T cell receptors (Winoto and Baltimore (1989) *EMBO J.* 8;729-733) and immunoglobulins (Banerji et at (1983) *Cell* 33:729-740; Queen and Baltimore (1983) *Cell* 33:741-748), neuron-specific promoters (e.g., the neurofilament promoter; Byme and Ruddle (1989) *Proc. Natl. Acad. Sci. USA* 86:5473-5477), pancreas-specific promoters (Edlund et al. (1985) *Science* 230:912-916), and mammary gland-specific promoters (e.g., milk whey promoter, U.S. Patent No. 4,873,316 and European Application Publication No. 264,166). Developmentally-regulated promoters are also encompassed, for example the murine hox promoters (Kessel and Cruss (1990) *Science* 249;374-379) and the á-fetoprotein promoter (Campes and Tilghman (1989) *Genes Dev*. 3:537-546).

The invention further provides a recombinant expression vector comprising a DNA molecule of the invention cloned into the expression vector in an antisense orientation. That is, the DNA molecule is operably linked to a regulatory sequence in a manner which allows for expression (by transcription of the DNA molecule) of an RNA molecule which is antisense to the mRNA encoding a polypeptide of the invention. Regulatory sequences operably linked to a nucleic acid cloned in the antisense orientation can be chosen which direct the continuous expression of the antisense RNA molecule in a variety of cell types, for instance viral promoters and/or enhancers, or regulatory sequences can be chosen which direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes see Weintraub et al. (*Reviews - Trends in Genetics,* Vol. 1(1) 1986).

Another aspect of the invention pertains to host cells into which a recombinant expression vector of the invention has been introduced. The terms "host cell" and "recombinant host cell" are used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic (e.g., *E. coli*) or eukaryotic cell (e.g., insect cells, yeast or mammalian cells).

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid into a host cell, including calcium phosphate or calcium chloride coprecipitation, DEAE-dextran-mediated transfection, lipofection, or electroporation. Suitable methods for transforming or transfecting host cells can be found in Sambrook, et al. (*supra*), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (e.g., for resistance to antibiotics) is generally introduced into the host cells along with the gene of interest. Preferred selectable markers include those which confer resistance to drugs, such as G418, hygromycin and methotrexate. Cells stably transfected with the introduced nucleic acid can be identified by drug selection (e.g., cells that have incorporated the selectable marker gene will survive, while the other cells die).

In another embodiment, the expression characteristics of an endogenous (*e*.*g*., TANGO 128, TANGO 140, TANGO 197, TANGO 212, TANGO 213, TANGO 224, and TANGO 239) nucleic acid within a cell, cell line or microorganism may be modified by inserting a DNA regulatory element heterologous to the endogenous gene of interest into the genome of a cell, stable cell line or cloned microorganism such that the inserted regulatory element is operatively linked with the endogenous gene (*e*.*g*., TANGO 128, TANGO 140, TANGO 197, TANGO 212, TANGO 213, TANGO 224, and TANGO 239) and controls, modulates or activates the endogenous gene. For example, endogenous TANGO 128, TANGO 140, TANGO 197, TANGO 212, TANGO 213, TANGO 224, and TANGO 239 which are normally "transcriptionally silent", *i*.*e*., TANGO 128, TANGO 140, TANGO 197, TANGO 212, TANGO 213, TANGO 224, and TANGO 239 genes which are normally not expressed, or are expressed only at very low levels in a cell line or microorganism, may be activated by inserting a regulatory element which is capable of promoting the expression of a normally expressed gene product in that cell line or microorganism. Alternatively, transcriptionally silent, endogenous TANGO 128, TANGO 140, TANGO 197, TANGO 212, TANGO 213, TANGO 224, and TANGO 239 genes may be activated by insertion of a promiscuous regulatory element that works across cell types.

A hetemlogous regulatory element may be inserted into a stable cell line or cloned microorganism, such that it is operatively linked with and activates expression of endogenous TANGO 128, TANGO 140, TANGO 197, TANGO 212, TANGO 213, TANGO 224, and TANGO 239 genes, using techniques, such as targeted homologous recombination, which are well known to those of skill in the art, and described *e.g*., in Chappel, U.S. Patent No. 5,272,071, PCT publication No. WO 91/06667, published May 16, 1991.

A host cell of the invention, such as a prokaryotic or eukaryotic host cell in culture, can be used to produce a polypeptide of the invention. Accordingly, the invention further provides methods for producing a polypeptide of the invention using the host cells of the invention. In one embodiment, the method comprises culturing the host cell of invention (into which a recombinant expression vector encoding a polypeptide of the invention has been introduced) in a suitable medium such that the polypeptide is produced. In another embodiment, the method further comprises isolating the polypeptide from the medium or the host cell.

The host cells of the invention can also be used to produce nonhuman transgenic animals. For example, in one embodiment, a host cell of the invention is a fertilized oocyte or an embryonic stem cell into which a sequences encoding apolypeptide of the invention have been introduced. Such host cells can then be used to create non-human transgenic animals in which exogenous sequences encoding a polypeptide of the invention have been introduced into their genome or homologous recombinant animals in which endogenous encoding a polypeptide of the invention sequences have been altered. Such animals are useful for studying the function and/or activity of the polypeptide and for identifying and/or evaluating modulators of polypeptide activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, amphibians, etc. A transgene is exogenous DNA which is integrated into the genome of a cell from which a transgenic animal develops and which remains in the genome of the mature animal, thereby directing the expression of an encoded gene product in one or more cell types or tissues of the transgenic animal. As used herein, an "homologous recombinant animal" is a non-human animal, preferably a mammal, more preferably a mouse, in which an endogenous gene has been altered by homologous recombination between the endogenous gene and an exogenous DNA molecule introduced into of the animal, e.g., an embryonic cell of the animal, prior to development of the animal.

A transgenic animal of the invention can be created by introducing nucleic acid encoding a polypeptide of the invention (or a homologue thereof) into the male pronuclei of a fertilized oocyte, e.g., by microinjection, retroviral infection, and allowing the oocyte to develop in a pseudopregnant female foster animal. Intronic sequences and polyadenylation signals can also be included in the transgene to increase the efficiency of expression of the transgene. A tissue-specific regulatory sequence(s) can be operably linked to the transgene to direct expression of the polypeptide of the invention to particular cells. Methods for generating transgenic animals via embryo manipulation and microinjection, particularly animals such as mice, have become conventional in the art and are described, for example, in U.S. Patent NOS. 4,736,866 and 4,870,009, U.S. Patent No. 4,873,191 and in Hogan, *Manipulating the Mouse Embryo*, (Cold Spring Harbor Laboratory Press, Cold Spring Harbor, N.Y., 1986). Similar methods are used for production of other transgenic animals. A transgenic founder animal can be identified based upon the presence of the transgene in its genome and/or expression of mRNA encoding the transgene in tissues or cells of the animals, A transgenic founder animal can then be used to breed additional animals carrying the transgeae. Moreover, transgenic animals carrying the transgene can further be bred to other transgenic animals carrying other transgenes.

To create an homologous recombinant animal, a vector is prepared which contains at least a portion of a gene encoding a polypeptide of the invention into which a deletion, addition or substitution has been introduced to thereby alter, e.g., functionally disrupt, the gene. In a preferred embodiment, the vector is designed such that, upon homologous recombination, the endogenous gene is functionally disrupted (i.e., no longer encodes a functional protein; also referred to as a "knock out" vector). Alternatively, the vector can be designed such that, upon homologous recombination, the endogenous gene is mutated or otherwise altered but still encodes functional protein (e.g., the upstream regulatory region can be altered to thereby alter the expression of the endogenous protein). In the homologous recombination vector, the altered potion of the gene is flanked at its 5' and 3' ends by additional nucleic acid of the gene to allow for homologous recombination to occur between the exogenous gene carried by the vector and an endogenous gene in an embryonic stem cell. The additional flanking nucleic acid sequences are of sufficient length for successful homologous recombination with the endogenous gene. Typically, several kilobases of flanking DNA (both at the 5' and 3' ends) are included in the vector (*see*, *e*.*g*., Thomas and Capecchi (1987) *Cell* 51;503 for a description of homologous recombination vectors). The vector is introduced into an embryonic stem cell line (e.g., by electroporation) and cells in which the introduced gene has homologously recombined with the endogenous gene are selected *(see, e.g.,* Li et aL (1992) *Cell* 69:915). The selected cells are then injected into a blastocyst of an animal (e.g., a mouse) to form aggregation chimeras (*see*, *e*.*g*., Bradley in *Teratocarcinomas and Embryonic Stem Cells: A Practical Approach*, Robertson, ed. (IRL, Oxford, 1987) pp. 113-152). A chimeric embryo can then be implanted into a suitable pseudopregnant female foster animal and the embryo brought to term. Progeny harboring the homologously recombined DNA in their germ cells can be used to breed animals in which all cells of the animal contain the homologously recombined DNA by germline transmission of the transgene. Methods for constructing homologous recombination vectors and homologous recombinant animals are described further in Bradley (1991) *Current Opinion in Bio*/*Technology* 2:823-829 and in PCT Publication NOS. WO 90/11354, WO 91/01140, WO 92/0968, and WO 93/04169.

In another embodiment, transgenic non-human animals can be produced which contain selected systems which allow for regulated expression of the transgene. One example of such a system is the *cre*/*loxP* recombinase system of bacteriophage P1. For a description of the *cre*/*loxP* recombinase system, *see, e*.*g*., Lakso et al. (1992) *Proc. Natl. Acad. Sci. USA* 89:6232-6236. Another example of a recombinase system is the FLP recombinase system of *Saccharomyces cerevisiae* (O'Gorman et al. (1991) *Science* 251:1351-1355, If a *cre*/*loxP* recombinase system is used to regulate expression of the transgene, animals containing transgenes encoding both the *Cre* recombinase and a selected protein are required. Such animals can be provided through the construction of "double" transgenic animals, e.g., by mating two tranagenic animals, one containing a transgene encoding a selected protein and the other containing a transgene encoding recombinase.

Clones of the non-human transgenic animals described herein can also be produced according to the methods described in Wilmut et aL (1997) *Nature* 385:810-813 and PCT Publication NOS. WO 97/07668 and WO 97/07669.

### IV. Pharmaceutical Compositions

The nucleic acid molecules, polypeptides, and antibodies (also referred to herein as "active compounds") of the invention can be incorporated into pharmaceutical compositions suitable for administration. Such compositions typically comprise the nucleic acid molecule, protein, or antibody and a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

The invention includes methods for preparing pharmaceutical compositions for modulating the expression or activity of a polypeptide or nucleic acid of the invention. Such methods comprise formulating a pharmaceutically acceptable carrier with an agent which modulates expression or activity of a polypeptide or nucleic acid of the invention. Such compositions can further include additional active agents. Thus, the invention further includes methods for preparing a pharmaceutical composition by formulating a pharmaceutically acceptable carrier with an agent which modulates expression or activity of a polypeptide or nucleic acid of the invention and one or more addtional active compounds.

A pharmaceutical composition of the invention is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxiwmts such as ascorbic acid or sodium bisulfite; chelating agents such as ethylenediaminetetraacetic acid; buffers such as acetates, citrates or phosphates and agents for the adjustment of tonicity such as sodium chloride or dextrose, pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersions. For intravenous administration, suitable carriers include physiological saline, bacteriostatic water, Cremophor EL (BASF; Parsippany, NJ) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol ana the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example; sugais, polyalcohols such as mannitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (e.g., a polypeptide or antibody) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed.

Pharmaceutically compatible binding agents, and/or adjuvant materials can be included as part of the composition. the tablcts, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Ptimogel, or com starch; a lubricant such as magnesium stearate or Sterotcs; a glidant such as colloidal silicon dioxide; a sweetening agent such as sucrose or saccharin; or a flavoring agent such as peppermint, methyl ealicylate, or orange flavoring.

For administration by inhalation, the compounds are delivered in the form of an aerosol spray from a pressurized container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art. The compounds can also be prepared in the form of suppositorics (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydndes, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811. It is especially advantageous to formulate oral or parenteral compositions in dosage unit form. for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

For antibodies, the preferred dosage is 0.1 mg/kg to 100 mg/kg of body weight (generally 10 mg/kg to 20 mg/kg). If the antibody is to act in the brain, a dosage of 50 mg/kg to 100 mg/kg is usually appropriate. Generally, partially human antibodies and fully human antibodies have a longer half-life within the human body than other antibodies. Accordingly, lower dosages and less frequent administration is often possible. Modifications such as lipidation can be used to stabilize antibodies and to enhance uptake and tissue penetration (e.g., into the brain). A method for lipidation of antibodies is described by Cruikshank et al. ((1997) *J. Acquired Immune Deficiency Syndromes and Human Retrovirology* 14:193).

The nucleic acid molecules of the invention can be inserted into vectors and used as gene therapy vectors. Gene therapy vectors can be delivered to a subject by, for example, intravenous injection, local administration (U.S. Patent 5,328,470) or by stereotactic injection (*see*, *e*.*g*., Chen et al. (1994) *Proc. Natl, Acad. Sci. USA* 91:3054-3057). The pharmaceutical preparation of the gene therapy vector can include the gene therapy vector in an acceptable, diluent, or can comprise a slow release matrix in which the gene delivery vehicle is imbedded. Alternatively, where the complete gene delivery vector can be produced intact from recombinant cells, e.g. retroviral vectors, the pharmaceutical preparation can include one or more cells which produce the gene delivery system.

The pharmaceutical compositions can be included in a container, pack, or dispenser together with instructions for administration.

### V. ses and Methods of the Invention

The nucleic acid molecules, proteins, protein homologues, and antibodies described herein can be used in one or more of the following methods: a) screening assays; b) detection assays (e.g., chromosomal mapping, tissue typing, forensic biology); c) predictive medicine (e.g., diagnostic assays, prognostic assays, monitoring clinical trials, and pharmacogenomics); and d) methods of treatment (e.g., therapeutic and prophylactic). For example, polypeptides of the invention can to used to (i) modulate cellular proliferation; (ii) modulate cell migration and chemotaxis; (iii) modulate cellular differentiation; and/of (iv) modulate angiogenesis. The isolated nucleic add molecules of the invention can be used to express proteins (e.g., via a recombinant expression vector in a host cell in gene therapy applications), to detect mRNA (e.g., in a biological sample) or a genetic lesion, and to modulate activity of a polypeptide of the invention. In addition, the polypeptides of the invention can be used to screen drugs or compounds which modulate activity or expression of a polypeptide of the invention as well as to treat disorders characterized by insufficient or excessive production of a protein of the invention or production of a form of a protein of the invention which has decreased or aberrant activity compared to the wild type protein. In addition, the antibodies of the invention can be used to detect and isolate a protein of the and modulate activity of a protein of the invention.

This invention further pertains to novel agents identified by the above-described screening assays and uses thereof for treatments as described herein.

### A. Screening Assays

The invention provides a method (also referred to herein as a "screening assay") for identifying modulators, i.e., candidate or test compounds or agents (e.g., peptides, peptidomimetics, small molecules or other drugs) which bind to polypeptide of the invention or have a stimulatory or inhibitory effect on, for example, expression or activity of a polypeptide of the invention.

In one embodiment, the invention provides assays for screening candidate or test compounds which bind to or modulate the activity of the membrane-bound form of a polypeptide of the invention or biologically active portion thereof The test compounds of the present invention can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds (Lam (1997) *Anticancer Drug Des*. 12:145).

Examples of methods for the synthesis of molecular libraries can be found in the art, for example in: DeWitt et al. (1993) *Proc. Natl. Acad. Sci. USA* 90:6909; Erb et al. (1994) *Proc. Natl. Acad. Sci. USA* 91:11422; Zuckermaan et al. (1994). *J. Med. Chem*. 37:2678; Cho et al. (1993) *Science* 261:1303; Carrell et al. (1994) *Angew. Chem. Int. Ed. Engl*. 33:2059; Carell et al. (1994) *Angew. Chem. Int. Ed. Engl*. 33:2061; and Gallop et al. (1994) *J. Med, Chem*. 37:1233.

Libraries of compounds may be presented in solution (e.g., Houghten (1992) *Bio*/*Techniques* 13:412-421), or on beads (Lam (1991) *Nature* 354:82-84), chips (Fodor (1993) *Nature* 364:555-556), bacteria (U.S. Patent No. 5,223,409), spores (Patent NOS. 5,571,698; 5,403,484; and 5,223,409), plasmids (Cull et al. (1992) *Proc. Natl. Acad. Sci*. *USA* 89:1865-1869) or phage (Scott and Smith (1990) *Science* 249:386-390; Devlin (1990) *Science* 249:404-406; Cwirla et al. (1990) *Proc. Natl. Acad. Sci. USA* 87:6378-6382; and Felici (1991) *J. Mol. Biol*. 222:301-310).

In one embodiment, an assay is a cell-based assay in which a cell which expresses a membrane-bound form of a polypeptide of the invention, or a biologically active portion thereof, on the cell surface is contacted with a test compound and the ability of the test compound to bind to the polypeptide determined. The cell, for example, can be a yeast cell or a cell of mammalian origin. Determining the ability of the test compound to bind to the polypeptide can be accomplished, for example, by coupling the test compound with a radioisotope or enzymatic label such that binding of the test compound to the polypeptide or biologically active portion thereof can be determined by detecting the labeled compound in a complex. For example, test compounds can be labeled with ¹²⁵I, ³⁵S, ¹⁴C, or ³H, either directly or indirectly, and the radioisotope detected by direct counting of radioemmission or by scintillation counting. Alternatively, test compounds can be enzymatically labeled with, for example, horseradish pemxidase, alkaline phosphatase, or luciferase, and the enzymatic label detected by determination of conversion of an appropriate substrate to product. In a preferred embodiment, the assay comprises contacting a cell which expresses a membrane-bound form of a polypeptide of the invention, or a biologically active portion thereof, on the cell surface with a known compound which binds the polypeptide to form an assay mixture, contacting the assay mixture with a test compound, and determining the ability of the test compound to interact with the polypeptide, wherein determining the ability of the test compound to interact with the polypeptide comprises determining the ability of the test compound to preferentially bind to the polypeptide or a biologically active portion thereof as compared to the known compound.

In another embodiment, an assay is a cell-based assay comprising contacting a cell expressing a membrane-bound form of a polypeptide of the invention, or a biologically active portion thereof on the cell surface with a test compound and determining the ability of the test compound to modulate (e.g., stimulate or inhibit) the activity of the polypeptide or biologically active portion thereof. Determining the ability of the test compound to modulate the activity of the polypeptide or a biologically active portion thereof can be accomplished, for example, by determining the ability of the polypeptide protein to bind to or interact with a target molecule.

Determining the ability of a polypeptide of the invention to bind to or interact with a target molecule can be accomplished by one of the methods described above for detesmining direct binding. As used herein, a "target molecule" is a molecule with which a selected polypeptide (e.g., a polypeptide of the invention binds or interacts Within nature, for example, a molecule on the surface of a cell which expresses the selected pivteia, a molecule on the surface of a second cell, a molecule in the extracellular milieu, a molecule associated with the internal surface of a cell membrane or a cytoplasmic molecule. A target molecule can be a polypeptide of the invention or some other polypeptide or protein. For example, a target molecule can be a coicponent of a signal transduction pathway which facilitates transduction of an extracellular signal (e.g., a signal generated by binding of a compound to a polypeptide of the invention) through the cell membrane and into the cell or a second intercellular protein which has catalytic activity or a protein which facilitates the association of downstream signaling molecules with a polypeptide of the invention. Determining the ability of a polypeptide of the invention to bind to or interact with a target molecule can be accomplished by determining the activity of the target molecule. For example, the activity of the target molecule can be determined by detecting induction of a cellular second messenger of the target (e.g., intracellular Ca²⁺, diacylglycerol, IP3, etc.), detecting catalytic/enzymatic activity of the target on an appropriate substrate, detecting the induction of a reporter gene (e.g., a regulatory element that is responsive to a polypeptide of the invention operably linked to a nucleic acid encoding a detectable marker, e.g. luciferase), or detecting a cellular response, for example, cellular differentiation, or cell proliferation.

In yet another embodiment, an assay of the present invention is a cell-free assay comprising contacting a polypeptide of the invention or biologically active portion thereof with a test compound and determining the ability of the test compound to bind to the polypeptide or biologically active portion thereof. Binding of the test compound to the polypeptide can be determined either directly or indirectly as described above. In a preferred embodiment, the assay includes contacting the polypeptide of the invention or biologically active portion thereof with known compound which binds the polypeptide to form an assay mixture, contacting the assay mixture with a test compound, and determining the ability of the test compound to interact with the polypeptide, wherein determining the ability of the test compound to interact with the polypeptide comprises determining the ability of the test compound to preferentially bind to the polypeptide or biologically active portion thereof as compared to the known compound.

In another embodiment, an assay is a cell-free assay comprising contacting a polypeptide of the invention or biologically active portion thereof with a test compound and determining the ability of the test compound to modulate (e.g., stimulate or inhibit) the activity of the polypeptide or biologically active portion thereof. Determining the ability of the test compound to modulate the activity of the polypeptide can be accomplished, for example, by determining the ability of the polypeptide to bind to a target molecule by one of the methods described above for determining direct binding. In an alternative embodiment, determining the ability of the test compound to modulate the activity of the polypeptide can be accomplished by determining the ability of the polypeptide of the invention to further modulate the target molecule. For example, the catalytic/enzymatic activity of the target molecule on an appropriate substrate can be determined as previously described.

In yet another embodiment, the cell-free assay comprises contacting a polypeptide of the invention or biologically active portion thereof with a known compound which binds the polypeptide to form an assay mixture, contacting the assay mixture with a test compound, and determining the ability of the test compound to interact with the polypeptide, wherein determining the ability of the test compound to interact with the polypeptide comprises determining the ability of the polypeptide to preferentially bind to or modulate the activity of a target molecule.

The cell-free assays of the present invention are amenable to use of both a soluble form or the membrane-bound form of a polypeptide of the invention. In the case of cell-free assays comprising the membrane-bound form of the polypeptide, it may be desirable to utilize a solubilizing agent such that the membrane-bound form of the polypeptide is maintained in solution. Examples of such solubilizing agents include non-ionic detergents such as n-octylgluroside, n-dodecylglucoside, n-octylmaltoside, octanoyl-N-methylglucamide, decanoyl-N-methylglucamide, Triton X-100, Triton X-114, Thesit, Isotridecypoly(ethylene glycol ether)n, 3-[(3-cholamidopropyl)dimethylamminio]-1-propane sulfonate (CHAPS), 3-[(3-cholamidopropyl)dimethylamminio]-2-hydroxy-1-propane sulfonate (CHAPSO), or N-dodecyl=N,N-dimethyl-3-ammonio-1-propane sulfonate.

In more than one embodiment of the above assay methods of the present invention, it may be desirable to immobilize either the polypeptide of the invention or its target molecule to facilitate separation of complexed from uncomplexed forms of one or both of the proteins, as well as to accommodate automation of the assay. Binding of a test compound to the polypeptide, or interaction of the polypeptide with a target molecule in the presence and absence of a candidate compound, can be accomplished in any vessel suitable for containing the reartants. Examples of such vessels include microtitre plates, test tubes, and micro-centrifuge tubes. In one embodiment, a fusion protein can be provided which adds a domain that allows one or both of the proteins to be bound to a matrix. For example, glutathione-S-transferase fusion proteins or glutathione-S-transferase fusion proteins can be adsorbed onto glutathione sepharose beads (Sigma Chemical; St. Louis, MO) or glutathione derivatized microtitre plates, which are then combined with the test compound or the test compound and either the non-adsorbed target protein or A polypeptide of the invention, and the mixture incubated under conditions conducive to complex formation (e.g., at physiological conditions for salt and pH). Following incubation, the beads or microtitre plate wells are washed to remove any unbound components and complex formation is measured either directly or indirectly, for example, as described above. Alternatively, the complexes can be dissociated from the matrix, and the level of binding or activity of the polypeptide of the invention can be determined using, standard techniques.

Other techniques for immobilizing proteins on matrices can also be used in the screening assays of the invention. For example, either the polypeptide of the invention or its target molecule can be immobilized utilizing conjugation ofbiotin and streptavidin. Biotinylated polypeptide of the invention or target molecules can be prepared from biotin-NHS (N-hydroxy-succinimide) using techniques well known in the art (e.g., biotinylation kit, Pierce Chemicals; Rockford, IL), and immobilized in the wells of streptavidin-coated 96 well plates (Pierce Chemical). Alternatively, antibodies reactive with the polypeptide of the invention or target molecules but which do not interfere with binding of the polypeptide of the invention to its target molecule can be derivatized to the wells of the plate, and unbound target or polypeptidede of the invention trapped in the wells by antibody conjugation. Methods for detecting such complexes, in addition to those described above for the GST-immobilized complexes, include immunodetection of complexes using antibodies reactive with the polypeptide of the invention or target molecule, as well as enzyme-linked assays which rely on detecting an enzymatic activity associated with the polypeptide of the invention or target molecule.

In another embodiment, modulators of expression of a polypeptide of the invention are identified in a method in which a cell is contacted with a candidate compound and the expression of the selected mRNA or protein (i.e., the mRNA or protein corresponding to a polypeptide or nucleic acid of the invention) in the cell is determined. The level of expression of the selected mRNA or protein in the presence of the candidate compound is compared to the level of expression of the selected mRNA or protein in the absence of the candidate compound. The candidate compound can then be identified as a modulator of expression of the polypeptide of the invention based on this comparison. For example, when expression of the selected mRNA or protein is greater (statistically significantly greater) in the presence of the candidate compound than in its absence, the candidate compound is identified as a stimulator of the selected mRNA or protein expression. Alternatively, when expression of the selected mRNA or protein is less (statistically significantly less) in the presence of the candidate compound than in its absence, the candidate compound is identified as an inhibitor of the selected mRNA or protein expression. The level of the selected mRNA or protein expression in the cells can be determined by methods described herein.

In yet another aspect of the invention, a polypeptide of the inventions can be used as "bait proteins" in a two-hybrid assay or three hybrid assay (*see*, *e.g.,* U.S. Patent No. 5,283,317; Zervos et al. (1993) *Cell* 72:223-232; Madura et al. (1993) *J*. *Biol. Chem.* 268:12046-12054; Bartel et al. (1993) *Bio*/*Techniques* 14:920-924; Iwabuchi et al. (1993) *Oncogene* 8:1693-1696; and PCT Publication No. WO 94/10300), to identify other proteins, which bind to or interact with the polypeptide of the invention and modulate activity of the polypeptide of the invention. Such binding proteins are also likely to be involved in the propagation of signals by the polypeptide of the inventions as, for example; upstream or downstream elements of a signaling Pathway involving the polypeptide of the invention.

This invention further pertains to novel agents identified by the above-described screening assays and uses thereof for treatments as described herein.

### B. Detection Assays

Portions or fragments of the cDNA sequences identified herein (and the corresponding complete gene sequences) can be used in numerous ways as polynucleotide reagents. For example, these sequences can be used to: (i) map their respective genes on a chromosome and, thus, locate gene regions associated with genetic disease; (ii) identify an individual from a minute biological sample (tissue typing); and (iii) aid in forensic identification of a biological sample. These applications are described in the subsections below.

### 1. Chromosome Mapping

Once the sequence (or a portion of the sequence) of a gene has been isolated, this sequence can be used to map the location of the gene on a chromosome. Accordingly, nucleic acid molecules described herein or fragments thereof, can be used to map the location of the corresponding genes on a chromosome. The mapping of the sequences to chromosomes is an important first step in correlating these sequences with genes associated with disease.

Briefly, genes can be mapped to chromosomes by preparing PCR primers (preferably 15-25 bp in length) from the sequence of a gene of the invention. Computer analysis of the sequence of a gene of the invention can be used to rapidly select primers that do not span more than one exon in the genomic DNA, thus complicating the amplification process. These primers can then be used for PCR screening of somatic cell hybrids containing individual human chromosomes. Only those hybrids containing the human gene corresponding to the gene sequences will yield an amplified fragment. For a review of this technique, see D'Eustachio et al. ((1983) *Science* 220:919-924).

PCR mapping of somatic cell hybrids is a rapid procedure for assigning a particular sequence to a particular chromosome. Three or more sequences can be assigned per day using a single thermal cycler. Using the nucleic acid sequences of the invention to design oligonucleotide primers, sublocalization can be achieved with panels of fragments from specific chromosomes. Other mapping strategies which can similarly be used to map a gene to its chromosome include *in situ* hybridization (described in Fan et al. (1990) *Proc. Natl. Acad. Sci. USA* 87:6223-27), pre-screening with labeled flow-sorted chromosomes, and pre-selection by hybridization to chromosome specific cDNA libraries. Fluorescence *in situ* hybridization (FISH) of a DNA sequence to a metaphase chromosomal spread can further be used to provide a precise chromosomal location in one step. For a review of this technique, see Verma et al. (Human Chromosomes: A Manual of Basic Techniques (Pergamon Press, New York, 1988)).

Reagents for chromosome mapping can be used individually to mark a single chromosome or a single site on that chromosome, or panels of reagents can be used for marking multiple sites and/or multiple chromosomes. Reagents corresponding to noncoding regions of the genes actually are preferred for mapping purposes. Coding sequences are more likely to be conserved within gene families, thus increasing the chance of cross hybridizations during chromosomal mapping.

Once a sequence has been mapped to a precise chromosomal location, the physical position of the sequence on the chromosome can be correlated with genetic map data. (Such data are found, for example, in V. McKusick, Mendelian Inheritance in Man, available on-line through Johns Hopkins University Welch Medical Library). The relationship between genes and disease, mapped to the same chromosomal region, can then be identified through linkage analysis (co-inheritance of physically adjacent genes), described in, e.g., Egeland et al. (1987) *Nature* 325:783-787.

Moreover, differences in the DNA sequences between individuals affected and unaffected with a disease associated with a gene of the invention can be determined. If a mutation is observed in some or all of the affected individuals but not in any unaffected individuals, then the mutation is likely to be the causative agent of the particular disease. Comparison of affected and unaffected individuals generally involves first looking for structural alterations in the chromosomes such as deletions or translocations that are visible from chromosome spreads or detectable using PCR based on that DNA sequence. Ultimately, complete sequencing of genes from several individuals can be performed to confirm the presence of a mutation and to distinguish mutations from polymorphisms.

In the instant case, the human gene for TANGO 128 was mapped on radiation hybrid panels to the long arm of chromosome 4, in the region q28-31. Flanking markers for this region are WI-3936 and AFMCO27ZB9. The FGC (fibrinogen gene cluster), GYP (glycophorin cluster), IL15 (interlukin 15), TDO2 (tryptophab oxygenase), and MLR (mineralcorticoid receptor) genes also map to this region of the human chromosome. This region is syntenic to mouse chromosome 8, The Q (quinky), pdw (proportional dwarf), and lyll (lymphoblastomic leukemia) loci also map to this region of the mouse chromosome. I115 (interlukin 15), mlr (mineralcorticoid receptor), ucp (uncoupling protein), and clgn (calmegin) genes also map to this region of the mouse chromosome.

In the instant case, the human gene for TANGO 213 was mapped on radiation hybrid panels to the long arm of chromosome 17, in the region p13.3. Flanking markers for this region are WI-5436 and WI-6584. The MDCR (Miller-Dieker syndrome), PEDF (pigment epithelium derived factor), and PFN1(profillin 1) genes also map to this region of the human chromosome. This region is syntenic to mouse chromosome 11, locus 46(g). The ti (tipsy) loci also maps to this region of the mouse chromosome. The pfn1 (profilin 1), htt (5-hydroxytryptamine (serotonin) transporter), acrb (acetylcholine receptor beta) genes also map to this region of the mouse chromosome.

### 2. Tissue Typing

The nucleic acid sequences of the present invention can also be used to identify individuals from minute biological samples. The United States military, for example, is considering the use of restriction fragment length polymorphism (RFLP) for identification of its personnel. In this technique, an individual's genomic DNA is digested with one or more restriction enzymes, and probed on a Southern blot to yield unique bands for identification. This method does not suffer from the current limitations of "Dog Tags" which can be lost, switched, or stolen, making positive identification difficult. The sequences of the present invention are useful as additional DNA markers for RFLP (described in U.S. Patent 5,272,057).

Furthermore, the sequences of the present invention can be used to provide an alternative technique which determines the actual base-by-base DNA sequence of selected portions of an individual's genome. Thus, the nucleic acid sequences described herein can be used to prepare two PCR primers from the 5' and 3' ends of the sequences. These primers can then be used to amplify an individual's DNA and subsequently sequence it.

Panels of corresponding DNA sequences from individuals, prepared in this manner, can provide unique individual identifications, as each individual will have a unique set of such DNA sequences due to allelic differences. The sequences of the present invention can be used to obtain such identification sequences from individuals and from tissue. The nucleic acid sequences of the invention uniquely represent portions of the human genome. Allelic variation occurs to some degree in the coding regions of these sequences, and to a greater degree in the noncoding regions. It is estimated that allelic variation between individual humans occurs with a frequency of about once per each 500 bases. Each of the sequences described herein can, to some degree, be used as a standard against which DNA from an individual can be compared for identification purposes. Because greater numbers of polymorphisms occur in the noncoding regions, fewer sequences are necessary to differentiate individuals. The noncoding sequences of SEQ ID NO:1, 4, 6, 8, 11, 14, 17, 20, 53, 56, 59, 62, 65, or 68 can comfortably provide positive individual identification with a panel of perhaps 10 to 1,000 primers which each yield a noncoding amplified sequence of 100 bases. If predicted coding sequences, such as those in SEQ ID NO:3, 10, 13, 16, 19, 22, 38, 3955, 58, 61, 64, 67, 70, or 73, are used, a more appropriate number of primers for positive individual identification would be 500-2,000.

If a panel of reagents from the nucleic acid sequences described herein is used to generate a unique identification database for an individual, those same reagents can later be used to identify tissue from that individual. Using the unique identification database, positive identification of the individual, living or dead, can be made from extremely small tissue samples.

### 3. Use of Partial Gene Sequences in Forensic Biology

DNA-based identification techniques can. also be used in forensic biology. Forensic biology is a scientific field employing genetic typing of biological evidence found at a crime scene as a means for positively identifying, for example, a perpetrator of a crime. To make such an idctttification, PCR technology can be used to amplify DNA sequences taken from very small biological samples such as tissues, e.g., hair or skin, or body fluids, e.g., blood, saliva, or semen found at a crime scene. The, amplified sequence can then be compared to a standard, thèreby allowing identification of the origin of the biological sample.

The sequences of the present invention can be used to provide polynucleotide reagents, e.g., PCR primers, targeted to specific loci in the human genome, which can enhance the reliability of DNA-based forensic identifications by, for example, providing another "identification marlcer" (i.e. another DNA sequence that is unique to a particular individual). As mentioned above, actual base sequence information can be used for identification as an accurate alternative to patterns formed by restriction enzyme generated fragments. Sequences targeted to noncoding regions are particularly appropriate for this use as greater numbers of polymorphisms occur in the noncoding regions, making it easier to differentiate individuals using this technique, Examples of polynucleotide reagents include the nucleic acid sequences of the invention or portions thereof, e.g., fragments derived from noncoding regions having a length of at least 20 or 30 bases.

The nucleic acid sequences described herein can further be used to provide polynucleotide reagents, e.g., labeled or labelable probes which can be used in, for example, an *in situ* hybridization technique, to identify a specific tissue, e.g., brain tissue. This can be very useful in cases where a forensic pathologist is presented with a tissue of unknown origin. Panels of such probes can be used to identify tissue by species and/or by organ type.

### C. Predictive Medicine

The present invention also pertains to the field of predictive medicine in which diagnostic assays, prognostic assays, pharmacogenomics, and monitoring clinical trails are used for prognostic (predictive) purposes to thereby treat an individual prophylactically. Accordingly, one aspect of the present invention relates to diaghosdc assays for determining expression of a polypeptide or nucleic acid of the invention and/or activity of a polypeptide of the invention, in the context of a biological sample (e.g., blood, serum, cells, tissue) to thereby determine whether an individual is afflicted with a disease or disorder, or is at risk of developing a disorder, associated with aberrant expression or activity of a polypeptide of the invention. The invention also provides for prognostic (or predictive) assays for determining whether an individual is at risk of developing a disorder associated with aberrant expression or activity of a polypeptide of the invention. For example, mutations in a gene of the invention can be assayed in a biological sample. Such assays can be used for prognostic or predictive purpose to thereby prophylactically treat an individual prior to the onset of a disorder characterized by or associated with aberrant expression or activity of a polypeptide of the invention.

Another aspect of the invention provides methods for expression of a nucleic acid or polypeptide of the invention or activity of a polypeptide of the invention in an individual to thereby select appropriate therapeutic or prophylactic agents for that individual (referred to herein as "pharmacogenomics"). Pharmacogenomics allows for the selection of agents (e.g., drugs) for therapeutic or prophylactic treatment of an individual based on the genotype of the individual (e.g., the genotype of the individual examined to determine the ability of the individual to respond to a particular agent).

Yet another aspect of the invention pertains to monitoring the influence of agents (e.g., drugs or other compounds) on the expression or activity of a polypeptide of the invention in clinical trials. These and other agents are described in further detail in the following sections.

### 1. Diagnostic Assays

An exemplary method for detecting the presence or absence of a polypeptide or nucleic acid of the invention in a biological sample involves obtaining a biological sample from a test subject and contacting the biological sample with a compound or an agent capable of detecting a polypeptide or nucleic acid (e.g., mRNA, genomic DNA) of the invention such that the presence of a polypeptide or nucleic acid of the invention is detected in the biological sample. A preferred agent for detecting mRNA or genomic DNA encoding a polypeptide of the invention is a labeled nucleic acid probe capable of hybridizing to mRNA or genomic DNA encoding a polypeptide of the invention. The nucleic acid probe can be, for example, a full-length cDNA, such as the nucleic acid of SEQ ID NO:1, 8, 11, 14, 17 or 20, or a portion thereof, such as an oligonucleotide of at least 15, 30, 50, 100, 250 or 500 nucleotides in length and sufficient to specifically hybridize under stringent conditions to a mRNA or genomic DNA encoding a polypeptide of the invention. Other suitable probes for use in the diagnostic assays of the invention are described herein.

A preferred agent for detecting a polypeptide of the invention is an antibody capable of binding to a polypeptide of the invention, preferably an antibody with a detectable label. Antibodies can be polyclonal, or more preferably, monoclonal. An intact antibody, or a fragment thereof (e.g., Fab or F(ab')₂) can be used. The term "labeled", with regard to the probe or antibody, is intended to encompass direct labeling of the probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin. The term "biological sample" is intended to include tissues, cells and biological fluids isolated from a subject, as well as tissues, cells and fluids present within a subject, That is, the detection method of the invention can be used to detect mRNA, protein, or genomic DNA in a biological sample *in vitro* as well as *in vivo.* For example, *in vitro* techniques for detection of mRNA include Northern hybridizations and *in situ* hybridizations. *In vitro* techniques for detection of a polypeptide of the invention include enzyme linked immunosorbent assays (ELISAs), Western blots, immunoprecipitations and immunofluorescence. *In vitro* techniques for detection of genomic DNA include Southern hybridizations. Furthermore, *in vivo* techniques for detection of a polypeptide of the invention include introducing into a subject a labeled antibody directed against the polypeptide. For example, the antibody can be labeled with a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject. A Preferred biological sample is a peripheral blood leukocyte sample isolated by conventional means from a subject.

In another embodiment, the methods further involve obtaining a control biological sample from a control subject, contacting the control sample with a compound or agent capable of detecting a polypeptide of the invention or mRNA or genomic DNA encoding a polypeptide of the invention, such that the presence of the polypeptide or mRNA or genomic DNA encoding the polypeptide is detected in the biological sample, and comparing the presence of the polypeptide or mRNA or genomic DNA encoding the polypeptide in the control sample with the presence of the polypeptide or mRNA or genomic DNA encoding the polypeptide in the test sample.

The invention also encompasses kits for detecting the presence of a polypeptide or nucleic acid of the invention in a biological sample (a test sample). Such kits can be used to determine if a subject is suffering from or is at increased risk of developing disorder associated with aberrant expression of a polypeptide of the invention (e.g., a proliferative disorder, e.g., psoriasis or cancer). For example, the kit can comprise a labeled compound or agent capable of detecting the polypeptide or mRNA encoding the polypeptide in a biological sample and means for determining the amount of the polypeptide or mRNA in the sample (e.g., an antibody which binds the polypeptide or an oligonucleotide probe which binds to DNA or mRNA encoding the polypeptide). Kits may also include instructions for observing that the tested subject is suffering from or is at risk of developing a disorder associated with aberrant expression of the polypeptide if the amount of the polypeptide or mRNA encoding the polypeptide is above or below a normal level.

For antibody-based kits, the kit may comprise, for example: (1) a first antibody (e.g., attached to a solid support) which binds to a polypeptide of the invention; and, optionally, (2) a second, different antibody which binds to either the polypeptide or the first antibody and is conjugated to a detectable agent.

For oligonucleotide-based kits, the kit may comprise, for example: (1) an oligonucleotide, e.g., a detectably labeled oligonucleotide, which hybridizes to a nucleic acid sequence encoding a polypeptide of the invention or (2) a pair of primers useful for amplifying a nucleic acid molecule encoding a polypeptide of the invention. The kit may also comprise, e.g., a buffering agent, a preservative, or a protein stabilizing agent. The kit may also comprise components necessary for detecting the detectable agent (e.g., an enzyme or a substrate). The kit may also contain a control sample or a series of control samples which can be assayed and compared to the test sample contained. Each component of the kit is usually enclosed within an individual container and all of the various containers are within a single package along with instructions for observing whether the tested subject is suffering from or is at risk of developing a disorder associated with aberrant expression of the polypeptide.

### 2. Prognostic Assays

The methods described herein can furthermore be utilized as diagnostic or prognostic assays to identify subjects having or at risk of developing a disease or disorder associated with aberrant expression or activity of a polypeptide of the invention. For example, the assays described herein, such as the preceding diagnostic assays or the following assays, can be utilized to identify a subject having or at risk of developing a disorder associated with aberrant expression or activity of a polypeptide of the invention, e.g., a proliferative disorder, e.g., psoriasis or cancer, or an angiogenic disorder. Alternatively, the prognostic assays can be utilized to identify a subject having or at risk for developing such a disease or disorder. Thus, the present invention provides a method in which a test sample is obtained from a subject and a polypeptide or nucleic acid (e.g., mRNA, genomic DNA) of the invention is detected, wherein the presence of the polypeptide or nucleic acid is diagnostic for a subject having or at risk of developing a disease or disorder associated with aberrant expression or activity of the polypeptide. As used herein, a "test sample" refers to a biological sample obtained from a subj ect of interest. For example, a test sample can be a biological fluid (e.g., serum), cell sample, or tissue.

Furthermore, the prognostic assays described herein can be used to determine whether a subject can be administered an agent (e.g., an agonist, antagonist, peptidomimetic, protein, peptide, nucleic acid, small molecule, or other drug candidate) to treat a disease or disorder associated with aberrant expression or activity of a polypeptide of the invention. For example, such methods can be used to determine whether a subject can be effectively treated with a specific agent or class of agents (e.g., agents of a type which decrease activity of the polypeptide). Thus, the present invention provides methods for determining whether a subject can be effectively treated with an agent for a disorder associated with aberrant expression or activity of a polypeptide of the invention in which a test sample is obtained and the polypeptide or nucleic acid encoding the polypeptide is detected (e.g., wherein the presence of the polypeptide or nucleic acid is diagnostic for a subject that can be administered the agent to treat a disorder associated with aberrant expression or activity of the polypeptide).

The methods of the invention can also be used to detect genetic lesions or mutations in a gene of the invention, thereby determining if a subject with the lesioned gene is at risk for a disorder characterized aberrant expression or activity of a polypeptide of the invention. In preferred embodiments, the methods include detecting, in a sample of cells from the subject, the presence or absence of a genetic lesion or mutation characterized by at least one of an alteration affecting the integrity of a gene encoding the polypeptide of the invention, or the mis-expression of the gone encoding the polypeptide of the invention. For example, such genetic lesions or mutations can be detected by ascertaining the existence of at least one of: 1) a deletion of one or more nucleotides from the gene; 2) an addition of one or more nucleotides to the gene; 3) a substitution of one or more nucleotides of the gene; 4) a chromosomal rearrangement of the gene; 5) an alteration in the level of a messenger RNA transcript of the gene; 6) an aberrant modification of the gene, such as of the methylation pattern of the genomic DNA; 7) the presence of a non-wild type splicing pattern of a messenger RNA transcript of the gene; 8) a non-wild type level of a the protein encoded by the gene; 9) an allelic loss of the gene; and 10) an inappropriate post-translational modification of the protein encoded by the gene. As described herein, there are a large number of assay techniques known in the art which can be used for detecting lesions in a gene.

In certain embodiments, detection of the lesion involves the use of a probe/primer in a polymerase chain reaction (PCR) (*see*, *e.g*., U.S. Patent Nos. 4,683,195 and 4,683,202), such as anchor PCR or RACE PCR, or, altemativoly, in a ligation chain reaction (LCR) (*see, e*.*g*., Landogran et al. (1988) *Science* 241:1077-1080; and Nakazawa et al. (1994) *Proc. Natl. Acad. Sci. USA* 91:360-364), the latter of which' can be particularly useful for detecting point mutations in a gene (*see*, *e*.*g*., Abravaya et al. (1995) *Nucleic Acids Res*. 23:675-682). This method can include the steps of collecting a sample of cells from a patient, isolating nucleic acid (e.g., genomic, mRNA or both) from the cells of the sample, contacting the nucleic acid sample with one or more primers which specifically hybridize to the selected gene under conditions such that hybridization and amplification of the gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (Guatelli et al. (1990) *Proc. Natl. Acad, Sci. USA.* 87:1874-1878), transcriptional amplification system (Kwoh, et al. (1989) *Proc. Natl. Acad. Sci, USA* 86:1173-1177), Q-Beta Replicase (Lizardi et al. (1988) *Bio*/*Technology* 6:1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in a selected gene from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes (*see, e*.*g*., U.S. Patent No. 5,498,531) can be used to score for the presence of specific mutations by development or loss of a ribozyme cleavage site.

In other embodiments, genetic mutations can be identified by hybridizing a sample and control nucleic acids, e.g., DNA or RNA, to high density arrays containing hundreds or thousands of oligonucleotides probes (Cronin et al. (1996) *Human Mutation* 7:244-255; Kozal et al. (1996) *Nature Medicine* 2:753-759). For example, genetic mutations can be identified in two-dunsasional arrays containing light-generated DNA probes as described in Cronin et al., *supra*. Briefly, a first hybridization array of probes can be used to scan through long stretches of DNA in a sample and control to identify base changes between the sequences by making linear arrays of sequential overlapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the selected gene and detect mutations by comparing the sequence of the sample nucleic acids with the corresponding wild-type (control) sequence. Examples of sequencing reactions include those based on techniques developed by Maxim and Gilbert ((1977) *Proc. Natl. Acad. Sci. USA* 74:560) or Sanger ((1977) *Proc. Natl. Acad. Sci. USA* 74:5463). It is also contemplated that any of a variety of automated sequencing procedures can be utilized when performing the diagnostic assays ((1995) *Bio*/*Techniques* 19:448), including sequetlciag by mess spectrometry (*see, e*.*g*., PCT Publication No. WO 94/16101; Cohen et al. (1996) *Adv. Chromatogr*. 36:127-162; and Griffin et al. (1993) *Appl. Biochem. Biotechnol*. 38:147-159).

Other methods for detecting mutations in a selected gene include methods in which protection from cleavage agents is used to detect mismatched bases in RNA/RNA or RNA/DNA heteroduplexes (Myers et al. (1985) *Science* 230:1242); In general, the technique of mismatch cleavage entails providing heteroduplexes formed by hybridizing (labeled) RNA or DNA containing the wild-type sequence with potentially mutant RNA or DNA obtained from a tissue sample. The double-stranded duplexes are treated with an agent which cleaves single-stranded regions of the duplex such as which will exist due to basepair mismatches between the control and sample strands. RNA/DNA duplexes can be treated with RNase to digest mismatched regions, and DNA/DNA hybrids can be treated with S1 nuclease to digest mismatched regions.

In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. *See*, *e*.*g*., Cotton et al. (1988) *Proc. Natl. Acad. Sci. USA* 85:4397; Saleeba et al. (1992) *Methods Enzymol*. 217:286-295. In a preferred embodiment, the control DNA or RNA can be labeled for detection.

In still another embodiment, the mismatch cleavage reaction employs one or more proteins that recognize mismatched base pairs in double-stranded DNA (so called DNA mismatch repair enzymes) in defined systems for detecting and mapping point mutations in cDNAs obtained from samples of cells. For example, the mutY enzyme of E. coli cleaves A at G/A mismatches and the thymidine DNA glycosylase from HeLa cells cleaves T at G/T mismatches (Hsu et al. (1994) *Carcinogenesis* 15:1657-1662). According to an exemplary embodiment, a probe based on a selected sequence, e.g., a wild-type sequence, is hybridized to a cDNA or other DNA product from a test cell(s). The duplex is treated with a DNA mismatch repair enzyme, and the cleavage products, if any, can be detected from, electrophoresis. protocols or the like. *See, e.g*., U.S. Patent No. 5,459,039.

In other embodiments, alterations in electrophoretic mobility will be used to identify mutations in genes. For example, single strand conformation polymorphism (SSCP) may be used to detect differences in electrophoretic mobility between mutant and wild type nucleic acids (Orita et al. (1989) *Proc. Natl. Acad. Sci. USA* 86:2766; *see also* Cotton (1993) *Mutat. Res*. 285:125-144; Hayashi (1992) *Genet. Anal. Tech., Appl.* 9:73-79). Single-stranded DNA fragments of sample and control nucleic acids will be denatured and allowed to renature. The secondary structure of single-smmded nucleic adds varies according to sequence, and the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a chaage in sequence. In a preferred embodiment, the subject method utilizes heteroduplex analysis to separate double stranded heteroduplex molecules on the basis of changes in electrophoretic mobility (Keen et al. (1991) *Trends Genet.* 7:5).

In yet another embodiment, the movement of mutant or wild-type fragments in polyacrylamide gels containing a gradient of denaturant is assayed using denaturing gradient gel electrophoresis (DGGE) (Myers et al. (1985) *Nature* 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA (Rosenbaum and Reissner (1987) *Biophys. Chem.* 265:12753).

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions which permit hybridization only if a perfect match is found (Saiki et al. (1986) *Nature* 324:163); Saiki et al. (1989) *Proc. Natl. Acad. Sci. USA* 86:6230). Such allele specific oligonucleotides are hybridized to PCR amplified target DNA or a number of different mutations when the oligonucleotides are attached to the hybridizing membrane and hybridized with labeled target DNA.

Alternatively, allele specific amplification technology which depends on selective PCR amplification may be used in conjunction with the instant invention. Oligonucleotides used as primers for specific amplification may cany the mutation of interest in the center of the molecule (so that amplification depends on differential hybridization) (Gbbs et al. (1989) *Nucleic Acids Res.* 17:2437-2448) or at the extreme 3' end of one primer where, under appropriate conditions, mismatch can prevent or reduce polymerase extension (Prossner (1993) *Tibtech* 11:238). In addition, it may be desirable to introduce a novel restriction site in the region of the mutation to create cleavage-based detection (Gasparini et al. (1992) *Mol. Cell Probes* 6:1). It is anticipated that in certain embodiments amplification may also be performed using Taq ligase for amplification (Barany (1991) *Proc. Nati. Acad. Sci. USA* 88:189). In such cases, ligation will occur only if there is a perfect match at the 3' end of the 5' sequence making it possible to detect the presence of a known mutation at a specific site by looking for the presence or absence of amplification.

The methods described herein may be performed, for example, by utilizing pre-packaged diagnostic kits comprising at least one probe nucleic acid or antibody reagent described herein, which may be conveniently used, e.g., in clinical settings to diagnose patients exhibiting symptoms or family history of a disease or illness involving a gene encoding a polypeptide of the invention. Furthermore, any cell type or tissue, preferably peripheral blood leukocytes, in which the polypeptide of the invention is expressed may be utilized in the prognostic assays described herein.

### 3. Pharmacogenomics

Agents, or modulators which have a stimulatory or inhibitory effect on activity or expression of a polypeptide of the invention as identified by a screening assay described herein can be administered to individuals to treat (prophylactically or therapeutically) disorders associated with aberrant activity of the polypeptide. In conjunction with such treatment, the pharmacogenomics (i.e., the study of the relationship between an individual's genotype and that individual's response to a foreign compound or drug) of the individual may be considered. Differences in metabolism of therapeutics can lead to severe toxicity or therapeutic failure by altering the relation between dose and blood concentration of the pharmacologically active drug. Thus, the pharmacogenomics of the individual permits the selection of effective agents (e.g., drugs) for prophylactic or therapeutic treatments based on a consideration of the individual's genotype. Such pharmacogenomics can further be used to determine appropriate dosages and therapeutic regimens. Accordingly, the activity of a polypeptide of the invention, expression of a nucleic acid of the invention, or mutation content of a gene of the invention in an individual can be determined to thereby select appropriate agent(s) for therapeutic or prophylactic treatment of the individual.

Pharmacogonomics deals with clinically significant hereditary variations in the response to drugs due to altered drug disposition and abnormal action in affected persons. *See, e*.*g*., Linder (1997) *Clin. Chem*. 43(2):254-266. In general, two types of pharmacogenetic conditions can be differentiated. Genetic conditions transmitted as a single factor altering the way drugs act on the body are referred to as "altered drug action." Genetic conditions transmitted as single factors altering the way the body acts on drugs are referred to as "altered drug metabolism". These pharmacogenetic conditions can occur either as rare defects or as polymorphisms. For example, glucose-6-phosphate dehydrogenase deficiency (G6PD) is a common inherited enzymopathy in which the main clinical complication is haemolysis after ingestion of oxidant drugs (anti-malarials, sulfonamides, analgesics, nitrofurans) and consumption of fava beans.

As an illustrative embodiment, the activity of drug metabolizing enzymes is a major determinant of both the intensity and duration of drug action. The discovery of genetic polymorphisms of drug metabolizing enzymes (e.g., N-acetyltransferase 2 (NAT 2) and cytochrome P450 enzymes CYP2D6 and CYP2C19) has provided an explanation as to why some patients do not obtain the expected drug effects or show exaggerated drug response and serious toxicity after taking the standard and safe dose of a drug. These polymorphisms are expressed in two phenotypes in the population, the extensive metabolizer (EM) and poor metabolizer (PM). The prevalence of PM is different among different populations. For example, the gene coding for CYP2D6 is highly polymorphic and several mutation have been identified in PM, which all lead to the absence of functional CYP2D6. Poor metabolizsrs of CYP2D6 and CYP2C19 quite frequently experience exaggerated drug response and side effects when they receive standard doses. If a metabolite is the active therapeutic moiety, a PM will show no therapeutic response, as demonstrated for the analgesic effect of codeine mediated by its CYP2D6-formed metabolite morphine. The other extreme are the so called ultra-rapid metabolizers who do not respond to standard doses. Recently, the molecular basis of ultra-rapid metabolism has been identified to be due to CYP2D6 gene amplification.

Thus, the activity of a polypeptide of the invention, expression of a nucleic acid encoding the polypeptide, or mutation content of a gene encoding the polypeptide in an individual can be determined to thereby select appropriate agent(s) for therapeutic or prophylactic treatment of the individual. In addition, pharmacogenetic studies can be used to apply genotyping of polymorphic alleles encoding drug-metabolizing enzymes to the identification of an individual's drug responsiveness phenotype. This knowledge, when applied to dosing or drug selection, can avoid adverse reactions or therapeutic failure and thus enhance therapeutic or prophylactic efficiency when treating a subject with a modulator of activity or expression of the polypeptide, such as a modulator identified by one of the exemplary screening assays described herein.

### 4. Monitoring of Effects During Clinical Trials

Monitoring the influence of agents (e.g., drugs, compounds) on the expression or activity of a polypeptide of the invention (e.g., the ability to modulate aberrant cell proliferation chemotaxis, and/or differentiation) can be applied not only in basic drug screening, but also in clinical trials. For example, the effectiveness of an agent, as determined by a screening assay as described herein, to increase gene expression, protein levels or protein activity, can be monitored in clinical trials of subjects exhibiting decreased gene expression, protein levels, or protein activity. Alternatively, the effectivenese of an agent, as determined by a screening assay, to decrease gene expression, protein levels or protein activity, can be monitorcd in clinical trials of subjects exhibiting increased gene expression, protein levels, or protein activity. In such clinical trials, expression or activity of a polypeptide of the invention and preferably, that of other polypeptide that have been implicated in for example, a cellular proliferation disorder, can be used as a marker of the immune responsiveness of a particular cell.

For example, and not by way of limitation, genes, including those of the invention, that are modulated in cells by treatment with an agent (e.g., compound, drug or small molecule) which modulates activity or expression of a polypeptide of the invention (e.g., as identified in a screening assay described herein) can be identified. Thus, to study the effect of agents on cellular proliferation disorders, for example, in a clinical trial, cells can be isolated and RNA prepared and analyzed for the levels of expression of a gene of the invention and other genes implicated in the disorder. The levels of gene expression (i.e., a gene expression pattern) can be quantified by Northern blot analysis or RT-PCR, as described herein, or alternatively by measuring the amount of protein produced, by one of the methods as described herein, or by measuring the levels of activity of a gene of the invention or other genes. In this way, the gene expression pattern can serve as a marker, indicative of the physiological response of the cells to the agent. Accordingly, this response state may be determined before, and at various points during, treatment of the individual with the agent.

In a preferred embodiment, the present invention provides a method for monitoring the effectiveness of treatment of a subject with an agent (e.g., an agonist, antagonist, peptidomimetic, protein, peptide, nucleic acid, small molecule, or other drug candidate identified by the screening assays described herein) comprising the steps of (i) obtaining a pre-administration sample from a subject prior to administration of the agent; (ii) detecting the level of the polypeptide or nucleic acid of the invention in the preadministration sample; (iii) obtaining one or more post-administration samples from the subject; (iv) detecting the level the of the polypeptide or nucleic acid of the invention in the post-administration samples; (v) comparing the level of the polypeptide or nucleic acid of the invention in the pre-administration sample with the level of the polypeptide or nucleic acid of the invention in the post-administration sample or samples; and (vi) altering the administration of the agent to the subject accordingly. For example, increased administration of the agent may be desirable to increase the expression or activity of the polypeptide to higher levels than detected, i.e., to increase the effectiveness of the agent. Alternatively, decreased administration of the agent may be desirable to decrease expression or activity of the polypeptide to lower levels than detected, i.e., to decrease the effectiveness of the agent.

### C. Methods of Treatment

The present invention provides for both prophylactic and therapeutic methods of treating a subject at risk of (or susceptible to) a disorder or having a disorder associated with aberrant expression or activity of a polypeptide of the invention. For example, disorders characterized by abberant expression or activity of the polypeptides of the invention include proliferative disorders such as psoriasis and cancer. In addition, the polypeptides of the invention can be used to promote hair growth, promote wound healing, as well as other uses described herein.

### 1. Prophylactic Methods

In one aspect, the invention provides a method for preventing in a subject, a disease or condition associated with an aberrant expression or activity of a polypeptide of the invention, by administering to the subject an agent which modulates expression or at least one activity of the polypeptide. Subjects at risk for a disease which is caused or contributed to by aberrant expression of activity of a polypeptide of the invention can be identified by, for example, any or a combination of diagnostic or prognostic assays as described herein. Administration of a prophylactic agent can occur prior to the manifestation of symptoms characteristic of the aberrancy, such that a disease or disorder is prevented or alternatively, delayed in its progression. Depending on the type of aberrancy, for example, an agonist or antagonist agent can be used for treating the subject. For example, an antagonist of an ELVIS protein may be used to treat a proliferative disorder, e.g., psoriasis, associated with abberant ELVIS expression or activity. The appropriate agent can be determined based on screening assays described herein.

### 2. Therapeutic Methods

Another aspect of the invention pertains to methods of modulating expression or activity of a polypeptide of the invention for therapeutic purposes. The modulatory method of the invention involves contacting a cell with an agent that modulates one or more of the activities of the polypeptide. An agent that modulates activity can be an agent as described herein, such as a nucleic acid or a protein, a naturally-occurring cognate ligand of the polypeptide, a peptide, a peptidomimetic, or other small molecule. In one embodiment, the agent stimulates one or more of the biological activities of the polypeptide. Examples of such stimulatory agents include the active polypeptide of the invention and a nucleic acid molecule encoding the polypeptide of the invention that has been introduced into the cell. In another embodiment, the agent inhibits one or more of the biological activities of the polypeptide of the invention. Examples of such inhibitory agents include antisense nucleic acid molecules and antibodies. These modulatory methods can be performed *in vitro* (e.g., by culturing the cell with the agent) or, alternatively, *in vivo* (e.g, by administering the agent to a subject). As such, the present invention provides methods of treating an individual afflicted with a disease or disorder characterized by aberrant expression or activity of a polypeptide of the invention. In one embodiment, the method involves administering an agent (e.g., an agent identified by a screening assay described herein), or combination of agents that modulates (e.g., upregulates or downregulates) expression or activity. In another embodiment, the method involves administering a polypeptide of the invention or a nucleic acid molecule of the invention as therapy to compensate for reduced or aberrant expression or activity of the polypeptide.

Stimulation of activity is desirable in situations in which activity or expression is abnoraially low or downregulaied and/or in which increased activity is likely to have a beneficial effect, e.g., in wound healing. Conversely, inhibition of activity is desirable in situations in which activity or expression is abnormally high or upregulated and/or in which decreased activity is likely to have a beneficial effect, e.g., in treatment of a proliferative disorder such as psoriasis.

This invention is further illuatrated by the following examples which should not be construed as limiting.

All publications, patents and patent applications mentioried in this specification are herein incorporated by reference in to the specification to the same extent as if each individual publication, patent or patent application was specifically and individually indicated to be incorporated herein by reference.

### Deposit of Clones

Clones containing cDNA molecules encoding TANGO 128, TANGO 140-1, TANGO 140-2, TANGO 197 and TANGO 239 were deposited with the American Type Culture Collection (Manassas, VA) as composite deposits.

Clones encoding TANGO 128, TANGO 140-1, TANGO 140-2, TANGO 197 and TANGO 239 were deposited on November 20, 1998 with the American Type Culture Collection under Accession Number ATCC 98999, (also referred to herein as mix EpDHMix1) from which each clone comprising a particular cDNA clone is obtainable. This deposit is a mixture of five strains, each carrying one recombinant plasmid harboring a particular cDNA clone. To distinguish the strains and isolate a strain harboring a particular cDNA clone, one can first streak out an aliquot of the mixture to single colonies on nutrient medium (e.g., LB plates) supplemented with 100µg/ml ampicillin, grow single colonies, and then extract the plasmid DNA using a standard minipreparation procedure. Next, one can digest a sample of the DNA minipreparation with a combination of the restriction enzymes *Sal* I and *Not* I and resolve the resultant products on a 0.8% agarose gel using standard DNA electrophoresis conditions. The digest will liberate fragments as follows:
TANGO 128 (EpDH237) 2.8 kb and 4.3 kb
TANGO 140-1 (EpDH137) 1.6 kb and 3.0 kb
TANGO 140-2 (EpDH185) 3.4 kb and 4.3 kb
TANGO 197 (EpDH213) 2.3 kb and 3.0 kb
TANGO 239 (EpDH233) 3.0 kb and 3.4 kb

The identity of the strains can be inferred from the fragments liberated.

### Equivalents

Those skilled in the art will recognize, or be able to ascertain using no more than routine experimentation, many equivalents to the specific embodiments of the invention described herein. Such equivalents are intended to be encompassed by the following claims.

## Claims

1. An isolated nucleic acid molecule selected from the group consisting of:
a) a nucleic acid molecule comprising a nucleotide sequence which is at least 55% identical to the nucleotide sequence of SEQ ID NO: 17, 19, 65, 67, 1, 3, 4, 6, 8, 10, 11, 13, 14,16, 20, 22, 38, 39, 53, 55, 56, 58, 59, 61, 62, 64, 68, 70, 71, 73, or the cDNA insert of the plasmid deposited with the ATCC as any of Accession Numbers 98966, 98999, 202171, 98965, or a complement thereof;
b) a nucleic acid molecule comprising a fragment of at least 300 nucleotides of the nucleotide sequence of SEQ ID NO: 17, 19, 65, 67, 1, 3, 4, 6, 8, 10, 11, 13, 14, 16, 20, 22, 38, 39, 53, 55, 56, 58, 59, 61, 62, 64, 68, 70, 71, 73, or the cDNA insert of the plasmid deposited with the ATCC as any of Accession Numbers 98966, 98999, 202171, 98965, or a complement thereof;
c) a nucleic acid molecule which encodes a polypeptide comprising the amino acid sequence of SEQ ID NO: 18, 66, 2, 5, 7, 9, 12, 15, 21, 54, 57, 60, 63, 69, 72, or amino acid sequence encoded by the cDNA insert of the plasmid deposited with the ATCC as any of Accession Numbers 98966, 98999, 202171, 98965;
d) a nucleic acid molecule which encodes a fragment of a polypeptide comprising the amino acid sequence of SEQ ID NO: 18, 66, 2, 5, 7, 9, 12, 15, 21, 54, 57, 60, 63, 69, 72, or the polypeptide encoded by the cDNA insert of the plasmid deposited with the ATCC as any of Accession Numbers 98966, 98999, 202171, 98965, wherein the fragment comprises at least 15 contiguous amino acids of SEQ ID NO: 18, 66, 2, 5, 7, 9, 12, 15, 21, 54, 57, 60, 63, 69, 72, or the polypeptide encoded by the cDNA insert of the plasmid deposited with the ATCC as any of Accession Numbers 98966, 98999, 202171, 98965; and
e) a nucleic acid molecule which encodes a naturally occurring allelic variant of a polypeptide comprising the amino acid sequence of SEQ ID NO: 18, 66, 2, 5, 7, 9, 12, 15, 21, 54, 57, 60, 63, 69, 72, or the amino acid sequence encoded by the cDNA insert of the plasmid deposited with the ATCC as any of Accession Numbers 98966, 98999, 202171, 98965, wherein the nucleic acid molecule hybridizes to a nucleic acid molecule comprising SEQ ID NO: 17, 19, 65, 67, 1, 3, 4, 6, 8, 10, 11, 13, 14, 16, 20, 22, 38, 39, 53, 55, 56, 58, 59, 61, 62, 64, 68, 70, 71, 73, or a complement thereof under stringent conditions.

2. The isolated nucleic acid molecule of claim 1, which is selected from the group consisting of:
a) a nucleic acid comprising the nucleotide sequence of SEQ ID NO: 17, 19, 65, 67, 1, 3, 4, 6, 8, 10, 11, 13, 14, 16, 20, 22, 38, 39, 53, 55, 56, 58, 59, 61, 62, 64, 68, 70, 71, 73, or the cDNA insert of the plasmid deposited with the ATCC as any of Accession Numbers 98966, 98999, 202171, 98965, or a complement thereof; and
b) a nucleic acid molecule which encodes a polypeptide comprising the amino acid sequence of SEQ ID NO: 18, 66, 2, 5, 7, 9, 12, 15, 21, 54, 57, 60, 63, 69, 72, or the amino acid sequence encoded by the cDNA insert of the plasmid deposited with the ATCC as any of Accession Numbers 98966, 98999, 202171, 98965.

3. The nucleic acid molecule of claim 1 further comprising vector nucleic acid sequences.

4. The nucleic acid molecule of claim 1 further comprising nucleic acid sequences encoding a heterologous polypeptide.

5. A host cell which contains the nucleic acid molecule of claim 1.

6. The host cell of claim 5 which is a mammalian host cell.

7. A non-human mammalian host cell containing the nucleic acid molecule of claim 1.

8. An isolated polypeptide selected from the group consisting of:
a) a fragment of a polypeptide comprising the amino acid sequence of SEQ ID NO: 18, 66, 2, 5, 7, 9, 12, 15, 21, 54, 57, 60, 63, 69, 72, wherein the fragment comprises at least 15 contiguous amino acids of SEQ ID NO: 18, 66, 2, 5, 7, 9, 12, 15, 21, 54, 57, 60, 63, 69, 72;
b) a naturally occurring allelic variant of a polypeptide comprising the amino acid sequence of SEQ ID NO: 18, 66, 2, 5, 7, 9, 12, 15, 21, 54, 57, 60, 63, 69, 72, or the amino acid sequence encoded by the cDNA insert of the plasmid deposited with the ATCC as any of Accession Numbers 98966, 98999, 202171, 98965, wherein the polypeptide is encoded by a nucleic acid molecule which hybridizes to a nucleic acid molecule comprising SEQ ID NO: 17, 19, 65, 67, 1, 3, 4, 6, 8, 10, 11, 13, 14, 16, 20, 22, 38, 39, 53, 55, 56, 58, 59, 61, 62, 64, 68, 70, 71, 73, or a complement thereof under stringent conditions; and
c) a polypeptide which is encoded by a nucleic acid molecule comprising a nucleotide sequence which is at least 65% identical to a nucleic acid comprising the nucleotide sequence of SEQ ID NO: 17, 19, 65, 67, 1, 3, 4, 6, 8, 10, 11, 13, 14, 16, 20, 22, 38, 39, 53, 55, 56, 58, 59, 61, 62, 64, 68, 70, 71, 73, or a complement thereof.

9. The isolated polypeptide of claim 8 comprising the amino acid sequence of SEQ ID NO: 18, 66, 2, 5, 7, 9, 12, 15, 21, 54, 57, 60, 63, 69, 72.

10. The polypeptide of claim 8 further comprising heterologous amino acid sequences.

11. An antibody which selectively binds to a polypeptide of claim 8.

12. A method for producing a polypeptide selected from the group consisting of:
a) a polypeptide comprising the amino acid sequence of SEQ ID NO: 18, 66, 2, 5, 7, 9, 12, 15, 21, 54, 57, 60, 63, 69, 72, or the amino acid sequence encoded by the cDNA insert of the plasmid deposited with the ATCC as any of Accession Numbers 98966, 98999, 202171, 98965;
b) a polypeptide comprising a fragment of the amino acid sequence of SEQ ID NO: 18, 66, 2, 5, 7, 9, 12, 15, 21, 54, 57, 60, 63, 69, 72, or the amino acid sequence encoded by the cDNA insert of the plasmid deposited with the ATCC as any of Accession Numbers 98966, 98999, 202171, 98965, wherein the fragment comprises at least 15 contiguous amino acids of SEQ ID NO: 18, 66, 2, 5, 7, 9, 12, 15, 21, 54, 57, 60, 63, 69, 72, or the amino acid sequence encoded by the cDNA insert of the plasmid deposited with the ATCC as any of Accession Numbers 98966, 98999, 202171, 98965; and
c) a naturally occurring allelic variant of a polypeptide comprising the amino acid sequence of SEQ ID NO: 18, 66, 2, 5, 7, 9, 12, 15, 21, 54, 57, 60, 63, 69, 72, or the amino acid sequence encoded by the cDNA insett of the plasmid deposited with the ATCC as any of Accession Numbers 98966, 98999, 202171, 98965, wherein the polypeptide is encoded by a nucleic acid molecule which hybridizes to a nucleic acid molecule comprising SEQ ID NO: 17, 19, 65, 67, 1, 3, 4, 6, 8, 10, 11, 13, 14, 16, 20, 22, 38, 39, 53, 55, 56, 58, 59, 61, 62, 64, 68, 70, 71, 73, or a complement thereof under stringent conditions;
comprising culturing the host cell of claim 5 under conditions in which the nucleic acid molecule is expressed.

13. A method for detecting the presence of a polypeptide of claim 8 in a sample, comprising:
a) contacting the sample with a compound which selectively binds to a polypeptide of claim 8; and
b) determining whether the compound binds to the polypeptide in the sample.

14. The method of claim 13, wherein the compound which binds to the polypeptide is an antibody.

15. A kit comprising a compound which selectively binds to a polypeptide of claim 8 and instructions for use.

16. A method for detecting the presence of a nucleic acid molecule of claim 1 in a sample, comprising the steps of:
a) contacting the sample with a nucleic acid probe or primer which selectively hybridizes to the nucleic acid molecule; and
b) determining whether the nucleic acid probe or primer binds to a nucleic acid molecule in the sample.

17. The method of claim 16, wherein the sample comprises mRNA molecules and is contacted with a nucleic acid probe.

18. A kit comprising a compound which selectively hybridizes to a nucleic acid molecule of claim 1 and instructions for use.

19. A method for identifying a compound which binds to a polypeptide of claim 8 comprising the steps of:
a) contacting a polypeptide, or a cell expressing a polypeptide of claim 8 with a test compdund; and
b) determining whether the polypeptide binds to the test compound.

20. The method of claim 19, wherein the binding of the test compound to the polypeptide is detected by a method selected from the group consisting of:
a) detection of binding by direct detecting of test compound/polypeptide binding;
b) detection of binding using a competition binding assay;
c) detection of binding Using an assay for TANGO 224, TANGO 128, TANGO 140, TANGO 197, TANGO 212, TANGO 213, or TANGO 239-mediated signal transduction.

21. A method for modulating the activity of a polypeptide of claim 8 comprising contacting a polypeptide or a cell expressing a polypeptide of claim 8 with a compound which binds to the polypeptide in a sufficient concentration to modulate the activity of the polypeptide.

22. A method for identifying a compound which modulates the activity of a polypeptide of claim 8, comprising:
a) contacting a polypeptide of claim 8 with a test compound; and
b) determining the effect of the test compound on the activity of the polypeptide to thereby identify a compound which modulates the activity of the polypeptide.


### Annex to the application documents - subsequently filed sequences listing

SEQUENCE LISTING

<110> Millennium Pharmaceuticals, Inc.

<120> Secreted Proteins and Nucleic Acids Encoding Them

<130> EPP290525A

<140> 05026823.4
<141> 1999-12-23

<150> PCT/US99/31025
<151> 1999-12-23

<150> 09/223,546
<151> 1998-12-30

<160> 135

<170> FastSEQ for Windows Version 4.0

<210> 1
<211> 2839
<212> DNA
<213> Homo sapiens

<220>
<221> CDS
<222> (288)...(1322)

<400> 1
gtcgacccac gcgtccggcc acagctcagg atttgtttaa accttgggaa actggttcag 60
gtccaggttt tgctttgatc cttttcaaaa actggagaca cagaagaggg ctctaggaaa 120
aagttttgga tgggattatg tggaaactac cctgcgattc tctgctgcca gagcaggctc 180
ggcgcttcca ccccagtgca gccttcccct ggcggtggtg aaagagactc gggagtcgct 240
gcttccaaag tgcccgccgt gagtgagctc tcaccccagt cagccaa atg agc ctc 296
Met Ser Leu
1

ttc ggg ctt ctc ctg ctg aca tct gcc ctg gcc ggc cag aga cag ggg 344
Phe Gly Leu Leu Leu Leu Thr Ser Ala Leu Ala Gly Gln Arg Gln Gly
    5                   10                  15

act cag gcg gaa tcc aac ctg agt agt aaa ttc cag ttt tcc agc aac 392
Thr Gln Ala Glu Ser Asn Leu Ser Ser Lys Phe Gln Phe Ser Ser Asn
20                  25                  30                  35

aag gaa cag aac gga gta caa gat cct cag cat gag aga att att act 440
Lys Glu Gln Asn Gly Val Gln Asp Pro Gln His Glu Arg Ile Ile Thr
                40                  45                  50

gtg tct act aat gga agt att cac agc cca agg ttt cct cat act tat 488
Val Ser Thr Asn Gly Ser Ile His Ser Pro Arg Phe Pro His Thr Tyr
            55                  60                  65

cca aga aat acg gtc ttg gta tgg aga tta gta gca gta gag gaa aat 536
Pro Arg Asn Thr Val Leu Val Trp Arg Leu Val Ala Val Glu Glu Asn
        70                  75                  80

gta tgg ata caa ctt acg ttt gat gaa aga ttt ggg ctt gaa gac cca 584
Val Trp Ile Gln Leu Thr Phe Asp Glu Arg Phe Gly Leu Glu Asp Pro
    85                  90                  95

gaa gat gac ata tgc aag tat gat ttt gta gaa gtt gag gaa ccc agt 632

Glu Asp Asp Ile Cys Lys Tyr Asp Phe Val Glu Val Glu Glu Pro Ser
100                 105                 110                 115

gat gga act ata tta ggg cgc tgg tgt ggt tct ggt act gta cca gga      680
Asp Gly Thr Ile Leu Gly Arg Trp Cys Gly Ser Gly Thr Val Pro Gly
            120                 125                 130

aaa cag att tct aaa gga aat caa att agg ata aga ttt gta tct gat      728
Lys Gln Ile Ser Lys Gly Asn Gln Ile Arg Ile Arg Phe Val Ser Asp
        135                 140                 145

gaa tat ttt cct tct gaa cca ggg ttc tgc atc cac tac aac att gtc      776
Glu Tyr Phe Pro Ser Glu Pro Gly Phe Cys Ile His Tyr Asn Ile Val
    150                 155                 160

atg cca caa ttc aca gaa gct gtg agt cct tca gtg cta ccc cct tca      824
Met Pro Gln Phe Thr Glu Ala Val Ser Pro Ser Val Leu Pro Pro Ser
    165                 170                 175

gct ttg cca ctg gac ctg ctt aat aat gct ata act gcc ttt agt acc      872
Ala Leu Pro Leu Asp Leu Leu Asn Asn Ala Ile Thr Ala Phe Ser Thr
180                 185                 190                 195

ttg gaa gac ctt att cga tat ctt gaa cca gag aga tgg cag ttg gac      920
Leu Glu Asp Leu Ile Arg Tyr Leu Glu Pro Glu Arg Trp Gln Leu Asp
                200                 205                 210

tta gaa gat cta tat agg cca act tgg caa ctt ctt ggc aag gct ttt      968
Leu Glu Asp Leu Tyr Arg Pro Thr Trp Gln Leu Leu Gly Lys Ala Phe
            215                 220                 225

gtt ttt gga aga aaa tcc aga gtg gtg gat ctg aac ctt cta aca gag     1016
Val Phe Gly Arg Lys Ser Arg Val Val Asp Leu Asn Leu Leu Thr Glu
        230                 235                 240

gag gta aga tta tac agc tgc aca cct cgt aac ttc tca gtg tcc ata     1064
Glu Val Arg Leu Tyr Ser Cys Thr Pro Arg Asn Phe Ser Val Ser Ile
    245                 250                 255

agg gaa gaa cta aag aga acc gat acc att ttc tgg cca ggt tgt ctc     1112
Arg Glu Glu Leu Lys Arg Thr Asp Thr Ile Phe Trp Pro Gly Cys Leu
260                 265                 270                 275

ctg gtt aaa cgc tgt ggt ggg aac tgt gcc tgt tgt ctc cac aat tgc     1160
Leu Val Lys Arg Cys Gly Gly Asn Cys Ala Cys Cys Leu His Asn Cys
                280                 285                 290

aat gaa tgt caa tgt gtc cca agc aaa gtt act aaa aaa tac cac gag     1208
Asn Glu Cys Gln Cys Val Pro Ser Lys Val Thr Lys Lys Tyr His Glu
            295                 300                 305

gtc ctt cag ttg aga cca aag acc ggt gtc agg gga ttg cac aaa tca     1256
Val Leu Gln Leu Arg Pro Lys Thr Gly Val Arg Gly Leu His Lys Ser
        310                 315                 320

ctc acc gac gtg gcc ctg gag cac cat gag gag tgt gac tgt gtg tgc     1304
Leu Thr Asp Val Ala Leu Glu His His Glu Glu Cys Asp Cys Val Cys
    325                 330                 335

aga ggg agc aca gga gga tagccgcatc accaccagca gctcttgccc            1352
Arg Gly Ser Thr Gly Gly
340                 345

agagctgtgc agtgcagtgg ctgattctat tagagaacgt atgcgttatc tccatcctta   1412
atctcagttg tttgcttcaa ggacctttca tcttcaggat ttacagtgca ttctgaaaga   1472
ggagacatca aacagaatta ggagttgtgc aacagctctt ttgagaggag gcctaaagga   1532

caggagaaaa ggtcttcaat cgtggaaaga aaattaaatg ttgtattaaa tagatcacca 1592
gctagtttca gagttaccat gtacgtattc cactagctgg gttctgtatt tcagttcttt 1652
cgatacggct tagggtaatg tcagtacagg aaaaaaactg tgcaagtgag cacctgattc 1712
cgttgccttg cttaactcta aagctccatg tcctgggcct aaaatcgtat aaaatctgga 1772
tttttttttt tttttttgct catattcaca tatgtaaacc agaacattct atgtactaca 1832
aacctggttt ttaaaaagga actatgttgc tatgaattaa acttgtgtcg tgctgatagg 1892
acagactgga tttttcatat ttcttattaa aatttctgcc atttagaaga agagaactac 1952
attcatggtt tggaagagat aaacctgaaa agaagagtgg ccttatcttc actttatcga 2012
taagtcagtt tatttgtttc attgtgtaca tttttatatt ctccttttga cattataact 2072
gttggctttt ctaatcttgt taaatatatc tatttttacc aaaggtattt aatattcttt 2132
tttatgacaa cttagatcaa ctatttttag cttggtaaat ttttctaaac acaattgtta 2192
tagccagagg aacaaagatg atataaaata ttgttgctct gacaaaaata catgtatttc 2252
attctcgtat ggtgctagag ttagattaat ctgcatttta aaaaactgaa ttggaataga 2312
attggtaagt tgcaaagact ttttgaaaat aattaaatta tcatatcttc cattcctgtt 2372
attggagatg aaaataaaaa gcaacttatg aaagtagaca ttcagatcca gccattacta 2432
acctattcct tttttgggga aatctgagcc tagctcagaa aaacataaag caccttgaaa 2492
aagacttggc agcttcctga taaagcgtgc tgtgctgtgc agtaggaaca catcctattt 2552
attgtgatgt tgtggtttta ttatcttaaa ctctgttcca tacacttgta taaatacatg 2612
gatattttta tgtacagaag tatgtctctt aaccagttca cttattgtac tctggcaatt 2672
taaaagaaaa tcagtaaaat attttgcttg taaaatgctt aatatcgtgc ctaggttatg 2732
tggtgactat ttgaatcaaa aatgtattga atcatcaaat aaaagaatgt ggctattttg 2792
gggagaaaaa aaaaaaaaaa aaaaaaaaaa aaaaaaaggg cggccgc 2839

<210> 2
<211> 345
<212> PRT
<213> Homo sapiens

<400> 2
Met Ser Leu Phe Gly Leu Leu Leu Leu Thr Ser Ala Leu Ala Gly Gln
1               5                   10                  15
Arg Gln Gly Thr Gln Ala Glu Ser Asn Leu Ser Ser Lys Phe Gln Phe
            20                  25                  30
Ser Ser Asn Lys Glu Gln Asn Gly Val Gln Asp Pro Gln His Glu Arg
        35                  40                  45
Ile Ile Thr Val Ser Thr Asn Gly Ser Ile His Ser Pro Arg Phe Pro
    50                  55                  60
His Thr Tyr Pro Arg Asn Thr Val Leu Val Trp Arg Leu Val Ala Val
65                  70                  75                  80
Glu Glu Asn Val Trp Ile Gln Leu Thr Phe Asp Glu Arg Phe Gly Leu
                85                  90                  95
Glu Asp Pro Glu Asp Asp Ile Cys Lys Tyr Asp Phe Val Glu Val Glu
            100                 105                 110
Glu Pro Ser Asp Gly Thr Ile Leu Gly Arg Trp Cys Gly Ser Gly Thr
        115                 120                 125
Val Pro Gly Lys Gln Ile Ser Lys Gly Asn Gln Ile Arg Ile Arg Phe
    130                 135                 140
Val Ser Asp Glu Tyr Phe Pro Ser Glu Pro Gly Phe Cys Ile His Tyr
145                 150                 155                 160
Asn Ile Val Met Pro Gln Phe Thr Glu Ala Val Ser Pro Ser Val Leu
                165                 170                 175
Pro Pro Ser Ala Leu Pro Leu Asp Leu Leu Asn Asn Ala Ile Thr Ala
            180                 185                 190
Phe Ser Thr Leu Glu Asp Leu Ile Arg Tyr Leu Glu Pro Glu Arg Trp
        195                 200                 205
Gln Leu Asp Leu Glu Asp Leu Tyr Arg Pro Thr Trp Gln Leu Leu Gly
    210                 215                 220
Lys Ala Phe Val Phe Gly Arg Lys Ser Arg Val Val Asp Leu Asn Leu
225                 230                 235                 240
Leu Thr Glu Glu Val Arg Leu Tyr Ser Cys Thr Pro Arg Asn Phe Ser
                245                 250                 255
Val Ser Ile Arg Glu Glu Leu Lys Arg Thr Asp Thr Ile Phe Trp Pro
            260                 265                 270
Gly Cys Leu Leu Val Lys Arg Cys Gly Gly Asn Cys Ala Cys Cys Leu
        275                 280                 285
His Asn Cys Asn Glu Cys Gln Cys Val Pro Ser Lys Val Thr Lys Lys
    290                 295                 300

Tyr His Glu Val Leu Gln Leu Arg Pro Lys Thr Gly Val Arg Gly Leu
305                 310                 315                 320
His Lys Ser Leu Thr Asp Val Ala Leu Glu His His Glu Glu Cys Asp
                325                 330                 335
Cys Val Cys Arg Gly Ser Thr Gly Gly
            340                 345

<210> 3
<211> 1035
<212> DNA
<213> Homo sapiens

<400> 3
atgagcctct tcgggcttct cctgctgaca tctgccctgg ccggccagag acaggggact      60
caggcggaat ccaacctgag tagtaaattc cagttttcca gcaacaagga acagaacgga     120
gtacaagatc ctcagcatga gagaattatt actgtgtcta ctaatggaag tattcacagc     180
ccaaggtttc ctcatactta tccaagaaat acggtcttgg tatggagatt agtagcagta     240
gaggaaaatg tatggataca acttacgttt gatgaaagat ttgggcttga agacccagaa     300
gatgacatat gcaagtatga ttttgtagaa gttgaggaac ccagtgatgg aactatatta     360
gggcgctggt gtggttctgg tactgtacca ggaaaacaga tttctaaagg aaatcaaatt     420
aggataagat ttgtatctga tgaatatttt ccttctgaac cagggttctg catccactac     480
aacattgtca tgccacaatt cacagaagct gtgagtcctt cagtgctacc cccttcagct     540
ttgccactgg acctgcttaa taatgctata actgccttta gtaccttgga agaccttatt     600
cgatatcttg aaccagagag atggcagttg gacttagaag atctatatag gccaacttgg     660
caacttcttg gcaaggcttt tgtttttgga agaaaatcca gagtggtgga tctgaacctt     720
ctaacagagg aggtaagatt atacagctgc acacctcgta acttctcagt gtccataagg     780
gaagaactaa agagaaccga taccattttc tggccaggtt gtctcctggt taaacgctgt     840
ggtgggaact gtgcctgttg tctccacaat tgcaatgaat gtcaatgtgt cccaagcaaa     900
gttactaaaa aataccacga ggtccttcag ttgagaccaa agaccggtgt caggggattg     960
cacaaatcac tcaccgacgt ggccctggag caccatgagg agtgtgactg tgtgtgcaga    1020
gggagcacag gagga                                                     1035

<210> 4
<211> 1550
<212> DNA
<213> Homo sapiens

<220>
<221> CDS
<222> (2)...(619)

<400> 4

g gaa ttt cat ata ctc ctt ccc acc atg gat tgc caa gaa aat gag tac      49
  Glu Phe His Ile Leu Leu Pro Thr Met Asp Cys Gln Glu Asn Glu Tyr
   1               5                  10                  15

tgg gac caa tgg gga cgg tgt gtc acc tgc caa cgg tgt ggt cct gga      97
Trp Asp Gln Trp Gly Arg Cys Val Thr Cys Gln Arg Cys Gly Pro Gly
            20                  25                  30

cag gag cta tcc aag gat tgt ggt tat gga gag ggt gga gat gcc tac     145
Gln Glu Leu Ser Lys Asp Cys Gly Tyr Gly Glu Gly Gly Asp Ala Tyr
        35                  40                  45

tgc aca gcc tgc cct cct cgc agg tac aaa agc agc tgg ggc cac cac     193
Cys Thr Ala Cys Pro Pro Arg Arg Tyr Lys Ser Ser Trp Gly His His
    50                  55                  60

aaa tgt cag agt tgc atc acc tgt gct gtc atc aat cgt gtt cag aag     241
Lys Cys Gln Ser Cys Ile Thr Cys Ala Val Ile Asn Arg Val Gln Lys
 65                  70                  75                  80

gtc aac tgc aca gct acc tct aat gct gtc tgt ggg gac tgt ttg ccc     289
Val Asn Cys Thr Ala Thr Ser Asn Ala Val Cys Gly Asp Cys Leu Pro

                85                  90                  95

agg ttc tac cga aag aca cgc att gga ggc ctg cag gac caa gag tgc 337
Arg Phe Tyr Arg Lys Thr Arg Ile Gly Gly Leu Gln Asp Gln Glu Cys
            100                 105                 110

atc ccg tgc acg aag cag acc ccc acc tct gag gtt caa tgt gcc ttc 385
Ile Pro Cys Thr Lys Gln Thr Pro Thr Ser Glu Val Gln Cys Ala Phe
        115                 120                 125

cag ttg agc tta gtg gag gca gat gca ccc aca gtg ccc cct cag gag 433
Gln Leu Ser Leu Val Glu Ala Asp Ala Pro Thr Val Pro Pro Gln Glu
    130                 135                 140

gcc aca ctt gtt gca ctg gtg agc agc ctg cta gtg gtg ttt acc ctg 481
Ala Thr Leu Val Ala Leu Val Ser Ser Leu Leu Val Val Phe Thr Leu
145                 150                 155                 160

gcc ttc ctg ggg ctc ttc ttc ctc tac tgc aag cag ttc ttc aac aga 529
Ala Phe Leu Gly Leu Phe Phe Leu Tyr Cys Lys Gln Phe Phe Asn Arg
                165                 170                 175

cat tgc cag cgt ggt aag ggt ggc tgt ttc atg ttt cac atg aat cag 577
His Cys Gln Arg Gly Lys Gly Gly Cys Phe Met Phe His Met Asn Gln
            180                 185                 190

gaa caa ggc tct tat tgg cag aag aga ggg atg ttc tgg ggt 619
Glu Gln Gly Ser Tyr Trp Gln Lys Arg Gly Met Phe Trp Gly
        195                 200                 205

tgagagacaa tgggcctttt ctaccatgct tgaaaacccc atgaatagtc ttctgttggc 679
ctggggaact atctggggct gcaatggaaa gcatgggact taagagatta ctgaagccaa 739
ccctgagagt ttagaagacc tggttcatta tgtttcaggg gattttaaag gaatgtggca 799
cggtgggaaa cccctcagga aaggaatcgg gagacctagg ttctatgtct acctctaaat 859
caccgtgtaa ccttagacaa gtcatctagc ctcttcagac ctctttcttc ctaatcttta 919
agataagaga taggattgga tttgttgatt tctgggttct tttccaattc tttatatcta 979
tactagtagg tataaattct agagcatgtc atgaattgag gaaggcttgg ctatatctga 1039
gatacgactg taataaaagt ggttgaggtc taaggccact gctatcagta taataacttc 1099
acaaatgggg ataactgaaa gccctatgtc cagagtagtt ggcaaacctg gactttctcc 1159
ctcagataat actgatgtca agaggtctag cccagtccat atcagacagc catgcaaaat 1219
tccagatgga aaatcatatt taaagtcaca gtaacattgt gatctctatt aagcacaact 1279
tcagctacta gacaagaatg tgggacagaa aagccagctc actgaggaca aagatggggg 1339
aggtgggaat gtggctgatc aggaccaggc aaagagttag aactccagcc aagagtacca 1399
aggttcagtg cagcttcatt gccattaagg gaactgagag ttaggcaggg acactgaaac 1459
aggaactcag atcagagaat gaagttgatt tataaggggg aaaaaaaaaa aaaaaaaaaa 1519
aaaaaaaaaa aaaaaaaaaa agggcggccg c 1550

<210> 5
<211> 206
<212> PRT
<213> Homo sapiens

<400> 5
Glu Phe His Ile Leu Leu Pro Thr Met Asp Cys Gln Glu Asn Glu Tyr
1               5                   10                  15
Trp Asp Gln Trp Gly Arg Cys Val Thr Cys Gln Arg Cys Gly Pro Gly
            20                  25                  30
Gln Glu Leu Ser Lys Asp Cys Gly Tyr Gly Glu Gly Gly Asp Ala Tyr
        35                  40                  45
Cys Thr Ala Cys Pro Pro Arg Arg Tyr Lys Ser Ser Trp Gly His His
    50                  55                  60
Lys Cys Gln Ser Cys Ile Thr Cys Ala Val Ile Asn Arg Val Gln Lys
65                  70                  75                  80
Val Asn Cys Thr Ala Thr Ser Asn Ala Val Cys Gly Asp Cys Leu Pro
                85                  90                  95
Arg Phe Tyr Arg Lys Thr Arg Ile Gly Gly Leu Gln Asp Gln Glu Cys
            100                 105                 110

Ile Pro Cys Thr Lys Gln Thr Pro Thr Ser Glu Val Gln Cys Ala Phe
        115                 120                 125
Gln Leu Ser Leu Val Glu Ala Asp Ala Pro Thr Val Pro Pro Gln Glu
    130                 135                 140
Ala Thr Leu Val Ala Leu Val Ser Ser Leu Leu Val Val Phe Thr Leu
145                 150                 155                 160
Ala Phe Leu Gly Leu Phe Phe Leu Tyr Cys Lys Gln Phe Phe Asn Arg
                165                 170                 175
His Cys Gln Arg Gly Lys Gly Gly Cys Phe Met Phe His Met Asn Gln
            180                 185                 190
Glu Gln Gly Ser Tyr Trp Gln Lys Arg Gly Met Phe Trp Gly
        195                 200                 205

<210> 6
<211> 3385
<212> DNA
<213> Homo sapiens

<220>
<221> CDS
<222> (1)...(591)

<400> 6
gga ttt gta ccg gag tcc cat ttg gga gca aga gcc atc tac tcg tcc 48
Gly Phe Val Pro Glu Ser His Leu Gly Ala Arg Ala Ile Tyr Ser Ser
1               5                   10                  15

gtt acc ggc ctt ccc acc atg gat tgc caa gaa aat gag tac tgg gac 96
Val Thr Gly Leu Pro Thr Met Asp Cys Gln Glu Asn Glu Tyr Trp Asp
            20                  25                  30

caa tgg gga cgg tgt gtc acc tgc caa cgg tgt ggt cct gga cag gag 144
Gln Trp Gly Arg Cys Val Thr Cys Gln Arg Cys Gly Pro Gly Gln Glu
        35                  40                  45

cta tcc aag gat tgt ggt tat gga gag ggt gga gat gcc tac tgc aca 192
Leu Ser Lys Asp Cys Gly Tyr Gly Glu Gly Gly Asp Ala Tyr Cys Thr
    50                  55                  60

gcc tgc cct cct cgc agg tac aaa agc agc tgg ggc cac cac aaa tgt 240
Ala Cys Pro Pro Arg Arg Tyr Lys Ser Ser Trp Gly His His Lys Cys
65                  70                  75                  80

cag agt tgc atc acc tgt gct gtc atc aat cgt gtt cag aag gtc aac 288
Gln Ser Cys Ile Thr Cys Ala Val Ile Asn Arg Val Gln Lys Val Asn
                85                  90                  95

tgc aca gct acc tct aat gct gtc tgt ggg gac tgt ttg ccc agg ttc 336
Cys Thr Ala Thr Ser Asn Ala Val Cys Gly Asp Cys Leu Pro Arg Phe
            100                 105                 110

tac cga aag aca cgc att gga ggc ctg cag gac caa gag tgc atc ccg 384
Tyr Arg Lys Thr Arg Ile Gly Gly Leu Gln Asp Gln Glu Cys Ile Pro
        115                 120                 125

tgc acg aag cag acc ccc acc tct gag gtt caa tgt gcc ttc cag ttg 432
Cys Thr Lys Gln Thr Pro Thr Ser Glu Val Gln Cys Ala Phe Gln Leu
    130                 135                 140

agc tta gtg gag gca gat gca ccc aca gtg ccc cct cag gag gcc aca 480
Ser Leu Val Glu Ala Asp Ala Pro Thr Val Pro Pro Gln Glu Ala Thr
145                 150                 155                 160

ctt gtt gca ctg ttg cag gag gtt tgc tgc agt ttg agg ctg ata aaa 528
Leu Val Ala Leu Leu Gln Glu Val Cys Cys Ser Leu Arg Leu Ile Lys
                165                 170                 175

cag caa agg agg aat ctc tct tcc ccg tgc cac cca gca agg aga cca     576
Gln Gln Arg Arg Asn Leu Ser Ser Pro Cys His Pro Ala Arg Arg Pro
        180                 185                 190

gtg ctg agt ccc aag tgagtgagaa catctttcag acccagccac ttaaccctat     631
Val Leu Ser Pro Lys
        195

cctcgaggac gactgcagct cgactagtgg cttccccaca caggagtcct ttaccatggc     691
ctcctgcacc tcagagagcc actcccactg ggtccacagc cccatcgaat gcacagagct     751
ggacctgcaa aagttttcca gctctgcctc ctatactgga gctgagacct tgggggggaaa     811
cacagtcgaa agcactggag acaggctgga gctcaatgtg ccctttgaag ttcccagccc     871
ttaactctaa tgaggtctct tgggcccctg gcagccttgc ccagttgttc tctctggact     931
ctgttcctat accacaacag cagcaggggc ctgaaatgtg atgtccacaa gagctaatac     991
cctacagatg gggcatatcc tatcccatcc caccagagga ttgattctcc atttcacaag    1051
gactgatctg gagcatttct tgcttccctg ttgtagtctg gggagccaga ttccacattc    1111
atgggactac cagacatgtt cctagctcaa cttgattata gagaagagga gagaggacag    1171
tgaatggggt agggttttca tgtctgcatt tttggtcagg taagcctctc aaaattgtgt    1231
tggcacatct acctagcact ttagggacaa aatcaaaccc ttctcccctt ttagctcctc    1291
cacactgcct ccctcctcaa cacacacaca cacacataca cacacatata catagacaca    1351
caaacacaca cacacacatt aatatctatc ttgggggagg cctcgtgcca taattcccaa    1411
gttcatgtct cagactgctg cattgcagca tgaggcaggg caaacacttt ccctctagat    1471
ccctggggcc tcaccctgta tttgaggttc tcaccaccct cagcagggag aagggctgaa    1531
gttcgccatt ttggaacctt acagaacatt tctgagccaa agtaatcttc cttctggggc    1591
ctgagttccc caaactaccc cacagcagtc cctcaaagac agccctcaat ccatgtaggg    1651
acatctgagt atgcctcttt ctattgaaat gtcaattcaa tcccagcttt ctcaccaccg    1711
ttcccctttg attctttctc aattgtcttt ttgcctttag ctcccaccta tacatctcat    1771
gctcagagaa aaacaagttc cttagaggtt gtattcttta ttctccaaga atctgtctga    1831
aacttgtaca gctagttcct gtcccacaac tattaagtgg tttattaagt acattaggca    1891
gaatgtgcac ttcatcacca ggttctagct ctggcaaagg agtgctgtct acagcaagat    1951
tttgcttttt agaattttat taactacatc ttttgggttc atccatctac aaacactgat    2011
taagggcccc tggggcaacc aattgatcag attactaaaa ggacttggga aaaagcaaaa    2071
aggtcccatt gtactggact gaggattaga agcaattgaa atacaagcct gtaccaagca    2131
agcagcctgg ccccacacag gtattagcaa atatgtggta accaaggttt taggccttgg    2191
cctttaggtt tcctgttttt tttttgtttt tgtttttgtt tttgtttttc tgcaacaggt    2251
tattcttatc tcactggctt tcactgatca tgtttagacc ttctggtaga agaaataata    2311
tccagacagg ggatgatttg gcttcagcag gctgcaggtg ttcaaaggtt gccatgtggc    2371
tggcagtggt tcaagcccac atttgacact gctgctctag aggaaagata atgatggtaa    2431
cacagtaata ataataataa taacaaaaat atgataaagt gaaagagtaa gacttcttca    2491
gtgtgcttgc tcatggcatg aatgctatgt ggacagccca agccataccc agaatcacct    2551
taattccaac tttttgaggt tcagcaattg gaggtggcaa ttggctttgc atttaaagt    2611
atttcgggta aaggtgaagt gaaggatttt cgtctttata atttctgttt ggccatggca    2671
aataccatag ttgagtattt gcttcaggag agttcttttt acagttttac ttttcaatgc    2731
tgaggcatat ttctttgagc actgtgcttt tatgtgtctt tctacaaagg ggttattggt    2791
cagtggaaga acaaagtaca cttgataaaa acattttcaa catacattga gcctaaacag    2851
cagttaagtt gtctctaaat gaactagcaa aaaaaaaaaa tgtagttttt gtttgtaagg    2911
aaggggaggt atttcctgag aatgaatttt tttttttttt ggattactgt ttttctctcc    2971
atataccttg acttggattt tgacaggagg gagtctggga aaataatttt ttcctccaag    3031
attctcagat ccaggttagg aaaggattca gcactacagc atacccctct acaacataca    3091
gccctgtcac attgagatca taatccctcc tgtcccactc ctctctacca accccaccct    3151
actagctagg tcttcagtgt tttacattga atattggtac atttaatta tttttctca    3211
taaatgggtt atttatagag attttgttaa ctcttgagcc atatgcatgt gtagatactg    3271
gcagggctat gtttgtttat gatgctctgc aaacatttca tattggccaa taaacagaaa    3331
tatatccaaa aaaaaaaaaa aaaaaaaaaa aaaaaaaaaa aaaagggcgg ccgc          3385

<210> 7
<211> 197
<212> PRT
<213> Homo sapiens

<400> 7
Gly Phe Val Pro Glu Ser His Leu Gly Ala Arg Ala Ile Tyr Ser Ser
1               5                   10                  15
Val Thr Gly Leu Pro Thr Met Asp Cys Gln Glu Asn Glu Tyr Trp Asp
            20                  25                  30
Gln Trp Gly Arg Cys Val Thr Cys Gln Arg Cys Gly Pro Gly Gln Glu
        35                  40                  45
Leu Ser Lys Asp Cys Gly Tyr Gly Glu Gly Gly Asp Ala Tyr Cys Thr

50                  55                  60
Ala Cys Pro Pro Arg Arg Tyr Lys Ser Ser Trp Gly His His Lys Cys
65                  70                  75                  80
Gln Ser Cys Ile Thr Cys Ala Val Ile Asn Arg Val Gln Lys Val Asn
                85                  90                  95
Cys Thr Ala Thr Ser Asn Ala Val Cys Gly Asp Cys Leu Pro Arg Phe
            100                 105                 110
Tyr Arg Lys Thr Arg Ile Gly Gly Leu Gln Asp Gln Glu Cys Ile Pro
        115                 120                 125
Cys Thr Lys Gln Thr Pro Thr Ser Glu Val Gln Cys Ala Phe Gln Leu
    130                 135                 140
Ser Leu Val Glu Ala Asp Ala Pro Thr Val Pro Pro Gln Glu Ala Thr
145                 150                 155                 160
Leu Val Ala Leu Leu Gln Glu Val Cys Cys Ser Leu Arg Leu Ile Lys
                165                 170                 175
Gln Gln Arg Arg Asn Leu Ser Ser Pro Cys His Pro Ala Arg Arg Pro
            180                 185                 190
Val Leu Ser Pro Lys
        195

<210> 8
<211> 2272
<212> DNA
<213> Homo sapiens

<220>
<221> CDS
<222> (213)...(1211)

<400> 8
   gtcgacccac gcgtcctcga cgcgtgggcg gagcggaaac cagaggggaa accttgaact      60
   cctccagaca attgcttccg gggagttgcg agggagcgag ggggaataaa ggacccgcga      120
   ggaagggccc gcggatggcg cgtccctgag ggtcgtggcg agttcgcgga gcgtgggaag      180
   gagcggaccc tgctctcccc gggctgcggg cc atg gcc acg gcg gag cgg aga      233
                                       Met Ala Thr Ala Glu Arg Arg
                                       1               5
   gcc ctc ggc atc ggc ttc cag tgg ctc tct ttg gcc act ctg gtg ctc      281
   Ala Leu Gly Ile Gly Phe Gln Trp Leu Ser Leu Ala Thr Leu Val Leu
           10                  15                  20
   atc tgc gcc ggg caa ggg gga cgc agg gag gat ggg ggt cca gcc tgc      329
   Ile Cys Ala Gly Gln Gly Gly Arg Arg Glu Asp Gly Gly Pro Ala Cys
       25                  30                  35
   tac ggc gga ttt gac ctg tac ttc att ttg gac aaa tca gga agt gtg      377
   Tyr Gly Gly Phe Asp Leu Tyr Phe Ile Leu Asp Lys Ser Gly Ser Val
   40                  45                  50                  55
   ctg cac cac tgg aat gaa atc tat tac ttt gtg gaa cag ttg gct cac      425
   Leu His His Trp Asn Glu Ile Tyr Tyr Phe Val Glu Gln Leu Ala His
                   60                  65                  70
   aaa ttc atc agc cca cag ttg aga atg tcc ttt att gtt ttc tcc acc      473
   Lys Phe Ile Ser Pro Gln Leu Arg Met Ser Phe Ile Val Phe Ser Thr
               75                  80                  85
   cga gga aca acc tta atg aaa ctg aca gaa gac aga gaa caa atc cgt      521
   Arg Gly Thr Thr Leu Met Lys Leu Thr Glu Asp Arg Glu Gln Ile Arg
           90                  95                  100
   caa ggc cta gaa gaa ctc cag aaa gtt ctg cca gga gga gac act tac      569
   Gln Gly Leu Glu Glu Leu Gln Lys Val Leu Pro Gly Gly Asp Thr Tyr
       105                 110                 115
   atg cat gaa gga ttt gaa agg gcc agt gag cag att tat tat gaa aac      617
   Met His Glu Gly Phe Glu Arg Ala Ser Glu Gln Ile Tyr Tyr Glu Asn
   120                 125                 130                 135
   aga caa ggg tac agg aca gcc agc gtc atc att gct ttg act gat gga 665
   Arg Gln Gly Tyr Arg Thr Ala Ser Val Ile Ile Ala Leu Thr Asp Gly
   140 145 150
   gaa ctc cat gaa gat ctc ttt ttc tat tca gag agg gag gct aat agg 713
   Glu Leu His Glu Asp Leu Phe Phe Tyr Ser Glu Arg Glu Ala Asn Arg
   155 160 165
   tct cga gat ctt ggt gca att gtt tac tgt gtt ggt gtg aaa gat ttc 761
   Ser Arg Asp Leu Gly Ala Ile Val Tyr Cys Val Gly Val Lys Asp Phe
   170 175 180
   aat gag aca cag ctg gcc cgg att gcg gac agt aag gat cat gtg ttt 809
   Asn Glu Thr Gln Leu Ala Arg Ile Ala Asp Ser Lys Asp His Val Phe
   185 190 195
   ccc gtg aat gac ggc ttt cag gct ctg caa ggc atc atc cac tca att 857
   Pro Val Asn Asp Gly Phe Gln Ala Leu Gln Gly Ile Ile His Ser Ile
   200 205 210 215
   ttg aag aag tcc tgc atc gaa att cta gca gct gaa cca tcc acc ata 905
   Leu Lys Lys Ser Cys Ile Glu Ile Leu Ala Ala Glu Pro Ser Thr Ile
   220 225 230
   tgt gca gga gag tca ttt caa gtt gtc gtg aga gga aac ggc ttc cga 953
   Cys Ala Gly Glu Ser Phe Gln Val Val Val Arg Gly Asn Gly Phe Arg
   235 240 245
   cat gcc cgc aac gtg gac agg gtc ctc tgc agc ttc aag atc aat gac 1001
   His Ala Arg Asn Val Asp Arg Val Leu Cys Ser Phe Lys Ile Asn Asp
   250 255 260
   tcg gtc aca ctc aat gag aag ccc ttt tct gtg gaa gat act tat tta 1049
   Ser Val Thr Leu Asn Glu Lys Pro Phe Ser Val Glu Asp Thr Tyr Leu
   265 270 275
   ctg tgt cca gcg cct atc tta aaa gaa gtt ggc atg aaa gct gca ctc 1097
   Leu Cys Pro Ala Pro Ile Leu Lys Glu Val Gly Met Lys Ala Ala Leu
   280 285 290 295
   cag gtc agc atg aac gat ggc ctc tct ttt atc tcc agt tct gtc atc 1145
   Gln Val Ser Met Asn Asp Gly Leu Ser Phe Ile Ser Ser Ser Val Ile
   300 305 310
   atc acc acc aca cac tgt agc ctc cac aaa att gca tca ggc ccc aca 1193
   Ile Thr Thr Thr His Cys Ser Leu His Lys Ile Ala Ser Gly Pro Thr
   315 320 325
   aca gct gct tgc atg gaa tagcagagaa taccgcctgc tccctccgga 1241
   Thr Ala Ala Cys Met Glu
   330
   cagcacactc ctgaaaacgg ggagagagga gccaaacatg ctcggtttac actttcctta 1301
   tttactgaat gagtggaggg cagagacagg cctggagtta cgcacactga gtgccccaac 1361
   atggaaagaa acatcaggag ggacaggaaa cgttccctcc ttaaccaaca gttttcaaga 1421
   ccttactgga ggcactttat tggctacata atcactccat gcggtgggca tcaggcagaa 1481
   tcctggtgca gacccaactt tgaggtggag gatttcacag tttctttatt ttgaacttcc 1541
   cccaggctcc cactaattcc tctccattct atcctcctcc ctttcccaca aaagaaaaca 1601
   gaaaggagca gcagtgtttg ataccgtatc atccagaggc ctggttctct cccattatag 1661
   ggcaaacaag ccctggcaag atatttcact cccgccccat gccatgcatt aaaaatccaa 1721
   aattgcctat attccacctg ccaagcaaga gatgctttca ttattgaagt tccaaatgta 1781
   tacctttgag aacagtgcct tctcgtctta aaagagaggt cctcatttgg tgagttggga 1841
   gcagagggaa ttaaagaaag ccatgatgca gggatttggc cattcaagcc gggcagcctt 1901
   cagagaatgt catccctaat gacacatgcc cgaatgaagg agcggggctg agcttgtcct 1961
   gccttcgtat tgaatgttgc ctgtctgcct ccttaatagc gggcctctgt gtgagcattt 2021
   gacaagactt aaaactattc attgaagaaa atggatgatc ccccaacagg aagatgcaac 2081
   cccatgggct gcctgcttga ccacagaagt gcttccagct ccagttgctc atctgagaac 2141
   tccccccacc acttgctgtt aaaattgtta aaattaaagg ccatgttgat tgatttttta 2201
   aaaaaaaaaa aaaaaaaaaa aaaaaaaaaa aaaaaaaaaa aaaaaaaaaa aaaaaaaaaa 2261
   ggggcggccg c 2272

<210> 9
<211> 333
<212> PRT
<213> Homo sapiens

<400> 9
Met Ala Thr Ala Glu Arg Arg Ala Leu Gly Ile Gly Phe Gln Trp Leu
1               5                   10                  15
Ser Leu Ala Thr Leu Val Leu Ile Cys Ala Gly Gln Gly Gly Arg Arg
            20                  25                  30
Glu Asp Gly Gly Pro Ala Cys Tyr Gly Gly Phe Asp Leu Tyr Phe Ile
        35                  40                  45
Leu Asp Lys Ser Gly Ser Val Leu His His Trp Asn Glu Ile Tyr Tyr
    50                  55                  60
Phe Val Glu Gln Leu Ala His Lys Phe Ile Ser Pro Gln Leu Arg Met
65                  70                  75                  80
Ser Phe Ile Val Phe Ser Thr Arg Gly Thr Thr Leu Met Lys Leu Thr
                85                  90                  95
Glu Asp Arg Glu Gln Ile Arg Gln Gly Leu Glu Glu Leu Gln Lys Val
            100                 105                 110
Leu Pro Gly Gly Asp Thr Tyr Met His Glu Gly Phe Glu Arg Ala Ser
        115                 120                 125
Glu Gln Ile Tyr Tyr Glu Asn Arg Gln Gly Tyr Arg Thr Ala Ser Val
    130                 135                 140
Ile Ile Ala Leu Thr Asp Gly Glu Leu His Glu Asp Leu Phe Phe Tyr
145                 150                 155                 160
Ser Glu Arg Glu Ala Asn Arg Ser Arg Asp Leu Gly Ala Ile Val Tyr
                165                 170                 175
Cys Val Gly Val Lys Asp Phe Asn Glu Thr Gln Leu Ala Arg Ile Ala
            180                 185                 190
Asp Ser Lys Asp His Val Phe Pro Val Asn Asp Gly Phe Gln Ala Leu
        195                 200                 205
Gln Gly Ile Ile His Ser Ile Leu Lys Lys Ser Cys Ile Glu Ile Leu
    210                 215                 220
Ala Ala Glu Pro Ser Thr Ile Cys Ala Gly Glu Ser Phe Gln Val Val
225                 230                 235                 240
Val Arg Gly Asn Gly Phe Arg His Ala Arg Asn Val Asp Arg Val Leu
                245                 250                 255
Cys Ser Phe Lys Ile Asn Asp Ser Val Thr Leu Asn Glu Lys Pro Phe
            260                 265                 270
Ser Val Glu Asp Thr Tyr Leu Leu Cys Pro Ala Pro Ile Leu Lys Glu
        275                 280                 285
Val Gly Met Lys Ala Ala Leu Gln Val Ser Met Asn Asp Gly Leu Ser
    290                 295                 300
Phe Ile Ser Ser Ser Val Ile Ile Thr Thr Thr His Cys Ser Leu His
305                 310                 315                 320
Lys Ile Ala Ser Gly Pro Thr Thr Ala Ala Cys Met Glu
                325                 330

<210> 10
<211> 999
<212> DNA
<213> Homo sapiens

<400> 10
atggccacgg cggagcggag agccctcggc atcggcttcc agtggctctc tttggccact 60
ctggtgctca tctgcgccgg gcaaggggga cgcagggagg atgggggtcc agcctgctac 120
ggcggatttg acctgtactt cattttggac aaatcaggaa gtgtgctgca ccactggaat 180
gaaatctatt actttgtgga acagttggct cacaaattca tcagcccaca gttgagaatg 240

tcctttattg ttttctccac ccgaggaaca accttaatga aactgacaga agacagagaa 300
caaatccgtc aaggcctaga agaactccag aaagttctgc caggaggaga cacttacatg 360
catgaaggat ttgaaagggc cagtgagcag atttattatg aaaacagaca agggtacagg 420
acagccagcg tcatcattgc tttgactgat ggagaactcc atgaagatct ctttttctat 480
tcagagaggg aggctaatag gtctcgagat cttggtgcaa ttgtttactg tgttggtgtg 540
aaagatttca atgagacaca gctggcccgg attgcggaca gtaaggatca tgtgtttccc 600
gtgaatgacg gctttcaggc tctgcaaggc atcatccact caattttgaa gaagtcctgc 660
atcgaaattc tagcagctga accatccacc atatgtgcag gagagtcatt tcaagttgtc 720
gtgagaggaa acggcttccg acatgcccgc aacgtggaca gggtcctctg cagcttcaag 780
atcaatgact cggtcacact caatgagaag cccttttctg tggaagatac ttatttactg 840
tgtccagcgc ctatcttaaa agaagttggc atgaaagctg cactccaggt cagcatgaac 900
gatggcctct cttttatctc cagttctgtc atcatcacca ccacacactg tagcctccac 960
aaaattgcat caggccccac aacagctgct tgcatggaa 999

<210> 11
<211> 2435
<212> DNA
<213> Homo sapiens

<220>
<221> CDS
<222> (269)...(1927)

<400> 11
gtcgacccac gcgtccgtcc agcttcatcc gcagaggagc ctcggccagg cttgccaggg 60
cgcccccagc ccctccccag gccgcgagcg cccctgccgc ggtgcctggc ctccccgccc 120
agactgcagg gacagcaccc ggtaactgcg agtggagcgg aggacccgag cggctgagga 180
gagaggaggc ggcggcttag ctgctacggg gtccggccgg cgccctcccg aggggggctc 240
aggaggagga aggaggaccc gtgcgaga atg cct ctg ccc tgg agc ctt gcg 292
Met Pro Leu Pro Trp Ser Leu Ala
1               5

ctc ccg ctg ctg ctc tcc tgg gtg gca ggt ggt ttc ggg aac gcg gcc 340
Leu Pro Leu Leu Leu Ser Trp Val Ala Gly Gly Phe Gly Asn Ala Ala
    10                  15                  20

agt gca agg cat cac ggg ttg tta gca tcg gca cgt cag cct ggg gtc 388
Ser Ala Arg His His Gly Leu Leu Ala Ser Ala Arg Gln Pro Gly Val
25                  30                  35                  40

tgt cac tat gga act aaa ctg gcc tgc tgc tac ggc tgg aga aga aac 436
Cys His Tyr Gly Thr Lys Leu Ala Cys Cys Tyr Gly Trp Arg Arg Asn
                45                  50                  55

agc aag gga gtc tgt gaa gct aca tgc gaa cct gga tgt aag ttt ggt 484
Ser Lys Gly Val Cys Glu Ala Thr Cys Glu Pro Gly Cys Lys Phe Gly
            60                  65                  70

gag tgc gtg gga cca aac aaa tgc aga tgc ttt cca gga tac acc ggg 532
Glu Cys Val Gly Pro Asn Lys Cys Arg Cys Phe Pro Gly Tyr Thr Gly
        75                  80                  85

aaa acc tgc agt caa gat gtg aat gag tgt gga atg aaa ccc cgg cca 580
Lys Thr Cys Ser Gln Asp Val Asn Glu Cys Gly Met Lys Pro Arg Pro
    90                  95                  100

tgc caa cac aga tgt gtg aat aca cac gga agc tac aag tgc ttt tgc 628
Cys Gln His Arg Cys Val Asn Thr His Gly Ser Tyr Lys Cys Phe Cys
105                 110                 115                 120

ctc agt ggc cac atg ctc atg cca gat gct acg tgt gtg aac tct agg 676
Leu Ser Gly His Met Leu Met Pro Asp Ala Thr Cys Val Asn Ser Arg
                125                 130                 135

aca tgt gcc atg ata aac tgt cag tac agc tgt gaa gac aca gaa gaa 724
Thr Cys Ala Met Ile Asn Cys Gln Tyr Ser Cys Glu Asp Thr Glu Glu
            140                 145                 150

ggg cca cag tgc ctg tgt cca tcc tca gga ctc cgc ctg gcc cca aat 772
Gly Pro Gln Cys Leu Cys Pro Ser Ser Gly Leu Arg Leu Ala Pro Asn
        155                 160                 165

gga aga gac tgt cta gat att gat gaa tgt gcc tct ggt aaa gtc atc 820
Gly Arg Asp Cys Leu Asp Ile Asp Glu Cys Ala Ser Gly Lys Val Ile
    170                 175                 180

tgt ccc tac aat cga aga tgt gtg aac aca ttt gga agc tac tac tgc 868
Cys Pro Tyr Asn Arg Arg Cys Val Asn Thr Phe Gly Ser Tyr Tyr Cys
185                 190                 195                 200

aaa tgt cac att ggt ttc gaa ctg caa tat atc agt gga cga tat gac 916
Lys Cys His Ile Gly Phe Glu Leu Gln Tyr Ile Ser Gly Arg Tyr Asp
                205                 210                 215

tgt ata gat ata aat gaa tgt act atg gat agc cat acg tgc agc cac 964
Cys Ile Asp Ile Asn Glu Cys Thr Met Asp Ser His Thr Cys Ser His
            220                 225                 230

cat gcc aat tgc ttc aat acc caa ggg tcc ttc aag tgt aaa tgc aag 1012
His Ala Asn Cys Phe Asn Thr Gln Gly Ser Phe Lys Cys Lys Cys Lys
        235                 240                 245

cag gga tat aaa ggc aat gga ctt cgg tgt tct gct atc cct gaa aat 1060
Gln Gly Tyr Lys Gly Asn Gly Leu Arg Cys Ser Ala Ile Pro Glu Asn
    250                 255                 260

tct gtg aag gaa gtc ctc aga gca cct ggt acc atc aaa gac aga atc 1108
Ser Val Lys Glu Val Leu Arg Ala Pro Gly Thr Ile Lys Asp Arg Ile
265                 270                 275                 280

aag aag ttg ctt gct cac aaa aac agc atg aaa aag aag gca aaa att 1156
Lys Lys Leu Leu Ala His Lys Asn Ser Met Lys Lys Lys Ala Lys Ile
                285                 290                 295

aaa aat gtt acc cca gaa ccc acc agg act cct acc cct aag gtg aac 1204
Lys Asn Val Thr Pro Glu Pro Thr Arg Thr Pro Thr Pro Lys Val Asn
            300                 305                 310

ttg cag ccc ttc aac tat gaa gag ata gtt tcc aga ggc ggg aac tct 1252
Leu Gln Pro Phe Asn Tyr Glu Glu Ile Val Ser Arg Gly Gly Asn Ser
        315                 320                 325

cat gga ggt aaa aaa ggg aat gaa gag aaa atg aaa gag ggg ctt gag 1300
His Gly Gly Lys Lys Gly Asn Glu Glu Lys Met Lys Glu Gly Leu Glu
    330                 335                 340

gat gag aaa aga gaa gag aaa gcc ctg aag aat gac ata gag gag cga 1348
Asp Glu Lys Arg Glu Glu Lys Ala Leu Lys Asn Asp Ile Glu Glu Arg
345                 350                 355                 360

agc ctg cga gga gat gtg ttt ttc cct aag gtg aat gaa gca ggt gaa 1396
Ser Leu Arg Gly Asp Val Phe Phe Pro Lys Val Asn Glu Ala Gly Glu
                365                 370                 375

ttc ggc ctg att ctg gtc caa agg aaa gcg cta act tcc aaa ctg gaa 1444
Phe Gly Leu Ile Leu Val Gln Arg Lys Ala Leu Thr Ser Lys Leu Glu
            380                 385                 390

cat aaa gat tta aat atc tcg gtt gac tgc agc ttc aat cat ggg atc 1492
His Lys Asp Leu Asn Ile Ser Val Asp Cys Ser Phe Asn His Gly Ile

        395                 400                 405

tgt gac tgg aaa cag gat aga gaa gat gat ttt gac tgg aat cct gct 1540
Cys Asp Trp Lys Gln Asp Arg Glu Asp Asp Phe Asp Trp Asn Pro Ala
    410                 415                 420

gat cga gat aat gct att ggc ttc tat atg gca gtt ccg gcc ttg gca 1588
Asp Arg Asp Asn Ala Ile Gly Phe Tyr Met Ala Val Pro Ala Leu Ala
425                 430                 435                 440

ggt cac aag aaa gac att ggc cga ttg aaa ctt ctc cta cct gac ctg 1636
Gly His Lys Lys Asp Ile Gly Arg Leu Lys Leu Leu Leu Pro Asp Leu
                445                 450                 455

caa ccc caa agc aac ttc tgt ttg ctc ttt gat tac cgg ctg gcc gga 1684
Gln Pro Gln Ser Asn Phe Cys Leu Leu Phe Asp Tyr Arg Leu Ala Gly
            460                 465                 470

gac aaa gtc ggg aaa ctt cga gtg ttt gtg aaa aac agt aac aat gcc 1732
Asp Lys Val Gly Lys Leu Arg Val Phe Val Lys Asn Ser Asn Asn Ala
        475                 480                 485

ctg gca tgg gag aag acc acg agt gag gat gaa aag tgg aag aca ggg 1780
Leu Ala Trp Glu Lys Thr Thr Ser Glu Asp Glu Lys Trp Lys Thr Gly
    490                 495                 500

aaa att cag ttg tat caa gga act gat gct acc aaa agc atc att ttt 1828
Lys Ile Gln Leu Tyr Gln Gly Thr Asp Ala Thr Lys Ser Ile Ile Phe
505                 510                 515                 520

gaa gca gaa cgt ggc aag ggc aaa acc ggc gaa atc gca gtg gat ggc 1876
Glu Ala Glu Arg Gly Lys Gly Lys Thr Gly Glu Ile Ala Val Asp Gly
                525                 530                 535

gtc ttg ctt gtt tca ggc tta tgt cca gat agc ctt tta tct gtg gat 1924
Val Leu Leu Val Ser Gly Leu Cys Pro Asp Ser Leu Leu Ser Val Asp
            540                 545                 550

gac tgaatgttac tatctttata tttgactttg tatgtcagtt ccctggtttt 1977
Asp

tttgatattg catcatagga cctctggcat tttagaatta ctagctgaaa aattgtaatg 2037
taccaacaga aatattattg taagatgcct ttcttgtata agatatgcca atatttgctt 2097
taaatatcat atcactgtat cttctcagtc atttctgaat ctttccacat tatattataa 2157
aatatggaaa tgtcagttta tctcccctcc tcagtatatc tgatttgtat aagtaagttg 2217
atgagcttct ctctacaaca tttctagaaa atagaaaaaa aagcacagag aaatgtttaa 2277
ctgtttgact cttatgatac ttcttggaaa ctatgacatc aaagatagac ttttgcctaa 2337
gtggcttagc tgggtctttc atagccaaac ttgtatattt aaattctttg taataataat 2397
atccaaatca taaaaaaaaa aaaaaaaagg gcggccgc 2435

<210> 12
<211> 553
<212> PRT
<213> Homo sapiens

<400> 12
Met Pro Leu Pro Trp Ser Leu Ala Leu Pro Leu Leu Leu Ser Trp Val
1               5                   10                  15
Ala Gly Gly Phe Gly Asn Ala Ala Ser Ala Arg His His Gly Leu Leu
            20                  25                  30
Ala Ser Ala Arg Gln Pro Gly Val Cys His Tyr Gly Thr Lys Leu Ala
        35                  40                  45
Cys Cys Tyr Gly Trp Arg Arg Asn Ser Lys Gly Val Cys Glu Ala Thr
    50                  55                  60
Cys Glu Pro Gly Cys Lys Phe Gly Glu Cys Val Gly Pro Asn Lys Cys

65                  70                  75                  80
Arg Cys Phe Pro Gly Tyr Thr Gly Lys Thr Cys Ser Gln Asp Val Asn
                85                  90                  95
Glu Cys Gly Met Lys Pro Arg Pro Cys Gln His Arg Cys Val Asn Thr
            100                 105                 110
His Gly Ser Tyr Lys Cys Phe Cys Leu Ser Gly His Met Leu Met Pro
        115                 120                 125
Asp Ala Thr Cys Val Asn Ser Arg Thr Cys Ala Met Ile Asn Cys Gln
    130                 135                 140
Tyr Ser Cys Glu Asp Thr Glu Glu Gly Pro Gln Cys Leu Cys Pro Ser
145                 150                 155                 160
Ser Gly Leu Arg Leu Ala Pro Asn Gly Arg Asp Cys Leu Asp Ile Asp
                165                 170                 175
Glu Cys Ala Ser Gly Lys Val Ile Cys Pro Tyr Asn Arg Arg Cys Val
            180                 185                 190
Asn Thr Phe Gly Ser Tyr Tyr Cys Lys Cys His Ile Gly Phe Glu Leu
        195                 200                 205
Gln Tyr Ile Ser Gly Arg Tyr Asp Cys Ile Asp Ile Asn Glu Cys Thr
    210                 215                 220
Met Asp Ser His Thr Cys Ser His His Ala Asn Cys Phe Asn Thr Gln
225                 230                 235                 240
Gly Ser Phe Lys Cys Lys Cys Lys Gln Gly Tyr Lys Gly Asn Gly Leu
                245                 250                 255
Arg Cys Ser Ala Ile Pro Glu Asn Ser Val Lys Glu Val Leu Arg Ala
            260                 265                 270
Pro Gly Thr Ile Lys Asp Arg Ile Lys Lys Leu Leu Ala His Lys Asn
        275                 280                 285
Ser Met Lys Lys Lys Ala Lys Ile Lys Asn Val Thr Pro Glu Pro Thr
    290                 295                 300
Arg Thr Pro Thr Pro Lys Val Asn Leu Gln Pro Phe Asn Tyr Glu Glu
305                 310                 315                 320
Ile Val Ser Arg Gly Gly Asn Ser His Gly Gly Lys Lys Gly Asn Glu
                325                 330                 335
Glu Lys Met Lys Glu Gly Leu Glu Asp Glu Lys Arg Glu Glu Lys Ala
            340                 345                 350
Leu Lys Asn Asp Ile Glu Glu Arg Ser Leu Arg Gly Asp Val Phe Phe
        355                 360                 365
Pro Lys Val Asn Glu Ala Gly Glu Phe Gly Leu Ile Leu Val Gln Arg
    370                 375                 380
Lys Ala Leu Thr Ser Lys Leu Glu His Lys Asp Leu Asn Ile Ser Val
385                 390                 395                 400
Asp Cys Ser Phe Asn His Gly Ile Cys Asp Trp Lys Gln Asp Arg Glu
                405                 410                 415
Asp Asp Phe Asp Trp Asn Pro Ala Asp Arg Asp Asn Ala Ile Gly Phe
            420                 425                 430

Tyr Met Ala Val Pro Ala Leu Ala Gly His Lys Lys Asp Ile Gly Arg
        435                 440                 445
Leu Lys Leu Leu Leu Pro Asp Leu Gln Pro Gln Ser Asn Phe Cys Leu
    450                 455                 460
Leu Phe Asp Tyr Arg Leu Ala Gly Asp Lys Val Gly Lys Leu Arg Val
465                 470                 475                 480
Phe Val Lys Asn Ser Asn Asn Ala Leu Ala Trp Glu Lys Thr Thr Ser
                485                 490                 495
Glu Asp Glu Lys Trp Lys Thr Gly Lys Ile Gln Leu Tyr Gln Gly Thr
            500                 505                 510
Asp Ala Thr Lys Ser Ile Ile Phe Glu Ala Glu Arg Gly Lys Gly Lys
        515                 520                 525
Thr Gly Glu Ile Ala Val Asp Gly Val Leu Leu Val Ser Gly Leu Cys
    530                 535                 540
Pro Asp Ser Leu Leu Ser Val Asp Asp
545                 550

<210> 13
<211> 1659
<212> DNA

<213> Homo sapiens

<400> 13
atgcctctgc cctggagcct tgcgctcccg ctgctgctct cctgggtggc aggtggtttc 60
gggaacgcgg ccagtgcaag gcatcacggg ttgttagcat cggcacgtca gcctggggtc 120
tgtcactatg gaactaaact ggcctgctgc tacggctgga gaagaaacag caagggagtc 180
tgtgaagcta catgcgaacc tggatgtaag tttggtgagt gcgtgggacc aaacaaatgc 240
agatgctttc caggatacac cgggaaaacc tgcagtcaag atgtgaatga gtgtggaatg 300
aaaccccggc catgccaaca cagatgtgtg aatacacacg gaagctacaa gtgcttttgc 360
ctcagtggcc acatgctcat gccagatgct acgtgtgtga actctaggac atgtgccatg 420
ataaactgtc agtacagctg tgaagacaca gaagaagggc cacagtgcct gtgtccatcc 480
tcaggactcc gcctggcccc aaatggaaga gactgtctag atattgatga atgtgcctct 540
ggtaaagtca tctgtcccta caatcgaaga tgtgtgaaca catttggaag ctactactgc 600
aaatgtcaca ttggtttcga actgcaatat atcagtggac gatatgactg tatagatata 660
aatgaatgta ctatggatag ccatacgtgc agccaccatg ccaattgctt caatacccaa 720
gggtccttca agtgtaaatg caagcaggga tataaaggca atggacttcg gtgttctgct 780
atccctgaaa attctgtgaa ggaagtcctc agagcacctg gtaccatcaa agacagaatc 840
aagaagttgc ttgctcacaa aaacagcatg aaaaagaagg caaaaattaa aaatgttacc 900
ccagaaccca ccaggactcc tacccctaag gtgaacttgc agcccttcaa ctatgaagag 960
atagtttcca gaggcgggaa ctctcatgga ggtaaaaaag ggaatgaaga gaaaatgaaa 1020
gaggggcttg aggatgagaa aagagaagag aaagccctga agaatgacat agaggagcga 1080
agcctgcgag gagatgtgtt tttccctaag gtgaatgaag caggtgaatt cggcctgatt 1140
ctggtccaaa ggaaagcgct aacttccaaa ctggaacata aagatttaaa tatctcggtt 1200
gactgcagct tcaatcatgg gatctgtgac tggaaacagg atagagaaga tgattttgac 1260
tggaatcctg ctgatcgaga taatgctatt ggcttctata tggcagttcc ggccttggca 1320
ggtcacaaga aagacattgg ccgattgaaa cttctcctac ctgacctgca accccaaagc 1380
aacttctgtt tgctctttga ttaccggctg gccggagaca aagtcgggaa acttcgagtg 1440
tttgtgaaaa acagtaacaa tgccctggca tgggagaaga ccacgagtga ggatgaaaag 1500
tggaagacag ggaaaattca gttgtatcaa ggaactgatg ctaccaaaag catcattttt 1560
gaagcagaac gtggcaaggg caaaaccggc gaaatcgcag tggatggcgt cttgcttgtt 1620
tcaggcttat gtccagatag ccttttatct gtggatgac 1659

<210> 14
<211> 1496
<212> DNA
<213> Homo sapiens

<220>
<221> CDS
<222> (58)...(870)

<400> 14
   gtcgacccac gcgtgcggcg gcaacctcgg aagtgcggag cgggtgggcc tatatag atg 60
   Met
   1
   ttg agg tgc gga ggc cgt ggg ctt ttg ttg ggc ctg gct gta gcc gca 108
   Leu Arg Cys Gly Gly Arg Gly Leu Leu Leu Gly Leu Ala Val Ala Ala
   5 10 15
   gca gcg gta atg gca gca cgg ctt atg ggc tgg tgg ggt ccc cgc gct 156
   Ala Ala Val Met Ala Ala Arg Leu Met Gly Trp Trp Gly Pro Arg Ala
   20 25 30
   ggc ttt cgc ctt ttc ata ccg gag gag ctg tct cgc tac cgc ggc ggc 204
   Gly Phe Arg Leu Phe Ile Pro Glu Glu Leu Ser Arg Tyr Arg Gly Gly
   35 40 45
   cca ggg gac ccg ggc ctg tac ttg gcg ttg ctc ggc cgt gtc tac gat 252
   Pro Gly Asp Pro Gly Leu Tyr Leu Ala Leu Leu Gly Arg Val Tyr Asp
   50 55 60 65
   gtg tcc tcc ggc cgg agg cac tac gag cct ggg tcc cac tat agc ggc 300
   Val Ser Ser Gly Arg Arg His Tyr Glu Pro Gly Ser His Tyr Ser Gly
   70 75 80
   ttc gca ggc cga gac gca tcc aga gct ttc gtg acc ggg gac tgt tct 348
   Phe Ala Gly Arg Asp Ala Ser Arg Ala Phe Val Thr Gly Asp Cys Ser
   85                  90                  95
   gaa gca ggc ctc gtg gat gac gta tcc gac ctg tca gcc gct gag atg          396
   Glu Ala Gly Leu Val Asp Asp Val Ser Asp Leu Ser Ala Ala Glu Met
           100                 105                 110
   ctg aca ctt cac aat tgg ctt tca ttc tat gag aag aat tat gtg tgt          444
   Leu Thr Leu His Asn Trp Leu Ser Phe Tyr Glu Lys Asn Tyr Val Cys
       115                 120                 125
   gtt ggg agg gtg aca gga cgg ttc tac gga gag gat ggg ctg ccc acc          492
   Val Gly Arg Val Thr Gly Arg Phe Tyr Gly Glu Asp Gly Leu Pro Thr
   130                 135                 140                 145
   ccg gca ctg acc cag gta gaa gct gcg atc acc aga ggc ttg gag gcc          540
   Pro Ala Leu Thr Gln Val Glu Ala Ala Ile Thr Arg Gly Leu Glu Ala
                   150                 155                 160
   aac aaa cta cag ctg caa gag aag cag aca ttc ccg ccg tgc aac gcg          588
   Asn Lys Leu Gln Leu Gln Glu Lys Gln Thr Phe Pro Pro Cys Asn Ala
               165                 170                 175
   gag tgg agc tca gcc agg ggc agc cgg ctc tgg tgc tcc cag aag agt          636
   Glu Trp Ser Ser Ala Arg Gly Ser Arg Leu Trp Cys Ser Gln Lys Ser
           180                 185                 190
   gga ggt gtg agc aga gac tgg att ggc gtc ccc agg aag ctg tat aag          684
   Gly Gly Val Ser Arg Asp Trp Ile Gly Val Pro Arg Lys Leu Tyr Lys
       195                 200                 205
   cca ggt gct aag gag ccc cgc tgc gtg tgt gtg aga acc acc ggc ccc          732
   Pro Gly Ala Lys Glu Pro Arg Cys Val Cys Val Arg Thr Thr Gly Pro
   210                 215                 220                 225
   cct agt ggc cag atg ccg gac aac ccc tcc aca cag aaa tcg tgg gga          780
   Pro Ser Gly Gln Met Pro Asp Asn Pro Ser Thr Gln Lys Ser Trp Gly
                   230                 235                 240
   cct gga cca ccc aaa ctt ggc aga gta cac agg ctg ccc acc gct agc          828
   Pro Gly Pro Pro Lys Leu Gly Arg Val His Arg Leu Pro Thr Ala Ser
               245                 250                 255
   cat cac atg ctc ctt tcc act cta agc cgt agc ctc ttc tgt              870
   His His Met Leu Leu Ser Thr Leu Ser Arg Ser Leu Phe Cys
           260                 265                 270
   taataacaca cagagagctc tgccaagcac ctgagtaggc ccttgacact tgtgtgccct     930
   gggatgcctc ctggcgcgaa tcaggaggtt ctggaaggac tctggctata ttctgcaaat     990
   gtggctcatg ccccttaccg tggctcggcg ttgtggtgcc tgagggacag ccggccacct    1050
   gcccagtact ggtcagcttt tcaacactat tccctttgac ctactggcca tcttcctcac    1110
   agccctcaga tatcaacggg cacaaataag accaactcaa tttccacttg aatttacaac    1170
   caaaagcctg ctgagttgat tacagctggg ccaatacagt acgaggcaat aacaaattag    1230
   tgtgggttga ttctggaatt ggaaaagctt ttgcttgtat ggatacagca aatccagatg    1290
   tctctgaaca aagcaacaat ttaaagcaac gacattttct gtcctttaag cacttaaaat    1350
   caggtgtggt gtgttttcaa aggcagaagt ctgcattttg agcaaaaggt ggcttcccag    1410
   ctctaacaag gtaactggtt agcatgacat taaagcttgg gcaaggcttc aaacttaaaa    1470
   aaaaaaaaaa aaaaaagggc ggccgc                                          1496

<210> 15
<211> 271
<212> PRT
<213> Homo sapiens

<400> 15
Met Leu Arg Cys Gly Gly Arg Gly Leu Leu Leu Gly Leu Ala Val Ala
1               5                   10                  15
Ala Ala Ala Val Met Ala Ala Arg Leu Met Gly Trp Trp Gly Pro Arg
            20                  25                  30

Ala Gly Phe Arg Leu Phe Ile Pro Glu Glu Leu Ser Arg Tyr Arg Gly
        35                  40                  45
Gly Pro Gly Asp Pro Gly Leu Tyr Leu Ala Leu Leu Gly Arg Val Tyr
    50                  55                  60
Asp Val Ser Ser Gly Arg Arg His Tyr Glu Pro Gly Ser His Tyr Ser
65                  70                  75                  80
Gly Phe Ala Gly Arg Asp Ala Ser Arg Ala Phe Val Thr Gly Asp Cys
                85                  90                  95
Ser Glu Ala Gly Leu Val Asp Asp Val Ser Asp Leu Ser Ala Ala Glu
            100                 105                 110
Met Leu Thr Leu His Asn Trp Leu Ser Phe Tyr Glu Lys Asn Tyr Val
        115                 120                 125
Cys Val Gly Arg Val Thr Gly Arg Phe Tyr Gly Glu Asp Gly Leu Pro
    130                 135                 140
Thr Pro Ala Leu Thr Gln Val Glu Ala Ala Ile Thr Arg Gly Leu Glu
145                 150                 155                 160
Ala Asn Lys Leu Gln Leu Gln Glu Lys Gln Thr Phe Pro Pro Cys Asn
                165                 170                 175
Ala Glu Trp Ser Ser Ala Arg Gly Ser Arg Leu Trp Cys Ser Gln Lys
            180                 185                 190
Ser Gly Gly Val Ser Arg Asp Trp Ile Gly Val Pro Arg Lys Leu Tyr
        195                 200                 205
Lys Pro Gly Ala Lys Glu Pro Arg Cys Val Cys Val Arg Thr Thr Gly
    210                 215                 220
Pro Pro Ser Gly Gln Met Pro Asp Asn Pro Ser Thr Gln Lys Ser Trp
225                 230                 235                 240
Gly Pro Gly Pro Pro Lys Leu Gly Arg Val His Arg Leu Pro Thr Ala
                245                 250                 255

Ser His His Met Leu Leu Ser Thr Leu Ser Arg Ser Leu Phe Cys
            260                 265                 270

<210> 16
<211> 813
<212> DNA
<213> Homo sapiens

<400> 16
atgttgaggt gcggaggccg tgggcttttg ttgggcctgg ctgtagccgc agcagcggta      60
atggcagcac ggcttatggg ctggtggggt ccccgcgctg gctttcgcct tttcataccg     120
gaggagctgt ctcgctaccg cggcggccca ggggacccgg gcctgtactt ggcgttgctc     180
ggccgtgtct acgatgtgtc ctccggccgg aggcactacg agcctgggtc ccactatagc     240
ggcttcgcag gccgagacgc atccagagct ttcgtgaccg gggactgttc tgaagcaggc     300
ctcgtggatg acgtatccga cctgtcagcc gctgagatgc tgacacttca caattggctt     360
tcattctatg agaagaatta tgtgtgtgtt gggagggtga caggacggtt ctacggagag     420
gatgggctgc ccaccccggc actgacccag gtagaagctg cgatcaccag aggcttggag     480
gccaacaaac tacagctgca agagaagcag acattcccgc cgtgcaacgc ggagtggagc     540
tcagccaggg gcagccggct ctggtgctcc cagaagagtg gaggtgtgag cagagactgg     600
attggcgtcc ccaggaagct gtataagcca ggtgctaagg agccccgctg cgtgtgtgtg     660
agaaccaccg gcccccctag tggccagatg ccggacaacc cctccacaca gaaatcgtgg     720
ggacctggac cacccaaact tggcagagta cacaggctgc ccaccgctag ccatcacatg     780
ctcctttcca ctctaagccg tagcctcttc tgt                                  813

<210> 17
<211> 2689
<212> DNA
<213> Homo sapiens

<220>
<221> CDS
<222> (1)...(1440)

<400> 17
cag cag gca gag gag cac tta gca gct tat tca gtg tcc gat tct gat      48
Gln Gln Ala Glu Glu His Leu Ala Ala Tyr Ser Val Ser Asp Ser Asp
 1               5                  10                  15

tcc ggc aag gat cca agc atg gaa tgc tgc cgt cgg gca act cct ggc      96

Ser Gly Lys Asp Pro Ser Met Glu Cys Cys Arg Arg Ala Thr Pro Gly
            20                  25                  30

aca ctg ctc ctc ttt ctg gct ttc ctg ctc ctg agt tcc agg acc gca      144
Thr Leu Leu Leu Phe Leu Ala Phe Leu Leu Leu Ser Ser Arg Thr Ala
        35                  40                  45

cgc tcc gag gag gac cgg gac ggc ctg tgg gat gcc tgg ggc cca tgg      192
Arg Ser Glu Glu Asp Arg Asp Gly Leu Trp Asp Ala Trp Gly Pro Trp
    50                  55                  60

agt gaa tgc tca cgc acc tgc ggg gga ggg gcc tcc tac tct ctg agg      240
Ser Glu Cys Ser Arg Thr Cys Gly Gly Gly Ala Ser Tyr Ser Leu Arg
65                  70                  75                  80

cgc tgc ctg agc agc aag agc tgt gaa gga aga aat atc cga tac aga      288
Arg Cys Leu Ser Ser Lys Ser Cys Glu Gly Arg Asn Ile Arg Tyr Arg
                85                  90                  95

aca tgc agt aat gtg gac tgc cca cca gaa gca ggt gat ttc cga gct      336
Thr Cys Ser Asn Val Asp Cys Pro Pro Glu Ala Gly Asp Phe Arg Ala
            100                 105                 110

cag caa tgc tca gct cat aat gat gtc aag cac cat ggc cag ttt tat      384
Gln Gln Cys Ser Ala His Asn Asp Val Lys His His Gly Gln Phe Tyr
        115                 120                 125

gaa tgg ctt cct gtg tct aat gac cct gac aac cca tgt tca ctc aag      432
Glu Trp Leu Pro Val Ser Asn Asp Pro Asp Asn Pro Cys Ser Leu Lys
    130                 135                 140

tgc caa gcc aaa gga aca acc ctg gtt gtt gaa cta gca cct aag gtc      480
Cys Gln Ala Lys Gly Thr Thr Leu Val Val Glu Leu Ala Pro Lys Val
145                 150                 155                 160

tta gat ggt acg cgt tgc tat aca gaa tct ttg gat atg tgc atc agt      528
Leu Asp Gly Thr Arg Cys Tyr Thr Glu Ser Leu Asp Met Cys Ile Ser
                165                 170                 175

ggt tta tgc caa att gtt ggc tgc gat cac cag ctg gga agc acc gtc      576
Gly Leu Cys Gln Ile Val Gly Cys Asp His Gln Leu Gly Ser Thr Val
            180                 185                 190

aag gaa gat aac tgt ggg gtc tgc aac gga gat ggg tcc acc tgc cgg      624
Lys Glu Asp Asn Cys Gly Val Cys Asn Gly Asp Gly Ser Thr Cys Arg
        195                 200                 205

ctg gtc cga ggg cag tat aaa tcc cag ctc tcc gca acc aaa tcg gat      672
Leu Val Arg Gly Gln Tyr Lys Ser Gln Leu Ser Ala Thr Lys Ser Asp
    210                 215                 220

gat act gtg gtt gca att ccc tat gga agt aga cat att cgc ctt gtc      720
Asp Thr Val Val Ala Ile Pro Tyr Gly Ser Arg His Ile Arg Leu Val
225                 230                 235                 240

tta aaa ggt cct gat cac tta tat ctg gaa acc aaa acc ctc cag ggg      768
Leu Lys Gly Pro Asp His Leu Tyr Leu Glu Thr Lys Thr Leu Gln Gly
                245                 250                 255

act aaa ggt gaa aac agt ctc agc tcc aca gga act ttc ctt gtg gac      816
Thr Lys Gly Glu Asn Ser Leu Ser Ser Thr Gly Thr Phe Leu Val Asp
            260                 265                 270

aat tct agt gtg gac ttc cag aaa ttt cca gac aaa gag ata ctg aga      864
Asn Ser Ser Val Asp Phe Gln Lys Phe Pro Asp Lys Glu Ile Leu Arg
        275                 280                 285

atg gct gga cca ctc aca gca gat ttc att gtc aag att cgt aac tcg      912

Met Ala Gly Pro Leu Thr Ala Asp Phe Ile Val Lys Ile Arg Asn Ser
    290                 295                 300

ggc tcc gct gac agt aca gtc cag ttc atc ttc tat caa ccc atc atc 960
Gly Ser Ala Asp Ser Thr Val Gln Phe Ile Phe Tyr Gln Pro Ile Ile
305                 310                 315                 320

cac cga tgg agg gag acg gat ttc ttt cct tgc tca gca acc tgt gga 1008
His Arg Trp Arg Glu Thr Asp Phe Phe Pro Cys Ser Ala Thr Cys Gly
                325                 330                 335

gga ggt tat cag ctg aca tcg gct gag tgc tac gat ctg agg agc aac 1056
Gly Gly Tyr Gln Leu Thr Ser Ala Glu Cys Tyr Asp Leu Arg Ser Asn
            340                 345                 350

cgt gtg gtt gct gac caa tac tgt cac tat tac cca gag aac atc aaa 1104
Arg Val Val Ala Asp Gln Tyr Cys His Tyr Tyr Pro Glu Asn Ile Lys
        355                 360                 365

ccc aaa ccc aag ctt cag gag tgc aac ttg gat cct tgt cca gcc aga 1152
Pro Lys Pro Lys Leu Gln Glu Cys Asn Leu Asp Pro Cys Pro Ala Arg
    370                 375                 380

ggg ttg gca tta ttg ttc cta aca gtg acg gat aca agc aga tca tgc 1200
Gly Leu Ala Leu Leu Phe Leu Thr Val Thr Asp Thr Ser Arg Ser Cys
385                 390                 395                 400

ctt atg acc tct acc atc ccc ttc ctc ggt ggg agg cca ccc cat gga 1248
Leu Met Thr Ser Thr Ile Pro Phe Leu Gly Gly Arg Pro Pro His Gly
                405                 410                 415

ccg cgt gct cct cct cgt gtg ggg ggg gca tcc aga gcc ggg cag ttt 1296
Pro Arg Ala Pro Pro Arg Val Gly Gly Ala Ser Arg Ala Gly Gln Phe
            420                 425                 430

cct gtg tgg agg agg aca tcc agg ggc atg tca ctt cag tgg aag agt 1344
Pro Val Trp Arg Arg Thr Ser Arg Gly Met Ser Leu Gln Trp Lys Ser
        435                 440                 445

gga aat gca tgt aca ccc cta aga tgc cca tcg cgc agc cct gca aca 1392
Gly Asn Ala Cys Thr Pro Leu Arg Cys Pro Ser Arg Ser Pro Ala Thr
    450                 455                 460

ttt ttg act gcc cta aat ggc tgg cac agg agt ggt ctc cgt gca cag 1440
Phe Leu Thr Ala Leu Asn Gly Trp His Arg Ser Gly Leu Arg Ala Gln
465                 470                 475                 480

tgacatgtgg ccagggcctc agataccgtg tggtcctctg catcgaccat cgaggaatgc 1500
acacaggagg ctgtagccca aaaacaaagc cccacataaa agaggaatgc atcgtaccca 1560
ctccctgcta taaacccaaa gagaaacttc cagtcgaggc caagttgcca tggttcaaac 1620
aagctcaaga gctagaagaa ggagctgctg tgtcagagga gccctcgttc atcccagagg 1680
cctggtcggc ctgcacagtc acctgtggtg tggggaccca ggtgcgaata gtcaggtgcc 1740
aggtgctcct gtctttctct cagtccgtgg ctgacctgcc tattgacgag tgtgaagggc 1800
ccaagccagc atcccagcgt gcctgttatg caggcccatg cagcggggaa attcctgagt 1860
tcaacccaga cgagacagat gggctctttg gtggcctgca ggatttcgac gagctgtatg 1920
actgggagta tgaggggttc accaagtgct ccgagtcctg tggaggaggt gtccaggagg 1980
ctgtggtgag ctgcttgaac aaacagactc gggagcctgc tgaggagaac ctgtgcgtga 2040
ccagccgccg gcccccacag ctcctgaagt cctgcaattt ggatccctgc ccagcaaggt 2100
gggaaattgg caagtggagt ccatgtagtc tcacatgtgg ggtcggccta cagaccagag 2160
acgtcttctg cagccacctg ctttccagag agatgaatga aacagtcatc ctggctgatg 2220
agctgtgtcg ccagcccaag cccagcacgg tgcaagcttg taaccgcttt aattgccccc 2280
cagcctggta ccctgcacag tggcagccgt gttccagaac gtgtggcggg ggtgttcaga 2340
aacgtgaggt tctttgcaag cagcgcatgg ctgatggcag cttcctggag cttcctgaga 2400
ccttctgttc agcttcaaaa cctgcctgcc agcaagcatg caagaaagat gactgtccca 2460
gcgagtggct tctctcagac tggacagagt gttccacaag ctgcggggaa ggcacccaga 2520
ctcgaagcgc catttgccga aagatgctga aaaccggcat ctcaacggtt gtcaattcca 2580

ccctgtgccc gcccctgcct ttctcttcct ccatcaggcc ctgtatgctg gcaacctgtg 2640
caaggcccgg gcggccatcc acgaagcaca gcccgcacat cgcggccgc 2689

<210> 18
<211> 480
<212> PRT
<213> Homo sapiens

<400> 18
Gln Gln Ala Glu Glu His Leu Ala Ala Tyr Ser Val Ser Asp Ser Asp
1               5                   10                  15
Ser Gly Lys Asp Pro Ser Met Glu Cys Cys Arg Arg Ala Thr Pro Gly
            20                  25                  30
Thr Leu Leu Leu Phe Leu Ala Phe Leu Leu Leu Ser Ser Arg Thr Ala
        35                  40                  45
Arg Ser Glu Glu Asp Arg Asp Gly Leu Trp Asp Ala Trp Gly Pro Trp
    50                  55                  60
Ser Glu Cys Ser Arg Thr Cys Gly Gly Gly Ala Ser Tyr Ser Leu Arg
65                  70                  75                  80
Arg Cys Leu Ser Ser Lys Ser Cys Glu Gly Arg Asn Ile Arg Tyr Arg
                85                  90                  95
Thr Cys Ser Asn Val Asp Cys Pro Pro Glu Ala Gly Asp Phe Arg Ala
            100                 105                 110
Gln Gln Cys Ser Ala His Asn Asp Val Lys His His Gly Gln Phe Tyr
        115                 120                 125
Glu Trp Leu Pro Val Ser Asn Asp Pro Asp Asn Pro Cys Ser Leu Lys
    130                 135                 140
Cys Gln Ala Lys Gly Thr Thr Leu Val Val Glu Leu Ala Pro Lys Val
145                 150                 155                 160
Leu Asp Gly Thr Arg Cys Tyr Thr Glu Ser Leu Asp Met Cys Ile Ser
                165                 170                 175

Gly Leu Cys Gln Ile Val Gly Cys Asp His Gln Leu Gly Ser Thr Val
            180                 185                 190
Lys Glu Asp Asn Cys Gly Val Cys Asn Gly Asp Gly Ser Thr Cys Arg
        195                 200                 205
Leu Val Arg Gly Gln Tyr Lys Ser Gln Leu Ser Ala Thr Lys Ser Asp
    210                 215                 220
Asp Thr Val Val Ala Ile Pro Tyr Gly Ser Arg His Ile Arg Leu Val
225                 230                 235                 240
Leu Lys Gly Pro Asp His Leu Tyr Leu Glu Thr Lys Thr Leu Gln Gly
                245                 250                 255
Thr Lys Gly Glu Asn Ser Leu Ser Ser Thr Gly Thr Phe Leu Val Asp
            260                 265                 270
Asn Ser Ser Val Asp Phe Gln Lys Phe Pro Asp Lys Glu Ile Leu Arg
        275                 280                 285
Met Ala Gly Pro Leu Thr Ala Asp Phe Ile Val Lys Ile Arg Asn Ser
    290                 295                 300
Gly Ser Ala Asp Ser Thr Val Gln Phe Ile Phe Tyr Gln Pro Ile Ile
305                 310                 315                 320
His Arg Trp Arg Glu Thr Asp Phe Phe Pro Cys Ser Ala Thr Cys Gly
                325                 330                 335
Gly Gly Tyr Gln Leu Thr Ser Ala Glu Cys Tyr Asp Leu Arg Ser Asn
            340                 345                 350
Arg Val Val Ala Asp Gln Tyr Cys His Tyr Tyr Pro Glu Asn Ile Lys
        355                 360                 365
Pro Lys Pro Lys Leu Gln Glu Cys Asn Leu Asp Pro Cys Pro Ala Arg
    370                 375                 380
Gly Leu Ala Leu Leu Phe Leu Thr Val Thr Asp Thr Ser Arg Ser Cys
385                 390                 395                 400
Leu Met Thr Ser Thr Ile Pro Phe Leu Gly Gly Arg Pro Pro His Gly
                405                 410                 415
Pro Arg Ala Pro Pro Arg Val Gly Gly Ala Ser Arg Ala Gly Gln Phe
            420                 425                 430
Pro Val Trp Arg Arg Thr Ser Arg Gly Met Ser Leu Gln Trp Lys Ser
        435                 440                 445
Gly Asn Ala Cys Thr Pro Leu Arg Cys Pro Ser Arg Ser Pro Ala Thr
    450                 455                 460

Phe Leu Thr Ala Leu Asn Gly Trp His Arg Ser Gly Leu Arg Ala Gln
465                 470                 475                 480

<210> 19
<211> 1440
<212> DNA
<213> Homo sapiens

<400> 19
cagcaggcag aggagcactt agcagcttat tcagtgtccg attctgattc cggcaaggat      60
ccaagcatgg aatgctgccg tcgggcaact cctggcacac tgctcctctt tctggctttc     120
ctgctcctga gttccaggac cgcacgctcc gaggaggacc gggacggcct gtgggatgcc     180
tggggcccat ggagtgaatg ctcacgcacc tgcgggggag gggcctccta ctctctgagg      240
cgctgcctga gcagcaagag ctgtgaagga agaaatatcc gatacagaac atgcagtaat     300
gtggactgcc caccagaagc aggtgatttc cgagctcagc aatgctcagc tcataatgat     360
gtcaagcacc atggccagtt ttatgaatgg cttcctgtgt ctaatgaccc tgacaaccca     420
tgttcactca agtgccaagc caaaggaaca accctggttg ttgaactagc acctaaggtc     480
ttagatggta cgcgttgcta tacagaatct ttggatatgt gcatcagtgg tttatgccaa     540
attgttggct gcgatcacca gctgggaagc accgtcaagg aagataactg tggggtctgc     600
aacggagatg ggtccacctg ccggctggtc cgagggcagt ataaatccca gctctccgca     660
accaaatcgg atgatactgt ggttgcaatt ccctatggaa gtagacatat tcgccttgtc     720
ttaaaaggtc ctgatcactt atatctggaa accaaaaccc tccaggggac taaaggtgaa     780
aacagtctca gctccacagg aactttcctt gtggacaatt ctagtgtgga cttccagaaa     840
tttccagaca aagagatact gagaatggct ggaccactca cagcagattt cattgtcaag     900
attcgtaact cgggctccgc tgacagtaca gtccagttca tcttctatca acccatcatc     960
caccgatgga gggagacgga tttctttcct tgctcagcaa cctgtggagg aggttatcag    1020
ctgacatcgg ctgagtgcta cgatctgagg agcaaccgtg tggttgctga ccaatactgt    1080
cactattacc cagagaacat caaacccaaa cccaagcttc aggagtgcaa cttggatcct    1140
tgtccagcca gagggttggc attattgttc ctaacagtga cggatacaag cagatcatgc    1200
cttatgacct ctaccatccc cttcctcggt gggaggccac cccatggacc gcgtgctcct    1260
cctcgtgtgg ggggggcatc cagagccggg cagtttcctg tgtggaggag gacatccagg     1320
ggcatgtcac ttcagtggaa gagtggaaat gcatgtacac ccctaagatg cccatcgcgc    1380
agccctgcaa catttttgac tgccctaaat ggctggcaca ggagtggtct ccgtgcacag    1440

<210> 20
<211> 3413
<212> DNA
<213> Homo sapiens

<220>
<221> CDS
<222> (344)...(1990)

<400> 20
gtcgacccac gcgtcccggg ctggcctttc aaagtgtgca gttgtctcct ccctgtccag      60
ccccatcgtc gcccaggacc agctgggccg cggtctgacc tgaggctgct gctcagcgcc     120
ggggcgctgg cgctctccat tcgagcacct tccagcatac cgctcggctc cgggagccgc     180
tctgcaaagt tgggcagctc agagcgcaag ctttgcctct cgacttctcc ctccttgggt     240
ccccggcgcc cccgcctccc acgatccctt tcactaggag cagccagtcc cagcgggctg     300
gcaacttgca ccccttccta gtcatcctcc ctgaaacgcg acc atg ctg tta agg      355
                                                Met Leu Leu Arg
                                                1

ggc gtc ctc ctg gcg ttg caa gcc ctg cag ctc gcc ggt gcc ctc gac      403
Gly Val Leu Leu Ala Leu Gln Ala Leu Gln Leu Ala Gly Ala Leu Asp
5                   10                  15                  20

ctg ccc gct ggg tcc tgt gcc ttt gaa gag agc act tgc ggc ttt gac      451
Leu Pro Ala Gly Ser Cys Ala Phe Glu Glu Ser Thr Cys Gly Phe Asp
                25                  30                  35

tcc gtg ttg gcc tct ctg ccg tgg att tta aat gag gaa ggc cat tac      499
Ser Val Leu Ala Ser Leu Pro Trp Ile Leu Asn Glu Glu Gly His Tyr
            40                  45                  50

att tat gtg gat acc tcc ttt ggc aag cag ggg gag aaa gct gtg ctg      547

Ile Tyr Val Asp Thr Ser Phe Gly Lys Gln Gly Glu Lys Ala Val Leu
        55                  60                  65

cta agt cct gac tta cag gct gag gaa tgg agc tgc ctc cgt ttg gtc      595
Leu Ser Pro Asp Leu Gln Ala Glu Glu Trp Ser Cys Leu Arg Leu Val
    70                  75                  80

tac cag ata acc aca tct tcg gag tct ctg tca gat ccc agc cag ctg      643
Tyr Gln Ile Thr Thr Ser Ser Glu Ser Leu Ser Asp Pro Ser Gln Leu
85                  90                  95                  100

aac ctc tac atg aga ttt gaa gat gaa agc ttt gat cgc ttg ctt tgg      691
Asn Leu Tyr Met Arg Phe Glu Asp Glu Ser Phe Asp Arg Leu Leu Trp
                105                 110                 115

tca gct aag gaa cct tca gac agc tgg ctc ata gcc agc ttg gat ttg      739
Ser Ala Lys Glu Pro Ser Asp Ser Trp Leu Ile Ala Ser Leu Asp Leu
            120                 125                 130

caa aac agt tcc aag aaa ttc aag att tta ata gaa ggt gta cta gga      787
Gln Asn Ser Ser Lys Lys Phe Lys Ile Leu Ile Glu Gly Val Leu Gly
        135                 140                 145

cag gga aac aca gcc agc atc gca cta ttt gaa atc aag atg aca acc      835
Gln Gly Asn Thr Ala Ser Ile Ala Leu Phe Glu Ile Lys Met Thr Thr
    150                 155                 160

ggc tac tgt att gaa tgt gac ttt gaa gaa aat cat ctc tgt ggc ttt      883
Gly Tyr Cys Ile Glu Cys Asp Phe Glu Glu Asn His Leu Cys Gly Phe
165                 170                 175                 180

gtg aac cgc tgg aat ccc aat gtg aac tgg ttt gtt gga gga gga agt      931
Val Asn Arg Trp Asn Pro Asn Val Asn Trp Phe Val Gly Gly Gly Ser
                185                 190                 195

att cgg aat gtc cac tcc att ctc cca cag gat cac acc ttc aag agt      979
Ile Arg Asn Val His Ser Ile Leu Pro Gln Asp His Thr Phe Lys Ser
            200                 205                 210

gaa ctg ggc cac tac atg tac gtg gac tca gtt tat gtg aag cac ttc      1027
Glu Leu Gly His Tyr Met Tyr Val Asp Ser Val Tyr Val Lys His Phe
        215                 220                 225

cag gag gtg gca cag ctc atc tcc ccg ttg acc acg gcc ccc atg gct      1075
Gln Glu Val Ala Gln Leu Ile Ser Pro Leu Thr Thr Ala Pro Met Ala
    230                 235                 240

ggc tgc ctg tca ttt tat tac cag atc cag cag ggg aat gac aat gtc      1123
Gly Cys Leu Ser Phe Tyr Tyr Gln Ile Gln Gln Gly Asn Asp Asn Val
245                 250                 255                 260

ttt tcc ctt tac act cgg gat gtg gct ggc ctt tac gag gaa atc tgg      1171
Phe Ser Leu Tyr Thr Arg Asp Val Ala Gly Leu Tyr Glu Glu Ile Trp
                265                 270                 275

aaa gca gac agg cca ggg aat gct gcc tgg aac ctt gcg gag gtc gag      1219
Lys Ala Asp Arg Pro Gly Asn Ala Ala Trp Asn Leu Ala Glu Val Glu
            280                 285                 290

ttc aat gct cct tac ccc atg gag gtt att ttt gaa gtt gct ttc aat      1267
Phe Asn Ala Pro Tyr Pro Met Glu Val Ile Phe Glu Val Ala Phe Asn
        295                 300                 305

ggt ccc aag gga ggt tat gtt gcc ctg gat gat att tca ttc tct cct      1315
Gly Pro Lys Gly Gly Tyr Val Ala Leu Asp Asp Ile Ser Phe Ser Pro
    310                 315                 320

gtt cac tgc cag aat cag aca gaa ctt ctg ttc agt gcc gtg gaa gcc     1363
Val His Cys Gln Asn Gln Thr Glu Leu Leu Phe Ser Ala Val Glu Ala
325                 330                 335                 340

agc tgc aat ttt gag caa gat ctc tgc aac ttt tac caa gat aaa gaa     1411
Ser Cys Asn Phe Glu Gln Asp Leu Cys Asn Phe Tyr Gln Asp Lys Glu
                345                 350                 355

ggt cca ggt tgg acc cga gtg aaa gta aaa cca aac atg tat cgg gct     1459
Gly Pro Gly Trp Thr Arg Val Lys Val Lys Pro Asn Met Tyr Arg Ala
            360                 365                 370

gga gac cac act aca ggc tta ggg tat tac ctg cta gcc aac aca aag     1507
Gly Asp His Thr Thr Gly Leu Gly Tyr Tyr Leu Leu Ala Asn Thr Lys
        375                 380                 385

ttc aca tct cag cct ggc tac att gga agg ctc tat ggg ccc tcc cta     1555
Phe Thr Ser Gln Pro Gly Tyr Ile Gly Arg Leu Tyr Gly Pro Ser Leu
    390                 395                 400

cca gga aac ttg cag tat tgt ctg cgt ttt cat tat gcc atc tat gga     1603
Pro Gly Asn Leu Gln Tyr Cys Leu Arg Phe His Tyr Ala Ile Tyr Gly
405                 410                 415                 420

ttt tta aaa atg agt gac acc cta gca gtt tac atc ttt gaa gag aac     1651
Phe Leu Lys Met Ser Asp Thr Leu Ala Val Tyr Ile Phe Glu Glu Asn
                425                 430                 435

cat gtg gtt caa gag aag atc tgg tct gtg ttg gag tcc cca agg ggt     1699
His Val Val Gln Glu Lys Ile Trp Ser Val Leu Glu Ser Pro Arg Gly
            440                 445                 450

gtt tgg atg caa gct gaa atc acc ttt aag aag ccc atg cct acc aag     1747
Val Trp Met Gln Ala Glu Ile Thr Phe Lys Lys Pro Met Pro Thr Lys
        455                 460                 465

gtg gtt ttc atg agc cta tgc aaa agt ttc tgg gac tgt ggg ctt gta     1795
Val Val Phe Met Ser Leu Cys Lys Ser Phe Trp Asp Cys Gly Leu Val
    470                 475                 480

gcc ctg gat gac att aca ata caa ttg gga agc tgc tca tct tca gag     1843
Ala Leu Asp Asp Ile Thr Ile Gln Leu Gly Ser Cys Ser Ser Ser Glu
485                 490                 495                 500

aaa ctt cca cct cac ctg gag agt gta ctt tcg agc aag atg aat gta     1891
Lys Leu Pro Pro His Leu Glu Ser Val Leu Ser Ser Lys Met Asn Val
                505                 510                 515

cat tta ctc agg aga aaa gaa acc gga gca gct ggc aca gga gga ggg     1939
His Leu Leu Arg Arg Lys Glu Thr Gly Ala Ala Gly Thr Gly Gly Gly
            520                 525                 530

gag aaa ctc cca ctt cct aca cag gac caa agg gag atc aca cta ctg     1987
Glu Lys Leu Pro Leu Pro Thr Gln Asp Gln Arg Glu Ile Thr Leu Leu
        535                 540                 545

ggg taggctacta catgtacatt gaggcctccc atatggtgta tggacaaaaa     2040
Gly

gcacgcctct tgtccaggcc tctgcgagga gtctctggaa aacactgctt gacctttttc     2100
taccacatgt atggaggggg cactggcctg ctgagtgttt atctgaaaaa ggaagaagac     2160
agtgaagagt ccctcttatg gaggagaaga ggtgaacaga gcatttcctg gctacgagca     2220
ctgattgaat acagctgtga gaggcaacac cagataattt ttgaagccat tcgaggagta     2280
tcaataagaa gtgatattgc cattgatgat gttaaatttc aggcaggacc ctgtggagaa     2340

atggaagata caactcaaca atcatcagga tattctgagg acttaaatga aattgagtat 2400
taagaaatga tctgcattgg atttactaga cgaaaaccat acctctcttc aatcaaaatg 2460
aaaacaaagc aaatgaatac tggacagtct taacaatttt ataagttata aaatgacttt 2520
agagcaccct ccttcattac ttttgcaaaa acatactgac tcagggctct ttttttcttt 2580
ttgcatatga caactgttac tagaaataca ggctactggt tttgcataga tcattcatct 2640
taattttggt accagttaaa aatacaaatg tactatattg tagtcatttt aaagtacaca 2700
aagggcacaa tcaaaatgag atgcactcat ttaaatctgc attcagtgaa tgtattggga 2760
gaaaaatagg tcttgcaggt ttccttttga attttaagta tcataaatat tttttaagta 2820
aataatacgg ggtgtcagta atatctgcag aatgaatgca gtctttcatg ctaatgagtt 2880
agtctggaaa aataaagtct tattttctat gttttattca tagaaatgga gtattaattt 2940
ttaatatttt caccatatgt gataacaaag gatctttcat gaatgtccaa gggtaagtca 3000
gtattaatta atgctgtatt acaaggcaat gctaccttct ttattccccc tttgaactac 3060
ctttgaagtc actatgagca catggataga aatttaactt tttttgtaa agcaagctta 3120
aaatgtttat gtatacatac ccagcaactt ttataaatgt gttaaacaat tttactgatt 3180
tttataataa atattttggt aagattttga ataatatgaa ttcaggcaga tatactaaac 3240
tgcttttatt tacttgttta gaaaattgta tatatatgtt tgtgtatcct aacagctgct 3300
atgaaattat aaaattacct aataaaaata atttgaaaat caaaaaaaaa aaaaaaaaaa 3360
aaaaaaaaaa aaaaaaaaaa aaaaaaaaaa aaaaaaaaaa aaaaaagggg ggg 3413

<210> 21
<211> 549
<212> PRT
<213> Homo sapiens

<400> 21
Met Leu Leu Arg Gly Val Leu Leu Ala Leu Gln Ala Leu Gln Leu Ala
1               5                   10                  15
Gly Ala Leu Asp Leu Pro Ala Gly Ser Cys Ala Phe Glu Glu Ser Thr
            20                  25                  30
Cys Gly Phe Asp Ser Val Leu Ala Ser Leu Pro Trp Ile Leu Asn Glu
        35                  40                  45
Glu Gly His Tyr Ile Tyr Val Asp Thr Ser Phe Gly Lys Gln Gly Glu
    50                  55                  60
Lys Ala Val Leu Leu Ser Pro Asp Leu Gln Ala Glu Glu Trp Ser Cys
65                  70                  75                  80
Leu Arg Leu Val Tyr Gln Ile Thr Thr Ser Ser Glu Ser Leu Ser Asp
                85                  90                  95
Pro Ser Gln Leu Asn Leu Tyr Met Arg Phe Glu Asp Glu Ser Phe Asp
            100                 105                 110
Arg Leu Leu Trp Ser Ala Lys Glu Pro Ser Asp Ser Trp Leu Ile Ala
        115                 120                 125
Ser Leu Asp Leu Gln Asn Ser Ser Lys Lys Phe Lys Ile Leu Ile Glu
    130                 135                 140
Gly Val Leu Gly Gln Gly Asn Thr Ala Ser Ile Ala Leu Phe Glu Ile
145                 150                 155                 160
Lys Met Thr Thr Gly Tyr Cys Ile Glu Cys Asp Phe Glu Glu Asn His
                165                 170                 175
Leu Cys Gly Phe Val Asn Arg Trp Asn Pro Asn Val Asn Trp Phe Val
            180                 185                 190
Gly Gly Gly Ser Ile Arg Asn Val His Ser Ile Leu Pro Gln Asp His
        195                 200                 205
Thr Phe Lys Ser Glu Leu Gly His Tyr Met Tyr Val Asp Ser Val Tyr
    210                 215                 220
Val Lys His Phe Gln Glu Val Ala Gln Leu Ile Ser Pro Leu Thr Thr
225                 230                 235                 240
Ala Pro Met Ala Gly Cys Leu Ser Phe Tyr Tyr Gln Ile Gln Gln Gly
                245                 250                 255
Asn Asp Asn Val Phe Ser Leu Tyr Thr Arg Asp Val Ala Gly Leu Tyr
            260                 265                 270
Glu Glu Ile Trp Lys Ala Asp Arg Pro Gly Asn Ala Ala Trp Asn Leu
        275                 280                 285
Ala Glu Val Glu Phe Asn Ala Pro Tyr Pro Met Glu Val Ile Phe Glu
    290                 295                 300
Val Ala Phe Asn Gly Pro Lys Gly Gly Tyr Val Ala Leu Asp Asp Ile
305                 310                 315                 320
Ser Phe Ser Pro Val His Cys Gln Asn Gln Thr Glu Leu Leu Phe Ser
                325                 330                 335
Ala Val Glu Ala Ser Cys Asn Phe Glu Gln Asp Leu Cys Asn Phe Tyr

340                 345                 350
Gln Asp Lys Glu Gly Pro Gly Trp Thr Arg Val Lys Val Lys Pro Asn
        355                 360                 365
Met Tyr Arg Ala Gly Asp His Thr Thr Gly Leu Gly Tyr Tyr Leu Leu
    370                 375                 380
Ala Asn Thr Lys Phe Thr Ser Gln Pro Gly Tyr Ile Gly Arg Leu Tyr
385                 390                 395                 400

Gly Pro Ser Leu Pro Gly Asn Leu Gln Tyr Cys Leu Arg Phe His Tyr
                405                 410                 415
Ala Ile Tyr Gly Phe Leu Lys Met Ser Asp Thr Leu Ala Val Tyr Ile
            420                 425                 430
Phe Glu Glu Asn His Val Val Gln Glu Lys Ile Trp Ser Val Leu Glu
        435                 440                 445
Ser Pro Arg Gly Val Trp Met Gln Ala Glu Ile Thr Phe Lys Lys Pro
    450                 455                 460
Met Pro Thr Lys Val Val Phe Met Ser Leu Cys Lys Ser Phe Trp Asp
465                 470                 475                 480
Cys Gly Leu Val Ala Leu Asp Asp Ile Thr Ile Gln Leu Gly Ser Cys
                485                 490                 495
Ser Ser Ser Glu Lys Leu Pro Pro His Leu Glu Ser Val Leu Ser Ser
            500                 505                 510
Lys Met Asn Val His Leu Leu Arg Arg Lys Glu Thr Gly Ala Ala Gly
        515                 520                 525
Thr Gly Gly Gly Glu Lys Leu Pro Leu Pro Thr Gln Asp Gln Arg Glu
    530                 535                 540
Ile Thr Leu Leu Gly
545

<210> 22
<211> 1647
<212> DNA
<213> Homo sapiens

<400> 22
atgctgttaa ggggcgtcct cctggcgttg caagccctgc agctcgccgg tgccctcgac      60
ctgcccgctg ggtcctgtgc ctttgaagag agcacttgcg gctttgactc cgtgttggcc     120
tctctgccgt ggattttaaa tgaggaaggc cattacattt atgtggatac ctcctttggc     180
aagcaggggg agaaagctgt gctgctaagt cctgacttac aggctgagga atggagctgc     240
ctccgtttgg tctaccagat aaccacatct tcggagtctc tgtcagatcc cagccagctg     300
aacctctaca tgagatttga agatgaaagc tttgatcgct tgctttggtc agctaaggaa     360
ccttcagaca gctggctcat agccagcttg gatttgcaaa acagttccaa gaaattcaag     420
attttaatag aaggtgtact aggacaggga aacacagcca gcatcgcact atttgaaatc     480
aagatgacaa ccggctactg tattgaatgt gactttgaag aaaatcatct ctgtggcttt     540
gtgaaccgct ggaatcccaa tgtgaactgg tttgttggag gaggaagtat tcggaatgtc     600
cactccattc tcccacagga tcacaccttc aagagtgaac tgggccacta catgtacgtg     660
gactcagttt atgtgaagca cttccaggag gtggcacagc tcatctcccc gttgaccacg     720
gcccccatgg ctggctgcct gtcattttat taccagatcc agcaggggaa tgacaatgtc     780
ttttcccttt acactcggga tgtggctggc ctttacgagg aaatctggaa agcagacagg     840
ccagggaatg ctgcctggaa ccttgcggag gtcgagttca atgctcctta ccccatggag     900
gttatttttg aagttgcttt caatggtccc aagggaggtt atgttgccct ggatgatatt     960
tcattctctc ctgttcactg ccagaatcag acagaacttc tgttcagtgc cgtggaagcc    1020
agctgcaatt ttgagcaaga tctctgcaac ttttaccaag ataaagaagg tccaggttgg    1080
acccgagtga aagtaaaacc aaacatgtat cgggctggag accacactac aggcttaggg    1140
tattacctgc tagccaacac aaagttcaca tctcagcctg gctacattgg aaggctctat    1200
gggccctccc taccaggaaa cttgcagtat tgtctgcgtt ttcattatgc catctatgga    1260
tttttaaaaa tgagtgacac cctagcagtt tacatctttg aagagaacca tgtggttcaa    1320
gagaagatct ggtctgtgtt ggagtcccca aggggtgttt ggatgcaagc tgaaatcacc    1380
tttaagaagc ccatgcctac caaggtggtt ttcatgagcc tatgcaaaag tttctgggac    1440
tgtgggcttg tagccctgga tgacattaca atacaattgg gaagctgctc atcttcagag    1500
aaacttccac ctcacctgga gagtgtactt tcgagcaaga tgaatgtaca tttactcagg    1560
agaaaagaaa ccggagcagc tggcacagga ggaggggaga aactcccact tcctacacag    1620
gaccaaaggg agatcacact actgggg                                        1647

<210> 23
<211> 22
<212> PRT
<213> Homo sapiens

<400> 23
Met Ser Leu Phe Gly Leu Leu Leu Leu Thr Ser Ala Leu Ala Gly Gln
1               5                   10                  15
Arg Gln Gly Thr Gln Ala
            20

<210> 24
<211> 27
<212> PRT
<213> Homo sapiens

<400> 24
Met Ala Thr Ala Glu Arg Arg Ala Leu Gly Ile Gly Phe Gln Trp Leu
1               5                   10                  15
Ser Leu Ala Thr Leu Val Leu Ile Cys Ala Gly
            20                  25

<210> 25
<211> 18
<212> PRT
<213> Homo sapiens

<400> 25
Met Pro Leu Pro Trp Ser Leu Ala Leu Pro Leu Leu Leu Ser Trp Val
1               5                   10                  15
Ala Gly

<210> 26
<211> 22
<212> PRT
<213> Homo sapiens

<400> 26
Met Leu Arg Cys Gly Gly Arg Gly Leu Leu Leu Gly Leu Ala Val Ala
1               5                   10                  15
Ala Ala Ala Val Met Ala
            20

<210> 27
<211> 28
<212> PRT
<213> Homo sapiens

<400> 27
Gln Gln Ala Glu Glu His Leu Ala Ala Tyr Ser Val Ser Asp Ser Asp
1               5                   10                  15
Ser Gly Lys Asp Pro Ser Met Glu Cys Cys Arg Arg
            20                  25

<210> 28
<211> 18
<212> PRT
<213> Homo sapiens

<400> 28
Met Leu Leu Arg Gly Val Leu Leu Ala Leu Gln Ala Leu Gln Leu Ala
1               5                   10                  15
Gly Ala

<210> 29
<211> 323
<212> PRT
<213> Homo sapiens

<400> 29
Glu Ser Asn Leu Ser Ser Lys Phe Gln Phe Ser Ser Asn Lys Glu Gln
1               5                   10                  15
Asn Gly Val Gln Asp Pro Gln His Glu Arg Ile Ile Thr Val Ser Thr
            20                  25                  30
Asn Gly Ser Ile His Ser Pro Arg Phe Pro His Thr Tyr Pro Arg Asn
        35                  40                  45
Thr Val Leu Val Trp Arg Leu Val Ala Val Glu Glu Asn Val Trp Ile
    50                  55                  60
Gln Leu Thr Phe Asp Glu Arg Phe Gly Leu Glu Asp Pro Glu Asp Asp
65                  70                  75                  80
Ile Cys Lys Tyr Asp Phe Val Glu Val Glu Glu Pro Ser Asp Gly Thr
                85                  90                  95
Ile Leu Gly Arg Trp Cys Gly Ser Gly Thr Val Pro Gly Lys Gln Ile
            100                 105                 110
Ser Lys Gly Asn Gln Ile Arg Ile Arg Phe Val Ser Asp Glu Tyr Phe
        115                 120                 125
Pro Ser Glu Pro Gly Phe Cys Ile His Tyr Asn Ile Val Met Pro Gln
    130                 135                 140
Phe Thr Glu Ala Val Ser Pro Ser Val Leu Pro Pro Ser Ala Leu Pro
145                 150                 155                 160
Leu Asp Leu Leu Asn Asn Ala Ile Thr Ala Phe Ser Thr Leu Glu Asp
                165                 170                 175
Leu Ile Arg Tyr Leu Glu Pro Glu Arg Trp Gln Leu Asp Leu Glu Asp
            180                 185                 190
Leu Tyr Arg Pro Thr Trp Gln Leu Leu Gly Lys Ala Phe Val Phe Gly
        195                 200                 205
Arg Lys Ser Arg Val Val Asp Leu Asn Leu Leu Thr Glu Glu Val Arg
    210                 215                 220

Leu Tyr Ser Cys Thr Pro Arg Asn Phe Ser Val Ser Ile Arg Glu Glu
225                 230                 235                 240
Leu Lys Arg Thr Asp Thr Ile Phe Trp Pro Gly Cys Leu Leu Val Lys
                245                 250                 255
Arg Cys Gly Gly Asn Cys Ala Cys Cys Leu His Asn Cys Asn Glu Cys
            260                 265                 270
Gln Cys Val Pro Ser Lys Val Thr Lys Lys Tyr His Glu Val Leu Gln
        275                 280                 285
Leu Arg Pro Lys Thr Gly Val Arg Gly Leu His Lys Ser Leu Thr Asp
    290                 295                 300
Val Ala Leu Glu His His Glu Glu Cys Asp Cys Val Cys Arg Gly Ser
305                 310                 315                 320
Thr Gly Gly


<210> 30
<211> 306
<212> PRT
<213> Homo sapiens

<400> 30
Gln Gly Gly Arg Arg Glu Asp Gly Gly Pro Ala Cys Tyr Gly Gly Phe
1               5                   10                  15
Asp Leu Tyr Phe Ile Leu Asp Lys Ser Gly Ser Val Leu His His Trp
            20                  25                  30
Asn Glu Ile Tyr Tyr Phe Val Glu Gln Leu Ala His Lys Phe Ile Ser
        35                  40                  45

Pro Gln Leu Arg Met Ser Phe Ile Val Phe Ser Thr Arg Gly Thr Thr
    50                  55                  60
Leu Met Lys Leu Thr Glu Asp Arg Glu Gln Ile Arg Gln Gly Leu Glu
65                  70                  75                  80
Glu Leu Gln Lys Val Leu Pro Gly Gly Asp Thr Tyr Met His Glu Gly
                85                  90                  95
Phe Glu Arg Ala Ser Glu Gln Ile Tyr Tyr Glu Asn Arg Gln Gly Tyr
            100                 105                 110
Arg Thr Ala Ser Val Ile Ile Ala Leu Thr Asp Gly Glu Leu His Glu

Asp Leu Phe Phe Tyr Ser Glu Arg Glu Ala Asn Arg Ser Arg Asp Leu
        115                 120                 125
Gly Ala Ile Val Tyr Cys Val Gly Val Lys Asp Phe Asn Glu Thr Gln
    130                 135                 140
Leu Ala Arg Ile Ala Asp Ser Lys Asp His Val Phe Pro Val Asn Asp
145                 150                 155                 160
Gly Phe Gln Ala Leu Gln Gly Ile Ile His Ser Ile Leu Lys Lys Ser
                165                 170                 175
Cys Ile Glu Ile Leu Ala Ala Glu Pro Ser Thr Ile Cys Ala Gly Glu
            180                 185                 190
Ser Phe Gln Val Val Val Arg Gly Asn Gly Phe Arg His Ala Arg Asn
        195                 200                 205
Val Asp Arg Val Leu Cys Ser Phe Lys Ile Asn Asp Ser Val Thr Leu
    210                 215                 220
Asn Glu Lys Pro Phe Ser Val Glu Asp Thr Tyr Leu Leu Cys Pro Ala
225                 230                 235                 240
Pro Ile Leu Lys Glu Val Gly Met Lys Ala Ala Leu Gln Val Ser Met
                245                 250                 255
Asn Asp Gly Leu Ser Phe Ile Ser Ser Ser Val Ile Ile Thr Thr Thr
            260                 265                 270
His Cys Ser Leu His Lys Ile Ala Ser Gly Pro Thr Thr Ala Ala Cys
        275                 280                 285
Met Glu
    290                 295                 300
305

<210> 31
<211> 535
<212> PRT
<213> Homo sapiens

<400> 31
Gly Phe Gly Asn Ala Ala Ser Ala Arg His His Gly Leu Leu Ala Ser
 1               5                  10                  15
Ala Arg Gln Pro Gly Val Cys His Tyr Gly Thr Lys Leu Ala Cys Cys
            20                  25                  30
Tyr Gly Trp Arg Arg Asn Ser Lys Gly Val Cys Glu Ala Thr Cys Glu
        35                  40                  45
Pro Gly Cys Lys Phe Gly Glu Cys Val Gly Pro Asn Lys Cys Arg Cys
    50                  55                  60
Phe Pro Gly Tyr Thr Gly Lys Thr Cys Ser Gln Asp Val Asn Glu Cys
65                  70                  75                  80
Gly Met Lys Pro Arg Pro Cys Gln His Arg Cys Val Asn Thr His Gly
                85                  90                  95
Ser Tyr Lys Cys Phe Cys Leu Ser Gly His Met Leu Met Pro Asp Ala
            100                 105                 110
Thr Cys Val Asn Ser Arg Thr Cys Ala Met Ile Asn Cys Gln Tyr Ser
        115                 120                 125
Cys Glu Asp Thr Glu Glu Gly Pro Gln Cys Leu Cys Pro Ser Ser Gly
    130                 135                 140
Leu Arg Leu Ala Pro Asn Gly Arg Asp Cys Leu Asp Ile Asp Glu Cys
145                 150                 155                 160
Ala Ser Gly Lys Val Ile Cys Pro Tyr Asn Arg Arg Cys Val Asn Thr
                165                 170                 175
Phe Gly Ser Tyr Tyr Cys Lys Cys His Ile Gly Phe Glu Leu Gln Tyr
            180                 185                 190
Ile Ser Gly Arg Tyr Asp Cys Ile Asp Ile Asn Glu Cys Thr Met Asp
        195                 200                 205
Ser His Thr Cys Ser His His Ala Asn Cys Phe Asn Thr Gln Gly Ser
    210                 215                 220
Phe Lys Cys Lys Cys Lys Gln Gly Tyr Lys Gly Asn Gly Leu Arg Cys
225                 230                 235                 240
Ser Ala Ile Pro Glu Asn Ser Val Lys Glu Val Leu Arg Ala Pro Gly
                245                 250                 255
Thr Ile Lys Asp Arg Ile Lys Lys Leu Leu Ala His Lys Asn Ser Met
            260                 265                 270
Lys Lys Lys Ala Lys Ile Lys Asn Val Thr Pro Glu Pro Thr Arg Thr

                275                 280                 285
Pro Thr Pro Lys Val Asn Leu Gln Pro Phe Asn Tyr Glu Glu Ile Val
    290                 295                 300
Ser Arg Gly Gly Asn Ser His Gly Gly Lys Lys Gly Asn Glu Glu Lys
305                 310                 315                 320
Met Lys Glu Gly Leu Glu Asp Glu Lys Arg Glu Glu Lys Ala Leu Lys
                325                 330                 335
Asn Asp Ile Glu Glu Arg Ser Leu Arg Gly Asp Val Phe Phe Pro Lys
            340                 345                 350
Val Asn Glu Ala Gly Glu Phe Gly Leu Ile Leu Val Gln Arg Lys Ala
        355                 360                 365
Leu Thr Ser Lys Leu Glu His Lys Asp Leu Asn Ile Ser Val Asp Cys
    370                 375                 380
Ser Phe Asn His Gly Ile Cys Asp Trp Lys Gln Asp Arg Glu Asp Asp
385                 390                 395                 400
Phe Asp Trp Asn Pro Ala Asp Arg Asp Asn Ala Ile Gly Phe Tyr Met
                405                 410                 415
Ala Val Pro Ala Leu Ala Gly His Lys Lys Asp Ile Gly Arg Leu Lys
            420                 425                 430

Leu Leu Leu Pro Asp Leu Gln Pro Gln Ser Asn Phe Cys Leu Leu Phe
        435                 440                 445
Asp Tyr Arg Leu Ala Gly Asp Lys Val Gly Lys Leu Arg Val Phe Val
    450                 455                 460
Lys Asn Ser Asn Asn Ala Leu Ala Trp Glu Lys Thr Thr Ser Glu Asp
465                 470                 475                 480
Glu Lys Trp Lys Thr Gly Lys Ile Gln Leu Tyr Gln Gly Thr Asp Ala
                485                 490                 495
Thr Lys Ser Ile Ile Phe Glu Ala Glu Arg Gly Lys Gly Lys Thr Gly
            500                 505                 510
Glu Ile Ala Val Asp Gly Val Leu Leu Val Ser Gly Leu Cys Pro Asp
        515                 520                 525
Ser Leu Leu Ser Val Asp Asp
    530                 535

<210> 32
<211> 249
<212> PRT
<213> Homo sapiens

<400> 32
Ala Arg Leu Met Gly Trp Trp Gly Pro Arg Ala Gly Phe Arg Leu Phe
1               5                   10                  15
Ile Pro Glu Glu Leu Ser Arg Tyr Arg Gly Gly Pro Gly Asp Pro Gly
            20                  25                  30
Leu Tyr Leu Ala Leu Leu Gly Arg Val Tyr Asp Val Ser Ser Gly Arg
        35                  40                  45

Arg His Tyr Glu Pro Gly Ser His Tyr Ser Gly Phe Ala Gly Arg Asp
    50                  55                  60
Ala Ser Arg Ala Phe Val Thr Gly Asp Cys Ser Glu Ala Gly Leu Val
65                  70                  75                  80
Asp Asp Val Ser Asp Leu Ser Ala Ala Glu Met Leu Thr Leu His Asn
                85                  90                  95
Trp Leu Ser Phe Tyr Glu Lys Asn Tyr Val Cys Val Gly Arg Val Thr
            100                 105                 110
Gly Arg Phe Tyr Gly Glu Asp Gly Leu Pro Thr Pro Ala Leu Thr Gln
        115                 120                 125
Val Glu Ala Ala Ile Thr Arg Gly Leu Glu Ala Asn Lys Leu Gln Leu
    130                 135                 140
Gln Glu Lys Gln Thr Phe Pro Pro Cys Asn Ala Glu Trp Ser Ser Ala
145                 150                 155                 160
Arg Gly Ser Arg Leu Trp Cys Ser Gln Lys Ser Gly Gly Val Ser Arg
                165                 170                 175
Asp Trp Ile Gly Val Pro Arg Lys Leu Tyr Lys Pro Gly Ala Lys Glu
            180                 185                 190
Pro Arg Cys Val Cys Val Arg Thr Thr Gly Pro Pro Ser Gly Gln Met
        195                 200                 205

Pro Asp Asn Pro Ser Thr Gln Lys Ser Trp Gly Pro Gly Pro Pro Lys
    210                 215                 220
Leu Gly Arg Val His Arg Leu Pro Thr Ala Ser His His Met Leu Leu
225                 230                 235                 240
Ser Thr Leu Ser Arg Ser Leu Phe Cys
                245

<210> 33
<211> 430
<212> PRT
<213> Homo sapiens

<400> 33
Ala Thr Pro Gly Thr Leu Leu Leu Phe Leu Ala Phe Leu Leu Leu Ser
1               5                   10                  15
Ser Arg Thr Ala Arg Ser Glu Glu Asp Arg Asp Gly Leu Trp Asp Ala
            20                  25                  30
Trp Gly Pro Trp Ser Glu Cys Ser Arg Thr Cys Gly Gly Gly Ala Ser
        35                  40                  45
Tyr Ser Leu Arg Arg Cys Leu Ser Ser Lys Ser Cys Glu Gly Arg Asn
    50                  55                  60
Ile Arg Tyr Arg Thr Cys Ser Asn Val Asp Cys Pro Pro Glu Ala Gly
65                  70                  75                  80
Asp Phe Arg Ala Gln Gln Cys Ser Ala His Asn Asp Val Lys His His
                85                  90                  95
Gly Gln Phe Tyr Glu Trp Leu Pro Val Ser Asn Asp Pro Asp Asn Pro
            100                 105                 110
Cys Ser Leu Lys Cys Gln Ala Lys Gly Thr Thr Leu Val Val Glu Leu
        115                 120                 125
Ala Pro Lys Val Leu Asp Gly Thr Arg Cys Tyr Thr Glu Ser Leu Asp
    130                 135                 140
Met Cys Ile Ser Gly Leu Cys Gln Ile Val Gly Cys Asp His Gln Leu
145                 150                 155                 160
Gly Ser Thr Val Lys Glu Asp Asn Cys Gly Val Cys Asn Gly Asp Gly
                165                 170                 175
Ser Thr Cys Arg Leu Val Arg Gly Gln Tyr Lys Ser Gln Leu Ser Ala
            180                 185                 190
Thr Lys Ser Asp Asp Thr Val Val Ala Ile Pro Tyr Gly Ser Arg His
        195                 200                 205
Ile Arg Leu Val Leu Lys Gly Pro Asp His Leu Tyr Leu Glu Thr Lys
    210                 215                 220

Thr Leu Gln Gly Thr Lys Gly Glu Asn Ser Leu Ser Ser Thr Gly Thr
225                 230                 235                 240
Phe Leu Val Asp Asn Ser Ser Val Asp Phe Gln Lys Phe Pro Asp Lys
                245                 250                 255
Glu Ile Leu Arg Met Ala Gly Pro Leu Thr Ala Asp Phe Ile Val Lys
            260                 265                 270
Ile Arg Asn Ser Gly Ser Ala Asp Ser Thr Val Gln Phe Ile Phe Tyr
        275                 280                 285
Gln Pro Ile Ile His Arg Trp Arg Glu Thr Asp Phe Phe Pro Cys Ser
    290                 295                 300
Ala Thr Cys Gly Gly Gly Tyr Gln Leu Thr Ser Ala Glu Cys Tyr Asp
305                 310                 315                 320
Leu Arg Ser Asn Arg Val Val Ala Asp Gln Tyr Cys His Tyr Tyr Pro
                325                 330                 335
Glu Asn Ile Lys Pro Lys Pro Lys Leu Gln Glu Cys Asn Leu Asp Pro
            340                 345                 350
Cys Pro Ala Arg Gly Leu Ala Leu Leu Phe Leu Thr Val Thr Asp Thr
        355                 360                 365
Ser Arg Ser Cys Leu Met Thr Ser Thr Ile Pro Phe Leu Gly Gly Arg
    370                 375                 380
Pro Pro His Gly Pro Arg Ala Pro Pro Arg Val Gly Gly Ala Ser Arg
385                 390                 395                 400
Ala Gly Gln Phe Pro Val Trp Arg Arg Thr Ser Arg Gly Met Ser Leu
                405                 410                 415

Gln Trp Lys Ser Gly Asn Ala Cys Thr Pro Leu Arg Cys Pro
            420                 425                 430

<210> 34
<211> 531
<212> PRT
<213> Homo sapiens

<400> 34
Leu Asp Leu Pro Ala Gly Ser Cys Ala Phe Glu Glu Ser Thr Cys Gly
1               5                   10                  15
Phe Asp Ser Val Leu Ala Ser Leu Pro Trp Ile Leu Asn Glu Glu Gly
            20                  25                  30
His Tyr Ile Tyr Val Asp Thr Ser Phe Gly Lys Gln Gly Glu Lys Ala
        35                  40                  45
Val Leu Leu Ser Pro Asp Leu Gln Ala Glu Glu Trp Ser Cys Leu Arg
    50                  55                  60
Leu Val Tyr Gln Ile Thr Thr Ser Ser Glu Ser Leu Ser Asp Pro Ser
65                  70                  75                  80
Gln Leu Asn Leu Tyr Met Arg Phe Glu Asp Glu Ser Phe Asp Arg Leu
                85                  90                  95
Leu Trp Ser Ala Lys Glu Pro Ser Asp Ser Trp Leu Ile Ala Ser Leu
            100                 105                 110
Asp Leu Gln Asn Ser Ser Lys Lys Phe Lys Ile Leu Ile Glu Gly Val
        115                 120                 125
Leu Gly Gln Gly Asn Thr Ala Ser Ile Ala Leu Phe Glu Ile Lys Met
    130                 135                 140
Thr Thr Gly Tyr Cys Ile Glu Cys Asp Phe Glu Glu Asn His Leu Cys
145                 150                 155                 160
Gly Phe Val Asn Arg Trp Asn Pro Asn Val Asn Trp Phe Val Gly Gly
                165                 170                 175
Gly Ser Ile Arg Asn Val His Ser Ile Leu Pro Gln Asp His Thr Phe
            180                 185                 190
Lys Ser Glu Leu Gly His Tyr Met Tyr Val Asp Ser Val Tyr Val Lys
        195                 200                 205
His Phe Gln Glu Val Ala Gln Leu Ile Ser Pro Leu Thr Thr Ala Pro
    210                 215                 220

Met Ala Gly Cys Leu Ser Phe Tyr Tyr Gln Ile Gln Gln Gly Asn Asp
225                 230                 235                 240
Asn Val Phe Ser Leu Tyr Thr Arg Asp Val Ala Gly Leu Tyr Glu Glu
                245                 250                 255
Ile Trp Lys Ala Asp Arg Pro Gly Asn Ala Ala Trp Asn Leu Ala Glu
            260                 265                 270
Val Glu Phe Asn Ala Pro Tyr Pro Met Glu Val Ile Phe Glu Val Ala
        275                 280                 285
Phe Asn Gly Pro Lys Gly Gly Tyr Val Ala Leu Asp Asp Ile Ser Phe
    290                 295                 300
Ser Pro Val His Cys Gln Asn Gln Thr Glu Leu Leu Phe Ser Ala Val
305                 310                 315                 320
Glu Ala Ser Cys Asn Phe Glu Gln Asp Leu Cys Asn Phe Tyr Gln Asp
                325                 330                 335
Lys Glu Gly Pro Gly Trp Thr Arg Val Lys Val Lys Pro Asn Met Tyr
            340                 345                 350
Arg Ala Gly Asp His Thr Thr Gly Leu Gly Tyr Tyr Leu Leu Ala Asn
        355                 360                 365
Thr Lys Phe Thr Ser Gln Pro Gly Tyr Ile Gly Arg Leu Tyr Gly Pro
    370                 375                 380
Ser Leu Pro Gly Asn Leu Gln Tyr Cys Leu Arg Phe His Tyr Ala Ile
385                 390                 395                 400
Tyr Gly Phe Leu Lys Met Ser Asp Thr Leu Ala Val Tyr Ile Phe Glu
                405                 410                 415
Glu Asn His Val Val Gln Glu Lys Ile Trp Ser Val Leu Glu Ser Pro
            420                 425                 430
Arg Gly Val Trp Met Gln Ala Glu Ile Thr Phe Lys Lys Pro Met Pro
        435                 440                 445
Thr Lys Val Val Phe Met Ser Leu Cys Lys Ser Phe Trp Asp Cys Gly
    450                 455                 460

Leu Val Ala Leu Asp Asp Ile Thr Ile Gln Leu Gly Ser Cys Ser Ser
465                 470                 475                 480
Ser Glu Lys Leu Pro Pro His Leu Glu Ser Val Leu Ser Ser Lys Met
                485                 490                 495
Asn Val His Leu Leu Arg Arg Lys Glu Thr Gly Ala Ala Gly Thr Gly
            500                 505                 510
Gly Gly Glu Lys Leu Pro Leu Pro Thr Gln Asp Gln Arg Glu Ile Thr
        515                 520                 525
Leu Leu Gly
    530

<210> 35
<211> 146
<212> PRT
<213> Homo sapiens

<400> 35
Glu Phe His Ile Leu Leu Pro Thr Met Asp Cys Gln Glu Asn Glu Tyr
 1               5                  10                  15
Trp Asp Gln Trp Gly Arg Cys Val Thr Cys Gln Arg Cys Gly Pro Gly
            20                  25                  30
Gln Glu Leu Ser Lys Asp Cys Gly Tyr Gly Glu Gly Gly Asp Ala Tyr
        35                  40                  45
Cys Thr Ala Cys Pro Pro Arg Arg Tyr Lys Ser Ser Trp Gly His His
    50                  55                  60
Lys Cys Gln Ser Cys Ile Thr Cys Ala Val Ile Asn Arg Val Gln Lys
65                  70                  75                  80
Val Asn Cys Thr Ala Thr Ser Asn Ala Val Cys Gly Asp Cys Leu Pro
                85                  90                  95
Arg Phe Tyr Arg Lys Thr Arg Ile Gly Gly Leu Gln Asp Gln Glu Cys
            100                 105                 110

Ile Pro Cys Thr Lys Gln Thr Pro Thr Ser Glu Val Gln Cys Ala Phe
        115                 120                 125
Gln Leu Ser Leu Val Glu Ala Asp Ala Pro Thr Val Pro Pro Gln Glu
    130                 135                 140
Ala Thr
145

<210> 36
<211> 24
<212> PRT
<213> Homo sapiens

<400> 36
Leu Val Ala Leu Val Ser Ser Leu Leu Val Val Phe Thr Leu Ala Phe
 1               5                  10                  15
Leu Gly Leu Phe Phe Leu Tyr Cys
            20

<210> 37
<211> 36
<212> PRT
<213> Homo sapiens

<400> 37
Lys Gln Phe Phe Asn Arg His Cys Gln Arg Gly Lys Gly Gly Cys Phe
 1               5                  10                  15
Met Phe His Met Asn Gln Glu Gln Gly Ser Tyr Trp Gln Lys Arg Gly
            20                  25                  30
Met Phe Trp Gly
        35


<210> 38
<211> 618
<212> DNA
<213> Homo sapiens

<400> 38
gaatttcata tactccttcc caccatggat tgccaagaaa atgagtactg ggaccaatgg 60
ggacggtgtg tcacctgcca acggtgtggt cctggacagg agctatccaa ggattgtggt 120
tatggagagg gtggagatgc ctactgcaca gcctgccctc ctcgcaggta caaaagcagc 180
tggggccacc acaaatgtca gagttgcatc acctgtgctg tcatcaatcg tgttcagaag 240
gtcaactgca cagctacctc taatgctgtc tgtggggact gtttgcccag gttctaccga 300
aagacacgca ttggaggcct gcaggaccaa gagtgcatcc cgtgcacgaa gcagaccccc 360
acctctgagg ttcaatgtgc cttccagttg agcttagtgg aggcagatgc acccacagtg 420
ccccctcagg aggccacact tgttgcactg gtgagcagcc tgctagtggt gtttaccctg 480
gccttcctgg ggctcttctt cctctactgc aagcagttct tcaacagaca ttgccagcgt 540
ggtaagggtg gctgtttcat gtttcacatg aatcaggaac aaggctctta ttggcagaag 600
agagggatgt tctggggt 618

<210> 39
<211> 591
<212> DNA
<213> Homo sapiens

<400> 39
ggatttgtac cggagtccca tttgggagca agagccatct actcgtccgt taccggcctt 60
cccaccatgg attgccaaga aaatgagtac tgggaccaat ggggacggtg tgtcacctgc 120
caacggtgtg gtcctggaca ggagctatcc aaggattgtg gttatggaga gggtggagat 180
gcctactgca cagcctgccc tcctcgcagg tacaaaagca gctggggcca ccacaaatgt 240
cagagttgca tcacctgtgc tgtcatcaat cgtgttcaga aggtcaactg cacagctacc 300
tctaatgctg tctgtgggga ctgtttgccc aggttctacc gaaagacacg cattggaggc 360
ctgcaggacc aagagtgcat cccgtgcacg aagcagaccc ccacctctga ggttcaatgt 420
gccttccagt tgagcttagt ggaggcagat gcacccacag tgccccctca ggaggccaca 480
cttgttgcac tgttgcagga ggtttgctgc agtttgaggc tgataaaaca gcaaaggagg 540
aatctctctt ccccgtgcca cccagcaagg agaccagtgc tgagtcccaa g 591

<210> 40
<211> 62
<212> PRT
<213> Homo sapiens

<400> 40
Leu Val Trp Pro Pro Cys Val Glu Val Lys Arg Cys Gly Gly Cys Cys
1               5                   10                  15
Asn Asp Glu Ser Val Glu Cys Val Pro Thr Glu Val Phe Asn Arg Thr
            20                  25                  30
Val Gln Val Met Lys Ile Glu Ile Val Arg Lys Lys Pro Lys Leu Lys
        35                  40                  45
Glu Val Ser Val Arg Leu Glu Gln His Leu Lys Cys Glu Cys
    50                  55                  60

<210> 41
<211> 118
<212> PRT
<213> Homo sapiens

<400> 41
Cys Gly Gly Asn Val Phe Thr Thr Ser Ser Gly Thr Ser Ile Thr Ser
1               5                   10                  15
Pro Asn Tyr Pro Asn Asp Tyr Pro Pro Asn Lys Glu Cys Val Trp Arg
            20                  25                  30
Ile Glu Ala Pro Pro Gly His Arg Val Val Val Glu Leu Thr Phe Gln
        35                  40                  45
Asp Ile Phe Asp Leu Glu Asp His Asp Gly Ala Pro Cys Arg Tyr Asp
    50                  55                  60
Tyr Leu Glu Ile Arg Asp Gly Asp Ser Asp Lys Pro Leu Leu Gly Arg
65                  70                  75                  80
Tyr Cys Gly Glu Arg Ser Glu Pro Pro Glu Asp Ile Val Ser Thr Ser
                85                  90                  95
Asn Arg Met Leu Leu Glu Phe Val Ser Asp Ala Ser Val Gln Lys Arg
            100                 105                 110
Gly Phe Lys Ala Arg Tyr
        115

<210> 42
<211> 42
<212> PRT
<213> Homo sapiens

<400> 42
Cys Glu Glu Gly Val Thr Tyr Thr Asp Glu Asn His Leu Glu Gln Cys
1               5                   10                  15
Leu Ser Cys Ser Arg Cys Glu Pro Glu Met Gly Gln Val Leu Val Ser
            20                  25                  30
Pro Cys Thr Ala Thr Gln Asn Thr Val Cys
        35                  40

<210> 43
<211> 200
<212> PRT
<213> Homo sapiens

<400> 43
   Asp Ile Val Phe Leu Leu Asp Gly Ser Gly Ser Ile Gly Ser Gln Asn
   1               5                   10                  15
   Phe Glu Arg Val Lys Asp Phe Val Glu Arg Val Val Glu Arg Leu Asp
               20                  25                  30
   Val Gly Pro Arg Asp Lys Lys Glu Glu Asp Ala Val Arg Val Gly Leu
           35                  40                  45
   Val Gln Tyr Ser Asp Asn Val Arg Thr Glu Ile Lys Phe Lys Leu Asn
       50                  55                  60
   Asp Tyr Gln Asn Lys Asp Glu Val Leu Gln Ala Leu Gln Lys Ile Arg
   65                  70                  75                  80
   Tyr Glu Asp Tyr Tyr Gly Gly Gly Gly Thr Asn Thr Gly Ala Ala Leu
                   85                  90                  95
   Gln Tyr Val Val Arg Asn Leu Phe Thr Glu Ala Ser Gly Ser Arg Ile
               100                 105                 110
   Glu Pro Val Ala Glu Glu Gly Ala Pro Lys Val Leu Val Val Leu Thr
           115                 120                 125
   Asp Gly Arg Ser Gln Asp Asp Pro Ser Pro Thr Ile Asp Ile Arg Asp
       130                 135                 140
   Val Leu Asn Glu Leu Lys Lys Glu Ala Gly Val Glu Val Phe Ala Ile
   145                 150                 155                 160
   Gly Val Gly Asn Ala Asp Asn Asn Asn Leu Glu Glu Leu Arg Glu Ile
                   165                 170                 175
   Ala Ser Lys Pro Asp Asp His Val Phe Lys Val Ser Asp Phe Glu Ala
               180                 185                 190
   Leu Asp Thr Leu Gln Glu Leu Leu
           195                 200

<210> 44
<211> 43
<212> PRT
<213> Homo sapiens

<400> 44
Cys Asn Pro Asn Thr Gly Pro Cys Leu Asn Gly Gly Thr Cys Val Asn
1               5                   10                  15
Thr Pro Gly Gly Ser Val Phe Gly Gly Tyr Thr Cys Glu Cys Pro Glu
            20                  25                  30
Gly Tyr Ala Leu Ser Tyr Thr Gly Lys Arg Cys
        35                  40

<210> 45
<211> 182
<212> PRT

<213> Homo sapiens

<400> 45
Asp Gly Cys Asp Phe Glu Asp Gly Asn Gln Lys Thr Val Cys Gly Tyr
1               5                   10                  15
Ile Gln Asp Leu Ser Asp Asp Ala Glu Trp Glu Arg Leu Asn Ser Ser
            20                  25                  30
Thr Pro Pro Pro Ser Thr Gly Pro Thr Gln Asp His Thr Leu Val Gly
        35                  40                  45
Gln Cys Lys Asp Ser Gly Phe Phe Met Leu Val Asn Thr Ser Glu Gly
    50                  55                  60
Ala Glu Gly Glu Arg Ala Arg Leu Leu Ser Pro Val Leu Lys Pro Lys
65                  70                  75                  80
Arg Asp Gln His Cys Leu Asp Phe Trp Tyr Tyr Met Ser Gly Lys Ser
                85                  90                  95
Asn Val Gly Pro Leu Ser Ile Asn Val Arg Val Asp Val Asn Glu Gly
            100                 105                 110

Lys Val Pro Leu Leu Asn Thr Ile Trp Thr Val Ser Gly Asn Pro Gly
        115                 120                 125
Arg Asn Trp Lys Arg Ala Glu Val Thr Leu Asn Thr Phe Glu Thr Lys
    130                 135                 140
Glu Val Gln Val Ile Phe Glu Gly Thr Lys Gly Asp Pro Gly Gly Ser
145                 150                 155                 160
Ser Gly Gly Ile Ala Ile Asp Asp Ile Lys Leu Thr Glu Thr Pro Ser
                165                 170                 175
Pro Ser Gln Cys Pro Ala
            180

<210> 46
<211> 54
<212> PRT
<213> Homo sapiens

<400> 46
Ser Pro Trp Ser Glu Trp Ser Pro Cys Ser Val Thr Cys Gly Lys Gly
1               5                   10                  15
Ile Arg Thr Arg Gln Arg Thr Cys Asn Ser Pro Ala Pro Gln Lys Lys
            20                  25                  30
Gly Gly Lys Pro Cys Thr Gly Asp Ala Gln Glu Glu Thr Glu Ala Cys
        35                  40                  45
Asp Met Met Asp Lys Cys
    50

<210> 47
<211> 17
<212> DNA
<213> Artificial Sequence

<220>
<223> Linker

<400> 47
gtcgacccac gcgtccg                                                17

<210> 48
<211> 10
<212> DNA
<213> Artificial Sequence

<220>
<223> Linker

<400> 48
gggcggccgc                                                        10

<210> 49
<211> 455
<212> DNA
<213> Rat

<220>
<221> misc_feature
<222> (1)...(455)
<223> n = A,T,C or G

<400> 49
tncgagaaca cgcttgttaa cgtgtaagtc cacgtctagc ctcctttgcc gccatctttg 60
ttgagggcgg ataagttgta agccaggacg gttttgcgta ctgttccact ttctcctcca 120
cgcttcacgt tcagaatcta ttctggaaaa gaaaaagagt cgcagatgaa gagtaaactt 180
tactgtaaag tctacagtgt cgcgctgctg acgtcacgac cagcgtctct gctgtcttag 240
tcgtagcttg agaggtggct acctccaaag tgtgtcttgt gtgttgctgt gtaaatgctg 300
aggatgcggt cttggtgtgg tgctgagcct tgctgtggcg gcggcggctg tcttggccgt 360
gtggttgatg gattggtggg ggttcccacc ccagtattcg cctcttcgta cccgaggagc 420
tagcccgcta tcgcggcggc caagggaccc gggtc 455

<210> 50
<211> 274
<212> PRT
<213> Homo sapiens

<400> 50
Gln Gly Gly Arg Arg Glu Asp Gly Gly Pro Ala Cys Tyr Gly Gly Phe
1               5                   10                  15
Asp Leu Tyr Phe Ile Leu Asp Lys Ser Gly Ser Val Leu His His Trp
            20                  25                  30
Asn Glu Ile Tyr Tyr Phe Val Glu Gln Leu Ala His Lys Phe Ile Ser
        35                  40                  45
Pro Gln Leu Arg Met Ser Phe Ile Val Phe Ser Thr Arg Gly Thr Thr
    50                  55                  60
Leu Met Lys Leu Thr Glu Asp Arg Glu Gln Ile Arg Gln Gly Leu Glu
65                  70                  75                  80
Glu Leu Gln Lys Val Leu Pro Gly Gly Asp Thr Tyr Met His Glu Gly
                85                  90                  95
Phe Glu Arg Ala Ser Glu Gln Ile Tyr Tyr Glu Asn Arg Gln Gly Tyr
            100                 105                 110
Arg Thr Ala Ser Val Ile Ile Ala Leu Thr Asp Gly Glu Leu His Glu
        115                 120                 125
Asp Leu Phe Phe Tyr Ser Glu Arg Glu Ala Asn Arg Ser Arg Asp Leu
    130                 135                 140
Gly Ala Ile Val Tyr Cys Val Gly Val Lys Asp Phe Asn Glu Thr Gln
145                 150                 155                 160
Leu Ala Arg Ile Ala Asp Ser Lys Asp His Val Phe Pro Val Asn Asp
                165                 170                 175
Gly Phe Gln Ala Leu Gln Gly Ile Ile His Ser Ile Leu Lys Lys Ser
            180                 185                 190
Cys Ile Glu Ile Leu Ala Ala Glu Pro Ser Thr Ile Cys Ala Gly Glu
        195                 200                 205
Ser Phe Gln Val Val Val Arg Gly Asn Gly Phe Arg His Ala Arg Asn
    210                 215                 220
Val Asp Arg Val Leu Cys Ser Phe Lys Ile Asn Asp Ser Val Thr Leu
225                 230                 235                 240
Asn Glu Lys Pro Phe Ser Val Glu Asp Thr Tyr Leu Leu Cys Pro Ala
                245                 250                 255
Pro Ile Leu Lys Glu Val Gly Met Lys Ala Ala Leu Gln Val Ser Met
            260                 265                 270
Asn Asp

<210> 51
<211> 306
<212> PRT
<213> Homo sapiens

<400> 51
Gln Gly Gly Arg Arg Glu Asp Gly Gly Pro Ala Cys Tyr Gly Gly Phe
1               5                   10                  15
Asp Leu Tyr Phe Ile Leu Asp Lys Ser Gly Ser Val Leu His His Trp
            20                  25                  30
Asn Glu Ile Tyr Tyr Phe Val Glu Gln Leu Ala His Lys Phe Ile Ser
        35                  40                  45

Pro Gln Leu Arg Met Ser Phe Ile Val Phe Ser Thr Arg Gly Thr Thr
    50                  55                  60
Leu Met Lys Leu Thr Glu Asp Arg Glu Gln Ile Arg Gln Gly Leu Glu
65                  70                  75                  80
Glu Leu Gln Lys Val Leu Pro Gly Gly Asp Thr Tyr Met His Glu Gly
                85                  90                  95
Phe Glu Arg Ala Ser Glu Gln Ile Tyr Tyr Glu Asn Arg Gln Gly Tyr
            100                 105                 110
Arg Thr Ala Ser Val Ile Ile Ala Leu Thr Asp Gly Glu Leu His Glu
        115                 120                 125
Asp Leu Phe Phe Tyr Ser Glu Arg Glu Ala Asn Arg Ser Arg Asp Leu
    130                 135                 140
Gly Ala Ile Val Tyr Cys Val Gly Val Lys Asp Phe Asn Glu Thr Gln
145                 150                 155                 160
Leu Ala Arg Ile Ala Asp Ser Lys Asp His Val Phe Pro Val Asn Asp
                165                 170                 175
Gly Phe Gln Ala Leu Gln Gly Ile Ile His Ser Ile Leu Lys Lys Ser
            180                 185                 190
Cys Ile Glu Ile Leu Ala Ala Glu Pro Ser Thr Ile Cys Ala Gly Glu
        195                 200                 205
Ser Phe Gln Val Val Val Arg Gly Asn Gly Phe Arg His Ala Arg Asn
    210                 215                 220
Val Asp Arg Val Leu Cys Ser Phe Lys Ile Asn Asp Ser Val Thr Leu
225                 230                 235                 240
Asn Glu Lys Pro Phe Ser Val Glu Asp Thr Tyr Leu Leu Cys Pro Ala
                245                 250                 255
Pro Ile Leu Lys Glu Val Gly Met Lys Ala Ala Leu Gln Val Ser Met
            260                 265                 270
Asn Asp Gly Leu Ser Phe Ile Ser Ser Ser Val Ile Ile Thr Thr Thr
        275                 280                 285
His Cys Ser Leu His Lys Ile Ala Ser Gly Pro Thr Thr Ala Ala Cys
    290                 295                 300
Met Glu
305

<210> 52
<211> 846
<212> PRT
<213> Homo sapiens

<400> 52
Glu Glu Asp Arg Asp Gly Leu Trp Asp Ala Trp Gly Pro Trp Ser Glu
1               5                   10                  15
Cys Ser Arg Thr Cys Gly Gly Gly Ala Ser Tyr Ser Leu Arg Arg Cys
            20                  25                  30
Leu Ser Ser Lys Ser Cys Glu Gly Arg Asn Ile Arg Tyr Arg Thr Cys
        35                  40                  45
Ser Asn Val Asp Cys Pro Pro Glu Ala Gly Asp Phe Arg Ala Gln Gln
    50                  55                  60
Cys Ser Ala His Asn Asp Val Lys His His Gly Gln Phe Tyr Glu Trp
65                  70                  75                  80
Leu Pro Val Ser Asn Asp Pro Asp Asn Pro Cys Ser Leu Lys Cys Gln
                85                  90                  95
Ala Lys Gly Thr Thr Leu Val Val Glu Leu Ala Pro Lys Val Leu Asp
            100                 105                 110
Gly Thr Arg Cys Tyr Thr Glu Ser Leu Asp Met Cys Ile Ser Gly Leu
        115                 120                 125
Cys Gln Ile Val Gly Cys Asp His Gln Leu Gly Ser Thr Val Lys Glu
    130                 135                 140
Asp Asn Cys Gly Val Cys Asn Gly Asp Gly Ser Thr Cys Arg Leu Val

145                 150                 155                 160

Arg Gly Gln Tyr Lys Ser Gln Leu Ser Ala Thr Lys Ser Asp Asp Thr
                165                 170                 175
Val Val Ala Ile Pro Tyr Gly Ser Arg His Ile Arg Leu Val Leu Lys
            180                 185                 190
Gly Pro Asp His Leu Tyr Leu Glu Thr Lys Thr Leu Gln Gly Thr Lys
        195                 200                 205
Gly Glu Asn Ser Leu Ser Ser Thr Gly Thr Phe Leu Val Asp Asn Ser
    210                 215                 220
Ser Val Asp Phe Gln Lys Phe Pro Asp Lys Glu Ile Leu Arg Met Ala
225                 230                 235                 240
Gly Pro Leu Thr Ala Asp Phe Ile Val Lys Ile Arg Asn Ser Gly Ser
                245                 250                 255
Ala Asp Ser Thr Val Gln Phe Ile Phe Tyr Gln Pro Ile Ile His Arg
            260                 265                 270
Trp Arg Glu Thr Asp Phe Phe Pro Cys Ser Ala Thr Cys Gly Gly Gly
        275                 280                 285
Tyr Gln Leu Thr Ser Ala Glu Cys Tyr Asp Leu Arg Ser Asn Arg Val
    290                 295                 300
Val Ala Asp Gln Tyr Cys His Tyr Tyr Pro Glu Asn Ile Lys Pro Lys
305                 310                 315                 320
Pro Lys Leu Gln Glu Cys Asn Leu Asp Pro Cys Pro Ala Gln Arg Val
                325                 330                 335
Gly Ile Ile Val Pro Asn Ser Asp Gly Tyr Lys Gln Ile Met Pro Tyr
            340                 345                 350
Asp Leu Tyr His Pro Leu Pro Arg Trp Glu Ala Thr Pro Trp Thr Ala
        355                 360                 365
Cys Ser Ser Ser Cys Gly Gly Gly Ile Gln Ser Arg Ala Val Ser Cys
    370                 375                 380
Val Glu Glu Asp Ile Gln Gly His Val Thr Ser Val Glu Glu Trp Lys
385                 390                 395                 400
Cys Met Tyr Thr Pro Lys Met Pro Ile Ala Gln Pro Cys Asn Ile Phe
                405                 410                 415
Asp Cys Pro Lys Trp Leu Ala Gln Glu Trp Ser Pro Cys Thr Val Thr
            420                 425                 430
Cys Gly Gln Gly Leu Arg Tyr Arg Val Val Leu Cys Ile Asp His Arg
        435                 440                 445
Gly Met His Thr Gly Gly Cys Ser Pro Lys Thr Lys Pro His Ile Lys
    450                 455                 460
Glu Glu Cys Ile Val Pro Thr Pro Cys Tyr Lys Pro Lys Glu Lys Leu
465                 470                 475                 480
Pro Val Glu Ala Lys Leu Pro Trp Phe Lys Gln Ala Gln Glu Leu Glu
                485                 490                 495
Glu Gly Ala Ala Val Ser Glu Glu Pro Ser Phe Ile Pro Glu Ala Trp
            500                 505                 510
Ser Ala Cys Thr Val Thr Cys Gly Val Gly Thr Gln Val Arg Ile Val
        515                 520                 525
Arg Cys Gln Val Leu Leu Ser Phe Ser Gln Ser Val Ala Asp Leu Pro
    530                 535                 540
Ile Asp Glu Cys Glu Gly Pro Lys Pro Ala Ser Gln Arg Ala Cys Tyr
545                 550                 555                 560
Ala Gly Pro Cys Ser Gly Glu Ile Pro Glu Phe Asn Pro Asp Glu Thr
                565                 570                 575
Asp Gly Leu Phe Gly Gly Leu Gln Asp Phe Asp Glu Leu Tyr Asp Trp
            580                 585                 590
Glu Tyr Glu Gly Phe Thr Lys Cys Ser Glu Ser Cys Gly Gly Gly Val
        595                 600                 605
Gln Glu Ala Val Val Ser Cys Leu Asn Lys Gln Thr Arg Glu Pro Ala
    610                 615                 620
Glu Glu Asn Leu Cys Val Thr Ser Arg Arg Pro Pro Gln Leu Leu Lys
625                 630                 635                 640
Ser Cys Asn Leu Asp Pro Cys Pro Ala Arg Trp Glu Ile Gly Lys Trp
                645                 650                 655
Ser Pro Cys Ser Leu Thr Cys Gly Val Gly Leu Gln Thr Arg Asp Val
            660                 665                 670
Phe Cys Ser His Leu Leu Ser Arg Glu Met Asn Glu Thr Val Ile Leu
        675                 680                 685

Ala Asp Glu Leu Cys Arg Gln Pro Lys Pro Ser Thr Val Gln Ala Cys
    690                 695                 700
Asn Arg Phe Asn Cys Pro Pro Ala Trp Tyr Pro Ala Gln Trp Gln Pro
705                 710                 715                 720
Cys Ser Arg Thr Cys Gly Gly Gly Val Gln Lys Arg Glu Val Leu Cys
                725                 730                 735
Lys Gln Arg Met Ala Asp Gly Ser Phe Leu Glu Leu Pro Glu Thr Phe
            740                 745                 750
Cys Ser Ala Ser Lys Pro Ala Cys Gln Gln Ala Cys Lys Lys Asp Asp
        755                 760                 765
Cys Pro Ser Glu Trp Leu Leu Ser Asp Trp Thr Glu Cys Ser Thr Ser
    770                 775                 780
Cys Gly Glu Gly Thr Gln Thr Arg Ser Ala Ile Cys Arg Lys Met Leu
785                 790                 795                 800
Lys Thr Gly Ile Ser Thr Val Val Asn Ser Thr Leu Cys Pro Pro Leu
                805                 810                 815
Pro Phe Ser Ser Ser Ile Arg Pro Cys Met Leu Ala Thr Cys Ala Arg
            820                 825                 830
Pro Gly Arg Pro Ser Thr Lys His Ser Pro His Ile Ala Ala
        835                 840                 845

<210> 53
<211> 764
<212> DNA
<213> Mouse

<220>
<221> CDS
<222> (211)...(750)

<221> misc_feature
<222> (1)...(764)
<223> n = A,T,C or G

<400> 53
ccgcgtccgg ctccctgcga gtcgccacga ctcatcgccg ctcccccgcg tccccacccc      60
ttctttcctc cctcgcctac ccccaccccc cgcacttcgg cacagctcag gatttgttta     120
aaccttggga aactggttca ggtccaggtt ttgctttgat ccttttcaaa aactggagac     180
acagaagagg gctctaggaa aaacttttgg atg gga tta tgt gga aac tac cct      234
                                 Met Gly Leu Cys Gly Asn Tyr Pro
                                 1               5

gcg att ctc tgc tgc caa aga acg ggg act cgg gct gag tcc aac ctg      282
Ala Ile Leu Cys Cys Gln Arg Thr Gly Thr Arg Ala Glu Ser Asn Leu
    10                  15                  20

agc agc aag ttg cag ctc tcc agc gac aag gaa cag aac gga gtg caa      330
Ser Ser Lys Leu Gln Leu Ser Ser Asp Lys Glu Gln Asn Gly Val Gln
25                  30                  35                  40

gat ccc cgg cat gag aga gtt gtc act ata tct ggt aat ggg agc atc      378
Asp Pro Arg His Glu Arg Val Val Thr Ile Ser Gly Asn Gly Ser Ile
                45                  50                  55

cac agc ccg aag ttt cct cat aca tac cca aga aat atg gtg ctg gtg      426
His Ser Pro Lys Phe Pro His Thr Tyr Pro Arg Asn Met Val Leu Val
            60                  65                  70

tgg aga tta gtt gca gta gat gaa aat gtg cgg atc cag ctg aca ttt      474
Trp Arg Leu Val Ala Val Asp Glu Asn Val Arg Ile Gln Leu Thr Phe
        75                  80                  85

gat gag aga ttt ggg ctg gaa gat cca gaa gac gat ata tgc aag tat      522
Asp Glu Arg Phe Gly Leu Glu Asp Pro Glu Asp Asp Ile Cys Lys Tyr
    90                  95                  100

gat ttt gta gga gtt gag gag ccc agt gat gga agc gtt tta gga cgc      570

Asp Phe Val Gly Val Glu Glu Pro Ser Asp Gly Ser Val Leu Gly Arg
105                 110                 115                 120

tgg tgt ggt tct ggg act gtg cca nga aag cag act tct aaa gga aat       618
Trp Cys Gly Ser Gly Thr Val Pro Xaa Lys Gln Thr Ser Lys Gly Asn
                125                 130                 135

cat atc agg ata aga ttt gca tct gat gag tat ttt cca tct gaa ccc       666
His Ile Arg Ile Arg Phe Ala Ser Asp Glu Tyr Phe Pro Ser Glu Pro
            140                 145                 150

gga ttc tgc atc cac tac agt att atc atg cca caa gtc aca gna acc       714
Gly Phe Cys Ile His Tyr Ser Ile Ile Met Pro Gln Val Thr Xaa Thr
        155                 160                 165

acg agt cct tng gtg ttg ccc cct tca tct ttt gtn nnttgggacc            760
Thr Ser Pro Xaa Val Leu Pro Pro Ser Ser Phe Val
    170                 175                 180

tgct                                                                  764

<210> 54
<211> 180
<212> PRT
<213> Mouse

<220>
<221> VARIANT
<222> (1)...(180)
<223> Xaa = Any Amino Acid

<400> 54
Met Gly Leu Cys Gly Asn Tyr Pro Ala Ile Leu Cys Cys Gln Arg Thr
 1               5                  10                  15
Gly Thr Arg Ala Glu Ser Asn Leu Ser Ser Lys Leu Gln Leu Ser Ser
            20                  25                  30
Asp Lys Glu Gln Asn Gly Val Gln Asp Pro Arg His Glu Arg Val Val
        35                  40                  45
Thr Ile Ser Gly Asn Gly Ser Ile His Ser Pro Lys Phe Pro His Thr
    50                  55                  60
Tyr Pro Arg Asn Met Val Leu Val Trp Arg Leu Val Ala Val Asp Glu
65                  70                  75                  80
Asn Val Arg Ile Gln Leu Thr Phe Asp Glu Arg Phe Gly Leu Glu Asp
                85                  90                  95
Pro Glu Asp Asp Ile Cys Lys Tyr Asp Phe Val Gly Val Glu Glu Pro
            100                 105                 110
Ser Asp Gly Ser Val Leu Gly Arg Trp Cys Gly Ser Gly Thr Val Pro
        115                 120                 125
Xaa Lys Gln Thr Ser Lys Gly Asn His Ile Arg Ile Arg Phe Ala Ser
    130                 135                 140
Asp Glu Tyr Phe Pro Ser Glu Pro Gly Phe Cys Ile His Tyr Ser Ile
145                 150                 155                 160

Ile Met Pro Gln Val Thr Xaa Thr Thr Ser Pro Xaa Val Leu Pro Pro
                165                 170                 175
Ser Ser Phe Val
            180

<210> 55
<211> 540
<212> DNA
<213> Mouse

<220>
<221> misc_feature
<222> (1)...(540)
<223> n = A,T,C or G

<400> 55
   atgggattat gtggaaacta ccctgcgatt ctctgctgcc aaagaacggg gactcgggct      60
   gagtccaacc tgagcagcaa gttgcagctc tccagcgaca aggaacagaa cggagtgcaa     120
   gatccccggc atgagagagt tgtcactata tctggtaatg ggagcatcca cagcccgaag     180
   tttcctcata catacccaag aaatatggtg ctggtgtgga gattagttgc agtagatgaa     240
   aatgtgcgga tccagctgac atttgatgag agatttgggc tggaagatcc agaagacgat     300
   atatgcaagt atgattttgt aggagttgag gagcccagtg atggaagcgt tttaggacgc     360
   tggtgtggtt ctgggactgt gccangaaag cagacttcta aaggaaatca tatcaggata     420
   agatttgcat ctgatgagta ttttccatct gaacccggat tctgcatcca ctacagtatt     480
   atcatgccac aagtcacagn aaccacgagt ccttnggtgt tgcccccttc atcttttgtn      540

<210> 56
<211> 4417
<212> DNA
<213> Mouse

<220>
<221> CDS
<222> (3)...(1145)

<400> 56
cc gcg tcc gct cag ttg gct cgg att gca gac agt aag gac cac gtg      47
   Ala Ser Ala Gln Leu Ala Arg Ile Ala Asp Ser Lys Asp His Val
    1               5                  10                  15

ttt cct gtg aac gac ggc ttc cag gct ctc caa ggc att atc cac tca      95
Phe Pro Val Asn Asp Gly Phe Gln Ala Leu Gln Gly Ile Ile His Ser
                20                  25                  30

att tta aag aaa tcc tgc atc gaa att ctg gcg gct gaa cca tcc acc     143
Ile Leu Lys Lys Ser Cys Ile Glu Ile Leu Ala Ala Glu Pro Ser Thr
            35                  40                  45

atc tgc gcg gga gag tcc ttt caa gtg gtc gta aga gga aat ggc ttc     191
Ile Cys Ala Gly Glu Ser Phe Gln Val Val Val Arg Gly Asn Gly Phe
        50                  55                  60

cga cat gcc cgc aat gtg gac agg gtc ctc tgc agc ttc aaa atc aat     239
Arg His Ala Arg Asn Val Asp Arg Val Leu Cys Ser Phe Lys Ile Asn
    65                  70                  75

gac tca gtc acg ctc aat gag aag ccc ttt gct gtg gaa gac act tat     287
Asp Ser Val Thr Leu Asn Glu Lys Pro Phe Ala Val Glu Asp Thr Tyr
80                  85                  90                  95

ttg ctg tgc cca gca cca atc ttg aaa gaa gtt ggc atg aaa gct gca     335
Leu Leu Cys Pro Ala Pro Ile Leu Lys Glu Val Gly Met Lys Ala Ala
                100                 105                 110

ctg cag gtc agc atg aac gac ggc ctg tcc ttc atc tcc agt tct gtc     383
Leu Gln Val Ser Met Asn Asp Gly Leu Ser Phe Ile Ser Ser Ser Val
            115                 120                 125

atc atc acc acc aca cac tgt tca gac ggc tcc atc ctg gcg att gct     431
Ile Ile Thr Thr Thr His Cys Ser Asp Gly Ser Ile Leu Ala Ile Ala
        130                 135                 140

ctg ctg gtc ctc ttc ctg ctg ctg gcc ctg gcg ctg ctc tgg tgg ttc     479
Leu Leu Val Leu Phe Leu Leu Leu Ala Leu Ala Leu Leu Trp Trp Phe
    145                 150                 155

tgg ccc ctc tgc tgc aca gtg atc atc aag gag gtc cct cca ccc cct     527
Trp Pro Leu Cys Cys Thr Val Ile Ile Lys Glu Val Pro Pro Pro Pro
160                 165                 170                 175

gtt gag gag agt gag gaa gaa gac gat gat ggt ttg cca aag aag aaa     575

Val Glu Glu Ser Glu Glu Glu Asp Asp Asp Gly Leu Pro Lys Lys Lys
                180                 185                 190

tgg ccc aca gta gat gcc tct tat tat ggt gga cgc ggt gtg gga ggc      623
Trp Pro Thr Val Asp Ala Ser Tyr Tyr Gly Gly Arg Gly Val Gly Gly
            195                 200                 205

att aaa aga atg gag gtc cgc tgg gga gaa aag ggc tcc aca gaa gaa      671
Ile Lys Arg Met Glu Val Arg Trp Gly Glu Lys Gly Ser Thr Glu Glu
        210                 215                 220

ggg gcg aag tta gaa aag gca aag aat gca cga gtc aag atg cca gag      719
Gly Ala Lys Leu Glu Lys Ala Lys Asn Ala Arg Val Lys Met Pro Glu
    225                 230                 235

caa gaa tat gag ttc cca gaa ccc cga aac ctc aac aac aac atg cgc      767
Gln Glu Tyr Glu Phe Pro Glu Pro Arg Asn Leu Asn Asn Asn Met Arg
240                 245                 250                 255

cgg cct tcc tcg cct cgg aag tgg tac tcg ccc atc aag gga aaa ctc      815
Arg Pro Ser Ser Pro Arg Lys Trp Tyr Ser Pro Ile Lys Gly Lys Leu
                260                 265                 270

gat gcc ttg tgg gtt ctg ctg aga aaa gga tat gac cga gtg tct gtg      863
Asp Ala Leu Trp Val Leu Leu Arg Lys Gly Tyr Asp Arg Val Ser Val
            275                 280                 285

atg agg cca cag cca gga gac acg gga cgc tgt atc aac ttc acc aga      911
Met Arg Pro Gln Pro Gly Asp Thr Gly Arg Cys Ile Asn Phe Thr Arg
        290                 295                 300

gtg aag aac agt cag cca gcc aag tat ccc ctg aac aac acc tac cac      959
Val Lys Asn Ser Gln Pro Ala Lys Tyr Pro Leu Asn Asn Thr Tyr His
    305                 310                 315

ccc agc tcc cca cct ccc gct cct atc tac aca ccc cca ccc cct gct      1007
Pro Ser Ser Pro Pro Pro Ala Pro Ile Tyr Thr Pro Pro Pro Pro Ala
320                 325                 330                 335

ccc cac tgc cct ccc cca gcc ccc agt gcc ccc act cct ccc att cct      1055
Pro His Cys Pro Pro Pro Ala Pro Ser Ala Pro Thr Pro Pro Ile Pro
                340                 345                 350

tcc cca cca tcc act ctc ccc cct cct cct cag gcc cca ccc cct aac      1103
Ser Pro Pro Ser Thr Leu Pro Pro Pro Pro Gln Ala Pro Pro Pro Asn
            355                 360                 365

agg gca cct ccc ccc tcc cga cct cct cca agg cct tct gtc              1145
Arg Ala Pro Pro Pro Ser Arg Pro Pro Pro Arg Pro Ser Val
        370                 375                 380

tagaacccaa agtccgagct ctgggctgcc tgagcaactc cagcaggagg cttctctgct    1205
gaaagaaaga tctgcccagc ctatgtggtg agtggcggct gatgtttgca cgatttaaaa    1265
gcaagtcgtg atgggcagaa caaaatgggc attttgaact gcctgaagac agacaatgag    1325
acaataacag tcacattata gcctgtgacc cctcacctct agaggaaggt tcccgagatg    1385
gccacattgc cacagtgctc tcagccagat tatgtcccat gaagaccagg aagaaagtga    1445
cttccaagaa tggaatgcag cattggataa gaagcacctg gctgagattc tgacctcact    1505
gatttgactc ttgattcttg gactgggagc caggccatct ccacccctgg taccacccag    1565
caactctgaa aatgtgcagt gtccctagta tgcatcgaat aggtatccaa ctgggatctg    1625
caggttgcct tataaagagc atatgctcta ttctctttcc cgaacttcct ggtttcccag    1685
tgatgaggga aggggaaagg tgttgccatg cttagaagtt agaggacgtc agtgctcagc    1745
actgatggag aagcgttgat gggagtgtcc agctcttaca tctagaaatg gctggcttca    1805
gcaggcacag ttcctaaacc aacaagcctt gtcattgtca aaggcaacct actaatgatt    1865
caccttaaac atcaaggttg actgtggcat aggtcagagc tgatcacaca gaaccttccc    1925

catgaaatcg caaggttcct catcttcaaa tacccaggac cccagagatt cctaaatcca 1985
gctaagagac agtagtcctg acttggcaag aaaaccattc ccagttgttt tactctgaaa 2045
caggccgttg tatgtatggt atatctctcc ttggcctttc aacctgctca caagtattac 2105
cagttatgaa gcaaggagaa atacatccag tgtgtaatag aaaagctctg cccgcaatcc 2165
ccatgtcact cctctacatt attctgaagc tgcttggtca gtgagccctt taacctcatg 2225
tagactctgg acactgtcac ccaatcatga aaacagaggt cattgtcaaa ggcagtgtat 2285
agcctgtaca aaaatgatgc ttccttcctc agtttccaca ggccccaaaa ttcctgtctt 2345
aggctcctaa acctctaaac tttttcctgg aacaaaagat ataaaacggg cataagtttt 2405
tatgttttgg gctgtgatct ccaaagatcc ttcaagaact caagttagcc tcattcttcc 2465
agcttgttta gaacagaggc atccaggtgt catgcactcc atagacacca atccttgttc 2525
ccaaggcaga cattattaat caatctcagc actagttctc aatttaatcc aattatattt 2585
ttccacagta cttcacatct cttatgacct gttggtcatc agttagaatt gagagagata 2645
aacactgttt gtaatcccta ccttagaaag aaaagcagag gagaatgggg gaaccaccag 2705
cataaaagtt attatctggg gaaaatcgac ctgaaagaac gcccaagtcc aagacctatg 2765
gtgctgacac caaagtaaca ctttcccaag tgtaccccag accccactct tctccctgtg 2825
gccaccactc cctgcttttc aggagttgtg aaaaagatct ccttcaccct tactgtgccc 2885
ccatattaga acaaggcttg tttagtgtag tccttgttaa acaggtgcca gaatgtctca 2945
gccacctgag atgacattgc tgggccccag aaaaccattc caaggagaat gggctcccca 3005
ggctcagagc atgcaactat gagcccatgg caactgtttt gactgctggc agtacaaaac 3065
gggccacccc acattacagc tgcaggattt gtgcagccat aagaaagtac gaaccaagat 3125
gctggcgttg ctgttcaaca agcatgggct tcggggaagg cagcagactc cgagagcagg 3185
ccttgtgcag tgtcccaagg ggctgtggtg aagtgtctga ggaaaaatga atgctgatac 3245
atggtgattc tgagaagaat ttgcaaggtt tgaccttaga atttatggaa tgtcttccct 3305
ggtcattcag aattatggct agaagtttct agaaaccgtc aaggttaata cctttcagag 3365
taggtgatta caggcaggaa gagctttgat gtggtttaca aagcccatca gttctgtgtc 3425
attccctgta agcaacagga gatggtggtt gtgattagca aactgcatgt gttatttgtt 3485
tgactccttg ttattgtcct tacggaggat tttttttata taagccaaat tttgttgtat 3545
atattcatat tccacgtgac agatggaagc acgtcctatc agtgtgaata aaaagaacag 3605
ttgtagtaaa ttattaaagc cagtgatttc atggcaggtt accctaccaa gctgtgcttg 3665
ttgatctccc atgaccatac tgcttttaca atgtacaaat agttcctagg tgacgagacc 3725
ctcctttaca taatgccgat gacagccttg ctgggaactg cggtccttct gctgtgacag 3785
ccagctcgaa aacaggtcct gcctggagct tgccacacac tttagggaga cataagagct 3845
gtctttcccc agcgtcaggg acaaagctac cataaagaag tggaaaagtc ttggctctcc 3905
agcctgggac agaggtctct ctggaacccc aaggaagagc agaaatgatc cttgcctgcc 3965
actgcacaca atgtgatggt ggaaaatcca tcaaggaata attgtgagat aatgaccgac 4025
agttcaggcg caaagggaat tcatgctgtg taaagtgggt ggaattcgtt tgcaagctat 4085
gcaaagcctg atcttactca ccaggaggat ggaaagggtt tttttagtta tctgagctca 4145
gctgagttat cacgcttgga gaaccgattt aaaggaatta gaatatgatt tctgaataca 4205
cataacatta aactcttctc tttttctatg gtaatttagt tatggacgtt cagcgtctct 4265
gagttattgt tataaaagac ttgtcatcac cgcactgtgc tgtaggagac tgggctgaac 4325
ctgtacaatg gtataccctg gaagttgctt ttttaaaaaa aaataataat aaacacctaa 4385
aatcagaaaa aaaaaaaaaa aagggcggcc gc 4417

<210> 57
<211> 381
<212> PRT
<213> Mouse

<400> 57
Ala Ser Ala Gln Leu Ala Arg Ile Ala Asp Ser Lys Asp His Val Phe
1               5                   10                  15
Pro Val Asn Asp Gly Phe Gln Ala Leu Gln Gly Ile Ile His Ser Ile
            20                  25                  30
Leu Lys Lys Ser Cys Ile Glu Ile Leu Ala Ala Glu Pro Ser Thr Ile
        35                  40                  45
Cys Ala Gly Glu Ser Phe Gln Val Val Val Arg Gly Asn Gly Phe Arg
    50                  55                  60
His Ala Arg Asn Val Asp Arg Val Leu Cys Ser Phe Lys Ile Asn Asp
65                  70                  75                  80
Ser Val Thr Leu Asn Glu Lys Pro Phe Ala Val Glu Asp Thr Tyr Leu
                85                  90                  95
Leu Cys Pro Ala Pro Ile Leu Lys Glu Val Gly Met Lys Ala Ala Leu
            100                 105                 110
Gln Val Ser Met Asn Asp Gly Leu Ser Phe Ile Ser Ser Ser Val Ile
        115                 120                 125
Ile Thr Thr Thr His Cys Ser Asp Gly Ser Ile Leu Ala Ile Ala Leu
    130                 135                 140
Leu Val Leu Phe Leu Leu Leu Ala Leu Ala Leu Leu Trp Trp Phe Trp

145                 150                 155                 160
Pro Leu Cys Cys Thr Val Ile Ile Lys Glu Val Pro Pro Pro Pro Val
                165                 170                 175
Glu Glu Ser Glu Glu Glu Asp Asp Asp Gly Leu Pro Lys Lys Lys Trp
            180                 185                 190
Pro Thr Val Asp Ala Ser Tyr Tyr Gly Gly Arg Gly Val Gly Gly Ile
        195                 200                 205
Lys Arg Met Glu Val Arg Trp Gly Glu Lys Gly Ser Thr Glu Glu Gly
    210                 215                 220
Ala Lys Leu Glu Lys Ala Lys Asn Ala Arg Val Lys Met Pro Glu Gln
225                 230                 235                 240
Glu Tyr Glu Phe Pro Glu Pro Arg Asn Leu Asn Asn Asn Met Arg Arg
                245                 250                 255
Pro Ser Ser Pro Arg Lys Trp Tyr Ser Pro Ile Lys Gly Lys Leu Asp
            260                 265                 270
Ala Leu Trp Val Leu Leu Arg Lys Gly Tyr Asp Arg Val Ser Val Met
        275                 280                 285
Arg Pro Gln Pro Gly Asp Thr Gly Arg Cys Ile Asn Phe Thr Arg Val
    290                 295                 300
Lys Asn Ser Gln Pro Ala Lys Tyr Pro Leu Asn Asn Thr Tyr His Pro
305                 310                 315                 320
Ser Ser Pro Pro Pro Ala Pro Ile Tyr Thr Pro Pro Pro Pro Ala Pro
                325                 330                 335
His Cys Pro Pro Pro Ala Pro Ser Ala Pro Thr Pro Pro Ile Pro Ser
            340                 345                 350
Pro Pro Ser Thr Leu Pro Pro Pro Pro Gln Ala Pro Pro Pro Asn Arg
        355                 360                 365
Ala Pro Pro Pro Ser Arg Pro Pro Pro Arg Pro Ser Val
    370                 375                 380

<210> 58
<211> 1143
<212> DNA
<213> Mouse

<400> 58
gcgtccgctc agttggctcg gattgcagac agtaaggacc acgtgtttcc tgtgaacgac      60
ggcttccagg ctctccaagg cattatccac tcaattttaa agaaatcctg catcgaaatt     120
ctggcggctg aaccatccac catctgcgcg ggagagtcct ttcaagtggt cgtaagagga     180
aatggcttcc gacatgcccg caatgtggac agggtcctct gcagcttcaa aatcaatgac     240
tcagtcacgc tcaatgagaa gccctttgct gtggaagaca cttatttgct gtgcccagca     300
ccaatcttga aagaagttgg catgaaagct gcactgcagg tcagcatgaa cgacggcctg     360
tccttcatct ccagttctgt catcatcacc accacacact gttcagacgg ctccatcctg     420
gcgattgctc tgctggtcct cttcctgctg ctggccctgg cgctgctctg gtggttctgg     480
cccctctgct gcacagtgat catcaaggag gtccctccac cccctgttga ggagagtgag     540
gaagaagacg atgatggttt gccaaagaag aaatggccca cagtagatgc ctcttattat     600
ggtggacgcg gtgtgggagg cattaaaaga atggaggtcc gctggggaga aaagggctcc     660
acagaagaag gggcgaagtt agaaaaggca aagaatgcac gagtcaagat gccagagcaa     720
gaatatgagt tcccagaacc ccgaaacctc aacaacaaca tgcgccggcc ttcctcgcct     780
cggaagtggt actcgcccat caagggaaaa ctcgatgcct tgtgggttct gctgagaaaa     840
ggatatgacc gagtgtctgt gatgaggcca cagccaggag acacgggacg ctgtatcaac     900
ttcaccagag tgaagaacag tcagccagcc aagtatcccc tgaacaacac ctaccacccc     960
agctccccac ctcccgctcc tatctacaca cccccacccc ctgctcccca ctgccctccc    1020
ccagccccca gtgcccccac tcctcccatt ccttccccac catccactct cccccctcct    1080
cctcaggccc caccccctaa cagggcacct cccccctccc gacctcctcc aaggccttct    1140
gtc                                                                  1143

<210> 59
<211> 1180
<212> DNA
<213> Mouse

<220>
<221> CDS
<222> (180)...(1179)

<400> 59
   cagccactca cagctccaaa cgttccccag ctctccacct ctgcgcgggc tgccacagag 60
   cctgccagct gcgcgcaaaa ccaaggtcct gaccagcgaa gcagagaaga ggcggtggcc 120
   cctctgtttc gtaggtcctg aggggctcag gacaagaaag gagcccaccc ccggccagt 179
   atg cag ccg ccc tgg ggc ctg gcg ctt cct ctg ctg ctc ccc tgg gtg 227
   Met Gln Pro Pro Trp Gly Leu Ala Leu Pro Leu Leu Leu Pro Trp Val
   1 5 10 15
   aca ggt gga gta ggg acc agt cca tgg gat tat ggg ttg tca gca ctg 275
   Thr Gly Gly Val Gly Thr Ser Pro Trp Asp Tyr Gly Leu Ser Ala Leu
   20 25 30
   gca cac cag cct ggg gtc tgt cag tat gga act aag atg gcc tgc tgc 323
   Ala His Gln Pro Gly Val Cys Gln Tyr Gly Thr Lys Met Ala Cys Cys
   35 40 45
   tat ggc tgg aaa agg aac aac aag gga gta tgc gaa gct atg tgt gag 371
   Tyr Gly Trp Lys Arg Asn Asn Lys Gly Val Cys Glu Ala Met Cys Glu
   50 55 60
   ccc agg tgc aag ttc ggt gag tgt gtg gga ccg aat aaa tgt aga tgc 419
   Pro Arg Cys Lys Phe Gly Glu Cys Val Gly Pro Asn Lys Cys Arg Cys
   65 70 75 80
   ttt cca gga tac acc ggg aag acc tgc act caa gat gtg aat gag tgt 467
   Phe Pro Gly Tyr Thr Gly Lys Thr Cys Thr Gln Asp Val Asn Glu Cys
   85 90 95
   gga gtc aaa ccc cgg ccg tgc cag cac aga tgt gtg aat aca cat ggt 515
   Gly Val Lys Pro Arg Pro Cys Gln His Arg Cys Val Asn Thr His Gly
   100 105 110
   agc tac aaa tgc ttt tgc ctc agc ggc cac atg ctt cta cca gat gct 563
   Ser Tyr Lys Cys Phe Cys Leu Ser Gly His Met Leu Leu Pro Asp Ala
   115 120 125
   aca tgt tca aac tcc agg acg tgt gcc aga cta aac tgc cag tac ggc 611
   Thr Cys Ser Asn Ser Arg Thr Cys Ala Arg Leu Asn Cys Gln Tyr Gly
   130 135 140
   tgt gaa gac aca gag gaa ggg cca cgg tgt gtg tgt cca tcc tct ggc 659
   Cys Glu Asp Thr Glu Glu Gly Pro Arg Cys Val Cys Pro Ser Ser Gly
   145 150 155 160
   ctc cgc ctg ggc cca aat gga aga gta tgc cta gat atc gat gaa tgt 707
   Leu Arg Leu Gly Pro Asn Gly Arg Val Cys Leu Asp Ile Asp Glu Cys
   165 170 175
   gcg tct agc aaa gca gtc tgc cct tcc aat cga aga tgc gtg aac acg 755
   Ala Ser Ser Lys Ala Val Cys Pro Ser Asn Arg Arg Cys Val Asn Thr
   180 185 190
   ttt gga agc tac tac tgc aaa tgt cac att ggt ttt gag ctg aaa tat 803
   Phe Gly Ser Tyr Tyr Cys Lys Cys His Ile Gly Phe Glu Leu Lys Tyr
   195 200 205
   atc ggt cgc cga tat gat tgt gta gat ata aat gag tgt gct ctg aat 851
   Ile Gly Arg Arg Tyr Asp Cys Val Asp Ile Asn Glu Cys Ala Leu Asn
   210 215 220
   acc cat ccg tgc agc ccc cat gcc aat tgc ctc aat acc cga gga tcc 899
   Thr His Pro Cys Ser Pro His Ala Asn Cys Leu Asn Thr Arg Gly Ser
   225 230 235 240
   ttc aag tgc aaa tgc aag cag gga tat agg ggc aat ggc ctg cag tgt 947
   Phe Lys Cys Lys Cys Lys Gln Gly Tyr Arg Gly Asn Gly Leu Gln Cys
   245 250 255
   tct gtg atc cct gaa cat tct gtg aag gaa ata ctc aca gca cct ggg 995
   Ser Val Ile Pro Glu His Ser Val Lys Glu Ile Leu Thr Ala Pro Gly
   260 265 270
   acc atc aaa gac cga atc aag aag tta ctg gct cac aag cgc acc atg 1043
   Thr Ile Lys Asp Arg Ile Lys Lys Leu Leu Ala His Lys Arg Thr Met
   275 280 285
   aag aaa aag gtg aaa cta aaa atg gtc acc cca aga ccc gcc agt aca 1091
   Lys Lys Lys Val Lys Leu Lys Met Val Thr Pro Arg Pro Ala Ser Thr
   290 295 300
   cgt gtc cct aag gtc aac ttg cct tac agc tct gag gag ggt gtt tcc 1139
   Arg Val Pro Lys Val Asn Leu Pro Tyr Ser Ser Glu Glu Gly Val Ser
   305 310 315 320
   agg ggc aga aac tat gat gga gaa caa aaa aaa aaa aaa a a 1180
   Arg Gly Arg Asn Tyr Asp Gly Glu Gln Lys Lys Lys Lys
   325 330

<210> 60
<211> 333
<212> PRT
<213> Mouse

<400> 60
Met Gln Pro Pro Trp Gly Leu Ala Leu Pro Leu Leu Leu Pro Trp Val
1               5                   10                  15
Thr Gly Gly Val Gly Thr Ser Pro Trp Asp Tyr Gly Leu Ser Ala Leu
            20                  25                  30
Ala His Gln Pro Gly Val Cys Gln Tyr Gly Thr Lys Met Ala Cys Cys
        35                  40                  45
Tyr Gly Trp Lys Arg Asn Asn Lys Gly Val Cys Glu Ala Met Cys Glu
    50                  55                  60
Pro Arg Cys Lys Phe Gly Glu Cys Val Gly Pro Asn Lys Cys Arg Cys
65                  70                  75                  80
Phe Pro Gly Tyr Thr Gly Lys Thr Cys Thr Gln Asp Val Asn Glu Cys
                85                  90                  95

Gly Val Lys Pro Arg Pro Cys Gln His Arg Cys Val Asn Thr His Gly
            100                 105                 110
Ser Tyr Lys Cys Phe Cys Leu Ser Gly His Met Leu Leu Pro Asp Ala
        115                 120                 125
Thr Cys Ser Asn Ser Arg Thr Cys Ala Arg Leu Asn Cys Gln Tyr Gly
    130                 135                 140
Cys Glu Asp Thr Glu Glu Gly Pro Arg Cys Val Cys Pro Ser Ser Gly
145                 150                 155                 160
Leu Arg Leu Gly Pro Asn Gly Arg Val Cys Leu Asp Ile Asp Glu Cys
                165                 170                 175
Ala Ser Ser Lys Ala Val Cys Pro Ser Asn Arg Arg Cys Val Asn Thr
            180                 185                 190
Phe Gly Ser Tyr Tyr Cys Lys Cys His Ile Gly Phe Glu Leu Lys Tyr
        195                 200                 205
Ile Gly Arg Arg Tyr Asp Cys Val Asp Ile Asn Glu Cys Ala Leu Asn
    210                 215                 220
Thr His Pro Cys Ser Pro His Ala Asn Cys Leu Asn Thr Arg Gly Ser
225                 230                 235                 240
Phe Lys Cys Lys Cys Lys Gln Gly Tyr Arg Gly Asn Gly Leu Gln Cys
                245                 250                 255
Ser Val Ile Pro Glu His Ser Val Lys Glu Ile Leu Thr Ala Pro Gly
            260                 265                 270
Thr Ile Lys Asp Arg Ile Lys Lys Leu Leu Ala His Lys Arg Thr Met
        275                 280                 285
Lys Lys Lys Val Lys Leu Lys Met Val Thr Pro Arg Pro Ala Ser Thr

290                 295                 300
Arg Val Pro Lys Val Asn Leu Pro Tyr Ser Ser Glu Glu Gly Val Ser
305                 310                 315                 320
Arg Gly Arg Asn Tyr Asp Gly Glu Gln Lys Lys Lys Lys
                325                 330

<210> 61
<211> 999
<212> DNA
<213> Mouse

<400> 61
atgcagccgc cctggggcct ggcgcttcct ctgctgctcc cctgggtgac aggtggagta      60
gggaccagtc catgggatta tgggttgtca gcactggcac accagcctgg ggtctgtcag     120
tatggaacta agatggcctg ctgctatggc tggaaaagga acaacaaggg agtatgcgaa     180
gctatgtgtg agcccaggtg caagttcggt gagtgtgtgg gaccgaataa atgtagatgc     240
tttccaggat acaccgggaa gacctgcact caagatgtga atgagtgtgg agtcaaaccc     300
cggccgtgcc agcacagatg tgtgaataca catggtagct acaaatgctt ttgcctcagc     360
ggccacatgc ttctaccaga tgctacatgt tcaaactcca ggacgtgtgc cagactaaac     420
tgccagtacg gctgtgaaga cacagaggaa gggccacggt gtgtgtgtcc atcctctggc     480
ctccgcctgg gcccaaatgg aagagtatgc ctagatatcg atgaatgtgc gtctagcaaa     540
gcagtctgcc cttccaatcg aagatgcgtg aacacgtttg gaagctacta ctgcaaatgt     600
cacattggtt ttgagctgaa atatatcggt cgccgatatg attgtgtaga tataaatgag     660
tgtgctctga atacccatcc gtgcagcccc catgccaatt gcctcaatac ccgaggatcc     720
ttcaagtgca aatgcaagca gggatatagg ggcaatggcc tgcagtgttc tgtgatccct     780
gaacattctg tgaaggaaat actcacagca cctgggacca tcaaagaccg aatcaagaag     840
ttactggctc acaagcgcac catgaagaaa aaggtgaaac taaaaatggt caccccaaga     900
cccgccagta cacgtgtccc taaggtcaac ttgccttaca gctctgagga gggtgtttcc     960
aggggcagaa actatgatgg agaacaaaaa aaaaaaaaa                            999

<210> 62
<211> 2154
<212> DNA
<213> Mouse

<220>
<221> CDS
<222> (41)...(616)

<400> 62
gtcgacccac gcgtccgcac ccgttaacgt atccgtctag ccg cct ctg ccg cca      55
                                            Pro Pro Leu Pro Pro
                                             1               5

tct ttg ttg agg cgg ata aac tgc cgg gat gca tcc aga gca ttt gtg     103
Ser Leu Leu Arg Arg Ile Asn Cys Arg Asp Ala Ser Arg Ala Phe Val
                10                  15                  20

aca gga gac tat tct gaa gcc ggt ctt gtg gat gat ata aat ggc ttg     151
Thr Gly Asp Tyr Ser Glu Ala Gly Leu Val Asp Asp Ile Asn Gly Leu
            25                  30                  35

tcc tcc tct gag ata ctg acg ctg cac aac tgg ctt tca ttc tac gag     199
Ser Ser Ser Glu Ile Leu Thr Leu His Asn Trp Leu Ser Phe Tyr Glu
        40                  45                  50

aaa aat tat gta ttt gtc gga aga ctg gtt gga agg ttc tac aga aag     247
Lys Asn Tyr Val Phe Val Gly Arg Leu Val Gly Arg Phe Tyr Arg Lys
    55                  60                  65

gat ggg tta ccc act tca gaa cta acc cag gta gaa gcc atg gtg act     295
Asp Gly Leu Pro Thr Ser Glu Leu Thr Gln Val Glu Ala Met Val Thr
70                  75                  80                  85

aaa ggc atg gag gca aat gaa cag gaa caa aga gaa aag cag aag ttc     343
Lys Gly Met Glu Ala Asn Glu Gln Glu Gln Arg Glu Lys Gln Lys Phe

90                  95                  100

cca cca tgc aat tcc gag tgg agc tct gct aag ggc agc agg ctc tgg      391
Pro Pro Cys Asn Ser Glu Trp Ser Ser Ala Lys Gly Ser Arg Leu Trp
            105                 110                 115

tgc tcc caa aag agt gga ggt gtg cac aga gac tgg att ggt gtc ccc      439
Cys Ser Gln Lys Ser Gly Gly Val His Arg Asp Trp Ile Gly Val Pro
        120                 125                 130

agg aag ttg tat aag cca ggg gcc aag gag ccc cat tgt gtg tgt gtg      487
Arg Lys Leu Tyr Lys Pro Gly Ala Lys Glu Pro His Cys Val Cys Val
    135                 140                 145

aga aca act ggc cct cct agt gac cag caa gac aac cct aga cac tca      535
Arg Thr Thr Gly Pro Pro Ser Asp Gln Gln Asp Asn Pro Arg His Ser
150                 155                 160                 165

aat cat ggg gac ttg gac aac ccc aac ttg gaa gaa tac aca ggc tgc      583
Asn His Gly Asp Leu Asp Asn Pro Asn Leu Glu Glu Tyr Thr Gly Cys
            170                 175                 180

cca cca ctg gct acc aca tgt tcc ttc cca ctc taagatggtg tcctgtatgt    636
Pro Pro Leu Ala Thr Thr Cys Ser Phe Pro Leu
            185                 190

ggctgacaca tggagaacct tccagatcta cagaaagccc tcgatcttgt gccctagcat    696
ggctcctggc ctgaaatagg agtgtcaagg actccaacca cactctgcaa atgtgggtca    756
caattctact ggctgagaca agagcctgat agcctatctc atatggtcag cttgttctga    816
cagtgtttgg aacctctgtc ctctctcatc ttccttcaga gcctgcagac attaatgaac    876
aaagtgaaat caacatagaa ttgatcagat aaggagtctg agagctccag aagggacttg    936
ccgtagactt ggtgttgcct gaatcagagc ttgcactctg cccatcatgc cagtcctgct    996
ggtacttgga gcaggactgt ggttctcagt ggaaaatcag acatttccca agaagatttt   1056
gcaccttagt aaaatgaagg taactgttct aagtcacagg gtaattgact gagctcttaa   1116
cttctgacat ttgtgacatt tctaaaacct ctactccagg gcaaactgac tccatgagaa   1176
ctgggatggt ccagtgagat gagataataa actagagtag cagattctgg ggcttgactt   1236
acacagatgg agcacaccat aatccccctg ccctggtgtg catcaaacag ctttctttac   1296
tcatattgct agtgaaaagt tgtccctcta tgactgccac acgctgtaag aggcttcgct   1356
gcaggtttct cacccccgct gcagaattcc gttctctgct gcacagccgc agctcttctc   1416
atctctttct ggttcttgtt ttcgttttcc agaaagggtc tcattatgta gctctagcta   1476
tcctggaagt caccaggtag acggagctga cctggaactc acagaaatcc acctgcctct   1536
gcctcccgag tattgggatt aaagacatgc accactatgt cctactaatt ttacctatct   1596
tgatctctgc tacttaaaaa aaaaattact tgactttatg aggcaggatc tcatgtttcc   1656
aggtcagcct tgaactcagt atgtaagcat gtgctcccat gcccagttta gtcagtagga   1716
ggggttgaat gaaccggagc cacatacatg ctaggcaagc actttaccaa ctgagctata   1776
tccctaggtc cttgagcttc attgctcttg actttttttt tttttttttc gtttttttga   1836
gacagggttt ctctgtgtag ctctggctgt cctggaactc actctgtaga ccaggctggc   1896
ctagaactca gaaatccacc tgtctctgcc tcccaagtgc taggattaaa ggcgtgtgcc   1956
accacgccca gcgctcttga ctttttgacc atgatgtcct gcaagatcct cataaagccc   2016
tcacttgctg cactggtccc atcccttagg ggttccttct gaggtagcat ctccaatcaa   2076
aaagcttgat aataaagatg tctgtgagtt ctgcaggcac tgtaactcca aaaaaaaaaa   2136
aaaaaaaagg gcggccgc                                                 2154

<210> 63
<211> 192
<212> PRT
<213> Mouse

<400> 63
Pro Pro Leu Pro Pro Ser Leu Leu Arg Arg Ile Asn Cys Arg Asp Ala
1               5                   10                  15
Ser Arg Ala Phe Val Thr Gly Asp Tyr Ser Glu Ala Gly Leu Val Asp
            20                  25                  30
Asp Ile Asn Gly Leu Ser Ser Ser Glu Ile Leu Thr Leu His Asn Trp
        35                  40                  45
Leu Ser Phe Tyr Glu Lys Asn Tyr Val Phe Val Gly Arg Leu Val Gly
    50                  55                  60
Arg Phe Tyr Arg Lys Asp Gly Leu Pro Thr Ser Glu Leu Thr Gln Val

65                  70                  75                  80
Glu Ala Met Val Thr Lys Gly Met Glu Ala Asn Glu Gln Glu Gln Arg
                85                  90                  95

Glu Lys Gln Lys Phe Pro Pro Cys Asn Ser Glu Trp Ser Ser Ala Lys
            100                 105                 110
Gly Ser Arg Leu Trp Cys Ser Gln Lys Ser Gly Gly Val His Arg Asp
        115                 120                 125
Trp Ile Gly Val Pro Arg Lys Leu Tyr Lys Pro Gly Ala Lys Glu Pro
    130                 135                 140
His Cys Val Cys Val Arg Thr Thr Gly Pro Pro Ser Asp Gln Gln Asp
145                 150                 155                 160
Asn Pro Arg His Ser Asn His Gly Asp Leu Asp Asn Pro Asn Leu Glu
                165                 170                 175
Glu Tyr Thr Gly Cys Pro Pro Leu Ala Thr Thr Cys Ser Phe Pro Leu
            180                 185                 190

<210> 64
<211> 576
<212> DNA
<213> Mouse

<400> 64
ccgcctctgc cgccatcttt gttgaggcgg ataaactgcc gggatgcatc cagagcattt      60
gtgacaggag actattctga agccggtctt gtggatgata taaatggctt gtcctcctct     120
gagatactga cgctgcacaa ctggctttca ttctacgaga aaaattatgt atttgtcgga     180
agactggttg gaaggttcta cagaaaggat gggttaccca cttcagaact aacccaggta     240
gaagccatgg tgactaaagg catggaggca aatgaacagg aacaaagaga aaagcagaag     300
ttcccaccat gcaattccga gtggagctct gctaagggca gcaggctctg gtgctcccaa     360
aagagtggag gtgtgcacag agactggatt ggtgtcccca ggaagttgta taagccaggg     420
gccaaggagc cccattgtgt gtgtgtgaga acaactggcc ctcctagtga ccagcaagac     480
aaccctagac actcaaatca tggggacttg gacaacccca acttggaaga atacacaggc     540
tgcccaccac tggctaccac atgttccttc ccactc                               576

<210> 65
<211> 2689
<212> DNA
<213> Homo sapiens

<220>
<221> CDS
<222> (67)...(2688)

<400> 65
cagcaggcag aggagcactt agcagcttat tcagtgtccg attctgattc cggcaaggat      60
ccaagc atg gaa tgc tgc cgt cgg gca act cct ggc aca ctg ctc ctc        108
       Met Glu Cys Cys Arg Arg Ala Thr Pro Gly Thr Leu Leu Leu
        1               5                   10

ttt ctg gct ttc ctg ctc ctg agt tcc agg acc gca cgc tcc gag gag       156
Phe Leu Ala Phe Leu Leu Leu Ser Ser Arg Thr Ala Arg Ser Glu Glu
 15                  20                  25                  30

gac cgg gac ggc ctg tgg gat gcc tgg ggc cca tgg agt gaa tgc tca       204
Asp Arg Asp Gly Leu Trp Asp Ala Trp Gly Pro Trp Ser Glu Cys Ser
                 35                  40                  45

cgc acc tgc ggg gga ggg gcc tcc tac tct ctg agg cgc tgc ctg agc       252
Arg Thr Cys Gly Gly Gly Ala Ser Tyr Ser Leu Arg Arg Cys Leu Ser
             50                  55                  60

agc aag agc tgt gaa gga aga aat atc cga tac aga aca tgc agt aat       300
Ser Lys Ser Cys Glu Gly Arg Asn Ile Arg Tyr Arg Thr Cys Ser Asn
         65                  70                  75

gtg gac tgc cca cca gaa gca ggt gat ttc cga gct cag caa tgc tca       348
Val Asp Cys Pro Pro Glu Ala Gly Asp Phe Arg Ala Gln Gln Cys Ser
     80                  85                  90

gct cat aat gat gtc aag cac cat ggc cag ttt tat gaa tgg ctt cct 396
Ala His Asn Asp Val Lys His His Gly Gln Phe Tyr Glu Trp Leu Pro
95                  100                 105                 110

gtg tct aat gac cct gac aac cca tgt tca ctc aag tgc caa gcc aaa 444
Val Ser Asn Asp Pro Asp Asn Pro Cys Ser Leu Lys Cys Gln Ala Lys
                115                 120                 125

gga aca acc ctg gtt gtt gaa cta gca cct aag gtc tta gat ggt acg 492
Gly Thr Thr Leu Val Val Glu Leu Ala Pro Lys Val Leu Asp Gly Thr
            130                 135                 140

cgt tgc tat aca gaa tct ttg gat atg tgc atc agt ggt tta tgc caa 540
Arg Cys Tyr Thr Glu Ser Leu Asp Met Cys Ile Ser Gly Leu Cys Gln
        145                 150                 155

att gtt ggc tgc gat cac cag ctg gga agc acc gtc aag gaa gat aac 588
Ile Val Gly Cys Asp His Gln Leu Gly Ser Thr Val Lys Glu Asp Asn
    160                 165                 170

tgt ggg gtc tgc aac gga gat ggg tcc acc tgc cgg ctg gtc cga ggg 636
Cys Gly Val Cys Asn Gly Asp Gly Ser Thr Cys Arg Leu Val Arg Gly
175                 180                 185                 190

cag tat aaa tcc cag ctc tcc gca acc aaa tcg gat gat act gtg gtt 684
Gln Tyr Lys Ser Gln Leu Ser Ala Thr Lys Ser Asp Asp Thr Val Val
                195                 200                 205

gca att ccc tat gga agt aga cat att cgc ctt gtc tta aaa ggt cct 732
Ala Ile Pro Tyr Gly Ser Arg His Ile Arg Leu Val Leu Lys Gly Pro
            210                 215                 220

gat cac tta tat ctg gaa acc aaa acc ctc cag ggg act aaa ggt gaa 780
Asp His Leu Tyr Leu Glu Thr Lys Thr Leu Gln Gly Thr Lys Gly Glu
        225                 230                 235

aac agt ctc agc tcc aca gga act ttc ctt gtg gac aat tct agt gtg 828
Asn Ser Leu Ser Ser Thr Gly Thr Phe Leu Val Asp Asn Ser Ser Val
    240                 245                 250

gac ttc cag aaa ttt cca gac aaa gag ata ctg aga atg gct gga cca 876
Asp Phe Gln Lys Phe Pro Asp Lys Glu Ile Leu Arg Met Ala Gly Pro
255                 260                 265                 270

ctc aca gca gat ttc att gtc aag att cgt aac tcg ggc tcc gct gac 924
Leu Thr Ala Asp Phe Ile Val Lys Ile Arg Asn Ser Gly Ser Ala Asp
                275                 280                 285

agt aca gtc cag ttc atc ttc tat caa ccc atc atc cac cga tgg agg 972
Ser Thr Val Gln Phe Ile Phe Tyr Gln Pro Ile Ile His Arg Trp Arg
            290                 295                 300

gag acg gat ttc ttt cct tgc tca gca acc tgt gga gga ggt tat cag 1020
Glu Thr Asp Phe Phe Pro Cys Ser Ala Thr Cys Gly Gly Gly Tyr Gln
        305                 310                 315

ctg aca tcg gct gag tgc tac gat ctg agg agc aac cgt gtg gtt gct 1068
Leu Thr Ser Ala Glu Cys Tyr Asp Leu Arg Ser Asn Arg Val Val Ala
    320                 325                 330

gac caa tac tgt cac tat tac cca gag aac atc aaa ccc aaa ccc aag 1116
Asp Gln Tyr Cys His Tyr Tyr Pro Glu Asn Ile Lys Pro Lys Pro Lys
335                 340                 345                 350

ctt cag gag tgc aac ttg gat cct tgt cca gcc cag agg gtt ggc att 1164

Leu Gln Glu Cys Asn Leu Asp Pro Cys Pro Ala Gln Arg Val Gly Ile
                355                 360                 365

att gtt cct aac agt gac gga tac aag cag atc atg cct tat gac ctc 1212
Ile Val Pro Asn Ser Asp Gly Tyr Lys Gln Ile Met Pro Tyr Asp Leu
            370                 375                 380

tac cat ccc ctt cct cgg tgg gag gcc acc cca tgg acc gcg tgc tcc 1260
Tyr His Pro Leu Pro Arg Trp Glu Ala Thr Pro Trp Thr Ala Cys Ser
        385                 390                 395

tcc tcg tgt ggg ggg ggc atc cag agc cgg gca gtt tcc tgt gtg gag 1308
Ser Ser Cys Gly Gly Gly Ile Gln Ser Arg Ala Val Ser Cys Val Glu
    400                 405                 410

gag gac atc cag ggg cat gtc act tca gtg gaa gag tgg aaa tgc atg 1356
Glu Asp Ile Gln Gly His Val Thr Ser Val Glu Glu Trp Lys Cys Met
415                 420                 425                 430

tac acc cct aag atg ccc atc gcg cag ccc tgc aac att ttt gac tgc 1404
Tyr Thr Pro Lys Met Pro Ile Ala Gln Pro Cys Asn Ile Phe Asp Cys
                435                 440                 445

cct aaa tgg ctg gca cag gag tgg tct ccg tgc aca gtg aca tgt ggc 1452
Pro Lys Trp Leu Ala Gln Glu Trp Ser Pro Cys Thr Val Thr Cys Gly
            450                 455                 460

cag ggc ctc aga tac cgt gtg gtc ctt tgc atc gac cat cga gga atg 1500
Gln Gly Leu Arg Tyr Arg Val Val Leu Cys Ile Asp His Arg Gly Met
        465                 470                 475

cac aca gga ggc tgt agc cca aaa aca aag ccc cac ata aaa gag gaa 1548
His Thr Gly Gly Cys Ser Pro Lys Thr Lys Pro His Ile Lys Glu Glu
    480                 485                 490

tgc atc gta ccc act ccc tgc tat aaa ccc aaa gag aaa ctt cca gtc 1596
Cys Ile Val Pro Thr Pro Cys Tyr Lys Pro Lys Glu Lys Leu Pro Val
495                 500                 505                 510

gag gcc aag ttg cca tgg ttc aaa caa gct caa gag cta gaa gaa gga 1644
Glu Ala Lys Leu Pro Trp Phe Lys Gln Ala Gln Glu Leu Glu Glu Gly
                515                 520                 525

gct gct gtg tca gag gag ccc tcg ttc atc cca gag gcc tgg tcg gcc 1692
Ala Ala Val Ser Glu Glu Pro Ser Phe Ile Pro Glu Ala Trp Ser Ala
            530                 535                 540

tgc aca gtc acc tgt ggt gtg ggg acc cag gtg cga ata gtc agg tgc 1740
Cys Thr Val Thr Cys Gly Val Gly Thr Gln Val Arg Ile Val Arg Cys
        545                 550                 555

cag gtg ctc ctg tct ttc tct cag tcc gtg gct gac ctg cct att gac 1788
Gln Val Leu Leu Ser Phe Ser Gln Ser Val Ala Asp Leu Pro Ile Asp
    560                 565                 570

gag tgt gaa ggg ccc aag cca gca tcc cag cgt gcc tgt tat gca ggc 1836
Glu Cys Glu Gly Pro Lys Pro Ala Ser Gln Arg Ala Cys Tyr Ala Gly
575                 580                 585                 590

cca tgc agc ggg gaa att cct gag ttc aac cca gac gag aca gat ggg 1884
Pro Cys Ser Gly Glu Ile Pro Glu Phe Asn Pro Asp Glu Thr Asp Gly
                595                 600                 605

ctc ttt ggt ggc ctg cag gat ttc gac gag ctg tat gac tgg gag tat 1932
Leu Phe Gly Gly Leu Gln Asp Phe Asp Glu Leu Tyr Asp Trp Glu Tyr
            610                 615                 620

gag ggg ttc acc aag tgc tcc gag tcc tgt gga gga ggt gtc cag gag 1980
Glu Gly Phe Thr Lys Cys Ser Glu Ser Cys Gly Gly Gly Val Gln Glu
        625                 630                 635

gct gtg gtg agc tgc ttg aac aaa cag act cgg gag cct gct gag gag 2028
Ala Val Val Ser Cys Leu Asn Lys Gln Thr Arg Glu Pro Ala Glu Glu
    640                 645                 650

aac ctg tgc gtg acc agc cgc cgg ccc cca cag ctc ctg aag tcc tgc 2076
Asn Leu Cys Val Thr Ser Arg Arg Pro Pro Gln Leu Leu Lys Ser Cys
655                 660                 665                 670

aat ttg gat ccc tgc cca gca agg tgg gaa att ggc aag tgg agt cca 2124
Asn Leu Asp Pro Cys Pro Ala Arg Trp Glu Ile Gly Lys Trp Ser Pro
                675                 680                 685

tgt agt ctc aca tgt ggg gtc ggc cta cag acc aga gac gtc ttc tgc 2172
Cys Ser Leu Thr Cys Gly Val Gly Leu Gln Thr Arg Asp Val Phe Cys
            690                 695                 700

agc cac ctg ctt tcc aga gag atg aat gaa aca gtc atc ctg gct gat 2220
Ser His Leu Leu Ser Arg Glu Met Asn Glu Thr Val Ile Leu Ala Asp
        705                 710                 715

gag ctg tgt cgc cag ccc aag ccc agc acg gtg caa gct tgt aac cgc 2268
Glu Leu Cys Arg Gln Pro Lys Pro Ser Thr Val Gln Ala Cys Asn Arg
    720                 725                 730

ttt aat tgc ccc cca gcc tgg tac cct gca cag tgg cag ccg tgt tcc 2316
Phe Asn Cys Pro Pro Ala Trp Tyr Pro Ala Gln Trp Gln Pro Cys Ser
735                 740                 745                 750

aga acg tgt ggc ggg ggt gtt cag aaa cgt gag gtt ctt tgc aag cag 2364
Arg Thr Cys Gly Gly Gly Val Gln Lys Arg Glu Val Leu Cys Lys Gln
                755                 760                 765

cgc atg gct gat ggc agc ttc ctg gag ctt cct gag acc ttc tgt tca 2412
Arg Met Ala Asp Gly Ser Phe Leu Glu Leu Pro Glu Thr Phe Cys Ser
            770                 775                 780

gct tca aaa cct gcc tgc cag caa gca tgc aag aaa gat gac tgt ccc 2460
Ala Ser Lys Pro Ala Cys Gln Gln Ala Cys Lys Lys Asp Asp Cys Pro
        785                 790                 795

agc gag tgg ctt ctc tca gac tgg aca gag tgt tcc aca agc tgc ggg 2508
Ser Glu Trp Leu Leu Ser Asp Trp Thr Glu Cys Ser Thr Ser Cys Gly
    800                 805                 810

gaa ggc acc cag act cga agc gcc att tgc cga aag atg ctg aaa acc 2556
Glu Gly Thr Gln Thr Arg Ser Ala Ile Cys Arg Lys Met Leu Lys Thr
815                 820                 825                 830

ggc atc tca acg gtt gtc aat tcc acc ctg tgc ccg ccc ctg cct ttc 2604
Gly Ile Ser Thr Val Val Asn Ser Thr Leu Cys Pro Pro Leu Pro Phe
                835                 840                 845

tct tcc tcc atc agg ccc tgt atg ctg gca acc tgt gca agg ccc ggg 2652
Ser Ser Ser Ile Arg Pro Cys Met Leu Ala Thr Cys Ala Arg Pro Gly
            850                 855                 860

cgg cca tcc acg aag cac agc ccg cac atc gcg gcc g 2689
Arg Pro Ser Thr Lys His Ser Pro His Ile Ala Ala
        865                 870

<210> 66
<211> 874
<212> PRT
<213> Homo sapiens

<400> 66
Met Glu Cys Cys Arg Arg Ala Thr Pro Gly Thr Leu Leu Leu Phe Leu
1               5                   10                  15
Ala Phe Leu Leu Leu Ser Ser Arg Thr Ala Arg Ser Glu Glu Asp Arg
            20                  25                  30
Asp Gly Leu Trp Asp Ala Trp Gly Pro Trp Ser Glu Cys Ser Arg Thr
        35                  40                  45
Cys Gly Gly Gly Ala Ser Tyr Ser Leu Arg Arg Cys Leu Ser Ser Lys
    50                  55                  60
Ser Cys Glu Gly Arg Asn Ile Arg Tyr Arg Thr Cys Ser Asn Val Asp
65                  70                  75                  80
Cys Pro Pro Glu Ala Gly Asp Phe Arg Ala Gln Gln Cys Ser Ala His
                85                  90                  95
Asn Asp Val Lys His His Gly Gln Phe Tyr Glu Trp Leu Pro Val Ser
            100                 105                 110
Asn Asp Pro Asp Asn Pro Cys Ser Leu Lys Cys Gln Ala Lys Gly Thr
        115                 120                 125
Thr Leu Val Val Glu Leu Ala Pro Lys Val Leu Asp Gly Thr Arg Cys
    130                 135                 140
Tyr Thr Glu Ser Leu Asp Met Cys Ile Ser Gly Leu Cys Gln Ile Val
145                 150                 155                 160
Gly Cys Asp His Gln Leu Gly Ser Thr Val Lys Glu Asp Asn Cys Gly
                165                 170                 175
Val Cys Asn Gly Asp Gly Ser Thr Cys Arg Leu Val Arg Gly Gln Tyr
            180                 185                 190
Lys Ser Gln Leu Ser Ala Thr Lys Ser Asp Asp Thr Val Val Ala Ile
        195                 200                 205
Pro Tyr Gly Ser Arg His Ile Arg Leu Val Leu Lys Gly Pro Asp His
    210                 215                 220
Leu Tyr Leu Glu Thr Lys Thr Leu Gln Gly Thr Lys Gly Glu Asn Ser
225                 230                 235                 240
Leu Ser Ser Thr Gly Thr Phe Leu Val Asp Asn Ser Ser Val Asp Phe
                245                 250                 255
Gln Lys Phe Pro Asp Lys Glu Ile Leu Arg Met Ala Gly Pro Leu Thr
            260                 265                 270
Ala Asp Phe Ile Val Lys Ile Arg Asn Ser Gly Ser Ala Asp Ser Thr
        275                 280                 285
Val Gln Phe Ile Phe Tyr Gln Pro Ile Ile His Arg Trp Arg Glu Thr
    290                 295                 300
Asp Phe Phe Pro Cys Ser Ala Thr Cys Gly Gly Gly Tyr Gln Leu Thr
305                 310                 315                 320
Ser Ala Glu Cys Tyr Asp Leu Arg Ser Asn Arg Val Val Ala Asp Gln
                325                 330                 335
Tyr Cys His Tyr Tyr Pro Glu Asn Ile Lys Pro Lys Pro Lys Leu Gln
            340                 345                 350
Glu Cys Asn Leu Asp Pro Cys Pro Ala Gln Arg Val Gly Ile Ile Val
        355                 360                 365
Pro Asn Ser Asp Gly Tyr Lys Gln Ile Met Pro Tyr Asp Leu Tyr His
    370                 375                 380
Pro Leu Pro Arg Trp Glu Ala Thr Pro Trp Thr Ala Cys Ser Ser Ser
385                 390                 395                 400
Cys Gly Gly Gly Ile Gln Ser Arg Ala Val Ser Cys Val Glu Glu Asp
                405                 410                 415
Ile Gln Gly His Val Thr Ser Val Glu Glu Trp Lys Cys Met Tyr Thr
            420                 425                 430
Pro Lys Met Pro Ile Ala Gln Pro Cys Asn Ile Phe Asp Cys Pro Lys
        435                 440                 445
Trp Leu Ala Gln Glu Trp Ser Pro Cys Thr Val Thr Cys Gly Gln Gly
    450                 455                 460
Leu Arg Tyr Arg Val Val Leu Cys Ile Asp His Arg Gly Met His Thr
465                 470                 475                 480
Gly Gly Cys Ser Pro Lys Thr Lys Pro His Ile Lys Glu Glu Cys Ile

                485                 490                 495
Val Pro Thr Pro Cys Tyr Lys Pro Lys Glu Lys Leu Pro Val Glu Ala
            500                 505                 510
Lys Leu Pro Trp Phe Lys Gln Ala Gln Glu Leu Glu Glu Gly Ala Ala
        515                 520                 525
Val Ser Glu Glu Pro Ser Phe Ile Pro Glu Ala Trp Ser Ala Cys Thr
    530                 535                 540
Val Thr Cys Gly Val Gly Thr Gln Val Arg Ile Val Arg Cys Gln Val
545                 550                 555                 560
Leu Leu Ser Phe Ser Gln Ser Val Ala Asp Leu Pro Ile Asp Glu Cys
                565                 570                 575
Glu Gly Pro Lys Pro Ala Ser Gln Arg Ala Cys Tyr Ala Gly Pro Cys
            580                 585                 590
Ser Gly Glu Ile Pro Glu Phe Asn Pro Asp Glu Thr Asp Gly Leu Phe
        595                 600                 605
Gly Gly Leu Gln Asp Phe Asp Glu Leu Tyr Asp Trp Glu Tyr Glu Gly
    610                 615                 620
Phe Thr Lys Cys Ser Glu Ser Cys Gly Gly Gly Val Gln Glu Ala Val
625                 630                 635                 640
Val Ser Cys Leu Asn Lys Gln Thr Arg Glu Pro Ala Glu Glu Asn Leu
                645                 650                 655
Cys Val Thr Ser Arg Arg Pro Pro Gln Leu Leu Lys Ser Cys Asn Leu
            660                 665                 670
Asp Pro Cys Pro Ala Arg Trp Glu Ile Gly Lys Trp Ser Pro Cys Ser
        675                 680                 685
Leu Thr Cys Gly Val Gly Leu Gln Thr Arg Asp Val Phe Cys Ser His
    690                 695                 700
Leu Leu Ser Arg Glu Met Asn Glu Thr Val Ile Leu Ala Asp Glu Leu
705                 710                 715                 720
Cys Arg Gln Pro Lys Pro Ser Thr Val Gln Ala Cys Asn Arg Phe Asn
                725                 730                 735
Cys Pro Pro Ala Trp Tyr Pro Ala Gln Trp Gln Pro Cys Ser Arg Thr
            740                 745                 750
Cys Gly Gly Gly Val Gln Lys Arg Glu Val Leu Cys Lys Gln Arg Met
        755                 760                 765
Ala Asp Gly Ser Phe Leu Glu Leu Pro Glu Thr Phe Cys Ser Ala Ser
    770                 775                 780
Lys Pro Ala Cys Gln Gln Ala Cys Lys Lys Asp Asp Cys Pro Ser Glu
785                 790                 795                 800
Trp Leu Leu Ser Asp Trp Thr Glu Cys Ser Thr Ser Cys Gly Glu Gly
                805                 810                 815
Thr Gln Thr Arg Ser Ala Ile Cys Arg Lys Met Leu Lys Thr Gly Ile
            820                 825                 830
Ser Thr Val Val Asn Ser Thr Leu Cys Pro Pro Leu Pro Phe Ser Ser
        835                 840                 845
Ser Ile Arg Pro Cys Met Leu Ala Thr Cys Ala Arg Pro Gly Arg Pro
    850                 855                 860
Ser Thr Lys His Ser Pro His Ile Ala Ala
865                 870

<210> 67
<211> 2622
<212> DNA
<213> Homo sapiens

<400> 67
atggaatgct gccgtcgggc aactcctggc acactgctcc tctttctggc tttcctgctc      60
ctgagttcca ggaccgcacg ctccgaggag gaccgggacg gcctgtggga tgcctggggc     120
ccatggagtg aatgctcacg cacctgcggg ggaggggcct cctactctct gaggcgctgc     180
ctgagcagca agagctgtga aggaagaaat atccgataca gaacatgcag taatgtggac     240
tgcccaccag aagcaggtga tttccgagct cagcaatgct cagctcataa tgatgtcaag     300
caccatggcc agttttatga atggcttcct gtgtctaatg accctgacaa cccatgttca     360
ctcaagtgcc aagccaaagg aacaaccctg gttgttgaac tagcacctaa ggtcttagat     420
ggtacgcgtt gctatacaga atctttggat atgtgcatca gtggtttatg ccaaattgtt     480
ggctgcgatc accagctggg aagcaccgtc aaggaagata actgtggggt ctgcaacgga     540
gatgggtcca cctgccggct ggtccgaggg cagtataaat cccagctctc cgcaaccaaa     600
tcggatgata ctgtggttgc aattccctat ggaagtagac atattcgcct tgtcttaaaa     660
ggtcctgatc acttatatct ggaaaccaaa accctccagg ggactaaagg tgaaaacagt     720

ctcagctcca caggaacttt ccttgtggac aattctagtg tggacttcca gaaatttcca 780
gacaaagaga tactgagaat ggctggacca ctcacagcag atttcattgt caagattcgt 840
aactcgggct ccgctgacag tacagtccag ttcatcttct atcaacccat catccaccga 900
tggagggaga cggatttctt tccttgctca gcaacctgtg gaggaggtta tcagctgaca 960
tcggctgagt gctacgatct gaggagcaac cgtgtggttg ctgaccaata ctgtcactat 1020
tacccagaga acatcaaacc caaacccaag cttcaggagt gcaacttgga tccttgtcca 1080
gcccagaggg ttggcattat tgttcctaac agtgacggat acaagcagat catgccttat 1140
gacctctacc atccccttcc tcggtgggag gccaccccat ggaccgcgtg ctcctcctcg 1200
tgtggggggg gcatccagag ccgggcagtt tcctgtgtgg aggaggacat ccaggggcat 1260
gtcacttcag tggaagagtg gaaatgcatg tacaccccta agatgcccat cgcgcagccc 1320
tgcaacattt ttgactgccc taaatggctg gcacaggagt ggtctccgtg cacagtgaca 1380
tgtggccagg gcctcagata ccgtgtggtc ctttgcatcg accatcgagg aatgcacaca 1440
ggaggctgta gcccaaaaac aaagccccac ataaaagagg aatgcatcgt acccactccc 1500
tgctataaac ccaaagagaa acttccagtc gaggccaagt tgccatggtt caaacaagct 1560
caagagctag aagaaggagc tgctgtgtca gaggagccct cgttcatccc agaggcctgg 1620
tcggcctgca cagtcacctg tggtgtgggg acccaggtgc gaatagtcag gtgccaggtg 1680
ctcctgtctt tctctcagtc cgtggctgac ctgcctattg acgagtgtga agggcccaag 1740
ccagcatccc agcgtgcctg ttatgcaggc ccatgcagcg ggaaaattcc tgagttcaac 1800
ccagacgaga cagatgggct ctttggtggc ctgcaggatt tcgacgagct gtatgactgg 1860
gagtatgagg ggttcaccaa gtgctccgag tcctgtggag gaggtgtcca ggaggctgtg 1920
gtgagctgct tgaacaaaca gactcgggag cctgctgagg agaacctgtg cgtgaccagc 1980
cgccggcccc cacagctcct gaagtcctgc aatttggatc cctgcccagc aaggtgggaa 2040
attggcaagt ggagtccatg tagtctcaca tgtggggtcg gcctacagac cagagacgtc 2100
ttctgcagcc acctgctttc cagagagatg aatgaaacag tcatcctggc tgatgagctg 2160
tgtcgccagc ccaagcccag cacggtgcaa gcttgtaacc gctttaattg cccccccagcc 2220
tggtaccctg cacagtggca gccgtgttcc agaacgtgtg gcgggggtgt tcagaaacgt 2280
gaggttcttt gcaagcagcg catggctgat ggcagcttcc tggagcttcc tgagaccttc 2340
tgttcagctt caaaacctgc ctgccagcaa gcatgcaaga aagatgactg tcccagcgag 2400
tggcttctct cagactggac agagtgttcc acaagctgcg ggaaggcac ccagactcga 2460
agcgccattt gccgaaagat gctgaaaacc ggcatctcaa cggttgtcaa ttccaccctg 2520
tgcccgcccc tgcctttctc ttcctccatc aggccctgta tgctggcaac ctgtgcaagg 2580
cccgggcggc catccacgaa gcacagcccg cacatcgcgg cc 2622

<210> 68
<211> 3413
<212> DNA
<213> Homo sapiens

<220>
<221> CDS
<222> (344)...(2401)

<400> 68
gtcgacccac gcgtcccggg ctggcctttc aaagtgtgca gttgtctcct ccctgtccag 60
ccccatcgtc gcccaggacc agctgggccg cggtctgacc tgaggctgct gctcagcgcc 120
ggggcgctgg cgctctccat tcgagcacct tccagcatac cgctcggctc cgggagccgc 180
tctgcaaagt tgggcagctc agagcgcaag ctttgcctct cgacttctcc ctccttgggt 240
ccccggcgcc cccgcctccc acgatccctt tcactaggag cagccagtcc cagcgggctg 300
gcaacttgca ccccttccta gtcatcctcc ctgaaacgcg acc atg ctg tta agg 355
Met Leu Leu Arg
1

ggc gtc ctc ctg gcg ttg caa gcc ctg cag ctc gcc ggt gcc ctc gac 403
Gly Val Leu Leu Ala Leu Gln Ala Leu Gln Leu Ala Gly Ala Leu Asp
5                   10                  15                  20

ctg ccc gct ggg tcc tgt gcc ttt gaa gag agc act tgc ggc ttt gac 451
Leu Pro Ala Gly Ser Cys Ala Phe Glu Glu Ser Thr Cys Gly Phe Asp
                25                  30                  35

tcc gtg ttg gcc tct ctg ccg tgg att tta aat gag gaa ggc cat tac 499
Ser Val Leu Ala Ser Leu Pro Trp Ile Leu Asn Glu Glu Gly His Tyr
            40                  45                  50

att tat gtg gat acc tcc ttt ggc aag cag ggg gag aaa gct gtg ctg 547
Ile Tyr Val Asp Thr Ser Phe Gly Lys Gln Gly Glu Lys Ala Val Leu
        55                  60                  65

cta agt cct gac tta cag gct gag gaa tgg agc tgc ctc cgt ttg gtc 595
Leu Ser Pro Asp Leu Gln Ala Glu Glu Trp Ser Cys Leu Arg Leu Val
    70                  75                  80

tac cag ata acc aca tct tcg gag tct ctg tca gat ccc agc cag ctg 643
Tyr Gln Ile Thr Thr Ser Ser Glu Ser Leu Ser Asp Pro Ser Gln Leu
85                  90                  95                  100

aac ctc tac atg aga ttt gaa gat gaa agc ttt gat cgc ttg ctt tgg 691
Asn Leu Tyr Met Arg Phe Glu Asp Glu Ser Phe Asp Arg Leu Leu Trp
                105                 110                 115

tca gct aag gaa cct tca gac agc tgg ctc ata gcc agc ttg gat ttg 739
Ser Ala Lys Glu Pro Ser Asp Ser Trp Leu Ile Ala Ser Leu Asp Leu
            120                 125                 130

caa aac agt tcc aag aaa ttc aag att tta ata gaa ggt gta cta gga 787
Gln Asn Ser Ser Lys Lys Phe Lys Ile Leu Ile Glu Gly Val Leu Gly
        135                 140                 145

cag gga aac aca gcc agc atc gca cta ttt gaa atc aag atg aca acc 835
Gln Gly Asn Thr Ala Ser Ile Ala Leu Phe Glu Ile Lys Met Thr Thr
    150                 155                 160

ggc tac tgt att gaa tgt gac ttt gaa gaa aat cat ctc tgt ggc ttt 883
Gly Tyr Cys Ile Glu Cys Asp Phe Glu Glu Asn His Leu Cys Gly Phe
165                 170                 175                 180

gtg aac cgc tgg aat ccc aat gtg aac tgg ttt gtt gga gga gga agt 931
Val Asn Arg Trp Asn Pro Asn Val Asn Trp Phe Val Gly Gly Gly Ser
                185                 190                 195

att cgg aat gtc cac tcc att ctc cca cag gat cac acc ttc aag agt 979
Ile Arg Asn Val His Ser Ile Leu Pro Gln Asp His Thr Phe Lys Ser
            200                 205                 210

gaa ctg ggc cac tac atg tac gtg gac tca gtt tat gtg aag cac ttc 1027
Glu Leu Gly His Tyr Met Tyr Val Asp Ser Val Tyr Val Lys His Phe
        215                 220                 225

cag gag gtg gca cag ctc atc tcc ccg ttg acc acg gcc ccc atg gct 1075
Gln Glu Val Ala Gln Leu Ile Ser Pro Leu Thr Thr Ala Pro Met Ala
    230                 235                 240

ggc tgc ctg tca ttt tat tac cag atc cag cag ggg aat gac aat gtc 1123
Gly Cys Leu Ser Phe Tyr Tyr Gln Ile Gln Gln Gly Asn Asp Asn Val
245                 250                 255                 260

ttt tcc ctt tac act cgg gat gtg gct ggc ctt tac gag gaa atc tgg 1171
Phe Ser Leu Tyr Thr Arg Asp Val Ala Gly Leu Tyr Glu Glu Ile Trp
                265                 270                 275

aaa gca gac agg cca ggg aat gct gcc tgg aac ctt gcg gag gtc gag 1219
Lys Ala Asp Arg Pro Gly Asn Ala Ala Trp Asn Leu Ala Glu Val Glu
            280                 285                 290

ttc aat gct cct tac ccc atg gag gtt att ttt gaa gtt gct ttc aat 1267
Phe Asn Ala Pro Tyr Pro Met Glu Val Ile Phe Glu Val Ala Phe Asn
        295                 300                 305

ggt ccc aag gga ggt tat gtt gcc ctg gat gat att tca ttc tct cct 1315
Gly Pro Lys Gly Gly Tyr Val Ala Leu Asp Asp Ile Ser Phe Ser Pro
    310                 315                 320

gtt cac tgc cag aat cag aca gaa ctt ctg ttc agt gcc gtg gaa gcc 1363
Val His Cys Gln Asn Gln Thr Glu Leu Leu Phe Ser Ala Val Glu Ala
325                 330                 335                 340

agc tgc aat ttt gag caa gat ctc tgc aac ttt tac caa gat aaa gaa 1411
Ser Cys Asn Phe Glu Gln Asp Leu Cys Asn Phe Tyr Gln Asp Lys Glu
                345                 350                 355

ggt cca ggt tgg acc cga gtg aaa gta aaa cca aac atg tat cgg gct 1459
Gly Pro Gly Trp Thr Arg Val Lys Val Lys Pro Asn Met Tyr Arg Ala
            360                 365                 370

gga gac cac act aca ggc tta ggg tat tac ctg cta gcc aac aca aag 1507
Gly Asp His Thr Thr Gly Leu Gly Tyr Tyr Leu Leu Ala Asn Thr Lys
        375                 380                 385

ttc aca tct cag cct ggc tac att gga agg ctc tat ggg ccc tcc cta 1555
Phe Thr Ser Gln Pro Gly Tyr Ile Gly Arg Leu Tyr Gly Pro Ser Leu
    390                 395                 400

cca gga aac ttg cag tat tgt ctg cgt ttt cat tat gcc atc tat gga 1603
Pro Gly Asn Leu Gln Tyr Cys Leu Arg Phe His Tyr Ala Ile Tyr Gly
405                 410                 415                 420

ttt tta aaa atg agt gac acc cta gca gtt tac atc ttt gaa gag aac 1651
Phe Leu Lys Met Ser Asp Thr Leu Ala Val Tyr Ile Phe Glu Glu Asn
                425                 430                 435

cat gtg gtt caa gag aag atc tgg tct gtg ttg gag tcc cca agg ggt 1699
His Val Val Gln Glu Lys Ile Trp Ser Val Leu Glu Ser Pro Arg Gly
            440                 445                 450

gtt tgg atg caa gct gaa atc acc ttt aag aag ccc atg cct acc aag 1747
Val Trp Met Gln Ala Glu Ile Thr Phe Lys Lys Pro Met Pro Thr Lys
        455                 460                 465

gtg gtt ttc atg agc cta tgc aaa agt ttc tgg gac tgt ggg ctt gta 1795
Val Val Phe Met Ser Leu Cys Lys Ser Phe Trp Asp Cys Gly Leu Val
    470                 475                 480

gcc ctg gat gac att aca ata caa ttg gga agc tgc tca tct tca gag 1843
Ala Leu Asp Asp Ile Thr Ile Gln Leu Gly Ser Cys Ser Ser Ser Glu
485                 490                 495                 500

aaa ctt cca ccc tca cct gga gag tgt act ttc gag caa gat gaa tgt 1891
Lys Leu Pro Pro Ser Pro Gly Glu Cys Thr Phe Glu Gln Asp Glu Cys
                505                 510                 515

aca ttt act cag gag aaa aga aac cgg agc agc tgg cac agg agg agg 1939
Thr Phe Thr Gln Glu Lys Arg Asn Arg Ser Ser Trp His Arg Arg Arg
            520                 525                 530

gga gaa act ccc act tcc tac aca gga cca aag gga gat cac act act 1987
Gly Glu Thr Pro Thr Ser Tyr Thr Gly Pro Lys Gly Asp His Thr Thr
        535                 540                 545

ggg gta ggc tac tac atg tac att gag gcc tcc cat atg gtg tat gga 2035
Gly Val Gly Tyr Tyr Met Tyr Ile Glu Ala Ser His Met Val Tyr Gly
    550                 555                 560

caa aaa gca cgc ctc ttg tcc agg cct ctg cga gga gtc tct gga aaa 2083
Gln Lys Ala Arg Leu Leu Ser Arg Pro Leu Arg Gly Val Ser Gly Lys
565                 570                 575                 580

cac tgc ttg acc ttt ttc tac cac atg tat gga ggg ggc act ggc ctg 2131
His Cys Leu Thr Phe Phe Tyr His Met Tyr Gly Gly Gly Thr Gly Leu
                585                 590                 595

ctg agt gtt tat ctg aaa aag gaa gaa gac agt gaa gag tcc ctc tta 2179

Leu Ser Val Tyr Leu Lys Lys Glu Glu Asp Ser Glu Glu Ser Leu Leu
            600                 605                 610

tgg agg aga aga ggt gaa cag agc att tcc tgg cta cga gca ctg att 2227
Trp Arg Arg Arg Gly Glu Gln Ser Ile Ser Trp Leu Arg Ala Leu Ile
        615                 620                 625

gaa tac agc tgt gag agg caa cac cag ata att ttt gaa gcc att cga 2275
Glu Tyr Ser Cys Glu Arg Gln His Gln Ile Ile Phe Glu Ala Ile Arg
    630                 635                 640

gga gta tca ata aga agt gat att gcc att gat gat gtt aaa ttt cag 2323
Gly Val Ser Ile Arg Ser Asp Ile Ala Ile Asp Asp Val Lys Phe Gln
645                 650                 655                 660

gca gga ccc tgt gga gaa atg gaa gat aca act caa caa tca tca gga 2371
Ala Gly Pro Cys Gly Glu Met Glu Asp Thr Thr Gln Gln Ser Ser Gly
                665                 670                 675

tat tct gag gac tta aat gaa att gag tat taagaaatga tctgcattgg 2421
Tyr Ser Glu Asp Leu Asn Glu Ile Glu Tyr
            680                 685

atttactaga cgaaaaccat acctctcttc aatcaaaatg aaaacaaagc aaatgaatac 2481
tggacagtct taacaatttt ataagttata aaatgacttt agagcaccct ccttcattac 2541
ttttgcaaaa acatactgac tcagggctct tttttctttt ttgcatatga caactgttac 2601
tagaaataca ggctactggt tttgcataga tcattcatct taattttggt accagttaaa 2661
aatacaaatg tactatattg tagtcatttt aaagtacaca aagggcacaa tcaaaatgag 2721
atgcactcat ttaaatctgc attcagtgaa tgtattggga gaaaaatagg tcttgcaggt 2781
ttccttttga attttaagta tcataaatat tttttaagta aataatacgg ggtgtcagta 2841
atatctgcag aatgaatgca gtctttcatg ctaatgagtt agtctggaaa aataaagtct 2901
tattttctat gttttattca tagaaatgga gtattaattt ttaatatttt caccatatgt 2961
gataacaaag gatctttcat gaatgtccaa gggtaagtca gtattaatta atgctgtatt 3021
acaaggcaat gctaccttct ttattccccc tttgaactac ctttgaagtc actatgagca 3081
catggataga aatttaactt ttttttgtaa agcaagctta aaatgtttat gtatacatac 3141
ccagcaactt ttataaatgt gttaaacaat tttactgatt tttataataa atattttggt 3201
aagattttga ataatatgaa ttcaggcaga tatactaaac tgcttttatt tacttgttta 3261
gaaaattgta tatatatgtt tgtgtatcct aacagctgct atgaaattat aaaattacct 3321
aataaaaata atttgaaaat caaaaaaaaa aaaaaaaaaa aaaaaaaaaa aaaaaaaaaa 3381
aaaaaaaaaa aaaaaaaaaa aaaaaagggg gg 3413

<210> 69
<211> 686
<212> PRT
<213> Homo sapiens

<400> 69
Met Leu Leu Arg Gly Val Leu Leu Ala Leu Gln Ala Leu Gln Leu Ala
1               5                   10                  15
Gly Ala Leu Asp Leu Pro Ala Gly Ser Cys Ala Phe Glu Glu Ser Thr
            20                  25                  30
Cys Gly Phe Asp Ser Val Leu Ala Ser Leu Pro Trp Ile Leu Asn Glu
        35                  40                  45
Glu Gly His Tyr Ile Tyr Val Asp Thr Ser Phe Gly Lys Gln Gly Glu
    50                  55                  60
Lys Ala Val Leu Leu Ser Pro Asp Leu Gln Ala Glu Glu Trp Ser Cys
65                  70                  75                  80
Leu Arg Leu Val Tyr Gln Ile Thr Thr Ser Ser Glu Ser Leu Ser Asp
                85                  90                  95
Pro Ser Gln Leu Asn Leu Tyr Met Arg Phe Glu Asp Glu Ser Phe Asp
            100                 105                 110
Arg Leu Leu Trp Ser Ala Lys Glu Pro Ser Asp Ser Trp Leu Ile Ala
        115                 120                 125
Ser Leu Asp Leu Gln Asn Ser Ser Lys Lys Phe Lys Ile Leu Ile Glu
    130                 135                 140
Gly Val Leu Gly Gln Gly Asn Thr Ala Ser Ile Ala Leu Phe Glu Ile

145                 150                 155                 160
Lys Met Thr Thr Gly Tyr Cys Ile Glu Cys Asp Phe Glu Glu Asn His
                165                 170                 175
Leu Cys Gly Phe Val Asn Arg Trp Asn Pro Asn Val Asn Trp Phe Val
            180                 185                 190
Gly Gly Gly Ser Ile Arg Asn Val His Ser Ile Leu Pro Gln Asp His
        195                 200                 205
Thr Phe Lys Ser Glu Leu Gly His Tyr Met Tyr Val Asp Ser Val Tyr
    210                 215                 220
Val Lys His Phe Gln Glu Val Ala Gln Leu Ile Ser Pro Leu Thr Thr
225                 230                 235                 240
Ala Pro Met Ala Gly Cys Leu Ser Phe Tyr Tyr Gln Ile Gln Gln Gly
                245                 250                 255
Asn Asp Asn Val Phe Ser Leu Tyr Thr Arg Asp Val Ala Gly Leu Tyr
            260                 265                 270
Glu Glu Ile Trp Lys Ala Asp Arg Pro Gly Asn Ala Ala Trp Asn Leu
        275                 280                 285
Ala Glu Val Glu Phe Asn Ala Pro Tyr Pro Met Glu Val Ile Phe Glu
    290                 295                 300
Val Ala Phe Asn Gly Pro Lys Gly Gly Tyr Val Ala Leu Asp Asp Ile
305                 310                 315                 320
Ser Phe Ser Pro Val His Cys Gln Asn Gln Thr Glu Leu Leu Phe Ser
                325                 330                 335
Ala Val Glu Ala Ser Cys Asn Phe Glu Gln Asp Leu Cys Asn Phe Tyr
            340                 345                 350
Gln Asp Lys Glu Gly Pro Gly Trp Thr Arg Val Lys Val Lys Pro Asn
        355                 360                 365
Met Tyr Arg Ala Gly Asp His Thr Thr Gly Leu Gly Tyr Tyr Leu Leu
    370                 375                 380
Ala Asn Thr Lys Phe Thr Ser Gln Pro Gly Tyr Ile Gly Arg Leu Tyr
385                 390                 395                 400
Gly Pro Ser Leu Pro Gly Asn Leu Gln Tyr Cys Leu Arg Phe His Tyr
                405                 410                 415
Ala Ile Tyr Gly Phe Leu Lys Met Ser Asp Thr Leu Ala Val Tyr Ile
            420                 425                 430
Phe Glu Glu Asn His Val Val Gln Glu Lys Ile Trp Ser Val Leu Glu
        435                 440                 445
Ser Pro Arg Gly Val Trp Met Gln Ala Glu Ile Thr Phe Lys Lys Pro
    450                 455                 460
Met Pro Thr Lys Val Val Phe Met Ser Leu Cys Lys Ser Phe Trp Asp
465                 470                 475                 480
Cys Gly Leu Val Ala Leu Asp Asp Ile Thr Ile Gln Leu Gly Ser Cys
                485                 490                 495
Ser Ser Ser Glu Lys Leu Pro Pro Ser Pro Gly Glu Cys Thr Phe Glu
            500                 505                 510
Gln Asp Glu Cys Thr Phe Thr Gln Glu Lys Arg Asn Arg Ser Ser Trp
        515                 520                 525
His Arg Arg Arg Gly Glu Thr Pro Thr Ser Tyr Thr Gly Pro Lys Gly
    530                 535                 540
Asp His Thr Thr Gly Val Gly Tyr Tyr Met Tyr Ile Glu Ala Ser His
545                 550                 555                 560
Met Val Tyr Gly Gln Lys Ala Arg Leu Leu Ser Arg Pro Leu Arg Gly
                565                 570                 575
Val Ser Gly Lys His Cys Leu Thr Phe Phe Tyr His Met Tyr Gly Gly
            580                 585                 590
Gly Thr Gly Leu Leu Ser Val Tyr Leu Lys Lys Glu Glu Asp Ser Glu
        595                 600                 605
Glu Ser Leu Leu Trp Arg Arg Arg Gly Glu Gln Ser Ile Ser Trp Leu
    610                 615                 620
Arg Ala Leu Ile Glu Tyr Ser Cys Glu Arg Gln His Gln Ile Ile Phe
625                 630                 635                 640
Glu Ala Ile Arg Gly Val Ser Ile Arg Ser Asp Ile Ala Ile Asp Asp
                645                 650                 655
Val Lys Phe Gln Ala Gly Pro Cys Gly Glu Met Glu Asp Thr Thr Gln
            660                 665                 670
Gln Ser Ser Gly Tyr Ser Glu Asp Leu Asn Glu Ile Glu Tyr
        675                 680                 685

<210> 70
<211> 2058
<212> DNA
<213> Homo sapiens

<400> 70
atgctgttaa ggggcgtcct cctggcgttg caagccctgc agctcgccgg tgccctcgac 60
ctgcccgctg ggtcctgtgc ctttgaagag agcacttgcg gctttgactc cgtgttggcc 120
tctctgccgt ggattttaaa tgaggaaggc cattacattt atgtggatac ctcctttggc 180
aagcaggggg agaaagctgt gctgctaagt cctgacttac aggctgagga atggagctgc 240
ctccgtttgg tctaccagat aaccacatct tcggagtctc tgtcagatcc cagccagctg 300
aacctctaca tgagatttga agatgaaagc tttgatcgct tgctttggtc agctaaggaa 360
ccttcagaca gctggctcat agccagcttg gatttgcaaa acagttccaa gaaattcaag 420
attttaatag aaggtgtact aggacaggga aacacagcca gcatcgcact atttgaaatc 480
aagatgacaa ccggctactg tattgaatgt gactttgaag aaaatcatct ctgtggcttt 540
gtgaaccgct ggaatcccaa tgtgaactgg tttgttggag gaggaagtat tcggaatgtc 600
cactccattc tcccacagga tcacaccttc aagagtgaac tgggccacta catgtacgtg 660
gactcagttt atgtgaagca cttccaggag gtggcacagc tcatctcccc gttgaccacg 720
gcccccatgg ctggctgcct gtcattttat taccagatcc agcaggggaa tgacaatgtc 780
ttttcccttt acactcggga tgtggctggc ctttacgagg aaatctggaa agcagacagg 840
ccagggaatg ctgcctggaa ccttgcggag gtcgagttca atgctcctta ccccatggag 900
gttatttttg aagttgcttt caatggtccc aagggaggtt atgttgccct ggatgatatt 960
tcattctctc ctgttcactg ccagaatcag acagaacttc tgttcagtgc cgtggaagcc 1020
agctgcaatt ttgagcaaga tctctgcaac ttttaccaag ataaagaagg tccaggttgg 1080
acccgagtga aagtaaaacc aaacatgtat cgggctggag accacactac aggcttaggg 1140
tattacctgc tagccaacac aaagttcaca tctcagcctg gctacattgg aaggctctat 1200
gggccctccc taccaggaaa cttgcagtat tgtctgcgtt ttcattatgc catctatgga 1260
tttttaaaaa tgagtgacac cctagcagtt tacatctttg aagagaacca tgtggttcaa 1320
gagaagatct ggtctgtgtt ggagtcccca aggggtgttt ggatgcaagc tgaaatcacc 1380
tttaagaagc ccatgcctac caaggtggtt ttcatgagcc tatgcaaaag tttctgggac 1440
tgtgggcttg tagccctgga tgacattaca atacaattgg gaagctgctc atcttcagag 1500
aaacttccac cctcacctgg agagtgtact ttcgagcaag atgaatgtac atttactcag 1560
gagaaaagaa accggagcag ctggcacagg aggaggggag aaactcccac ttcctacaca 1620
ggaccaaagg gagatcacac tactggggta ggctactaca tgtacattga ggcctcccat 1680
atggtgtatg gacaaaaagc acgcctcttg tccaggcctc tgcgaggagt ctctggaaaa 1740
cactgcttga cctttttcta ccacatgtat ggagggggca ctggcctgct gagtgtttat 1800
ctgaaaaagg aagaagacag tgaagagtcc ctcttatgga ggagaagagg tgaacagagc 1860
atttcctggc tacgagcact gattgaatac agctgtgaga ggcaacacca gataattttt 1920
gaagccattc gaggagtatc aataagaagt gatattgcca ttgatgatgt taaatttcag 1980
gcaggaccct gtggagaaat ggaagataca actcaacaat catcaggata ttctgaggac 2040
ttaaatgaaa ttgagtat 2058

<210> 71
<211> 1029
<212> DNA
<213> Mouse

<220>
<221> CDS
<222> (209)...(370)

<400> 71
gtcgacccac gcgtccgccg ggctacgagt ggccggacgc tacagccttg cgcagcgcgc 60
tctgctcctc agactcttcg aatttgagca gcctgtggca tcccccagca ggtcccccag 120
ctccttgcct agcaccctcc cttccctagg agcagcgggc cacagtgagc cagcagccct 180
cgcgggtcct cctgcctgaa gttcaact atg cta cta gaa ggg gtc ctg ctg 232
Met Leu Leu Glu Gly Val Leu Leu
1               5

gta gtg caa gcc ttg cag ctt gcc aat gcc cta gac ctg ccc gct ggc 280
Val Val Gln Ala Leu Gln Leu Ala Asn Ala Leu Asp Leu Pro Ala Gly
    10                  15                  20

tcc tgc gcc ttt gaa gaa gac acg tgt ggc ttt gac tcc gtg ttt gcg 328
Ser Cys Ala Phe Glu Glu Asp Thr Cys Gly Phe Asp Ser Val Phe Ala
25                  30                  35                  40

ttt ctg cct tgg ata cta aat gag gaa ggt aag ggg act tcg 370

Phe Leu Pro Trp Ile Leu Asn Glu Glu Gly Lys Gly Thr Ser
                45                  50

tagaaagatg ctcgaggtga actttcttca cgtcttgttc ctcccaaccc cccggaagta 430
aagatatctt ggagttactt ccctttggga ggaaaagtgt gtgagtcatg aaacctcctt 490
ccaactctcc tgcagcaaag agtggccagg gaaaccacgg gaaagggggc ggaggggaac 550
agctgtgtac ctggctctga gcatgcgctc ctacccccag cacaccctat tgaaagggac 610
aaaggggatt ctgctaatga ttgttgcccc tagccgtgtg ccccctgcag gctgatagcc 670
ttgctagtct cagtggctac ttgcccgagc tgagattgtc aaacggacta gctcacagga 730
agctttgcag aaattttcca cacggttgtg agcgtcctct gtgctaagct ctcccacttt 790
ggtccaccca cagcagtttt acctgtgatt catcctttcc cattgtatct aattcagcac 850
tggacaaaag agttaactcc accacggagt ccctgaagcc actgggctag ggccaattga 910
tcagtcacat tactctgcac cgctggggtt ccggtgacaa cgtttaagtg aaaaggagtc 970
tgtgatgtgt tttcttaccc ttcattgtta cagtaaaaaa aaaaaaaaag ggcggccgc 1029

<210> 72
<211> 54
<212> PRT
<213> Mouse

<400> 72
Met Leu Leu Glu Gly Val Leu Leu Val Val Gln Ala Leu Gln Leu Ala
1               5                   10                  15
Asn Ala Leu Asp Leu Pro Ala Gly Ser Cys Ala Phe Glu Glu Asp Thr
            20                  25                  30
Cys Gly Phe Asp Ser Val Phe Ala Phe Leu Pro Trp Ile Leu Asn Glu
        35                  40                  45
Glu Gly Lys Gly Thr Ser
    50

<210> 73
<211> 162
<212> DNA
<213> Mouse

<400> 73
atgctactag aaggggtcct gctggtagtg caagccttgc agcttgccaa tgccctagac 60
ctgcccgctg gctcctgcgc ctttgaagaa gacacgtgtg gctttgactc cgtgtttgcg 120
tttctgcctt ggatactaaa tgaggaaggt aaggggactt cg 162

<210> 74
<211> 16
<212> PRT
<213> Homo sapiens

<400> 74
Pro Gly Cys Leu Leu Val Lys Arg Cys Gly Gly Asn Cys Ala Cys Cys
1               5                   10                  15

<210> 75
<211> 69
<212> PRT
<213> Homo sapiens

<400> 75
Ile Phe Trp Pro Gly Cys Leu Leu Val Lys Arg Cys Gly Gly Asn Cys
1               5                   10                  15
Ala Cys Cys Leu His Asn Cys Asn Glu Cys Gln Cys Val Pro Ser Lys
            20                  25                  30
Val Thr Lys Lys Tyr His Glu Val Leu Gln Leu Arg Pro Lys Thr Gly
        35                  40                  45
Val Arg Gly Leu His Lys Ser Leu Thr Asp Val Ala Leu Glu His His
    50                  55                  60
Glu Glu Cys Asp Cys
65

<210> 76
<211> 49

<212> PRT
<213> Homo sapiens

<400> 76
Gly Ser Ile His Ser Pro Arg Phe Pro His Thr Tyr Pro Arg Asn Thr
1               5                   10                  15
Val Leu Val Trp Arg Leu Val Ala Val Glu Glu Asn Val Trp Ile Gln
            20                  25                  30

Leu Thr Phe Asp Glu Arg Phe Gly Leu Glu Asp Pro Glu Asp Asp Ile
        35                  40                  45
Cys


<210> 77
<211> 113
<212> PRT
<213> Homo sapiens

<400> 77
Arg Ile Ile Thr Val Ser Thr Asn Gly Ser Ile His Ser Pro Arg Phe
1               5                   10                  15
Pro His Thr Tyr Pro Arg Asn Thr Val Leu Val Trp Arg Leu Val Ala
            20                  25                  30
Val Glu Glu Asn Val Trp Ile Gln Leu Thr Phe Asp Glu Arg Phe Gly
        35                  40                  45
Leu Glu Asp Pro Glu Asp Asp Ile Cys Lys Tyr Asp Phe Val Glu Val
    50                  55                  60
Glu Glu Pro Ser Asp Gly Thr Ile Leu Gly Arg Trp Cys Gly Ser Gly
65                  70                  75                  80
Thr Val Pro Gly Lys Gln Ile Ser Lys Gly Asn Gln Ile Arg Ile Arg
                85                  90                  95
Phe Val Ser Asp Glu Tyr Phe Pro Ser Glu Pro Gly Phe Cys Ile His
            100                 105                 110
Tyr


<210> 78
<211> 764
<212> DNA
<213> Mouse

<220>
<221> CDS
<222> (211)...(750)

<221> misc_feature
<222> (1)...(764)
<223> n = A,T,C or G

<400> 78
ccgcgtccgg ctccctgcga gtcgccacga ctcatcgccg ctcccccgcg tccccacccc      60
ttctttcctc cctcgcctac ccccaccccc cgcacttcgg cacagctcag gatttgttta     120
aaccttggga aactggttca ggtccaggtt ttgctttgat ccttttcaaa aactggagac     180
acagaagagg gctctaggaa aaacttttgg atg gga tta tgt gga aac tac cct      234
                                 Met Gly Leu Cys Gly Asn Tyr Pro
                                 1               5

gcg att ctc tgc tgc caa aga acg ggg act cgg gct gag tcc aac ctg      282
Ala Ile Leu Cys Cys Gln Arg Thr Gly Thr Arg Ala Glu Ser Asn Leu
    10                  15                  20

agc agc aag ttg cag ctc tcc agc gac aag gaa cag aac gga gtg caa      330
Ser Ser Lys Leu Gln Leu Ser Ser Asp Lys Glu Gln Asn Gly Val Gln
25                  30                  35                  40

gat ccc cgg cat gag aga gtt gtc act ata tct ggt aat ggg agc atc      378
Asp Pro Arg His Glu Arg Val Val Thr Ile Ser Gly Asn Gly Ser Ile

                    45                  50                  55

cac agc ccg aag ttt cct cat aca tac cca aga aat atg gtg ctg gtg        426
His Ser Pro Lys Phe Pro His Thr Tyr Pro Arg Asn Met Val Leu Val
            60                  65                  70

tgg aga tta gtt gca gta gat gaa aat gtg cgg atc cag ctg aca ttt        474
Trp Arg Leu Val Ala Val Asp Glu Asn Val Arg Ile Gln Leu Thr Phe
        75                  80                  85

gat gag aga ttt ggg ctg gaa gat cca gaa gac gat ata tgc aag tat        522
Asp Glu Arg Phe Gly Leu Glu Asp Pro Glu Asp Asp Ile Cys Lys Tyr
    90                  95                  100

gat ttt gta gga gtt gag gag ccc agt gat gga agc gtt tta gga cgc        570
Asp Phe Val Gly Val Glu Glu Pro Ser Asp Gly Ser Val Leu Gly Arg
105                 110                 115                 120

tgg tgt ggt tct ggg act gtg cca gga aag cag act tct aaa gga aat        618
Trp Cys Gly Ser Gly Thr Val Pro Gly Lys Gln Thr Ser Lys Gly Asn
                125                 130                 135

cat atc agg ata aga ttt gca tct gat gag tat ttt cca tct gaa ccc        666
His Ile Arg Ile Arg Phe Ala Ser Asp Glu Tyr Phe Pro Ser Glu Pro
            140                 145                 150

gga ttc tgc atc cac tac agt att atc atg cca caa gtc aca gna acc        714
Gly Phe Cys Ile His Tyr Ser Ile Ile Met Pro Gln Val Thr Xaa Thr
        155                 160                 165

acg agt cct tng gtg ttg ccc cct tca tct ttt gtn nnttgggacc             760
Thr Ser Pro Xaa Val Leu Pro Pro Ser Ser Phe Val
    170                 175                 180

tgct                                                                   764

<210> 79
<211> 180
<212> PRT
<213> Mouse

<220>
<221> VARIANT
<222> (1)...(180)
<223> Xaa = Any Amino Acid

<400> 79
Met Gly Leu Cys Gly Asn Tyr Pro Ala Ile Leu Cys Cys Gln Arg Thr
 1               5                  10                  15
Gly Thr Arg Ala Glu Ser Asn Leu Ser Ser Lys Leu Gln Leu Ser Ser
            20                  25                  30
Asp Lys Glu Gln Asn Gly Val Gln Asp Pro Arg His Glu Arg Val Val
        35                  40                  45
Thr Ile Ser Gly Asn Gly Ser Ile His Ser Pro Lys Phe Pro His Thr
    50                  55                  60
Tyr Pro Arg Asn Met Val Leu Val Trp Arg Leu Val Ala Val Asp Glu
65                  70                  75                  80
Asn Val Arg Ile Gln Leu Thr Phe Asp Glu Arg Phe Gly Leu Glu Asp
                85                  90                  95
Pro Glu Asp Asp Ile Cys Lys Tyr Asp Phe Val Gly Val Glu Glu Pro
            100                 105                 110
Ser Asp Gly Ser Val Leu Gly Arg Trp Cys Gly Ser Gly Thr Val Pro
        115                 120                 125
Gly Lys Gln Thr Ser Lys Gly Asn His Ile Arg Ile Arg Phe Ala Ser
    130                 135                 140
Asp Glu Tyr Phe Pro Ser Glu Pro Gly Phe Cys Ile His Tyr Ser Ile
145                 150                 155                 160
Ile Met Pro Gln Val Thr Xaa Thr Thr Ser Pro Xaa Val Leu Pro Pro

165                 170                 175
Ser Ser Phe Val
            180

<210> 80
<211> 764
<212> DNA
<213> Mouse

<220>
<221> CDS
<222> (211)...(750)

<221> misc_feature
<222> (1)...(764)
<223> n = A,T,C or G

<400> 80
ccgcgtccgg ctccctgcga gtcgccacga ctcatcgccg ctcccccgcg tccccacccc      60
ttctttcctc cctcgcctac ccccaccccc cgcacttcgg cacagctcag gatttgttta     120
aaccttggga aactggttca ggtccaggtt ttgctttgat ccttttcaaa aactggagac     180
acagaagagg gctctaggaa aaacttttgg atg gga tta tgt gga aac tac cct      234
                                 Met Gly Leu Cys Gly Asn Tyr Pro
                                  1               5

gcg att ctc tgc tgc caa aga acg ggg act cgg gct gag tcc aac ctg      282
Ala Ile Leu Cys Cys Gln Arg Thr Gly Thr Arg Ala Glu Ser Asn Leu
    10                  15                  20

agc agc aag ttg cag ctc tcc agc gac aag gaa cag aac gga gtg caa      330
Ser Ser Lys Leu Gln Leu Ser Ser Asp Lys Glu Gln Asn Gly Val Gln
25                  30                  35                  40

gat ccc cgg cat gag aga gtt gtc act ata tct ggt aat ggg agc atc      378
Asp Pro Arg His Glu Arg Val Val Thr Ile Ser Gly Asn Gly Ser Ile
                45                  50                  55

cac agc ccg aag ttt cct cat aca tac cca aga aat atg gtg ctg gtg      426
His Ser Pro Lys Phe Pro His Thr Tyr Pro Arg Asn Met Val Leu Val
            60                  65                  70

tgg aga tta gtt gca gta gat gaa aat gtg cgg atc cag ctg aca ttt      474
Trp Arg Leu Val Ala Val Asp Glu Asn Val Arg Ile Gln Leu Thr Phe
        75                  80                  85

gat gag aga ttt ggg ctg gaa gat cca gaa gac gat ata tgc aag tat      522
Asp Glu Arg Phe Gly Leu Glu Asp Pro Glu Asp Asp Ile Cys Lys Tyr
    90                  95                  100

gat ttt gta gga gtt gag gag ccc agt gat gga agc gtt tta gga cgc      570
Asp Phe Val Gly Val Glu Glu Pro Ser Asp Gly Ser Val Leu Gly Arg
105                 110                 115                 120

tgg tgt ggt tct ggg act gtg cca cga aag cag act tct aaa gga aat      618
Trp Cys Gly Ser Gly Thr Val Pro Arg Lys Gln Thr Ser Lys Gly Asn
                125                 130                 135

cat atc agg ata aga ttt gca tct gat gag tat ttt cca tct gaa ccc      666
His Ile Arg Ile Arg Phe Ala Ser Asp Glu Tyr Phe Pro Ser Glu Pro
            140                 145                 150

gga ttc tgc atc cac tac agt att atc atg cca caa gtc aca gna acc      714
Gly Phe Cys Ile His Tyr Ser Ile Ile Met Pro Gln Val Thr Xaa Thr
        155                 160                 165

acg agt cct tng gtg ttg ccc cct tca tct ttt gtn nnttgggacc           760

Thr Ser Pro Xaa Val Leu Pro Pro Ser Ser Phe Val
    170                 175                 180

tgct                                                                 764

<210> 81
<211> 180
<212> PRT
<213> Mouse

<220>
<221> VARIANT
<222> (1)...(180)
<223> Xaa = Any Amino Acid

<400> 81
Met Gly Leu Cys Gly Asn Tyr Pro Ala Ile Leu Cys Cys Gln Arg Thr
 1               5                  10                  15
Gly Thr Arg Ala Glu Ser Asn Leu Ser Ser Lys Leu Gln Leu Ser Ser
            20                  25                  30
Asp Lys Glu Gln Asn Gly Val Gln Asp Pro Arg His Glu Arg Val Val
        35                  40                  45
Thr Ile Ser Gly Asn Gly Ser Ile His Ser Pro Lys Phe Pro His Thr
    50                  55                  60
Tyr Pro Arg Asn Met Val Leu Val Trp Arg Leu Val Ala Val Asp Glu
65                  70                  75                  80
Asn Val Arg Ile Gln Leu Thr Phe Asp Glu Arg Phe Gly Leu Glu Asp
                85                  90                  95
Pro Glu Asp Asp Ile Cys Lys Tyr Asp Phe Val Gly Val Glu Glu Pro
            100                 105                 110
Ser Asp Gly Ser Val Leu Gly Arg Trp Cys Gly Ser Gly Thr Val Pro
        115                 120                 125
Arg Lys Gln Thr Ser Lys Gly Asn His Ile Arg Ile Arg Phe Ala Ser
    130                 135                 140
Asp Glu Tyr Phe Pro Ser Glu Pro Gly Phe Cys Ile His Tyr Ser Ile
145                 150                 155                 160
Ile Met Pro Gln Val Thr Xaa Thr Thr Ser Pro Xaa Val Leu Pro Pro
                165                 170                 175
Ser Ser Phe Val
            180

<210> 82
<211> 764
<212> DNA
<213> Mouse

<220>
<221> CDS
<222> (211)...(750)

<221> misc_feature
<222> (1)...(764)
<223> n = A,T,C or G

<400> 82
ccgcgtccgg ctccctgcga gtcgccacga ctcatcgccg ctcccccgcg tccccacccc      60
ttctttcctc cctcgcctac ccccaccccc cgcacttcgg cacagctcag gatttgttta     120
aaccttggga aactggttca ggtccaggtt ttgctttgat ccttttcaaa aactggagac     180
acagaagagg gctctaggaa aaacttttgg atg gga tta tgt gga aac tac cct      234
                                 Met Gly Leu Cys Gly Asn Tyr Pro
                                  1               5

gcg att ctc tgc tgc caa aga acg ggg act cgg gct gag tcc aac ctg      282
Ala Ile Leu Cys Cys Gln Arg Thr Gly Thr Arg Ala Glu Ser Asn Leu
    10                  15                  20

agc agc aag ttg cag ctc tcc agc gac aag gaa cag aac gga gtg caa      330
Ser Ser Lys Leu Gln Leu Ser Ser Asp Lys Glu Gln Asn Gly Val Gln

25                  30                  35                  40

gat ccc cgg cat gag aga gtt gtc act ata tct ggt aat ggg agc atc        378
Asp Pro Arg His Glu Arg Val Val Thr Ile Ser Gly Asn Gly Ser Ile
                45                  50                  55

cac agc ccg aag ttt cct cat aca tac cca aga aat atg gtg ctg gtg        426
His Ser Pro Lys Phe Pro His Thr Tyr Pro Arg Asn Met Val Leu Val
            60                  65                  70

tgg aga tta gtt gca gta gat gaa aat gtg cgg atc cag ctg aca ttt        474
Trp Arg Leu Val Ala Val Asp Glu Asn Val Arg Ile Gln Leu Thr Phe
        75                  80                  85

gat gag aga ttt ggg ctg gaa gat cca gaa gac gat ata tgc aag tat        522
Asp Glu Arg Phe Gly Leu Glu Asp Pro Glu Asp Asp Ile Cys Lys Tyr
    90                  95                  100

gat ttt gta gga gtt gag gag ccc agt gat gga agc gtt tta gga cgc        570
Asp Phe Val Gly Val Glu Glu Pro Ser Asp Gly Ser Val Leu Gly Arg
105                 110                 115                 120

tgg tgt ggt tct ggg act gtg cca tga aag cag act tct aaa gga aat        618
Trp Cys Gly Ser Gly Thr Val Pro  *  Lys Gln Thr Ser Lys Gly Asn
                125                 130                 135

cat atc agg ata aga ttt gca tct gat gag tat ttt cca tct gaa ccc        666
His Ile Arg Ile Arg Phe Ala Ser Asp Glu Tyr Phe Pro Ser Glu Pro
                140                 145                 150

gga ttc tgc atc cac tac agt att atc atg cca caa gtc aca gna acc        714
Gly Phe Cys Ile His Tyr Ser Ile Ile Met Pro Gln Val Thr Xaa Thr
                155                 160                 165

acg agt cct tng gtg ttg ccc cct tca tct ttt gtn nnttgggacc             760
Thr Ser Pro Xaa Val Leu Pro Pro Ser Ser Phe Val
        170                 175

tgct                                                                   764

<210> 83
<211> 128
<212> PRT
<213> Mouse

<400> 83
Met Gly Leu Cys Gly Asn Tyr Pro Ala Ile Leu Cys Cys Gln Arg Thr
1               5                   10                  15
Gly Thr Arg Ala Glu Ser Asn Leu Ser Ser Lys Leu Gln Leu Ser Ser
            20                  25                  30
Asp Lys Glu Gln Asn Gly Val Gln Asp Pro Arg His Glu Arg Val Val
        35                  40                  45
Thr Ile Ser Gly Asn Gly Ser Ile His Ser Pro Lys Phe Pro His Thr
    50                  55                  60
Tyr Pro Arg Asn Met Val Leu Val Trp Arg Leu Val Ala Val Asp Glu
65                  70                  75                  80
Asn Val Arg Ile Gln Leu Thr Phe Asp Glu Arg Phe Gly Leu Glu Asp
                85                  90                  95
Pro Glu Asp Asp Ile Cys Lys Tyr Asp Phe Val Gly Val Glu Glu Pro
            100                 105                 110
Ser Asp Gly Ser Val Leu Gly Arg Trp Cys Gly Ser Gly Thr Val Pro
        115                 120                 125

<210> 84
<211> 764
<212> DNA
<213> Mouse

<220>
<221> CDS
<222> (211)...(750)

<221> misc_feature
<222> (1)...(764)
<223> n = A,T,C or G

<400> 84
   ccgcgtccgg ctccctgcga gtcgccacga ctcatcgccg ctcccccgcg tccccacccc      60
   ttctttcctc cctcgcctac ccccaccccc cgcacttcgg cacagctcag gatttgttta     120
   aaccttggga aactggttca ggtccaggtt ttgctttgat ccttttcaaa aactggagac     180
   acagaagagg gctctaggaa aaacttttgg atg gga tta tgt gga aac tac cct 234
   Met Gly Leu Cys Gly Asn Tyr Pro
   1 5
   gcg att ctc tgc tgc caa aga acg ggg act cgg gct gag tcc aac ctg       282
   Ala Ile Leu Cys Cys Gln Arg Thr Gly Thr Arg Ala Glu Ser Asn Leu
        10                  15                  20
   agc agc aag ttg cag ctc tcc agc gac aag gaa cag aac gga gtg caa       330
   Ser Ser Lys Leu Gln Leu Ser Ser Asp Lys Glu Gln Asn Gly Val Gln
    25                  30                  35                  40
   gat ccc cgg cat gag aga gtt gtc act ata tct ggt aat ggg agc atc       378
   Asp Pro Arg His Glu Arg Val Val Thr Ile Ser Gly Asn Gly Ser Ile
                    45                  50                  55
   cac agc ccg aag ttt cct cat aca tac cca aga aat atg gtg ctg gtg       426
   His Ser Pro Lys Phe Pro His Thr Tyr Pro Arg Asn Met Val Leu Val
                60                  65                  70
   tgg aga tta gtt gca gta gat gaa aat gtg cgg atc cag ctg aca ttt       474
   Trp Arg Leu Val Ala Val Asp Glu Asn Val Arg Ile Gln Leu Thr Phe
            75                  80                  85
   gat gag aga ttt ggg ctg gaa gat cca gaa gac gat ata tgc aag tat       522
   Asp Glu Arg Phe Gly Leu Glu Asp Pro Glu Asp Asp Ile Cys Lys Tyr
        90                  95                 100
   gat ttt gta gga gtt gag gag ccc agt gat gga agc gtt tta gga cgc       570
   Asp Phe Val Gly Val Glu Glu Pro Ser Asp Gly Ser Val Leu Gly Arg
   105                 110                 115                 120
   tgg tgt ggt tct ggg act gtg cca nga aag cag act tct aaa gga aat       618
   Trp Cys Gly Ser Gly Thr Val Pro Xaa Lys Gln Thr Ser Lys Gly Asn
                   125                 130                 135
   cat atc agg ata aga ttt gca tct gat gag tat ttt cca tct gaa ccc       666
   His Ile Arg Ile Arg Phe Ala Ser Asp Glu Tyr Phe Pro Ser Glu Pro
               140                 145                 150
   gga ttc tgc atc cac tac agt att atc atg cca caa gtc aca gta acc       714
   Gly Phe Cys Ile His Tyr Ser Ile Ile Met Pro Gln Val Thr Val Thr
           155                 160                 165
   acg agt cct tng gtg ttg ccc cct tca tct ttt gtn nnttgggacc            760
   Thr Ser Pro Xaa Val Leu Pro Pro Ser Ser Phe Val
       170                 175                 180
   tgct                                                                  764

<210> 85
<211> 180
<212> PRT
<213> Mouse

<220>
<221> VARIANT
<222> (1)...(180)
<223> Xaa = Any Amino Acid

<400> 85
Met Gly Leu Cys Gly Asn Tyr Pro Ala Ile Leu Cys Cys Gln Arg Thr
 1               5                  10                  15
Gly Thr Arg Ala Glu Ser Asn Leu Ser Ser Lys Leu Gln Leu Ser Ser
            20                  25                  30
Asp Lys Glu Gln Asn Gly Val Gln Asp Pro Arg His Glu Arg Val Val
        35                  40                  45
Thr Ile Ser Gly Asn Gly Ser Ile His Ser Pro Lys Phe Pro His Thr
    50                  55                  60
Tyr Pro Arg Asn Met Val Leu Val Trp Arg Leu Val Ala Val Asp Glu
65                  70                  75                  80
Asn Val Arg Ile Gln Leu Thr Phe Asp Glu Arg Phe Gly Leu Glu Asp
                85                  90                  95
Pro Glu Asp Asp Ile Cys Lys Tyr Asp Phe Val Gly Val Glu Glu Pro
            100                 105                 110
Ser Asp Gly Ser Val Leu Gly Arg Trp Cys Gly Ser Gly Thr Val Pro
        115                 120                 125
Xaa Lys Gln Thr Ser Lys Gly Asn His Ile Arg Ile Arg Phe Ala Ser
    130                 135                 140
Asp Glu Tyr Phe Pro Ser Glu Pro Gly Phe Cys Ile His Tyr Ser Ile
145                 150                 155                 160

Ile Met Pro Gln Val Thr Val Thr Thr Ser Pro Xaa Val Leu Pro Pro
                165                 170                 175
Ser Ser Phe Val
            180

<210> 86
<211> 764
<212> DNA
<213> Mouse

<220>
<221> CDS
<222> (211)...(750)

<221> misc_feature
<222> (1)...(764)
<223> n = A,T,C or G

<400> 86
   ccgcgtccgg ctccctgcga gtcgccacga ctcatcgccg ctcccccgcg tccccacccc      60
   ttctttcctc cctcgcctac ccccaccccc cgcacttcgg cacagctcag gatttgttta     120
   aaccttggga aactggttca ggtccaggtt ttgctttgat ccttttcaaa aactggagac     180
   acagaagagg gctctaggaa aaacttttgg atg gga tta tgt gga aac tac cct     234
                                    Met Gly Leu Cys Gly Asn Tyr Pro
                                     1               5
   gcg att ctc tgc tgc caa aga acg ggg act cgg gct gag tcc aac ctg       282
   Ala Ile Leu Cys Cys Gln Arg Thr Gly Thr Arg Ala Glu Ser Asn Leu
       10                  15                  20
   agc agc aag ttg cag ctc tcc agc gac aag gaa cag aac gga gtg caa       330
   Ser Ser Lys Leu Gln Leu Ser Ser Asp Lys Glu Gln Asn Gly Val Gln
    25                  30                  35                  40
   gat ccc cgg cat gag aga gtt gtc act ata tct ggt aat ggg agc atc       378
   Asp Pro Arg His Glu Arg Val Val Thr Ile Ser Gly Asn Gly Ser Ile
                   45                  50                  55
   cac agc ccg aag ttt cct cat aca tac cca aga aat atg gtg ctg gtg 426
   His Ser Pro Lys Phe Pro His Thr Tyr Pro Arg Asn Met Val Leu Val
   60 65 70
   tgg aga tta gtt gca gta gat gaa aat gtg cgg atc cag ctg aca ttt 474
   Trp Arg Leu Val Ala Val Asp Glu Asn Val Arg Ile Gln Leu Thr Phe
   75 80 85
   gat gag aga ttt ggg ctg gaa gat cca gaa gac gat ata tgc aag tat 522
   Asp Glu Arg Phe Gly Leu Glu Asp Pro Glu Asp Asp Ile Cys Lys Tyr
   90 95 100
   gat ttt gta gga gtt gag gag ccc agt gat gga agc gtt tta gga cgc 570
   Asp Phe Val Gly Val Glu Glu Pro Ser Asp Gly Ser Val Leu Gly Arg
   105 110 115 120
   tgg tgt ggt tct ggg act gtg cca nga aag cag act tct aaa gga aat 618
   Trp Cys Gly Ser Gly Thr Val Pro Xaa Lys Gln Thr Ser Lys Gly Asn
   125 130 135
   cat atc agg ata aga ttt gca tct gat gag tat ttt cca tct gaa ccc 666
   His Ile Arg Ile Arg Phe Ala Ser Asp Glu Tyr Phe Pro Ser Glu Pro
   140 145 150
   gga ttc tgc atc cac tac agt att atc atg cca caa gtc aca gca acc 714
   Gly Phe Cys Ile His Tyr Ser Ile Ile Met Pro Gln Val Thr Ala Thr
   155 160 165
   acg agt cct tng gtg ttg ccc cct tca tct ttt gtn nnttgggacc 760
   Thr Ser Pro Xaa Val Leu Pro Pro Ser Ser Phe Val
   170 175 180
   tgct 764

<210> 87
<211> 180
<212> PRT
<213> Mouse

<220>
<221> VARIANT
<222> (1)...(180)
<223> Xaa = Any Amino Acid

<400> 87
Met Gly Leu Cys Gly Asn Tyr Pro Ala Ile Leu Cys Cys Gln Arg Thr
1               5                   10                  15
Gly Thr Arg Ala Glu Ser Asn Leu Ser Ser Lys Leu Gln Leu Ser Ser
            20                  25                  30
Asp Lys Glu Gln Asn Gly Val Gln Asp Pro Arg His Glu Arg Val Val
        35                  40                  45
Thr Ile Ser Gly Asn Gly Ser Ile His Ser Pro Lys Phe Pro His Thr
    50                  55                  60
Tyr Pro Arg Asn Met Val Leu Val Trp Arg Leu Val Ala Val Asp Glu
65                  70                  75                  80
Asn Val Arg Ile Gln Leu Thr Phe Asp Glu Arg Phe Gly Leu Glu Asp
                85                  90                  95
Pro Glu Asp Asp Ile Cys Lys Tyr Asp Phe Val Gly Val Glu Glu Pro
            100                 105                 110
Ser Asp Gly Ser Val Leu Gly Arg Trp Cys Gly Ser Gly Thr Val Pro
        115                 120                 125
Xaa Lys Gln Thr Ser Lys Gly Asn His Ile Arg Ile Arg Phe Ala Ser
    130                 135                 140
Asp Glu Tyr Phe Pro Ser Glu Pro Gly Phe Cys Ile His Tyr Ser Ile
145                 150                 155                 160
Ile Met Pro Gln Val Thr Ala Thr Thr Ser Pro Xaa Val Leu Pro Pro
                165                 170                 175

Ser Ser Phe Val
            180

<210> 88
<211> 764
<212> DNA
<213> Mouse

<220>
<221> CDS
<222> (211)...(750)

<221> misc_feature
<222> (1)...(764)
<223> n = A,T,C or G

<400> 88
ccgcgtccgg ctccctgcga gtcgccacga ctcatcgccg ctcccccgcg tccccacccc      60
ttctttcctc cctcgcctac ccccaccccc cgcacttcgg cacagctcag gatttgttta     120
aaccttggga aactggttca ggtccaggtt ttgctttgat ccttttcaaa aactggagac     180
acagaagagg gctctaggaa aaacttttgg atg gga tta tgt gga aac tac cct      234
                                  Met Gly Leu Cys Gly Asn Tyr Pro
                                   1               5

gcg att ctc tgc tgc caa aga acg ggg act cgg gct gag tcc aac ctg      282
Ala Ile Leu Cys Cys Gln Arg Thr Gly Thr Arg Ala Glu Ser Asn Leu
    10                  15                  20

agc agc aag ttg cag ctc tcc agc gac aag gaa cag aac gga gtg caa      330
Ser Ser Lys Leu Gln Leu Ser Ser Asp Lys Glu Gln Asn Gly Val Gln
 25                  30                  35                  40

gat ccc cgg cat gag aga gtt gtc act ata tct ggt aat ggg agc atc      378
Asp Pro Arg His Glu Arg Val Val Thr Ile Ser Gly Asn Gly Ser Ile
                 45                  50                  55

cac agc ccg aag ttt cct cat aca tac cca aga aat atg gtg ctg gtg      426
His Ser Pro Lys Phe Pro His Thr Tyr Pro Arg Asn Met Val Leu Val
             60                  65                  70

tgg aga tta gtt gca gta gat gaa aat gtg cgg atc cag ctg aca ttt      474
Trp Arg Leu Val Ala Val Asp Glu Asn Val Arg Ile Gln Leu Thr Phe
         75                  80                  85

gat gag aga ttt ggg ctg gaa gat cca gaa gac gat ata tgc aag tat      522
Asp Glu Arg Phe Gly Leu Glu Asp Pro Glu Asp Asp Ile Cys Lys Tyr
     90                  95                 100

gat ttt gta gga gtt gag gag ccc agt gat gga agc gtt tta gga cgc      570
Asp Phe Val Gly Val Glu Glu Pro Ser Asp Gly Ser Val Leu Gly Arg
105                 110                 115                 120

tgg tgt ggt tct ggg act gtg cca nga aag cag act tct aaa gga aat      618
Trp Cys Gly Ser Gly Thr Val Pro Xaa Lys Gln Thr Ser Lys Gly Asn
                125                 130                 135

cat atc agg ata aga ttt gca tct gat gag tat ttt cca tct gaa ccc      666
His Ile Arg Ile Arg Phe Ala Ser Asp Glu Tyr Phe Pro Ser Glu Pro
            140                 145                 150

gga ttc tgc atc cac tac agt att atc atg cca caa gtc aca gaa acc      714
Gly Phe Cys Ile His Tyr Ser Ile Ile Met Pro Gln Val Thr Glu Thr
        155                 160                 165

acg agt cct tng gtg ttg ccc cct tca tct ttt gtn nnttgggacc           760
Thr Ser Pro Xaa Val Leu Pro Pro Ser Ser Phe Val
    170                 175                 180

tgct                                                                764

<210> 89
<211> 180
<212> PRT
<213> Mouse

<220>
<221> VARIANT
<222> (1)...(180)
<223> Xaa = Any Amino Acid

<400> 89
Met Gly Leu Cys Gly Asn Tyr Pro Ala Ile Leu Cys Cys Gln Arg Thr
 1               5                  10                  15
Gly Thr Arg Ala Glu Ser Asn Leu Ser Ser Lys Leu Gln Leu Ser Ser
            20                  25                  30

Asp Lys Glu Gln Asn Gly Val Gln Asp Pro Arg His Glu Arg Val Val
        35                  40                  45
Thr Ile Ser Gly Asn Gly Ser Ile His Ser Pro Lys Phe Pro His Thr
    50                  55                  60
Tyr Pro Arg Asn Met Val Leu Val Trp Arg Leu Val Ala Val Asp Glu
65                  70                  75                  80
Asn Val Arg Ile Gln Leu Thr Phe Asp Glu Arg Phe Gly Leu Glu Asp
                85                  90                  95
Pro Glu Asp Asp Ile Cys Lys Tyr Asp Phe Val Gly Val Glu Glu Pro
            100                 105                 110
Ser Asp Gly Ser Val Leu Gly Arg Trp Cys Gly Ser Gly Thr Val Pro
        115                 120                 125
Xaa Lys Gln Thr Ser Lys Gly Asn His Ile Arg Ile Arg Phe Ala Ser
    130                 135                 140
Asp Glu Tyr Phe Pro Ser Glu Pro Gly Phe Cys Ile His Tyr Ser Ile
145                 150                 155                 160
Ile Met Pro Gln Val Thr Glu Thr Thr Ser Pro Xaa Val Leu Pro Pro
                165                 170                 175
Ser Ser Phe Val
            180

<210> 90
<211> 764
<212> DNA
<213> Mouse

<220>
<221> CDS
<222> (211)...(750)

<221> misc_feature
<222> (1)...(764)
<223> n = A,T,C or G

<400> 90
ccgcgtccgg ctccctgcga gtcgccacga ctcatcgccg ctcccccgcg tccccacccc      60
ttctttcctc cctcgcctac ccccaccccc cgcacttcgg cacagctcag gatttgttta     120
aaccttggga aactggttca ggtccaggtt ttgctttgat ccttttcaaa aactggagac     180
acagaagagg gctctaggaa aaacttttgg atg gga tta tgt gga aac tac cct      234
                                 Met Gly Leu Cys Gly Asn Tyr Pro
                                  1               5

gcg att ctc tgc tgc caa aga acg ggg act cgg gct gag tcc aac ctg      282
Ala Ile Leu Cys Cys Gln Arg Thr Gly Thr Arg Ala Glu Ser Asn Leu
     10                  15                  20

agc agc aag ttg cag ctc tcc agc gac aag gaa cag aac gga gtg caa      330
Ser Ser Lys Leu Gln Leu Ser Ser Asp Lys Glu Gln Asn Gly Val Gln
 25                  30                  35                  40

gat ccc cgg cat gag aga gtt gtc act ata tct ggt aat ggg agc atc     378
Asp Pro Arg His Glu Arg Val Val Thr Ile Ser Gly Asn Gly Ser Ile
                45                  50                  55

cac agc ccg aag ttt cct cat aca tac cca aga aat atg gtg ctg gtg     426
His Ser Pro Lys Phe Pro His Thr Tyr Pro Arg Asn Met Val Leu Val
            60                  65                  70

tgg aga tta gtt gca gta gat gaa aat gtg cgg atc cag ctg aca ttt     474
Trp Arg Leu Val Ala Val Asp Glu Asn Val Arg Ile Gln Leu Thr Phe
        75                  80                  85

gat gag aga ttt ggg ctg gaa gat cca gaa gac gat ata tgc aag tat     522
Asp Glu Arg Phe Gly Leu Glu Asp Pro Glu Asp Asp Ile Cys Lys Tyr
    90                  95                  100

gat ttt gta gga gtt gag gag ccc agt gat gga agc gtt tta gga cgc     570
Asp Phe Val Gly Val Glu Glu Pro Ser Asp Gly Ser Val Leu Gly Arg
105                 110                 115                 120

tgg tgt ggt tct ggg act gtg cca nga aag cag act tct aaa gga aat     618
Trp Cys Gly Ser Gly Thr Val Pro Xaa Lys Gln Thr Ser Lys Gly Asn
                125                 130                 135

cat atc agg ata aga ttt gca tct gat gag tat ttt cca tct gaa ccc     666
His Ile Arg Ile Arg Phe Ala Ser Asp Glu Tyr Phe Pro Ser Glu Pro
            140                 145                 150

gga ttc tgc atc cac tac agt att atc atg cca caa gtc aca gga acc     714
Gly Phe Cys Ile His Tyr Ser Ile Ile Met Pro Gln Val Thr Gly Thr
        155                 160                 165

acg agt cct tng gtg ttg ccc cct tca tct ttt gtn nnttgggacc          760
Thr Ser Pro Xaa Val Leu Pro Pro Ser Ser Phe Val
    170                 175                 180

tgct                                                                764

<210> 91
<211> 180
<212> PRT
<213> Mouse

<220>
<221> VARIANT
<222> (1)...(180)
<223> Xaa = Any Amino Acid

<400> 91
Met Gly Leu Cys Gly Asn Tyr Pro Ala Ile Leu Cys Cys Gln Arg Thr
 1               5                  10                  15
Gly Thr Arg Ala Glu Ser Asn Leu Ser Ser Lys Leu Gln Leu Ser Ser
            20                  25                  30
Asp Lys Glu Gln Asn Gly Val Gln Asp Pro Arg His Glu Arg Val Val
        35                  40                  45
Thr Ile Ser Gly Asn Gly Ser Ile His Ser Pro Lys Phe Pro His Thr
    50                  55                  60
Tyr Pro Arg Asn Met Val Leu Val Trp Arg Leu Val Ala Val Asp Glu
65                  70                  75                  80
Asn Val Arg Ile Gln Leu Thr Phe Asp Glu Arg Phe Gly Leu Glu Asp
                85                  90                  95
Pro Glu Asp Asp Ile Cys Lys Tyr Asp Phe Val Gly Val Glu Glu Pro
            100                 105                 110
Ser Asp Gly Ser Val Leu Gly Arg Trp Cys Gly Ser Gly Thr Val Pro
        115                 120                 125
Xaa Lys Gln Thr Ser Lys Gly Asn His Ile Arg Ile Arg Phe Ala Ser
    130                 135                 140
Asp Glu Tyr Phe Pro Ser Glu Pro Gly Phe Cys Ile His Tyr Ser Ile

145                 150                 155                 160

Ile Met Pro Gln Val Thr Gly Thr Thr Ser Pro Xaa Val Leu Pro Pro
                165                 170                 175
Ser Ser Phe Val
            180

<210> 92
<211> 764
<212> DNA
<213> Mouse

<220>
<221> CDS
<222> (211)...(750)

<221> misc_feature
<222> (1)...(764)
<223> n = A,T,C or G

<400> 92
ccgcgtccgg ctccctgcga gtcgccacga ctcatcgccg ctcccccgcg tccccacccc      60
ttctttcctc cctcgcctac ccccaccccc cgcacttcgg cacagctcag gatttgttta     120
aaccttggga aactggttca ggtccaggtt ttgctttgat ccttttcaaa aactggagac     180
acagaagagg gctctaggaa aaacttttgg atg gga tta tgt gga aac tac cct      234
                                 Met Gly Leu Cys Gly Asn Tyr Pro
                                  1               5

gcg att ctc tgc tgc caa aga acg ggg act cgg gct gag tcc aac ctg      282
Ala Ile Leu Cys Cys Gln Arg Thr Gly Thr Arg Ala Glu Ser Asn Leu
    10                  15                  20

agc agc aag ttg cag ctc tcc agc gac aag gaa cag aac gga gtg caa      330
Ser Ser Lys Leu Gln Leu Ser Ser Asp Lys Glu Gln Asn Gly Val Gln
25                  30                  35                  40

gat ccc cgg cat gag aga gtt gtc act ata tct ggt aat ggg agc atc      378
Asp Pro Arg His Glu Arg Val Val Thr Ile Ser Gly Asn Gly Ser Ile
                45                  50                  55

cac agc ccg aag ttt cct cat aca tac cca aga aat atg gtg ctg gtg      426
His Ser Pro Lys Phe Pro His Thr Tyr Pro Arg Asn Met Val Leu Val
            60                  65                  70

tgg aga tta gtt gca gta gat gaa aat gtg cgg atc cag ctg aca ttt      474
Trp Arg Leu Val Ala Val Asp Glu Asn Val Arg Ile Gln Leu Thr Phe
        75                  80                  85

gat gag aga ttt ggg ctg gaa gat cca gaa gac gat ata tgc aag tat      522
Asp Glu Arg Phe Gly Leu Glu Asp Pro Glu Asp Asp Ile Cys Lys Tyr
    90                  95                  100

gat ttt gta gga gtt gag gag ccc agt gat gga agc gtt tta gga cgc      570
Asp Phe Val Gly Val Glu Glu Pro Ser Asp Gly Ser Val Leu Gly Arg
105                 110                 115                 120

tgg tgt ggt tct ggg act gtg cca nga aag cag act tct aaa gga aat      618
Trp Cys Gly Ser Gly Thr Val Pro Xaa Lys Gln Thr Ser Lys Gly Asn
                125                 130                 135

cat atc agg ata aga ttt gca tct gat gag tat ttt cca tct gaa ccc      666
His Ile Arg Ile Arg Phe Ala Ser Asp Glu Tyr Phe Pro Ser Glu Pro
            140                 145                 150

gga ttc tgc atc cac tac agt att atc atg cca caa gtc aca gna acc      714
Gly Phe Cys Ile His Tyr Ser Ile Ile Met Pro Gln Val Thr Xaa Thr
        155                 160                 165

acg agt cct ttg gtg ttg ccc cct tca tct ttt gtn nnttgggacc 760
Thr Ser Pro Leu Val Leu Pro Pro Ser Ser Phe Val
    170                 175                 180

tgct 764

<210> 93
<211> 180
<212> PRT
<213> Mouse

<220>
<221> VARIANT
<222> (1)...(180)
<223> Xaa = Any Amino Acid

<400> 93
Met Gly Leu Cys Gly Asn Tyr Pro Ala Ile Leu Cys Cys Gln Arg Thr
1               5                   10                  15
Gly Thr Arg Ala Glu Ser Asn Leu Ser Ser Lys Leu Gln Leu Ser Ser
            20                  25                  30
Asp Lys Glu Gln Asn Gly Val Gln Asp Pro Arg His Glu Arg Val Val
        35                  40                  45
Thr Ile Ser Gly Asn Gly Ser Ile His Ser Pro Lys Phe Pro His Thr
    50                  55                  60
Tyr Pro Arg Asn Met Val Leu Val Trp Arg Leu Val Ala Val Asp Glu
65                  70                  75                  80
Asn Val Arg Ile Gln Leu Thr Phe Asp Glu Arg Phe Gly Leu Glu Asp
                85                  90                  95
Pro Glu Asp Asp Ile Cys Lys Tyr Asp Phe Val Gly Val Glu Glu Pro
            100                 105                 110
Ser Asp Gly Ser Val Leu Gly Arg Trp Cys Gly Ser Gly Thr Val Pro
        115                 120                 125
Xaa Lys Gln Thr Ser Lys Gly Asn His Ile Arg Ile Arg Phe Ala Ser
    130                 135                 140
Asp Glu Tyr Phe Pro Ser Glu Pro Gly Phe Cys Ile His Tyr Ser Ile
145                 150                 155                 160
Ile Met Pro Gln Val Thr Xaa Thr Thr Ser Pro Leu Val Leu Pro Pro
                165                 170                 175
Ser Ser Phe Val
            180

<210> 94
<211> 764
<212> DNA
<213> Mouse

<220>
<221> CDS
<222> (211)...(750)

<221> misc_feature
<222> (1)...(764)
<223> n = A,T,C or G

<400> 94
   ccgcgtccgg ctccctgcga gtcgccacga ctcatcgccg ctcccccgcg tccccacccc 60
   ttctttcctc cctcgcctac ccccaccccc cgcacttcgg cacagctcag gatttgttta 120
   aaccttggga aactggttca ggtccaggtt ttgctttgat ccttttcaaa aactggagac 180
   acagaagagg gctctaggaa aaactttggg atg gga tta tgt gga aac tac cct 234
   Met Gly Leu Cys Gly Asn Tyr Pro
   1 5
   gcg att ctc tgc tgc caa aga acg ggg act cgg gct gag tcc aac ctg 282
   Ala Ile Leu Cys Cys Gln Arg Thr Gly Thr Arg Ala Glu Ser Asn Leu
   10 15 20
   agc agc aag ttg cag ctc tcc agc gac aag gaa cag aac gga gtg caa 330
   Ser Ser Lys Leu Gln Leu Ser Ser Asp Lys Glu Gln Asn Gly Val Gln
   25                  30                  35                  40
   gat ccc cgg cat gag aga gtt gtc act ata tct ggt aat ggg agc atc      378
   Asp Pro Arg His Glu Arg Val Val Thr Ile Ser Gly Asn Gly Ser Ile
                   45                  50                  55
   cac agc ccg aag ttt cct cat aca tac cca aga aat atg gtg ctg gtg      426
   His Ser Pro Lys Phe Pro His Thr Tyr Pro Arg Asn Met Val Leu Val
               60                  65                  70
   tgg aga tta gtt gca gta gat gaa aat gtg cgg atc cag ctg aca ttt      474
   Trp Arg Leu Val Ala Val Asp Glu Asn Val Arg Ile Gln Leu Thr Phe
           75                  80                  85
   gat gag aga ttt ggg ctg gaa gat cca gaa gac gat ata tgc aag tat      522
   Asp Glu Arg Phe Gly Leu Glu Asp Pro Glu Asp Asp Ile Cys Lys Tyr
       90                  95                  100
   gat ttt gta gga gtt gag gag ccc agt gat gga agc gtt tta gga cgc      570
   Asp Phe Val Gly Val Glu Glu Pro Ser Asp Gly Ser Val Leu Gly Arg
   105                 110                 115                 120
   tgg tgt ggt tct ggg act gtg cca nga aag cag act tct aaa gga aat      618
   Trp Cys Gly Ser Gly Thr Val Pro Xaa Lys Gln Thr Ser Lys Gly Asn
                   125                 130                 135
   cat atc agg ata aga ttt gca tct gat gag tat ttt cca tct gaa ccc      666
   His Ile Arg Ile Arg Phe Ala Ser Asp Glu Tyr Phe Pro Ser Glu Pro
               140                 145                 150
   gga ttc tgc atc cac tac agt att atc atg cca caa gtc aca gna acc      714
   Gly Phe Cys Ile His Tyr Ser Ile Ile Met Pro Gln Val Thr Xaa Thr
           155                 160                 165
   acg agt cct tcg gtg ttg ccc cct tca tct ttt gtn nnttgggacc           760
   Thr Ser Pro Ser Val Leu Pro Pro Ser Ser Phe Val
       170                 175                 180
   tgct                                                                 764

<210> 95
<211> 180
<212> PRT
<213> Mouse

<220>
<221> VARIANT
<222> (1)...(180)
<223> Xaa = Any Amino Acid

<400> 95
Met Gly Leu Cys Gly Asn Tyr Pro Ala Ile Leu Cys Cys Gln Arg Thr
1               5                   10                  15
Gly Thr Arg Ala Glu Ser Asn Leu Ser Ser Lys Leu Gln Leu Ser Ser
            20                  25                  30

Asp Lys Glu Gln Asn Gly Val Gln Asp Pro Arg His Glu Arg Val Val
        35                  40                  45
Thr Ile Ser Gly Asn Gly Ser Ile His Ser Pro Lys Phe Pro His Thr
    50                  55                  60
Tyr Pro Arg Asn Met Val Leu Val Trp Arg Leu Val Ala Val Asp Glu
65                  70                  75                  80
Asn Val Arg Ile Gln Leu Thr Phe Asp Glu Arg Phe Gly Leu Glu Asp
                85                  90                  95
Pro Glu Asp Asp Ile Cys Lys Tyr Asp Phe Val Gly Val Glu Glu Pro
            100                 105                 110
Ser Asp Gly Ser Val Leu Gly Arg Trp Cys Gly Ser Gly Thr Val Pro

Xaa Lys Gln Thr Ser Lys Gly Asn His Ile Arg Ile Arg Phe Ala Ser
        115                 120                 125
    130                 135                 140
Asp Glu Tyr Phe Pro Ser Glu Pro Gly Phe Cys Ile His Tyr Ser Ile
145                 150                 155                 160
Ile Met Pro Gln Val Thr Xaa Thr Thr Ser Pro Ser Val Leu Pro Pro
                165                 170                 175
Ser Ser Phe Val
            180

<210> 96
<211> 764
<212> DNA
<213> Mouse

<220>
<221> CDS
<222> (211)...(750)

<221> misc_feature
<222> (1)...(764)
<223> n = A,T,C or G

<400> 96
ccgcgtccgg ctccctgcga gtcgccacga ctcatcgccg ctcccccgcg tccccacccc      60
ttctttcctc cctcgcctac ccccaccccc cgcacttcgg cacagctcag gatttgttta     120
aaccttggga aactggttca ggtccaggtt ttgctttgat ccttttcaaa aactggagac     180
acagaagagg gctctaggaa aaacttttgg atg gga tta tgt gga aac tac cct      234
                                 Met Gly Leu Cys Gly Asn Tyr Pro
                                   1               5

gcg att ctc tgc tgc caa aga acg ggg act cgg gct gag tcc aac ctg      282
Ala Ile Leu Cys Cys Gln Arg Thr Gly Thr Arg Ala Glu Ser Asn Leu
     10                  15                  20

agc agc aag ttg cag ctc tcc agc gac aag gaa cag aac gga gtg caa      330
Ser Ser Lys Leu Gln Leu Ser Ser Asp Lys Glu Gln Asn Gly Val Gln
 25                  30                  35                  40

gat ccc cgg cat gag aga gtt gtc act ata tct ggt aat ggg agc atc      378
Asp Pro Arg His Glu Arg Val Val Thr Ile Ser Gly Asn Gly Ser Ile
                 45                  50                  55

cac agc ccg aag ttt cct cat aca tac cca aga aat atg gtg ctg gtg      426
His Ser Pro Lys Phe Pro His Thr Tyr Pro Arg Asn Met Val Leu Val
             60                  65                  70

tgg aga tta gtt gca gta gat gaa aat gtg cgg atc cag ctg aca ttt      474
Trp Arg Leu Val Ala Val Asp Glu Asn Val Arg Ile Gln Leu Thr Phe
         75                  80                  85

gat gag aga ttt ggg ctg gaa gat cca gaa gac gat ata tgc aag tat      522
Asp Glu Arg Phe Gly Leu Glu Asp Pro Glu Asp Asp Ile Cys Lys Tyr
     90                  95                 100

gat ttt gta gga gtt gag gag ccc agt gat gga agc gtt tta gga cgc      570
Asp Phe Val Gly Val Glu Glu Pro Ser Asp Gly Ser Val Leu Gly Arg
105                 110                 115                 120

tgg tgt ggt tct ggg act gtg cca nga aag cag act tct aaa gga aat      618
Trp Cys Gly Ser Gly Thr Val Pro Xaa Lys Gln Thr Ser Lys Gly Asn
                125                 130                 135

cat atc agg ata aga ttt gca tct gat gag tat ttt cca tct gaa ccc      666
His Ile Arg Ile Arg Phe Ala Ser Asp Glu Tyr Phe Pro Ser Glu Pro
            140                 145                 150

gga ttc tgc atc cac tac agt att atc atg cca caa gtc aca gna acc      714

Gly Phe Cys Ile His Tyr Ser Ile Ile Met Pro Gln Val Thr Xaa Thr
        155                 160                 165

acg agt cct tag gtg ttg ccc cct tca tct ttt gtn nnttgggacc          760
Thr Ser Pro  *  Val Leu Pro Pro Ser Ser Phe Val
    170                 175

tgct                                                                764

<210> 97
<211> 171
<212> PRT
<213> Mouse

<220>
<221> VARIANT
<222> (1)...(171)
<223> Xaa = Any Amino Acid

<400> 97
Met Gly Leu Cys Gly Asn Tyr Pro Ala Ile Leu Cys Cys Gln Arg Thr
 1               5                  10                  15
Gly Thr Arg Ala Glu Ser Asn Leu Ser Ser Lys Leu Gln Leu Ser Ser
            20                  25                  30
Asp Lys Glu Gln Asn Gly Val Gln Asp Pro Arg His Glu Arg Val Val
        35                  40                  45
Thr Ile Ser Gly Asn Gly Ser Ile His Ser Pro Lys Phe Pro His Thr
    50                  55                  60
Tyr Pro Arg Asn Met Val Leu Val Trp Arg Leu Val Ala Val Asp Glu
65                  70                  75                  80
Asn Val Arg Ile Gln Leu Thr Phe Asp Glu Arg Phe Gly Leu Glu Asp
                85                  90                  95
Pro Glu Asp Asp Ile Cys Lys Tyr Asp Phe Val Gly Val Glu Glu Pro
            100                 105                 110
Ser Asp Gly Ser Val Leu Gly Arg Trp Cys Gly Ser Gly Thr Val Pro
        115                 120                 125
Xaa Lys Gln Thr Ser Lys Gly Asn His Ile Arg Ile Arg Phe Ala Ser
    130                 135                 140
Asp Glu Tyr Phe Pro Ser Glu Pro Gly Phe Cys Ile His Tyr Ser Ile
145                 150                 155                 160

Ile Met Pro Gln Val Thr Xaa Thr Thr Ser Pro
                165                 170


<210> 98
<211> 764
<212> DNA
<213> Mouse

<220>
<221> CDS
<222> (211)...(750)

<221> misc_feature
<222> (1)...(764)
<223> n = A,T,C or G

<400> 98
ccgcgtccgg ctccctgcga gtcgccacga ctcatcgccg ctcccccgcg tccccacccc     60
ttctttcctc cctcgcctac ccccaccccc cgcacttcgg cacagctcag gatttgttta    120
aaccttggga aactggttca ggtccaggtt ttgctttgat ccttttcaaa aactggagac    180
acagaagagg gctctaggaa aaacttttgg atg gga tta tgt gga aac tac cct     234
                                 Met Gly Leu Cys Gly Asn Tyr Pro
                                  1               5

gcg att ctc tgc tgc caa aga acg ggg act cgg gct gag tcc aac ctg      282
Ala Ile Leu Cys Cys Gln Arg Thr Gly Thr Arg Ala Glu Ser Asn Leu

10                          15                          20

agc agc aag ttg cag ctc tcc agc gac aag gaa cag aac gga gtg caa          330
Ser Ser Lys Leu Gln Leu Ser Ser Asp Lys Glu Gln Asn Gly Val Gln
 25                      30                          35                      40

gat ccc cgg cat gag aga gtt gtc act ata tct ggt aat ggg agc atc          378
Asp Pro Arg His Glu Arg Val Val Thr Ile Ser Gly Asn Gly Ser Ile
                    45                          50                          55

cac agc ccg aag ttt cct cat aca tac cca aga aat atg gtg ctg gtg          426
His Ser Pro Lys Phe Pro His Thr Tyr Pro Arg Asn Met Val Leu Val
                60                          65                          70

tgg aga tta gtt gca gta gat gaa aat gtg cgg atc cag ctg aca ttt          474
Trp Arg Leu Val Ala Val Asp Glu Asn Val Arg Ile Gln Leu Thr Phe
            75                          80                          85

gat gag aga ttt ggg ctg gaa gat cca gaa gac gat ata tgc aag tat          522
Asp Glu Arg Phe Gly Leu Glu Asp Pro Glu Asp Asp Ile Cys Lys Tyr
        90                          95                          100

gat ttt gta gga gtt gag gag ccc agt gat gga agc gtt tta gga cgc          570
Asp Phe Val Gly Val Glu Glu Pro Ser Asp Gly Ser Val Leu Gly Arg
105                         110                         115                         120

tgg tgt ggt tct ggg act gtg cca nga aag cag act tct aaa gga aat          618
Trp Cys Gly Ser Gly Thr Val Pro Xaa Lys Gln Thr Ser Lys Gly Asn
                    125                         130                         135

cat atc agg ata aga ttt gca tct gat gag tat ttt cca tct gaa ccc          666
His Ile Arg Ile Arg Phe Ala Ser Asp Glu Tyr Phe Pro Ser Glu Pro
                140                         145                         150

gga ttc tgc atc cac tac agt att atc atg cca caa gtc aca gna acc          714
Gly Phe Cys Ile His Tyr Ser Ile Ile Met Pro Gln Val Thr Xaa Thr
            155                         160                         165

acg agt cct tgg gtg ttg ccc cct tca tct ttt gtn nnttgggacc               760
Thr Ser Pro Trp Val Leu Pro Pro Ser Ser Phe Val
        170                         175                         180

tgct                                                                     764

<210> 99
<211> 180
<212> PRT
<213> Mouse

<220>
<221> VARIANT
<222> (1)...(180)
<223> Xaa = Any Amino Acid

<400> 99
Met Gly Leu Cys Gly Asn Tyr Pro Ala Ile Leu Cys Cys Gln Arg Thr
 1               5                   10                  15
Gly Thr Arg Ala Glu Ser Asn Leu Ser Ser Lys Leu Gln Leu Ser Ser
            20                  25                  30
Asp Lys Glu Gln Asn Gly Val Gln Asp Pro Arg His Glu Arg Val Val
        35                  40                  45
Thr Ile Ser Gly Asn Gly Ser Ile His Ser Pro Lys Phe Pro His Thr
    50                  55                  60
Tyr Pro Arg Asn Met Val Leu Val Trp Arg Leu Val Ala Val Asp Glu
65                  70                  75                  80
Asn Val Arg Ile Gln Leu Thr Phe Asp Glu Arg Phe Gly Leu Glu Asp

85                  90                  95
Pro Glu Asp Asp Ile Cys Lys Tyr Asp Phe Val Gly Val Glu Glu Pro
            100                 105                 110
Ser Asp Gly Ser Val Leu Gly Arg Trp Cys Gly Ser Gly Thr Val Pro
        115                 120                 125
Xaa Lys Gln Thr Ser Lys Gly Asn His Ile Arg Ile Arg Phe Ala Ser
    130                 135                 140
Asp Glu Tyr Phe Pro Ser Glu Pro Gly Phe Cys Ile His Tyr Ser Ile
145                 150                 155                 160
Ile Met Pro Gln Val Thr Xaa Thr Thr Ser Pro Trp Val Leu Pro Pro
                165                 170                 175
Ser Ser Phe Val
            180

<210> 100
<211> 39
<212> PRT
<213> Homo sapiens

<400> 100
Cys Gln Glu Asn Glu Tyr Trp Asp Gln Trp Gly Arg Cys Val Thr Cys
1               5                   10                  15
Gln Arg Cys Gly Pro Gly Gln Glu Leu Ser Lys Asp Cys Gly Tyr Gly
            20                  25                  30
Glu Gly Gly Asp Ala Tyr Cys
        35

<210> 101
<211> 40
<212> PRT
<213> Homo sapiens

<400> 101
Cys Pro Pro Arg Arg Tyr Lys Ser Ser Trp Gly His His Lys Cys Gln
1               5                   10                  15
Ser Cys Ile Thr Cys Ala Val Ile Asn Arg Val Gln Lys Val Asn Cys
            20                  25                  30
Thr Ala Thr Ser Asn Ala Val Cys
        35                  40

<210> 102
<211> 39
<212> PRT
<213> Homo sapiens

<400> 102
Cys Gln Glu Asn Glu Tyr Trp Asp Gln Trp Gly Arg Cys Val Thr Cys
1               5                   10                  15
Gln Arg Cys Gly Pro Gly Gln Glu Leu Ser Lys Asp Cys Gly Tyr Gly
            20                  25                  30
Glu Gly Gly Asp Ala Tyr Cys
        35

<210> 103
<211> 40
<212> PRT
<213> Homo sapiens

<400> 103
Cys Pro Pro Arg Arg Tyr Lys Ser Ser Trp Gly His His Lys Cys Gln
1               5                   10                  15
Ser Cys Ile Thr Cys Ala Val Ile Asn Arg Val Gln Lys Val Asn Cys
            20                  25                  30
Thr Ala Thr Ser Asn Ala Val Cys
        35                  40

<210> 104

<211> 22
<212> PRT
<213> Homo sapiens

<400> 104
Asp Leu Tyr Phe Ile Leu Asp Lys Ser Gly Ser Val Leu His His Trp
1               5                   10                  15
Asn Glu Ile Tyr Tyr Phe
            20

<210> 105
<211> 172
<212> PRT
<213> Homo sapiens

<400> 105
Asp Leu Tyr Phe Ile Leu Asp Lys Ser Gly Ser Val Leu His His Trp
1               5                   10                  15
Asn Glu Ile Tyr Tyr Phe Val Glu Gln Leu Ala His Lys Phe Ile Ser
            20                  25                  30
Pro Gln Leu Arg Met Ser Phe Ile Val Phe Ser Thr Arg Gly Thr Thr
        35                  40                  45
Leu Met Lys Leu Thr Glu Asp Arg Glu Gln Ile Arg Gln Gly Leu Glu
    50                  55                  60
Glu Leu Gln Lys Val Leu Pro Gly Gly Asp Thr Tyr Met His Glu Gly
65                  70                  75                  80

Phe Glu Arg Ala Ser Glu Gln Ile Tyr Tyr Glu Asn Arg Gln Gly Tyr
                85                  90                  95
Arg Thr Ala Ser Val Ile Ile Ala Leu Thr Asp Gly Glu Leu His Glu
            100                 105                 110
Asp Leu Phe Phe Tyr Ser Glu Arg Glu Ala Asn Arg Ser Arg Asp Leu
        115                 120                 125
Gly Ala Ile Val Tyr Cys Val Gly Val Lys Asp Phe Asn Glu Thr Gln
    130                 135                 140
Leu Ala Arg Ile Ala Asp Ser Lys Asp His Val Phe Pro Val Asn Asp
145                 150                 155                 160
Gly Phe Gln Ala Leu Gln Gly Ile Ile His Ser Ile
                165                 170

<210> 106
<211> 12
<212> PRT
<213> Homo sapiens

<400> 106
Cys Arg Cys Phe Pro Gly Tyr Thr Gly Lys Thr Cys
1               5                   10

<210> 107
<211> 17
<212> PRT
<213> Homo sapiens

<400> 107
Cys Leu Cys Pro Ser Ser Gly Leu Arg Leu Ala Pro Asn Gly Arg Asp
1               5                   10                  15
Cys


<210> 108
<211> 18
<212> PRT
<213> Homo sapiens

<400> 108
Cys Lys Cys His Ile Gly Phe Glu Leu Gln Tyr Ile Ser Gly Arg Tyr
1               5                   10                  15

Asp Cys

<210> 109
<211> 14
<212> PRT
<213> Homo sapiens

<400> 109
Cys Lys Cys Lys Gln Gly Tyr Lys Gly Asn Gly Leu Arg Cys
1               5                   10

<210> 110
<211> 31
<212> PRT
<213> Homo sapiens

<400> 110
Cys Glu Ala Thr Cys Glu Pro Gly Cys Lys Phe Gly Glu Cys Val Gly
1               5                   10                  15

Pro Asn Lys Cys Arg Cys Phe Pro Gly Tyr Thr Gly Lys Thr Cys
            20                  25                  30

<210> 111
<211> 35
<212> PRT
<213> Homo sapiens

<400> 111
Cys Gly Met Lys Pro Arg Pro Cys Gln His Arg Cys Val Asn Thr His
1               5                   10                  15
Gly Ser Tyr Lys Cys Phe Cys Leu Ser Gly His Met Leu Met Pro Asp
            20                  25                  30
Ala Thr Cys
        35

<210> 112
<211> 35
<212> PRT
<213> Homo sapiens

<400> 112
Cys Ala Met Ile Asn Cys Gln Tyr Ser Cys Glu Asp Thr Glu Glu Gly
1               5                   10                  15
Pro Gln Cys Leu Cys Pro Ser Ser Gly Leu Arg Leu Ala Pro Asn Gly
            20                  25                  30
Arg Asp Cys
        35

<210> 113
<211> 40
<212> PRT
<213> Homo sapiens

<400> 113
Cys Ala Ser Gly Lys Val Ile Cys Pro Tyr Asn Arg Arg Cys Val Asn
1               5                   10                  15
Thr Phe Gly Ser Tyr Tyr Cys Lys Cys His Ile Gly Phe Glu Leu Gln
            20                  25                  30
Tyr Ile Ser Gly Arg Tyr Asp Cys
        35                  40

<210> 114
<211> 36
<212> PRT
<213> Homo sapiens

<400> 114
Cys Thr Met Asp Ser His Thr Cys Ser His His Ala Asn Cys Phe Asn
1               5                   10                  15
Thr Gln Gly Ser Phe Lys Cys Lys Cys Lys Gln Gly Tyr Lys Gly Asn
            20                  25                  30
Gly Leu Arg Cys
        35

<210> 115
<211> 42
<212> PRT
<213> Homo sapiens

<400> 115
Gly Phe Tyr Met Ala Val Pro Ala Leu Ala Gly His Lys Lys Asp Ile
1               5                   10                  15
Gly Arg Leu Lys Leu Leu Leu Pro Asp Leu Gln Pro Gln Ser Asn Phe
            20                  25                  30
Cys Leu Leu Phe Asp Tyr Arg Leu Ala Gly
        35                  40

<210> 116
<211> 147
<212> PRT
<213> Homo sapiens

<400> 116
Val Asp Cys Ser Phe Asn His Gly Ile Cys Asp Trp Lys Gln Asp Arg
1               5                   10                  15
Glu Asp Asp Phe Asp Trp Asn Pro Ala Asp Arg Asp Asn Ala Ile Gly
            20                  25                  30
Phe Tyr Met Ala Val Pro Ala Leu Ala Gly His Lys Lys Asp Ile Gly
        35                  40                  45
Arg Leu Lys Leu Leu Leu Pro Asp Leu Gln Pro Gln Ser Asn Phe Cys
    50                  55                  60
Leu Leu Phe Asp Tyr Arg Leu Ala Gly Asp Lys Val Gly Lys Leu Arg
65                  70                  75                  80
Val Phe Val Lys Asn Ser Asn Asn Ala Leu Ala Trp Glu Lys Thr Thr
                85                  90                  95
Ser Glu Asp Glu Lys Trp Lys Thr Gly Lys Ile Gln Leu Tyr Gln Gly
            100                 105                 110
Thr Asp Ala Thr Lys Ser Ile Ile Phe Glu Ala Glu Arg Gly Lys Gly
        115                 120                 125
Lys Thr Gly Glu Ile Ala Val Asp Gly Val Leu Leu Val Ser Gly Leu
    130                 135                 140
Cys Pro Asp
145

<210> 117
<211> 40
<212> PRT
<213> Homo sapiens

<400> 117
Trp Ser Glu Cys Ser Arg Thr Cys Gly Gly Gly Ala Ser Tyr Ser Leu
1               5                   10                  15
Arg Arg Cys Leu Ser Ser Lys Ser Cys Glu Gly Arg Asn Ile Arg Tyr
            20                  25                  30
Arg Thr Cys Ser Asn Val Asp Cys
        35                  40

<210> 118
<211> 45
<212> PRT
<213> Homo sapiens

<400> 118
Asp Ala Trp Gly Pro Trp Ser Glu Cys Ser Arg Thr Cys Gly Gly Gly
1               5                   10                  15
Ala Ser Tyr Ser Leu Arg Arg Cys Leu Ser Ser Lys Ser Cys Glu Gly
            20                  25                  30
Arg Asn Ile Arg Tyr Arg Thr Cys Ser Asn Val Asp Cys
        35                  40                  45

<210> 119
<211> 41
<212> PRT
<213> Homo sapiens

<400> 119
Gly His Tyr Ile Tyr Val Asp Thr Ser Phe Gly Lys Gln Gly Glu Lys
1               5                   10                  15
Ala Val Leu Leu Ser Pro Asp Leu Gln Ala Glu Glu Trp Ser Cys Leu
            20                  25                  30
Arg Leu Val Tyr Gln Ile Thr Thr Ser
        35                  40

<210> 120
<211> 42
<212> PRT
<213> Homo sapiens

<400> 120
Gly His Tyr Met Tyr Val Asp Ser Val Tyr Val Lys His Phe Gln Glu
1               5                   10                  15
Val Ala Gln Leu Ile Ser Pro Leu Thr Thr Ala Pro Met Ala Gly Cys
            20                  25                  30
Leu Ser Phe Tyr Tyr Gln Ile Gln Gln Gly
        35                  40

<210> 121
<211> 41
<212> PRT
<213> Homo sapiens

<400> 121
Gly Tyr Tyr Leu Leu Ala Asn Thr Lys Phe Thr Ser Gln Pro Gly Tyr
1               5                   10                  15
Ile Gly Arg Leu Tyr Gly Pro Ser Leu Pro Gly Asn Leu Gln Tyr Cys
            20                  25                  30
Leu Arg Phe His Tyr Ala Ile Tyr Gly
        35                  40

<210> 122
<211> 144
<212> PRT
<213> Homo sapiens

<400> 122
Cys Ala Phe Glu Glu Ser Thr Cys Gly Phe Asp Ser Val Leu Ala Ser
1               5                   10                  15
Leu Pro Trp Ile Leu Asn Glu Glu Gly His Tyr Ile Tyr Val Asp Thr
            20                  25                  30
Ser Phe Gly Lys Gln Gly Glu Lys Ala Val Leu Leu Ser Pro Asp Leu
        35                  40                  45
Gln Ala Glu Glu Trp Ser Cys Leu Arg Leu Val Tyr Gln Ile Thr Thr
    50                  55                  60
Ser Ser Glu Ser Leu Ser Asp Pro Ser Gln Leu Asn Leu Tyr Met Arg
65                  70                  75                  80
Phe Glu Asp Glu Ser Phe Asp Arg Leu Leu Trp Ser Ala Lys Glu Pro
                85                  90                  95
Ser Asp Ser Trp Leu Ile Ala Ser Leu Asp Leu Gln Asn Ser Ser Lys
            100                 105                 110

Lys Phe Lys Ile Leu Ile Glu Gly Val Leu Gly Gln Gly Asn Thr Ala
        115                 120                 125

Ser Ile Ala Leu Phe Glu Ile Lys Met Thr Thr Gly Tyr Cys Ile Glu
    130                 135                 140

<210> 123
<211> 160
<212> PRT
<213> Homo sapiens

<400> 123
Cys Asp Phe Glu Glu Asn His Leu Cys Gly Phe Val Asn Arg Trp Asn
 1               5                  10                  15
Pro Asn Val Asn Trp Phe Val Gly Gly Gly Ser Ile Arg Asn Val His
            20                  25                  30
Ser Ile Leu Pro Gln Asp His Thr Phe Lys Ser Glu Leu Gly His Tyr
        35                  40                  45
Met Tyr Val Asp Ser Val Tyr Val Lys His Phe Gln Glu Val Ala Gln
    50                  55                  60
Leu Ile Ser Pro Leu Thr Thr Ala Pro Met Ala Gly Cys Leu Ser Phe
65                  70                  75                  80
Tyr Tyr Gln Ile Gln Gln Gly Asn Asp Asn Val Phe Ser Leu Tyr Thr
                85                  90                  95
Arg Asp Val Ala Gly Leu Tyr Glu Glu Ile Trp Lys Ala Asp Arg Pro
            100                 105                 110
Gly Asn Ala Ala Trp Asn Leu Ala Glu Val Glu Phe Asn Ala Pro Tyr
        115                 120                 125
Pro Met Glu Val Ile Phe Glu Val Ala Phe Asn Gly Pro Lys Gly Gly
    130                 135                 140
Tyr Val Ala Leu Asp Asp Ile Ser Phe Ser Pro Val His Cys Gln Asn
145                 150                 155                 160

<210> 124
<211> 157
<212> PRT
<213> Homo sapiens

<400> 124
Cys Asn Phe Glu Gln Asp Leu Cys Asn Phe Tyr Gln Asp Lys Glu Gly
 1               5                  10                  15
Pro Gly Trp Thr Arg Val Lys Val Lys Pro Asn Met Tyr Arg Ala Gly
            20                  25                  30
Asp His Thr Thr Gly Leu Gly Tyr Tyr Leu Leu Ala Asn Thr Lys Phe
        35                  40                  45
Thr Ser Gln Pro Gly Tyr Ile Gly Arg Leu Tyr Gly Pro Ser Leu Pro
    50                  55                  60
Gly Asn Leu Gln Tyr Cys Leu Arg Phe His Tyr Ala Ile Tyr Gly Phe
65                  70                  75                  80
Leu Lys Met Ser Asp Thr Leu Ala Val Tyr Ile Phe Glu Glu Asn His
                85                  90                  95
Val Val Gln Glu Lys Ile Trp Ser Val Leu Glu Ser Pro Arg Gly Val
            100                 105                 110
Trp Met Gln Ala Glu Ile Thr Phe Lys Lys Pro Met Pro Thr Lys Val
        115                 120                 125
Val Phe Met Ser Leu Cys Lys Ser Phe Trp Asp Cys Gly Leu Val Ala
    130                 135                 140
Leu Asp Asp Ile Thr Ile Gln Leu Gly Ser Cys Ser Ser
145                 150                 155

<210> 125
<211> 18
<212> PRT
<213> Homo sapiens

<400> 125
Met Leu Leu Arg Gly Val Leu Leu Ala Leu Gln Ala Leu Gln Leu Ala
 1               5                  10                  15

Gly Ala

<210> 126
<211> 668
<212> PRT
<213> Homo sapiens

<400> 126
Leu Asp Leu Pro Ala Gly Ser Cys Ala Phe Glu Glu Ser Thr Cys Gly
1               5                   10                  15
Phe Asp Ser Val Leu Ala Ser Leu Pro Trp Ile Leu Asn Glu Glu Gly
            20                  25                  30
His Tyr Ile Tyr Val Asp Thr Ser Phe Gly Lys Gln Gly Glu Lys Ala
        35                  40                  45
Val Leu Leu Ser Pro Asp Leu Gln Ala Glu Glu Trp Ser Cys Leu Arg
    50                  55                  60
Leu Val Tyr Gln Ile Thr Thr Ser Ser Glu Ser Leu Ser Asp Pro Ser
65                  70                  75                  80
Gln Leu Asn Leu Tyr Met Arg Phe Glu Asp Glu Ser Phe Asp Arg Leu
                85                  90                  95
Leu Trp Ser Ala Lys Glu Pro Ser Asp Ser Trp Leu Ile Ala Ser Leu
            100                 105                 110
Asp Leu Gln Asn Ser Ser Lys Lys Phe Lys Ile Leu Ile Glu Gly Val
        115                 120                 125
Leu Gly Gln Gly Asn Thr Ala Ser Ile Ala Leu Phe Glu Ile Lys Met
    130                 135                 140
Thr Thr Gly Tyr Cys Ile Glu Cys Asp Phe Glu Glu Asn His Leu Cys
145                 150                 155                 160
Gly Phe Val Asn Arg Trp Asn Pro Asn Val Asn Trp Phe Val Gly Gly
                165                 170                 175
Gly Ser Ile Arg Asn Val His Ser Ile Leu Pro Gln Asp His Thr Phe
            180                 185                 190
Lys Ser Glu Leu Gly His Tyr Met Tyr Val Asp Ser Val Tyr Val Lys
        195                 200                 205
His Phe Gln Glu Val Ala Gln Leu Ile Ser Pro Leu Thr Thr Ala Pro
    210                 215                 220
Met Ala Gly Cys Leu Ser Phe Tyr Tyr Gln Ile Gln Gln Gly Asn Asp
225                 230                 235                 240
Asn Val Phe Ser Leu Tyr Thr Arg Asp Val Ala Gly Leu Tyr Glu Glu
                245                 250                 255
Ile Trp Lys Ala Asp Arg Pro Gly Asn Ala Ala Trp Asn Leu Ala Glu
            260                 265                 270
Val Glu Phe Asn Ala Pro Tyr Pro Met Glu Val Ile Phe Glu Val Ala
        275                 280                 285
Phe Asn Gly Pro Lys Gly Gly Tyr Val Ala Leu Asp Asp Ile Ser Phe
    290                 295                 300
Ser Pro Val His Cys Gln Asn Gln Thr Glu Leu Leu Phe Ser Ala Val
305                 310                 315                 320
Glu Ala Ser Cys Asn Phe Glu Gln Asp Leu Cys Asn Phe Tyr Gln Asp
                325                 330                 335
Lys Glu Gly Pro Gly Trp Thr Arg Val Lys Val Lys Pro Asn Met Tyr
            340                 345                 350
Arg Ala Gly Asp His Thr Thr Gly Leu Gly Tyr Tyr Leu Leu Ala Asn
        355                 360                 365
Thr Lys Phe Thr Ser Gln Pro Gly Tyr Ile Gly Arg Leu Tyr Gly Pro
    370                 375                 380
Ser Leu Pro Gly Asn Leu Gln Tyr Cys Leu Arg Phe His Tyr Ala Ile
385                 390                 395                 400
Tyr Gly Phe Leu Lys Met Ser Asp Thr Leu Ala Val Tyr Ile Phe Glu
                405                 410                 415
Glu Asn His Val Val Gln Glu Lys Ile Trp Ser Val Leu Glu Ser Pro
            420                 425                 430
Arg Gly Val Trp Met Gln Ala Glu Ile Thr Phe Lys Lys Pro Met Pro
        435                 440                 445
Thr Lys Val Val Phe Met Ser Leu Cys Lys Ser Phe Trp Asp Cys Gly
    450                 455                 460
Leu Val Ala Leu Asp Asp Ile Thr Ile Gln Leu Gly Ser Cys Ser Ser

465                 470                 475                 480
Ser Glu Lys Leu Pro Pro Ser Pro Gly Glu Cys Thr Phe Glu Gln Asp
                485                 490                 495
Glu Cys Thr Phe Thr Gln Glu Lys Arg Asn Arg Ser Ser Trp His Arg
            500                 505                 510
Arg Arg Gly Glu Thr Pro Thr Ser Tyr Thr Gly Pro Lys Gly Asp His
        515                 520                 525
Thr Thr Gly Val Gly Tyr Tyr Met Tyr Ile Glu Ala Ser His Met Val
    530                 535                 540
Tyr Gly Gln Lys Ala Arg Leu Leu Ser Arg Pro Leu Arg Gly Val Ser
545                 550                 555                 560
Gly Lys His Cys Leu Thr Phe Phe Tyr His Met Tyr Gly Gly Gly Thr
                565                 570                 575
Gly Leu Leu Ser Val Tyr Leu Lys Lys Glu Glu Asp Ser Glu Glu Ser
            580                 585                 590
Leu Leu Trp Arg Arg Arg Gly Glu Gln Ser Ile Ser Trp Leu Arg Ala
        595                 600                 605
Leu Ile Glu Tyr Ser Cys Glu Arg Gln His Gln Ile Ile Phe Glu Ala
    610                 615                 620
Ile Arg Gly Val Ser Ile Arg Ser Asp Ile Ala Ile Asp Asp Val Lys
625                 630                 635                 640
Phe Gln Ala Gly Pro Cys Gly Glu Met Glu Asp Thr Thr Gln Gln Ser
                645                 650                 655
Ser Gly Tyr Ser Glu Asp Leu Asn Glu Ile Glu Tyr
            660                 665

<210> 127
<211> 2004
<212> DNA
<213> Homo sapiens

<400> 127
ctcgacctgc ccgctgggtc ctgtgccttt gaagagagca cttgcggctt tgactccgtg      60
ttggcctctc tgccgtggat tttaaatgag gaaggccatt acatttatgt ggatacctcc     120
tttggcaagc agggggagaa agctgtgctg ctaagtcctg acttacaggc tgaggaatgg     180
agctgcctcc gtttggtcta ccagataacc acatcttcgg agtctctgtc agatcccagc     240
cagctgaacc tctacatgag atttgaagat gaaagctttg atcgcttgct ttggtcagct     300
aaggaacctt cagacagctg gctcatagcc agcttggatt tgcaaaacag ttccaagaaa     360
ttcaagattt taatagaagg tgtactagga cagggaaaca cagccagcat cgcactattt     420
gaaatcaaga tgacaaccgg ctactgtatt gaatgtgact ttgaagaaaa tcatctctgt     480
ggctttgtga accgctggaa tcccaatgtg aactggtttg ttggaggagg aagtattcgg     540
aatgtccact ccattctccc acaggatcac accttcaaga gtgaactggg ccactacatg     600
tacgtggact cagtttatgt gaagcacttc caggaggtgg cacagctcat ctccccgttg     660
accacggccc ccatggctgg ctgcctgtca ttttattacc agatccagca ggggaatgac     720
aatgtctttt ccctttacac tcgggatgtg gctggccttt acgaggaaat ctggaaagca     780
gacaggccag ggaatgctgc ctggaacctt gcggaggtcg agttcaatgc tccttacccc     840
atggaggtta tttttgaagt tgctttcaat ggtcccaagg gaggttatgt tgccctggat     900
gatatttcat tctctcctgt tcactgccag aatcagacag aacttctgtt cagtgccgtg     960
gaagccagct gcaattttga gcaagatctc tgcaactttt accaagataa agaaggtcca    1020
ggttggaccc gagtgaaagt aaaaccaaac atgtatcggg ctggagacca cactacaggc    1080
ttagggtatt acctgctagc caacacaaag ttcacatctc agcctggcta cattggaagg    1140
ctctatgggc cctccctacc aggaaacttg cagtattgtc tgcgttttca ttatgccatc    1200
tatggatttt taaaaatgag tgacacccta gcagtttaca tctttgaaga gaaccatgtg    1260
gttcaagaga agatctggtc tgtgttggag tccccaaggg gtgtttggat gcaagctgaa    1320
atcaccttta agaagcccat gcctaccaag gtggttttca tgagcctatg caaaagtttc    1380
tgggactgtg ggcttgtagc cctggatgac attacaatac aattgggaag ctgctcatct    1440
tcagagaaac ttccaccctc acctggagag tgtactttcg agcaagatga atgtacattt    1500
actcaggaga aaagaaaccg gagcagctgg cacaggagga ggggagaaac tcccacttcc    1560
tacacaggac caaagggaga tcacactact ggggtaggct actacatgta cattgaggcc    1620
tcccatatgg tgtatggaca aaaagcacgc ctcttgtcca ggcctctgcg aggagtctct    1680
ggaaaacact gcttgacctt tttctaccac atgtatggag gggcactggc cctgctgagt    1740
gtttatctga aaaaggaaga agacagtgaa gagtccctct tatggaggag aagaggtgaa    1800
cagagcattt cctggctacg agcactgatt gaatacagct gtgagaggca acaccagata    1860
atttttgaag ccattcgagg agtatcaata agaagtgata ttgccattga tgatgttaaa    1920
tttcaggcag gaccctgtgg agaaatggaa gatacaactc aacaatcatc aggatattct    1980
gaggacttaa atgaaattga gtat                                          2004

<210> 128

<211> 158
<212> PRT
<213> Homo sapiens

<400> 128
Cys Thr Phe Glu Gln Asp Glu Cys Thr Phe Thr Gln Glu Lys Arg Asn
1               5                   10                  15
Arg Ser Ser Trp His Arg Arg Arg Gly Glu Thr Pro Thr Ser Tyr Thr
            20                  25                  30
Gly Pro Lys Gly Asp His Thr Thr Gly Val Gly Tyr Tyr Met Tyr Ile
        35                  40                  45
Glu Ala Ser His Met Val Tyr Gly Gln Lys Ala Arg Leu Leu Ser Arg
    50                  55                  60
Pro Leu Arg Gly Val Ser Gly Lys His Cys Leu Thr Phe Phe Tyr His
65                  70                  75                  80
Met Tyr Gly Gly Gly Thr Gly Leu Leu Ser Val Tyr Leu Lys Lys Glu
                85                  90                  95
Glu Asp Ser Glu Glu Ser Leu Leu Trp Arg Arg Arg Gly Glu Gln Ser
            100                 105                 110
Ile Ser Trp Leu Arg Ala Leu Ile Glu Tyr Ser Cys Glu Arg Gln His
        115                 120                 125
Gln Ile Ile Phe Glu Ala Ile Arg Gly Val Ser Ile Arg Ser Asp Ile
    130                 135                 140
Ala Ile Asp Asp Val Lys Phe Gln Ala Gly Pro Cys Gly Glu
145                 150                 155

<210> 129
<211> 123
<212> PRT
<213> Homo sapiens

<400> 129
Ser Arg Glu Met Asn Glu Thr Val Ile Leu Ala Asp Glu Leu Cys Arg
1               5                   10                  15
Gln Pro Lys Pro Ser Thr Val Gln Ala Cys Asn Arg Phe Asn Cys Pro
            20                  25                  30
Pro Ala Trp Tyr Pro Ala Gln Trp Gln Pro Cys Ser Arg Thr Cys Gly
        35                  40                  45
Gly Gly Val Gln Lys Arg Glu Val Leu Cys Lys Gln Arg Met Ala Asp
    50                  55                  60
Gly Ser Phe Leu Glu Leu Pro Glu Thr Phe Cys Ser Ala Ser Lys Pro
65                  70                  75                  80
Ala Cys Gln Gln Ala Cys Lys Lys Asp Asp Cys Pro Ser Glu Trp Leu
                85                  90                  95
Leu Ser Asp Trp Thr Glu Cys Ser Thr Ser Cys Gly Glu Gly Thr Gln
            100                 105                 110

Thr Arg Ser Ala Ile Cys Arg Lys Met Leu Lys
        115                 120

<210> 130
<211> 18
<212> PRT
<213> Homo sapiens

<400> 130
Gly Leu Ser Phe Ile Ser Ser Ser Val Ile Ile Thr Thr Thr His Cys
1               5                   10                  15
Ser Leu


<210> 131
<211> 14
<212> PRT
<213> Homo sapiens

<400> 131
His Lys Ile Ala Ser Gly Pro Thr Thr Ala Ala Cys Met Glu

1                   5                   10

<210> 132
<211> 301
<212> PRT
<213> Homo sapiens

<400> 132
Met Ala Thr Ala Glu Arg Arg Ala Leu Gly Ile Gly Phe Gln Trp Leu
1               5                   10                  15
Ser Leu Ala Thr Leu Val Leu Ile Cys Ala Gly Gln Gly Gly Arg Arg
            20                  25                  30
Glu Asp Gly Gly Pro Ala Cys Tyr Gly Gly Phe Asp Leu Tyr Phe Ile
        35                  40                  45
Leu Asp Lys Ser Gly Ser Val Leu His His Trp Asn Glu Ile Tyr Tyr
    50                  55                  60
Phe Val Glu Gln Leu Ala His Lys Phe Ile Ser Pro Gln Leu Arg Met
65                  70                  75                  80
Ser Phe Ile Val Phe Ser Thr Arg Gly Thr Thr Leu Met Lys Leu Thr
                85                  90                  95
Glu Asp Arg Glu Gln Ile Arg Gln Gly Leu Glu Glu Leu Gln Lys Val
            100                 105                 110
Leu Pro Gly Gly Asp Thr Tyr Met His Glu Gly Phe Glu Arg Ala Ser
        115                 120                 125
Glu Gln Ile Tyr Tyr Glu Asn Arg Gln Gly Tyr Arg Thr Ala Ser Val
    130                 135                 140
Ile Ile Ala Leu Thr Asp Gly Glu Leu His Glu Asp Leu Phe Phe Tyr
145                 150                 155                 160
Ser Glu Arg Glu Ala Asn Arg Ser Arg Asp Leu Gly Ala Ile Val Tyr
                165                 170                 175
Cys Val Gly Val Lys Asp Phe Asn Glu Thr Gln Leu Ala Arg Ile Ala
            180                 185                 190
Asp Ser Lys Asp His Val Phe Pro Val Asn Asp Gly Phe Gln Ala Leu
        195                 200                 205
Gln Gly Ile Ile His Ser Ile Leu Lys Lys Ser Cys Ile Glu Ile Leu
    210                 215                 220
Ala Ala Glu Pro Ser Thr Ile Cys Ala Gly Glu Ser Phe Gln Val Val
225                 230                 235                 240
Val Arg Gly Asn Gly Phe Arg His Ala Arg Asn Val Asp Arg Val Leu
                245                 250                 255
Cys Ser Phe Lys Ile Asn Asp Ser Val Thr Leu Asn Glu Lys Pro Phe
            260                 265                 270
Ser Val Glu Asp Thr Tyr Leu Leu Cys Pro Ala Pro Ile Leu Lys Glu
        275                 280                 285
Val Gly Met Lys Ala Ala Leu Gln Val Ser Met Asn Asp
    290                 295                 300

<210> 133
<211> 221
<212> PRT
<213> Mouse

<400> 133
Pro Leu Cys Cys Thr Val Ile Ile Lys Glu Val Pro Pro Pro Pro Val
1               5                   10                  15
Glu Glu Ser Glu Glu Glu Asp Asp Asp Gly Leu Pro Lys Lys Lys Trp
            20                  25                  30
Pro Thr Val Asp Ala Ser Tyr Tyr Gly Gly Arg Gly Val Gly Gly Ile
        35                  40                  45
Lys Arg Met Glu Val Arg Trp Gly Glu Lys Gly Ser Thr Glu Glu Gly
    50                  55                  60
Ala Lys Leu Glu Lys Ala Lys Asn Ala Arg Val Lys Met Pro Glu Gln
65                  70                  75                  80
Glu Tyr Glu Phe Pro Glu Pro Arg Asn Leu Asn Asn Asn Met Arg Arg
                85                  90                  95
Pro Ser Ser Pro Arg Lys Trp Tyr Ser Pro Ile Lys Gly Lys Leu Asp
            100                 105                 110

Ala Leu Trp Val Leu Leu Arg Lys Gly Tyr Asp Arg Val Ser Val Met
        115                 120                 125
Arg Pro Gln Pro Gly Asp Thr Gly Arg Cys Ile Asn Phe Thr Arg Val
    130                 135                 140
Lys Asn Ser Gln Pro Ala Lys Tyr Pro Leu Asn Asn Thr Tyr His Pro
145                 150                 155                 160
Ser Ser Pro Pro Pro Ala Pro Ile Tyr Thr Pro Pro Pro Pro Ala Pro
                165                 170                 175
His Cys Pro Pro Pro Ala Pro Ser Ala Pro Thr Pro Pro Ile Pro Ser
            180                 185                 190
Pro Pro Ser Thr Leu Pro Pro Pro Pro Gln Ala Pro Pro Pro Asn Arg
        195                 200                 205
Ala Pro Pro Pro Ser Arg Pro Pro Pro Arg Pro Ser Val
    210                 215                 220

<210> 134
<211> 22
<212> PRT
<213> Mouse

<400> 134
Ile Leu Ala Ile Ala Leu Leu Val Leu Phe Leu Leu Leu Ala Leu Ala
1               5                   10                  15
Leu Leu Trp Trp Phe Trp
            20

<210> 135
<211> 138
<212> PRT
<213> Mouse

<400> 135
Ala Ser Ala Gln Leu Ala Arg Ile Ala Asp Ser Lys Asp His Val Phe
1               5                   10                  15

Pro Val Asn Asp Gly Phe Gln Ala Leu Gln Gly Ile Ile His Ser Ile
            20                  25                  30
Leu Lys Lys Ser Cys Ile Glu Ile Leu Ala Ala Glu Pro Ser Thr Ile
        35                  40                  45
Cys Ala Gly Glu Ser Phe Gln Val Val Val Arg Gly Asn Gly Phe Arg
    50                  55                  60
His Ala Arg Asn Val Asp Arg Val Leu Cys Ser Phe Lys Ile Asn Asp
65                  70                  75                  80
Ser Val Thr Leu Asn Glu Lys Pro Phe Ala Val Glu Asp Thr Tyr Leu
                85                  90                  95
Leu Cys Pro Ala Pro Ile Leu Lys Glu Val Gly Met Lys Ala Ala Leu
            100                 105                 110
Gln Val Ser Met Asn Asp Gly Leu Ser Phe Ile Ser Ser Ser Val Ile
        115                 120                 125
Ile Thr Thr Thr His Cys Ser Asp Gly Ser
    130                 135